(19) Europäisches Patentamt
European Patent Office
Office européen des brevets

(11) EP 4 127 157 B1

(12) **EUROPEAN PATENT SPECIFICATION**

(45) Date of publication and mention of the grant of the patent:
**11.02.2026 Bulletin 2026/07**

(21) Application number: **21775942.2**

(22) Date of filing: **19.03.2021**

(51) International Patent Classification (IPC):
*C12N 9/48* (2006.01) *G01N 33/68* (2006.01)
*C12N 9/64* (2006.01)

(52) Cooperative Patent Classification (CPC):
**C12N 9/6408; C12Y 304/14; C12Y 304/15**

(86) International application number:
**PCT/US2021/023347**

(87) International publication number:
**WO 2021/194908 (30.09.2021 Gazette 2021/39)**

(54) **MODIFIED DIPEPTIDE CLEAVASES, USES THEREOF AND RELATED KITS**

MODIFIZIERTE DIPEPTID-CLEAVASEN, VERWENDUNGEN DAVON UND ZUGEHÖRIGE KITS

CLIVASES DIPEPTIDIQUES MODIFIÉES, UTILISATIONS CORRESPONDANTES ET KITS CORRESPONDANTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.03.2020 US 202062994216 P**
**30.09.2020 US 202063085977 P**

(43) Date of publication of application:
**08.02.2023 Bulletin 2023/06**

(73) Proprietor: **Encodia, Inc.**
**San Diego, CA 92121 (US)**

(72) Inventors:
- **GUNDERSON, Kevin, L.**
  **San Diego, CA 92121 (US)**
- **JAMES, Robert, C.**
  **San Diego, CA 92121 (US)**
- **SHI, Lei**
  **San Diego, CA 92121 (US)**
- **VERESPY, Stephen, III**
  **San Diego, CA 92121 (US)**
- **DESAI, Kevin**
  **San Diego, CA 92121 (US)**

(74) Representative: **Kuttenkeuler, David**
**SKM-IP PartGmbB**
**Oberanger 45**
**80331 München (DE)**

(56) References cited:
**WO-A1-2010/065322**
**WO-A1-2014/067746**
**WO-A1-2017/192633**
**WO-A1-2017/192633**
**WO-A1-2020/198264**
**US-A1- 2002 164 759**
**US-A1- 2002 164 759**
**US-A1- 2018 195 082**
**US-A1- 2019 246 664**

- **SUZUKI YOSHIYUKI ET AL: "Identification of the Catalytic Triad of Family S46 Exopeptidases, Closely Related to Clan PA Endopeptidases", SCIENTIFIC REPORTS, vol. 4, no. 1, 1 May 2015 (2015-05-01), XP055974091, DOI: 10.1038/ srep04292**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 127 157 B1

## Description

CROSS-REFERENCE TO RELATED APPLICATIONS

This disclosure claims priority to U.S. Provisional Patent Application No. 62/994,216, filed on March 24, 2020 and U.S. Provisional Patent Application No. 63/085,977, filed on September 30, 2020.

STATEMENT REGARDING FEDERALLY SPONSORED RESEARCH

This invention was made with Government support awarded by National Institute of General Medical Sciences of the National Institutes of Health under Grant No. 1R43GM130185-01 and Grant No. 5R44GM123836-03. The United States Government has certain rights in this invention pursuant to these grants.

SEQUENCE LISTING ON ASCII TEXT

This patent or application file contains a Sequence Listing submitted in computer readable ASCII text format (file name: 4614-2002240_SeqList_ST25.txt, date recorded: March 17, 2021, size: 211,194 bytes). The content of the Sequence Listing file is incorporated herein by reference in its entirety.

TECHNICAL FIELD

The present disclosure relates to modified dipeptide cleavases for cleaving amino acids from peptides, polypeptides, and proteins, including modified peptides, polypeptides, and proteins. Also provided are methods of using the modified dipeptide cleavases for treating polypeptides, and kits comprising the modified dipeptide cleavase. The methods and the kits also include other components for macromolecule sequencing and/or analysis.

BACKGROUND

Enzymes that are involved in degradation of peptides and proteins, *e.g.,* aminopeptidases, dipeptidyl peptidases, carboxypeptidases, endopeptidases, and others, hydrolyze peptide bonds (Sanderink et al., J. Clin. Chem. Clin. Biochem. (1988) 26:795-807). Various peptidases have been isolated and discovered in a number of organisms and from various tissues. Aminopeptidases naturally occur as monomeric and multimeric enzymes, and may be metal or ATP-dependent. Some substrate-specific peptidases specifically remove one or two amino acid residues at a time from the amino-terminus of the peptide while others remove from the carboxy-terminus of the protein or peptide. Natural aminopeptidases generally have limited specificity and eliminate amino acids in a processive manner, eliminating one amino acid one after another.

In some embodiments, methods for peptide degradation are useful for applications in protein analysis and/or sequencing. For example, peptide sequencing may involve Edman degradation to achieve stepwise degradation of the N-terminal amino acid (NTAA) on a peptide through a series of chemical modifications and downstream HPLC analysis or mass spectrometry analysis. However, in general, Edman degradation peptide sequencing may be limited, for example, typical Edman degradation requires deployment of high temperature and harsh chemical conditions (e.g., strong acids; anhydrous TFA) for long incubation times. In some cases, Edman degradation may not be compatible with processes for protein analysis methods which may be sensitive to harsh chemical conditions, such as analysis methods which employ nucleic acids *(e.g.,* DNA).

Suzuki, Yoshiyuki, et al. (in "Identification of the catalytic triad of family S46 exopeptidases, closely related to clan PA endopeptidases." Scientific Reports 4.1 (2014): 4292.) disclosed catalytic residues in the dipeptidyl aminopeptidase DAP BII.

US 2002/164759 disclosed isolated polypeptides, dipeptidylpeptidases, active analogs, active fragments, or active modifications thereof, having amidolytic activity for cleavage of a peptide bond between the second and third amino acids from the N-terminal end of a target polypeptide.

WO 2017/192633 disclosed a method for analyzing macromolecules, including peptides, polypeptides, and proteins, employing nucleic acid encoding.

US 2019/246664 disclosed a site-specific mutagenesis modified yeast dipeptidyl peptidase III.

Accordingly, there remains a need for improved reagents for degradation of amino acids. For example, enzymatic methods for removing, eliminating, or cleaving amino acids from polypeptides may be desired. Furthermore, the availability of additional substrate-specific enzymes which can bind and remove desired amino acids from a polypeptide is also desired. In some cases, such improved reagents for removing amino acids are useful for protein sequencing and/or analysis. The present disclosure fulfills these and other related needs.

These and other aspects of the invention will be apparent upon reference to the following detailed description. To this end, various references are set forth herein which describe in more detail certain background information, procedures, compounds and/or compositions.

## BRIEF SUMMARY

The present invention is disclosed in independent claims 1, 8 13 and 14. Preferred embodiments are disclosed in the dependent claims.

In one aspect, disclosed herein is a modified dipeptide cleavase comprising three or more amino acid substitutions in a substrate binding site of a dipeptidyl aminopeptidase, wherein:

(i) the dipeptidyl aminopeptidase removes or is configured to remove two terminal amino acids from a polypeptide; and
(ii) the modified dipeptide cleavase removes or is configured to remove from the polypeptide having a terminal amino acid residue labeled with a chemical reagent (a) the single labeled terminal amino acid residue or (b) a labeled terminal dipeptide, wherein the dipeptidyl aminopeptidase comprises an amino acid sequence having at least 30 % sequence identity to the amino acid sequence of SEQ ID NO: 31 and also comprises an asparagine residue at a position corresponding to position 191 of SEQ ID NO: 31, a tryptophan or phenylalanine residue at a position corresponding to position 192 of SEQ ID NO: 31, an arginine residue at a position corresponding to position 196 of SEQ ID NO: 31, an asparagine residue at a position corresponding to position 306 of SEQ ID NO: 31, an aspartate residue at a position corresponding to position 650 of SEQ ID NO: 31; and wherein the modified dipeptide cleavase comprises three or more amino acid substitutions in residues corresponding to positions 191, 192, 196, 306, 650 of SEQ ID NO: 31.

According to an embodiment, the modified dipeptide cleavase does not remove an unlabeled terminal dipeptide from the polypeptide.

In yet another embodiment, the modified dipeptide cleavase comprises at least four amino acid substitutions in residues corresponding to positions 191, 192, 196, 306, 650 of SEQ ID NO: 31.

In yet another embodiment, the modified dipeptide cleavase removes or is configured to remove a single N-terminally labeled amino acid of the polypeptide.

In yet another embodiment, the modified dipeptide cleavase further comprises one or more amino acid substitutions in residues corresponding to positions 310, 628, 648, 651, 659, 669.

In yet another embodiment, the dipeptidyl aminopeptidase is a protein classified in MEROPS S46, or a functional homolog or fragment thereof.

In yet another embodiment, the single terminal amino acid or terminal dipeptide is labeled with a N-terminal modification that comprises a N-terminal blocking group ($NTM_{blk}$) and, optionally, a natural or unnatural amino acid portion (NTMaa), wherein the NTMaa comprises a compound selected from the group consisting of: a naturally-occurring amino acid residue, 3-(3'-pyridyl)-L-alanine, L-cyclohexylglycine, α-aminoisobutyric acid, 3-(4'-pyridyl)-L-alanine, L-azetidine-2-carboxylic acid, isonipecotic acid, L-phenylglycine, β-(2-thienyl)-L-alanine, 3-(4-thiazolyl)-L-alanine, 1-aminocyclopentane-1-carboxylic acid, (2-trifluoromethyl)-L-Phenylalanine, L-cyclopropylalanine, 3-(2'-pyridyl)-L-alanine, beta-cyano-L-alanine, α-methyl-L-4-Fluorophenylalanine, α-methyl-D-4-fluorophenylalanine, 3-amino-2,2-difluoro-propionic acid, O-sulfo-L-tyrosine sodium salt, L-2-furylalanine, 1-aminocyclopropane-1-carboxylic acid, 3,5-dinitro-L-tyrosine, pentafluoro-L-phenylalanine, 3,5-difluoro-L-phenylalanine, 3-fluoro-L-phenylalanine, N-cyclopentylglycine, 1-(amino)cyclohexanecarboxylic acid, N-methylalanine, 4-amino-tetrahydropyran-4-carboxylic acid, 4-amino-1,1-dioxothiane-4-carboxylic acid, 4-amino-1-methyl-4-piperidinecarboxylic acid, 2-amino-N-(2,4-dimethoxybenzyl)acetamido)acetic acid, or N-alkylated derivatives; and the $NTM_{blk}$ comprises a compound selected from the group consisting of: 4-methylbenzoic acid, 4-(dimethylamio)benzoic acid, nicotinic acid, 3-aminonicotinic acid, 2-pyrazinecarbooxylic acid, 5-amino-2-fluoroisonicotinic acid, 2,3-pyrazinedicarboxylic acid, 4,7-Difluoroisobenzofuran-1,3-dicarboxylic acid, 4-chloro-2-aminobenzoic acid, 4-nitro-2-aminobenzoic acid, 7-methoxy-1h-benzo[d][1,3]oxazine-2,4-dione, 4-carboxy-2-aminobenzoic acid, 6-(Trifluoromethyl)-2,4-dihydro-1h-3, 1-benzoxazine-2,4-dione, 7-(Trifluoromethyl)-1h-benzo[d][1,3]oxazine-2,4-dione, 6-fluoro-2-aminobenzoic acid, 4-fluoro-2-aminobenzoic acid, 5-methoxy-2-aminobenzoic acid, 4-fluorobenzoic acid, 4-(trifluoromethyl)benzoic acid, 2-ethynyl-6-fluorobenzaldehyde, 2-aminobenzoic acid, Succinic anhydride, 3,6-Difluoropyridine-2-carboxylic acid, 2-Fluoronicotinic acid, 5-Bromo-2-hydroxynicotinic acid, 4-(Trifluoromethyl)pyrimidine-5-carboxylic acid, 2-Oxo-1,2-dihydropyridine-3-carboxylic acid, 5-Methyl-2-aminobenzoic acid, 6-Fluoropicolinic acid, 3-Methyl-2-aminobenzoic acid, 4-Methyl-2-aminobenzoic acid, 2-Amino-6-methylbenzoic acid, 2-Amino-6-fluorobenzoic acid, 2-Amino-5-fluorobenzoic acid, 2-Amino-3-fluorobenzoic acid, 2-Amino-4-fluorobenzoic acid, 2-Aminonicotinic acid, 4-Aminonicotinic acid, 3-Aminopicolinic acid, 2-Amino-4,5-difluorobenzoic acid, 3,4-difluorobenzoic acid, 3,4,5-difluorobenzoic acid, 3-(Methoxycarbonyl)bicyclo[1.1.1]pentane-1-carboxylic acid, 3,3-Difluorocyclobutane-1-carboxylic acid, 1-Methyl-2-oxo-piperidine-4-carboxylic acid, Tetrahydropyran-4-carboxylic acid, 5-Fluoroorotic acid, 3-Fluoro-4-nitrobenzoic acid, 3-(Difluoromethyl)-1-methyl-1H-pyrazole-4-carboxylic acid, 4-(Difluoromethoxy)benzoic acid, 1-(Difluoromethyl)-1h-pyrazole-3-carboxylic acid, 4-(Methanesulfonylamino)benzoic acid, 5-Fluoro-6-methoxynicotinic acid, Tetrahydro-2H-thiopyran-4-carboxylic acid 1,1-dioxide, 4-(1H-Tetrazol-5-yl)benzoic acid, 1,2,3-Thiadiazole-4-carboxylic acid, 1,3-Benzodioxole-4-carboxylic acid, 2,1,3-Benzoxadiazole-5-carboxylic acid, 1-Benzyl-3-methyl-1h-pyrazole-5-carboxylic acid, 1-Cyclopropyl-6,7-difluoro-1,4-dihydro-4-oxoquinoline-3-carboxylic acid, 3,4-Dichlorobenzoic acid, 5-Fluoro-6-methylpyridine-2-carboxylic acid, 4,5-Dimethyl-2-(1h-pyrrol-1-yl)thiophene-3-carboxylic acid, 1,3-Dimethyl-1h-thieno[2,3-c]pyrazole-5-carboxylic acid, 1-[(4-Fluorobenzene)sulfonyl]piperidine-3-carboxylic acid, 1-(4-Fluorobenzyl)-5-oxopyrrolidine-3-carboxylic acid, 3-Fluoro-4-methoxybenzoic acid, 4-Fluoro-3-nitrobenzoic acid, 6-Fluoro-4-oxochromene-2-carboxylic acid, 3-Fluorophenylacetic acid, 4-Fluoro-3-(trifluoromethyl)benzoic acid, 5-Furan-2-yl-isoxazole-3-carboxylic acid, 1-Isopropyl-2-(trifluoromethyl)-1h-benzimidazole-5-carboxylic acid, Levofloxacin carboxylic acid, 3,5,7-Trifluoroadamantane-1-carboxylic acid, 3,4,5-Trimethoxybenzoic acid, 2-Oxo-2,3-dihydro-1h-benzo[d]imidazole-4-carboxylic acid, 1-Methyl-3-(trifluoromethyl)-1h-pyrazole-5-carboxylic acid, 2-Morpholin-4-yl-isonicotinic acid, 1,3-Oxazole-4-carboxylic acid, 4-Carboxybenzenesulfonamide, 3,4-difluorobenzenesulfonyl chloride.

In another aspect, disclosed herein is a method of treating a polypeptide, comprising the following steps:

labeling a terminal amino acid of the polypeptide with a chemical reagent to produce a labeled polypeptide; and contacting the labeled polypeptide with a modified dipeptide cleavase, wherein the modified dipeptide cleavase comprises three or more amino acid substitutions in a substrate binding site of a dipeptidyl aminopeptidase, wherein:

(i) the dipeptidyl aminopeptidase removes or is configured to remove two terminal amino acids from the polypeptide upon contacting; and

(ii) the modified dipeptide cleavase removes or is configured to remove from the polypeptide having a terminal amino acid labeled with a chemical reagent (a) the single labeled terminal amino acid or (b) a labeled terminal dipeptide, wherein the dipeptidyl aminopeptidase comprises an amino acid sequence having at least 30 % sequence identity to the amino acid sequence of SEQ ID NO: 31 and also comprises an asparagine residue at a position corresponding to position 191 of SEQ ID NO: 31, a tryptophan or phenylalanine residue at a position corresponding to position 192 of SEQ ID NO: 31, an arginine residue at a position corresponding to position 196 of SEQ ID NO: 31, an asparagine residue at a position corresponding to position 306 of SEQ ID NO: 31, an aspartate residue at a position corresponding to position 650 of SEQ ID NO: 31; and wherein the modified dipeptide cleavase comprises three or more amino acid substitutions in residues corresponding to positions 191, 192, 196, 306, 650 of SEQ ID NO: 31.

In an embodiment of the method, the modified dipeptide cleavase does not remove an unlabeled terminal dipeptide from the polypeptide.

In yet another embodiment of the method, the modified dipeptide cleavase comprises at least four amino acid substitutions in residues corresponding to positions 191, 192, 196, 306, 650 of SEQ ID NO: 31.

In yet another embodiment of the method, the single terminal amino acid or terminal dipeptide is labeled with a N-terminal modification that comprises a N-terminal blocking group ($NTM_{blk}$) and, optionally, a natural or unnatural amino acid portion (NTMaa), wherein the NTMaa comprises a compound selected from the group consisting of: a naturally-occurring amino acid residue, 3-(3'-pyridyl)-L-alanine, L-cyclohexylglycine, α-aminoisobutyric acid, 3-(4'-pyridyl)-L-alanine, L-azetidine-2-carboxylic acid, isonipecotic acid, L-phenylglycine, β-(2-thienyl)-L-alanine, 3-(4-thiazolyl)-L-alanine, 1-aminocyclopentane-1-carboxylic acid, (2-trifluoromethyl)-L-Phenylalanine, L-cyclopropylalanine, 3-(2'-pyridyl)-L-alanine, beta-cyano-L-alanine, α-methyl-L-4-Fluorophenylalanine, α-methyl-D-4-fluorophenylalanine, 3-amino-2,2-difluoro-propionic acid, O-sulfo-L-tyrosine sodium salt, L-2-furylalanine, 1-aminocyclopropane-1-carboxylic acid, 3,5-dinitro-L-tyrosine, pentafluoro-L-phenylalanine, 3,5-difluoro-L-phenylalanine, 3-fluoro-L-phenylalanine, N-cyclopentylglycine, 1-(amino)cyclohexanecarboxylic acid, N-methylalanine, 4-amino-tetrahydropyran-4-carboxylic acid, 4-amino-1,1-dioxothiane-4-carboxylic acid, 4-amino-1-methyl-4-piperidinecarboxylic acid, 2-amino-N-(2,4-dimethoxybenzyl) acetamido)acetic acid, or N-alkylated derivatives; and the $NTM_{blk}$ comprises a compound selected from the group consisting of: 4-methylbenzoic acid, 4-(dimethylamio)benzoic acid, nicotinic acid, 3-aminonicotinic acid, 2-pyrazinecarbooxylic acid, 5-amino-2-fluoro-isonicotinic acid, 2,3-pyrazinedicarboxylic acid, 4,7-Difluoroisobenzofuran-1,3-dicarboxylic acid, 4-chloro-2-aminobenzoic acid, 4-nitro-2-aminobenzoic acid, 7-methoxy-1h-benzo[d][1,3]oxazine-2,4-dione, 4-carboxy-2-aminobenzoic acid, 6-(Trifluoromethyl)-2,4-dihydro-1h-3, 1-benzoxazine-2,4-dione, 7-(Trifluoromethyl)-1h-benzo[d][1,3]oxazine-2,4-dione, 6-fluoro-2-aminobenzoic acid, 4-fluoro-2-aminobenzoic acid, 5-methoxy-2-aminobenzoic acid, 4-fluorobenzoic acid, 4-(trifluoromethyl)benzoic acid, 2-ethynyl-6-fluorobenzaldehyde, 2-aminobenzoic acid, Succinic anhydride, 3,6-Difluoropyridine-2-carboxylic acid, 2-Fluoronicotinic acid, 5-Bromo-2-hydroxynicotinic acid, 4-(Trifluoromethyl)pyrimidine-5-carboxylic acid, 2-Oxo-1,2-dihydropyridine-3-carboxylic acid, 5-Methyl-2-aminobenzoic acid, 6-Fluoropicolinic acid, 3-Methyl-2-aminobenzoic acid, 4-Methyl-2-aminobenzoic acid, 2-Amino-6-methylbenzoic acid, 2-Amino-6-fluorobenzoic acid, 2-Amino-5-fluorobenzoic acid, 2-Amino-3-fluorobenzoic acid, 2-Amino-4-fluorobenzoic acid, 2-Aminonicotinic acid, 4-Aminonicotinic acid, 3-Aminopicolinic acid, 2-Amino-4,5-difluorobenzoic acid, 3,4-difluorobenzoic acid, 3,4,5-difluorobenzoic acid, 3-(Methoxycarbonyl)bicyclo[1.1.1]pentane-1-carboxylic acid, 3,3-Difluorocyclobutane-1-carboxylic acid, 1-Methyl-2-oxo-piperidine-4-carboxylic acid, Tetrahydropyran-4-carboxylic acid, 5-Fluoroorotic acid, 3-Fluoro-4-nitrobenzoic acid, 3-(Difluoromethyl)-1-methyl-1H-pyrazole-4-carboxylic acid, 4-(Difluoromethoxy)benzoic acid, 1-(Difluoromethyl)-1h-pyrazole-3-carboxylic acid, 4-(Methanesulfonylamino)benzoic acid, 5-Fluoro-6-methoxynicotinic acid, Tetrahydro-2H-thiopyran-4-carboxylic acid 1,1-dioxide, 4-(1H-Tetrazol-5-yl)benzoic acid, 1,2,3-Thiadiazole-4-carboxylic acid, 1,3-Benzodioxole-4-carboxylic acid, 2,1,3-Benzoxadiazole-5-carboxylic acid, 1-Benzyl-3-methyl-1h-pyrazole-5-carboxylic acid, 1-Cyclopropyl-6,7-difluoro-1,4-dihydro-4-oxoquinoline-3-carboxylic acid, 3,4-Dichlorobenzoic acid, 5-Fluoro-6-methylpyridine-2-carboxylic acid, 4,5-Dimethyl-2-(1h-pyrrol-1-yl)thiophene-3-carboxylic acid, 1,3-Dimethyl-1h-thieno[2,3-c]pyrazole-5-carboxylic acid, 1-[(4-Fluorobenzene)sulfonyl]piperidine-3-carboxylic acid, 1-(4-Fluorobenzyl)-5-oxopyrrolidine-3-carboxylic acid, 3-Fluoro-4-methoxybenzoic acid, 4-Fluoro-3-nitrobenzoic acid, 6-Fluoro-4-oxochromene-2-carboxylic acid, 3-Fluorophenylacetic acid, 4-Fluoro-3-(trifluoromethyl)benzoic acid, 5-Furan-2-yl-isoxazole-3-carboxylic acid, 1-Isopropyl-2-(trifluoromethyl)-1h-benzimidazole-5-carboxylic acid, Levofloxacin carboxylic acid, 3,5,7-Trifluoroadamantane-1-carboxylic acid, 3,4,5-Trimethoxybenzoic acid, 2-Oxo-2,3-dihydro-1h-benzo[d]imidazole-4-carboxylic acid, 1-Methyl-3-(trifluoromethyl)-1h-pyrazole-5-carboxylic acid, 2-Morpholin-4-yl-isonicotinic acid, 1,3-Oxazole-4-carboxylic acid, 4-Carboxybenzenesulfonamide, 3,4-difluorobenzenesulfonyl chloride.

In yet another embodiment, the method further comprises a step of contacting the polypeptide with a binding agent configured to bind to the single labeled terminal amino acid or to the labeled terminal dipeptide, wherein the step of labeling the terminal amino acid of the polypeptide is before the step of contacting the polypeptide with the binding agent; and the step of contacting the polypeptide with the binding agent is before the step of contacting the polypeptide with the modified dipeptide cleavase.

In yet another aspect, disclosed herein is a set of dipeptide cleavase enzymes, comprising at least two different modified dipeptide cleavases, wherein:

(i) each of the modified dipeptide cleavases from the set of dipeptide cleavase enzymes comprises three or more amino acid substitutions in a substrate binding site of a dipeptidyl aminopeptidase, wherein the dipeptidyl aminopeptidase is configured to remove two terminal amino acids from an unlabeled polypeptide;
(ii) each of the modified dipeptide cleavases from the set of dipeptide cleavase enzymes is configured to remove a single labeled terminal amino acid from a polypeptide having the terminal amino acid labeled with a chemical reagent, wherein
the dipeptidyl aminopeptidase comprises an amino acid sequence having at least 30 % sequence identity to the amino acid sequence of SEQ ID NO: 31 and also comprises an asparagine residue at a position corresponding to position 191 of SEQ ID NO: 31, a tryptophan or phenylalanine residue at a position corresponding to position 192 of SEQ ID NO: 31, an arginine residue at a position corresponding to position 196 of SEQ ID NO: 31, an asparagine residue at a position corresponding to position 306 of SEQ ID NO: 31, an aspartate residue at a position corresponding to position 650 of SEQ ID NO: 31; and wherein the modified dipeptide cleavase comprises three or more amino acid substitutions in residues corresponding to positions 191, 192, 196, 306, 650 of SEQ ID NO: 31; and
(iii) the modified dipeptide cleavases from the set of dipeptide cleavase enzymes have different specificities for the labeled terminal amino acids, which the modified dipeptide cleavases are configured to remove.

In yet another aspect, disclosed herein is a kit for treating a polypeptide, comprising:

(a) a chemical reagent for labeling a terminal amino acid of the polypeptide; and
(b) a set of dipeptide cleavase enzymes, comprising at least two different modified dipeptide cleavases, wherein:

(i) each of the modified dipeptide cleavases from the set of dipeptide cleavase enzymes comprises three or more amino acid substitutions in a substrate binding site of a dipeptidyl aminopeptidase, wherein the dipeptidyl aminopeptidase is configured to remove two terminal amino acids from an unlabeled polypeptide;
(ii) each of the modified dipeptide cleavases from the set of dipeptide cleavase enzymes is configured to remove a single labeled terminal amino acid from a polypeptide having the terminal amino acid labeled with a chemical reagent, wherein
the dipeptidyl aminopeptidase comprises an amino acid sequence having at least 30 % sequence identity to the amino acid sequence of SEQ ID NO: 31 and also comprises an asparagine residue at a position corresponding to position 191 of SEQ ID NO: 31, a tryptophan or phenylalanine residue at a position corresponding to position 192 of SEQ ID NO: 31, an arginine residue at a position corresponding to position 196 of SEQ ID NO: 31, an asparagine residue at a position corresponding to position 306 of SEQ ID NO: 31, an aspartate residue at a position corresponding to position 650 of SEQ ID NO: 31; and wherein the modified dipeptide cleavase comprises three or more amino acid substitutions in residues corresponding to positions 191, 192, 196, 306, 650 of SEQ ID

NO: 31; and

(iii) the modified dipeptide cleavases from the set of dipeptide cleavase enzymes have different specificities for the labeled terminal amino acids, which the modified dipeptide cleavases are configured to remove.

In an embodiment of the kit, (i) the chemical reagent is configured to attach a N-terminal modification to the terminal amino acid of the polypeptide; (ii) the N-terminal modification comprises a N-terminal blocking group ($NTM_{blk}$) and, optionally, a natural or unnatural amino acid portion (NTMaa); (iii) the NTMaa comprises a compound selected from the group consisting of: a naturally-occurring amino acid residue, 3-(3'-pyridyl)-L-alanine, L-cyclohexylglycine, α-aminoisobutyric acid, 3-(4'-pyridyl)-L-alanine, L-azetidine-2-carboxylic acid, isonipecotic acid, L-phenylglycine, β-(2-thienyl)-L-alanine, 3-(4-thiazolyl)-L-alanine, 1-aminocyclopentane-1-carboxylic acid, (2-trifluoromethyl)-L-Phenylalanine, L-cyclopropylalanine, 3-(2'-pyridyl)-L-alanine, beta-cyano-L-alanine, α-methyl-L-4-Fluorophenylalanine, α-methyl-D-4-fluorophenylalanine, 3-amino-2,2-difluoro-propionic acid, O-sulfo-L-tyrosine sodium salt, L-2-furylalanine, 1-aminocyclopropane-1-carboxylic acid, 3,5-dinitro-L-tyrosine, pentafluoro-L-phenylalanine, 3,5-difluoro-L-phenylalanine, 3-fluoro-L-phenylalanine, N-cyclopentylglycine, 1-(amino)cyclohexanecarboxylic acid, N-methylalanine, 4-amino-tetrahydropyran-4-carboxylic acid, 4-amino-1,1-dioxothiane-4-carboxylic acid, 4-amino-1-methyl-4-piperidinecarboxylic acid, 2-amino-N-(2,4-dimethoxybenzyl)acetamido)acetic acid, or N-alkylated derivatives; and (iv) the $NTM_{blk}$ comprises a compound selected from the group consisting of: 4-methylbenzoic acid, 4-(dimethylamio)benzoic acid, nicotinic acid, 3-aminonicotinic acid, 2-pyrazinecarbooxylic acid, 5-amino-2-fluoro-isonicotinic acid, 2,3-pyrazinedicarboxylic acid, 4,7-Difluoroisobenzofuran-1,3-dicarboxylic acid, 4-chloro-2-aminobenzoic acid, 4-nitro-2-aminobenzoic acid, 7-methoxy-1h-benzo[d][1,3]oxazine-2,4-dione, 4-carboxy-2-aminobenzoic acid, 6-(Trifluoromethyl)-2,4-dihydro-1h-3, 1-benzoxazine-2,4-dione, 7-(Trifluoromethyl)-1h-benzo[d][1,3]oxazine-2,4-dione, 6-fluoro-2-aminobenzoic acid, 4-fluoro-2-aminobenzoic acid, 5-methoxy-2-aminobenzoic acid, 4-fluorobenzoic acid, 4-(trifluoromethyl)benzoic acid, 2-ethynyl-6-fluorobenzaldehyde, 2-aminobenzoic acid, Succinic anhydride, 3,6-Difluoropyridine-2-carboxylic acid, 2-Fluoronicotinic acid, 5-Bromo-2-hydroxynicotinic acid, 4-(Trifluoromethyl)pyrimidine-5-carboxylic acid, 2-Oxo-1,2-dihydropyridine-3-carboxylic acid, 5-Methyl-2-aminobenzoic acid, 6-Fluoropicolinic acid, 3-Methyl-2-aminobenzoic acid, 4-Methyl-2-aminobenzoic acid, 2-Amino-6-methylbenzoic acid, 2-Amino-6-fluorobenzoic acid, 2-Amino-5-fluorobenzoic acid, 2-Amino-3-fluorobenzoic acid, 2-Amino-4-fluorobenzoic acid, 2-Aminonicotinic acid, 4-Aminonicotinic acid, 3-Aminopicolinic acid, 2-Amino-4,5-difluorobenzoic acid, 3,4-difluorobenzoic acid, 3,4,5-difluorobenzoic acid, 3-(Methoxycarbonyl)bicyclo[1.1.1]pentane-1-carboxylic acid, 3,3-Difluorocyclobutane-1-carboxylic acid, 1-Methyl-2-oxo-piperidine-4-carboxylic acid, Tetrahydropyran-4-carboxylic acid, 5-Fluoroorotic acid, 3-Fluoro-4-nitrobenzoic acid, 3-(Difluoromethyl)-1-methyl-1H-pyrazole-4-carboxylic acid, 4-(Difluoromethoxy)benzoic acid, 1-(Difluoromethyl)-1h-pyrazole-3-carboxylic acid, 4-(Methanesulfonylamino)benzoic acid, 5-Fluoro-6-methoxynicotinic acid, Tetrahydro-2H-thiopyran-4-carboxylic acid 1,1-dioxide, 4-(1H-Tetrazol-5-yl)benzoic acid, 1,2,3-Thiadiazole-4-carboxylic acid, 1,3-Benzodioxole-4-carboxylic acid, 2,1,3-Benzoxadiazole-5-carboxylic acid, 1-Benzyl-3-methyl-1h-pyrazole-5-carboxylic acid, 1-Cyclopropyl-6,7-difluoro-1,4-dihydro-4-oxoquinoline-3-carboxylic acid, 3,4-Dichlorobenzoic acid, 5-Fluoro-6-methylpyridine-2-carboxylic acid, 4,5-Dimethyl-2-(1h-pyrrol-1-yl)thiophene-3-carboxylic acid, 1,3-Dimethyl-1h-thieno[2,3-c]pyrazole-5-carboxylic acid, 1-[(4-Fluorobenzene)sulfonyl]piperidine-3-carboxylic acid, 1-(4-Fluorobenzyl)-5-oxopyrrolidine-3-carboxylic acid, 3-Fluoro-4-methoxybenzoic acid, 4-Fluoro-3-nitrobenzoic acid, 6-Fluoro-4-oxochromene-2-carboxylic acid, 3-Fluorophenylacetic acid, 4-Fluoro-3-(trifluoromethyl)benzoic acid, 5-Furan-2-yl-isoxazole-3-carboxylic acid, 1-Isopropyl-2-(trifluoromethyl)-1h-benzimidazole-5-carboxylic acid, Levofloxacin carboxylic acid, 3,5,7-Trifluoroadamantane-1-carboxylic acid, 3,4,5-Trimethoxybenzoic acid, 2-Oxo-2,3-dihydro-1h-benzo[d]imidazole-4-carboxylic acid, 1-Methyl-3-(trifluoromethyl)-1h-pyrazole-5-carboxylic acid, 2-Morpholin-4-yl-isonicotinic acid, 1,3-Oxazole-4-carboxylic acid, 4-Carboxybenzenesulfonamide, 3,4-difluorobenzenesulfonyl chloride.

## BRIEF DESCRIPTION OF THE DRAWINGS

Accompanying figures are intended to support the invention; the figures are schematic and are not intended to be drawn to scale. For purposes of illustration, not every component is labeled in every figure, nor is every component of each embodiment of the invention shown where illustration is not necessary to allow those of ordinary skill in the art to understand the invention.

**FIG.** 1 is a schematic depicting the removal of a single modified amino acid by exemplary modified dipeptide cleavases as provided herein. In **FIG. 1** on the left, an exemplary unmodified dipeptide cleavase removes two amino acids as a dipeptide from the N-terminus of the polypeptide, cleaving the bond between the penultimate (P2) and antepenultimate amino acid (P3) residues. On the right, an exemplary modified dipeptide cleavase removes a labeled dipeptide including the terminal labeled amino acid from the N-terminus of the polypeptide, cleaving the bond between the penultimate terminal amino acid residue (P2) and the antepenultimate amino acid residue (P3).

**FIG. 2A-2C** is a schematic depicting a cycle of terminal amino acid removal using the modified dipeptide cleavase and

terminal amino acid labeling. In **FIG. 2A-2B,** a polypeptide with a labeled N-terminal amino acid residue is cleaved at the bond between the penultimate amino acid and antepenultimate amino acid by the modified dipeptide cleavase and the terminal dipeptide, including the label or modification (diamond), is released. In **FIG. 2C,** the new terminal amino acid is labeled and the modified penultimate cleavase is able to recognize the new labeled terminal amino acid for further cleavage and release of the terminal dipeptide following the next cleavage step.

**FIG. 3.** depicts N-terminal amino acid (NTAA) conversion efficiency with different exemplary reagents for labeling the N-terminal amino acid. Two different peptides were tested in solution: N-Terminal G (NT-G) = GRFSGIY (SEQ ID NO:29); N-Terminal W (NT-W) = WTQIFGA (SEQ ID NO:30). LC-MS was used to quantitate conversion efficiency.

**FIG. 4A-4C** depicts results from a WebLogo analysis of sequence conservation of DAP BII homologs with 60% sequence similarity or identity. The height of each stack indicates the sequence conservation at that position (measured in bits), and the height of symbols within the stack reflects the relative frequency of the corresponding amino acid at the indicated position (in reference to SEQ ID NO: 20).

**FIG. 5** shows a graph of Michaelis-Menten kinetics for two modified dipeptide cleavases (containing the amino acid sequences as set forth in SEQ ID NO: 18 and SEQ ID NO: 27) tested at various concentrations.

**Fig. 6** depicts a model of an exemplary anticalin scaffold bound with N-terminal modified amino acid. The modification is shown in orange spheres the preferably occupy part of a surface accessible pocket. The P1 sidechain *(i.e.,* Leucine in magenta) is surrounded by amino acids (shown in blue stick) that can be mutated to provide specificity.

**Fig. 7A-7H** illustrates exemplary Luminex-based binding affinity profile of anticalin clones chosen from a phage display screen against M15-L-P1 peptides. Eight exemplary engineered anticalin binders are shown to have mostly mono-specificity for P1 residues except for the I/L binder. The anticalin clones are isolated from phage library panning. Clones with specificity to different P1 residues, such as E, F, G, H, I, L, P, W, as well as clones with specificity to two different P1 residues, such as T/S, A/T/S, T/V/I/A, F/L, were successfully isolated.

**Fig. 8A-B**illustrates exemplary analysis of P2 dependence via ProteoCode™ encoding assay. **Fig. 8A** shows Encoding versus Luminex binding signal for M15-L-G clone shown in Fig. 4. **Fig. 8B** shows P2 dependence determined by ProteoCode™ encoding assay using the M15-L-G binder clone on various M15-L-G-P2 peptides.

**Fig. 9** shows ProteoCode™ encoding assay with modified NTAA binders and modified cleavases used for high-throughput polypeptide sequencing. Polypeptide molecules are each labeled with a DNA recording tag and attached to a solid support (beads) at a low molecular density, a sparsity that permits only intramolecular information transfer to occur. **(1)** At the beginning of a sequencing cycle, the polypeptide N-terminal amino acid (NTAA) is functionalized with a N-terminal modification (NTM) or label. **(2)** Next, an engineered NTAA binding agent labelled with a DNA coding tag binds to the labeled NTAA residue. After binding and washing, the coding tag information is transferred enzymatically to the recording tag (by extension or ligation). **(3)** Removal of the NTM-labeled N-terminal residue is accomplished by using a modified Cleavase enzyme that specifically cleaves the NTM-labeled N-terminal residue. After n cycles, a DNA library element representing the n amino acids of the polypeptide sequence is formed as a part of extended recording tag and can be sequenced by a next-generation sequencing (NGS) method. A representative structure of an NGS library element after 7 cycles is shown.

**Fig. 10A** illustrates exemplary cleavage of M15-L-modified NTAAs of a model polypeptide (M15-L-P1-AR) with Cleavase enzymes. A compilation of seven different modified Cleavase clones was used to generate the spectrum of cleavage profile across the M15-L-modified NTAAs as shown. Data were generated by HPLC analysis (UV absorbance) of cleaved versus intact peptides after cleavase assay. **Fig. 10B** shows the same cleavage events using SDS-PAGE analysis. **Fig. 10C** shows a cleavage profile for an exemplary set of two selected modified Cleavase clones, M15-L_Z001, having specificity towards A, I, L, M, Q, V in the P1 position (cleavage efficiency of M15-L_Z001 is shown by the left columns for each amino acid), and M15-L_Z002, having specificity towards D and E in the P1 position (cleavage efficiency of M15-L_Z002 is shown by the right columns for each amino acid).

**Fig. 11** illustrates cleavage of an exemplary polypeptide by unmodified dipeptide cleavase (dipeptidyl aminopeptidase DAP BII, SEQ ID NO: 13). 1-5 correspond to cleavage results at the following time points: 0 min, 5 min, 30 min, 45 min, 60 min.

**Fig. 12A-B** . Exemplary N-terminal modifications (NTMs) to enable NTM-NTAA cleavage at P1 residue by modified dipeptide cleavases. **Fig. 12A.** Structures of a bipartite NTM comprised of an amino acid-like portion (NTMaa) and a N-terminal blocking group ($NTM_{blk}$) connected by an amide bond (upper) and other possible NTMs that would accommodate modified substrate binding pockets of cleavases. NTM can also be a small chemical entity ($NTM_B$) with a similar bipartite shape configuration as $NTM_A$, or a differently shaped $NTM_C$. **Fig. 12B.** NTMs are activated using standard methods (activated ester) and are coupled to the N-terminal amine on the P1 residue of a polypeptide. The arrow indicates a cleavage site of the modified dipeptide cleavase enzyme.

**Fig. 13.** The cleavage efficiency of the NTM-labeled NTAA of a target polypeptide depends on a particular NTM.

**Fig. 14A-B** . Time course of cleavage reactions of two labeled peptides (M15-LAAR and M19-LAAR, cleavage efficiencies are shown in left and right columns, respectively, for each time point) by two modified dipeptidyl cleavases selected using M15 NTM **(Fig. 14A)** and M19 NTM **(Fig. 14B).**

**Fig. 15** depicts results from a WebLogo analysis of sequence conservation of DAP BII homologs. The height of each stack indicates the sequence conservation at that position (measured in bits), and the height of symbols within the stack reflects the relative frequency of the corresponding amino acid at the indicated position (in reference to positions of SEQ ID NO: 20). Conservation of N215, W216(F), R220, N330, D674 positions is highlighted.

**Fig. 16.** Similarity distribution relative to DapBII (Pseudoxanthomonas mexicana) of 2125 sequences clustered at 80% sequence identity.

DETAILED DESCRIPTION

Provided herein are modified dipeptide cleavases comprising a mutation (*e.g*., one or more modifications in an unmodified dipeptide cleavase) and related methods of selecting, engineering, and using the modified dipeptide cleavases. Also provided are kits comprising the modified dipeptide cleavases. In some embodiments, the kits comprising the modified dipeptide cleavase is used for treating peptides, polypeptides, and proteins, such as for sequencing and/or analysis. In some embodiments, protein analysis using the modified dipeptide cleavase employs barcoding and nucleic acid encoding of molecular recognition events, and/or detectable labels. In some embodiments, the kits also include other components for treating the polypeptides, including tags (*e.g.,* DNA tag or DNA recording tag), solid supports, and other reagents for preparing the polypeptides and other reagents for polypeptide analysis.

Various enzymes that degrade peptides and proteins by hydrolyzing peptide bonds, (*e.g.,* aminopeptidases, dipeptidyl peptidases, carboxypeptidases, endopeptidases) have been isolated and discovered in a number of organisms and from various tissues. However, natural aminopeptidases may have limited specificity, and generically eliminate N-terminal amino acids in a processive manner, eliminating one amino acid off after another. Some substrate-specific peptidases specifically remove one or two amino acid residues at a time from the amino-terminus or carboxy-terminus of peptides.

In some embodiments, methods for peptide degradation are useful for applications in protein analysis and/or sequencing. For example, peptide sequencing may involve Edman degradation to achieve stepwise degradation of the N-terminal amino acid on a peptide through a series of chemical modifications and downstream HPLC analysis or mass spectrometry analysis. However, in general, Edman degradation peptide sequencing may be limited, for example, typical Edman degradation requires deployment of high temperature and harsh chemical conditions (*e.g.,* strong acids; anhydrous TFA) for long incubation times. In some cases, Edman degradation may not be compatible with processes for protein analysis methods which may be sensitive to harsh chemical conditions, such as analysis methods which employ nucleic acids (*e.g.,* DNA).

Accordingly, there remains a need for improved reagents and techniques for degradation of amino acids from a polypeptide. For example, enzymatic methods for removing, eliminating, or cleaving amino acids from polypeptides may be desired. Provided herein are modified dipeptide cleavases that meet such needs. In some embodiments, provided herein are enzymatic methods and reagents for removing amino acids as dipeptides or as single labeled amino acids from polypeptides. In some cases, the removal of amino acids by the provided modified enzymes (*e.g.,* dipeptide cleavases) are used for stepwise degradation of amino acids from polypeptides. In some embodiments, the removal of amino acids by the provided modified dipeptide cleavase are suitable for cyclic removal of dipeptides or single amino acids from the polypeptide. In some embodiments, the modified dipeptide cleavase removes or is configured to remove a labeled terminal dipeptide from a polypeptide. In some embodiments, the modified dipeptide cleavase removes or is configured to remove a single N-terminally modified amino acid from a target polypeptide. In some embodiments, the modified dipeptide cleavase removes a labeled dipeptide (the terminal and penultimate terminal amino acids) from the C-terminus or N-terminus of a polypeptide. In some embodiments, the modified dipeptide cleavase is derived from a wild-type or unmodified dipeptide cleavase. For example, the unmodified dipeptide cleavase is a protein classified in EC 3.4.14, EC 3.4.15, MEROPS S9, MEROPS S46, MEROPS M49, or a functional homolog or fragment thereof. For example, the modified dipeptide cleavase is derived from a wild-type or unmodified dipeptide cleavase (*e.g.,* a dipeptidyl peptidase, a dipeptidyl aminopeptidase, a peptidyl-dipeptidase, or a dipeptidyl carboxypeptidase).

The present disclosure also relates to a binder that specifically binds to an N-terminally modified polypeptide and modified or an engineered cleavase that removes or is configured to remove a single N-terminally modified amino acid from a polypeptide. Also provided herein is a method and related kits for treating a polypeptide using or comprising the binder and/or modified cleavase.

In some embodiments, peptidases may be engineered to possess specific binding or catalytic activity to specific terminal amino acids only when modified with a label. For example, a cleavase may be engineered or modified, compared to a wild-type or unmodified dipeptide cleavase, such than it only eliminates a terminal amino acid if it is labeled by a chemical label. Using this exemplary approach, the modified dipeptide cleavase eliminates only terminal single labeled amino acids or dipeptides containing a labeled amino acid from the terminus of the polypeptide, and allows control of degradation in a desired manner. In some embodiments, the modified dipeptide cleavase is configured to remove a labeled terminal dipeptide (including the terminal and penultimate terminal amino acids) from the C-terminus or N-terminus of a polypeptide. In some embodiments, the modified dipeptide cleavase is non-selective as to amino acid residue identity while being selective for the label (*e.g.,* will remove any single labeled amino acids or terminal dipeptides containing any two amino acids associated with a label or modification). In some other embodiments, the modified dipeptide cleavase exhibits some preference for certain amino acid residues or classes of amino acids (*e.g.* at the P1 and/or P2 terminal positions of the polypeptide). In some cases, two or more modified dipeptide cleavases with different preferences for certain amino acids (or classes of amino acids) may be used in combination. In some embodiments, the modified dipeptide cleavase binds and removes single labeled amino acids dipeptides from the N-terminus of the polypeptide. In some embodiments, the modified dipeptide cleavase binds and removes dipeptides from the C-terminus of the polypeptide.

In some embodiments, known peptidases may be modified to achieve specific characteristics for binding and/or cleaving. An example of a model of modifying the specificity of enzymatic N-terminal amino acid (NTAA) degradation involves a methionine aminopeptidase converted into a leucine aminopeptidase (Borgo et al., Protein Sci. (2014) 23(3):312-320). In another example, aminopeptidase mutants were engineered to bind to and eliminate individual or small groups of labelled (biotinylated) NTAAs (*see,* PCT Publication No. WO2010/065322). Provided herein are modified dipeptide cleavases which are selected or modified to remove terminal dipeptides that are labeled, such as dipeptides containing a chemically-modified terminal amino acid on a polypeptide. In some embodiments, a wild-type cleavase is engineered *(e.g.,* using structural-function based-design and/or directed evolution) to cleave or remove only a terminal dipeptide containing an N-terminal amino acid having a chemical group present as the label (e.g., PTC/DNP/acetyl/Cbz).

The unmodified dipeptide cleavase may be from any suitable organism. In some examples, the wild-type or unmodified dipeptide cleavase is from a mammal, *e.g., Homo sapiens,* a fungus or yeast, *e.g., Saccharomyces cerevisiae,* or a bacterium, *e.g., Bacteroides thetaiotaomicron, Porphyromonas gingivalis, Pseudomonas sp., Pseudoxanthomonas mexicana or Caldithrix abyssi.* In some cases, these enzymes are stable, robust, and active at room temperature and at or around pH 8.0, and thus compatible with mild conditions preferred for peptide analysis. In some embodiments, it is preferred to have a thermophilic cleavase capable of removing labeled single amino acids or terminal dipeptides at elevated temperatures to minimize peptide secondary structure.

In another embodiment, cyclic elimination or removal of amino acids is attained by engineering the dipeptide cleavase to be active only in the presence of a terminal amino acid label. In some embodiments, the label is a chemical label. Moreover, the dipeptide cleavase may be engineered to be non-specific, such that it does not selectively recognize particular amino acids over another, but recognizes any amino acid at the terminus that has a label. **In** some embodiments, the modified dipeptide cleavase is selective for one or more, two or more, three or more, four or more, five or more, ten or more, fifteen or more, twenty or more etc. amino acids.

In some embodiments, the provided modified dipeptide cleavases are used for treating polypeptides obtained from a sample. In some cases, the sample and/or the polypeptide obtained from the sample is treated with other reagents for processing the polypeptides, such as digesting the polypeptides. In some aspects, the polypeptides are treated with a reagent for labeling the terminal amino acid (*e.g.,* a chemical reagent) prior to treating the polypeptide with the modified dipeptide cleavase provided. In some embodiments, the polypeptides comprise a plurality of polypeptides obtained from a sample. In some embodiments, the sample is obtained from a subject.

In certain embodiments, the modified dipeptide cleavase is derived from a metallopeptidase, a zinc-dependent metallopeptidase, or a zinc-dependent hydrolase. In some cases, the modified dipeptide cleavase is a metallo-peptidase and requires a metal ion for activation. In some embodiments, the use of a monomeric metallo-aminopeptidase provides the advantage of having controllable activity can be turned on/off at will by adding or removing the appropriate metal cation.

Numerous specific details are set forth in the following description in order to provide a thorough understanding of the present disclosure. These details are provided for the purpose of example and the claimed subject matter may be practiced according to the claims without some or all of these specific details. It is to be understood that other embodiments can be used and structural changes can be made without departing from the scope of the claimed subject matter. It should be understood that the various features and functionality described in one or more of the individual embodiments are not limited in their applicability to the particular embodiment with which they are described. They instead can, be applied, alone or in some combination, to one or more of the other embodiments of the disclosure, whether or not such embodiments are described, and whether or not such features are presented as being a part of a described embodiment. For the purpose of clarity, technical material that is known in the technical fields related to the claimed subject matter has not been described in detail so that the claimed subject matter is not unnecessarily obscured.

Citation of the publications or documents is not intended as an admission that any of them is pertinent prior art, nor does it constitute any admission as to the contents or date of these publications or documents.

All headings are for the convenience of the reader and should not be used to limit the meaning of the text that follows the heading, unless so specified.

DEFINITIONS

Unless defined otherwise, all technical and scientific terms used herein have the same meaning as is commonly understood by one of ordinary skill in the art to which the present disclosure belongs. If a definition set forth in this section is contrary to or otherwise inconsistent with a definition set forth in the patents, applications, published applications and other publications that are herein cited, the definition set forth in this section prevails over the definition of the cited reference.

As used herein, the singular forms "a," "an" and "the" include plural referents unless the context clearly dictates otherwise. Thus, for example, reference to "a peptide" includes one or more peptides, or mixtures of peptides. Also, and unless specifically stated or obvious from context, as used herein, the term "or" is understood to be inclusive and covers both "or" and "and".

The term "about" as used herein refers to the usual error range for the respective value readily known to the skilled person in this technical field. Reference to "about" a value or parameter herein includes (and describes) embodiments that are directed to that value or parameter *per se.* For example, description referring to "about X" includes description of "X.

The term "antibody" herein is used in the broadest sense and includes polyclonal and monoclonal antibodies, including intact antibodies and functional (antigen-binding) antibody fragments, including fragment antigen binding (Fab) fragments, $F(ab')_2$ fragments, Fab' fragments, Fv fragments, recombinant IgG (rIgG) fragments, single chain antibody fragments, including single chain variable fragments (scFv), and single domain antibodies (*e.g.*, sdAb, sdFv, nanobody) fragments. The term encompasses genetically engineered and/or otherwise modified forms of immunoglobulins, such as intrabodies, peptibodies, chimeric antibodies, fully human antibodies, humanized antibodies, and heteroconjugate antibodies, multispecific, e.g., bispecific, antibodies, diabodies, triabodies, and tetrabodies, tandem di-scFv, tandem tri-scFv. Unless otherwise stated, the term "antibody" should be understood to encompass functional antibody fragments thereof. The term also encompasses intact or full-length antibodies, including antibodies of any class or sub-class, including IgG and sub-classes thereof, IgM, IgE, IgA, and IgD.

An "individual" or "subject" includes a mammal. Mammals include, but are not limited to, domesticated animals (e.g., cows, sheep, cats, dogs, and horses), primates (e.g., humans and non-human primates such as monkeys), rabbits, and rodents (e.g., mice and rats). An "individual" or "subject" may include birds such as chickens, vertebrates such as fish and mammals such as mice, rats, rabbits, cats, dogs, pigs, cows, ox, sheep, goats, horses, monkeys and other non-human primates. In certain embodiments, the individual or subject is a human.

As used herein, the term "sample" refers to anything which may contain an analyte for which an analyte assay is desired. As used herein, a "sample" can be a solution, a suspension, liquid, powder, a paste, aqueous, non-aqueous or any combination thereof. The sample may be a biological sample, such as a biological fluid or a biological tissue. Examples of biological fluids include urine, blood, plasma, serum, saliva, semen, stool, sputum, cerebral spinal fluid, tears, mucus, amniotic fluid or the like. Biological tissues are aggregate of cells, usually of a particular kind together with their intercellular substance that form one of the structural materials of a human, animal, plant, bacterial, fungal or viral structure, including connective, epithelium, muscle and nerve tissues. Examples of biological tissues also include organs, tumors, lymph nodes, arteries and individual cell(s).

In some embodiments, the sample is a biological sample. A biological sample of the present disclosure encompasses a sample in the form of a solution, a suspension, a liquid, a powder, a paste, an aqueous sample, or a non-aqueous sample. As used herein, a "biological sample" includes any sample obtained from a living or viral (or prion) source or other source of macromolecules and biomolecules, and includes any cell type or tissue of a subject from which nucleic acid, protein and/or other macromolecule can be obtained. The biological sample can be a sample obtained directly from a biological source or a sample that is processed. For example, isolated nucleic acids that are amplified constitute a biological sample. Biological samples include, but are not limited to, body fluids, such as blood, plasma, serum, cerebrospinal fluid, synovial fluid, urine and sweat, tissue and organ samples from animals and plants and processed samples derived therefrom. In some embodiments, the sample can be derived from a tissue or a body fluid, for example, a connective, epithelium, muscle or nerve tissue; a tissue selected from the group consisting of brain, lung, liver, spleen, bone marrow, thymus, heart, lymph, blood, bone, cartilage, pancreas, kidney, gall bladder, stomach, intestine, testis, ovary, uterus, rectum, nervous system, gland, and internal blood vessels; or a body fluid selected from the group consisting of blood, urine, saliva, bone marrow, sperm, an ascitic fluid, and subfractions thereof, *e.g.*, serum or plasma.

The terms "level" or "levels" are used to refer to the presence and/or amount of a target, *e.g.,* a substance or an organism that is part of the etiology of a disease or disorder, and can be determined qualitatively or quantitatively. A "qualitative" change in the target level refers to the appearance or disappearance of a target that is not detectable or is present in samples obtained from normal controls. A "quantitative" change in the levels of one or more targets refers to a measurable increase or decrease in the target levels when compared to a healthy control.

As used herein, the term "polypeptide" encompasses peptides and proteins, and refers to a molecule comprising a chain of two or more amino acids joined by peptide bonds. **In** some embodiments, a polypeptide comprises 2 to 50 amino acids, *e.g.*, having more than 20-30 amino acids. In some embodiments, a peptide does not comprise a secondary, tertiary, or higher structure. In some embodiments, the polypeptide is a protein. In some embodiments, a protein comprises 30 or

more amino acids, e.g. having more than 50 amino acids. In some embodiments, in addition to a primary structure, a protein comprises a secondary, tertiary, or higher structure. The amino acids of the polypeptides are most typically L-amino acids, but may also be D-amino acids, modified amino acids, amino acid analogs, amino acid mimetics, or any combination thereof. Polypeptides may be naturally occurring, synthetically produced, or recombinantly expressed. Polypeptides may be synthetically produced, isolated, recombinantly expressed, or be produced by a combination of methodologies as described above. Polypeptides may also comprise additional groups modifying the amino acid chain, for example, functional groups added via post-translational modification. The polymer may be linear or branched, it may comprise modified amino acids, and it may be interrupted by non-amino acids. The term also encompasses an amino acid polymer that has been modified naturally or by intervention; for example, disulfide bond formation, glycosylation, lipidation, acetylation, phosphorylation, or any other manipulation or modification, such as conjugation with a labeling component.

As used herein, the term "amino acid" refers to an organic compound comprising an amine group, a carboxylic acid group, and a side-chain specific to each amino acid, which serve as a monomeric subunit of a peptide. An amino acid includes the 20 standard, naturally occurring or canonical amino acids as well as non-standard amino acids. The standard, naturally-occurring amino acids include Alanine (A or Ala), Cysteine (C or Cys), Aspartic Acid (D or Asp), Glutamic Acid (E or Glu), Phenylalanine (F or Phe), Glycine (G or Gly), Histidine (H or His), Isoleucine (I or Ile), Lysine (K or Lys), Leucine (L or Leu), Methionine (M or Met), Asparagine (N or Asn), Proline (P or Pro), Glutamine (Q or Gln), Arginine (R or Arg), Serine (S or Ser), Threonine (T or Thr), Valine (V or Val), Tryptophan (W or Trp), and Tyrosine (Y or Tyr). An amino acid may be an L-amino acid or a D-amino acid. Non-standard amino acids may be modified amino acids, amino acid analogs, amino acid mimetics, non-standard proteinogenic amino acids, or non-proteinogenic amino acids that occur naturally or are chemically synthesized. Examples of non-standard amino acids include, but are not limited to, selenocysteine, pyrrolysine, and N-formylmethionine, β-amino acids, Homo-amino acids, Proline and Pyruvic acid derivatives, 3-substituted alanine derivatives, glycine derivatives, ring-substituted phenylalanine and tyrosine derivatives, linear core amino acids, N-methyl amino acids.

As used herein, the term "post-translational modification" refers to modifications that occur on a peptide after its translation, *e.g.*, translation by ribosomes, is complete. A post-translational modification may be a covalent chemical modification or enzymatic modification. Examples of post-translation modifications include, but are not limited to, acylation, acetylation, alkylation (including methylation), biotinylation, butyrylation, carbamylation, carbonylation, deamidation, deiminiation, diphthamide formation, disulfide bridge formation, eliminylation, flavin attachment, formylation, gamma-carboxylation, glutamylation, glycylation, glycosylation, glypiation, heme C attachment, hydroxylation, hypusine formation, iodination, isoprenylation, lipidation, lipoylation, malonylation, methylation, myristolylation, oxidation, palmitoylation, pegylation, phosphopantetheinylation, phosphorylation, prenylation, propionylation, retinylidene Schiff base formation, S-glutathionylation, S-nitrosylation, S-sulfenylation, selenation, succinylation, sulfination, ubiquitination, and C-terminal amidation. A post-translational modification includes modifications of the amino terminus and/or the carboxyl terminus of a peptide. Modifications of the terminal amino group include, but are not limited to, des-amino, N-lower alkyl, N-di-lower alkyl, and N-acyl modifications. Modifications of the terminal carboxy group include, but are not limited to, amide, lower alkyl amide, dialkyl amide, and lower alkyl ester modifications (*e.g.*, wherein lower alkyl is $C_1$-$C_4$ alkyl). A post-translational modification also includes modifications, such as but not limited to those described above, of amino acids falling between the amino and carboxy termini. The term post-translational modification can also include peptide modifications that include one or more detectable labels.

As used herein, the term "binding agent" or "binder" refers to a nucleic acid molecule, a peptide, a polypeptide, a protein, carbohydrate, or a small molecule that binds to, associates, unites with, recognizes, or combines with a binding target, *e.g.,* a polypeptide or a component or feature of a polypeptide. A binding agent may form a covalent association or non-covalent association with the polypeptide or component or feature of a polypeptide. A binding agent may also be a chimeric binding agent, composed of two or more types of molecules, such as a nucleic acid molecule-peptide chimeric binding agent or a carbohydrate-peptide chimeric binding agent. A binding agent may be a naturally occurring, synthetically produced, or recombinantly expressed molecule. A binding agent may bind to a single monomer or subunit of a polypeptide (*e.g.,* a single amino acid of a polypeptide) or bind to a plurality of linked subunits of a polypeptide (*e.g.,* a di-peptide, tri-peptide, or higher order peptide of a longer peptide, polypeptide, or protein molecule). A binding agent may bind to a linear molecule or a molecule having a three-dimensional structure (also referred to as conformation). For example, an antibody binding agent may bind to linear peptide, polypeptide, or protein, or bind to a conformational peptide, polypeptide, or protein. A binding agent may bind to an N-terminal peptide, a C-terminal peptide, or an intervening peptide of a peptide, polypeptide, or protein molecule. A binding agent may bind to an N-terminal amino acid, C-terminal amino acid, or an intervening amino acid of a peptide molecule. A binding agent may preferably bind to a chemically modified or labeled amino acid (*e.g.,* an amino acid that has been labeled by a reagent comprising a compound of any one of Formula (I)-(IV) as described herein) over a non-modified or unlabeled amino acid. For example, a binding agent may preferably bind to an amino acid that has been labeled or modified over an amino acid that is unlabeled or unmodified. A binding agent may bind to a post-translational modification of a peptide molecule. A binding agent may exhibit selective binding to a component or feature of a polypeptide (*e.g.,* a binding agent may selectively bind to one of the 20 possible natural amino acid residues

and with bind with very low affinity or not at all to the other 19 natural amino acid residues). A binding agent may exhibit less selective binding, where the binding agent is capable of binding or configured to bind to a plurality of components or features of a polypeptide (*e.g.,* a binding agent may bind with similar affinity to two or more different amino acid residues). A binding agent may comprise a coding tag, which may be joined to the binding agent by a linker.

As used herein, the term "linker" refers to one or more of a nucleotide, a nucleotide analog, an amino acid, a peptide, a polypeptide, a polymer, or a non-nucleotide chemical moiety that is used to join two molecules. A linker may be used to join a binding agent with a coding tag, a recording tag with a polypeptide, a polypeptide with a solid support, a recording tag with a solid support, etc. In certain embodiments, a linker joins two molecules via enzymatic reaction or chemistry reaction (*e.g*., click chemistry).

The term "ligand" as used herein refers to any molecule or moiety connected to the compounds described herein. "Ligand" may refer to one or more ligands attached to a compound. In some embodiments, the ligand is a pendant group or binding site (*e.g.,* the site to which the binding agent binds).

As used herein, the term "proteome" can include the entire set of proteins, polypeptides, or peptides (including conjugates or complexes thereof) expressed by a genome, cell, tissue, or organism at a certain time, of any organism. In one aspect, it is the set of expressed proteins in a given type of cell or organism, at a given time, under defined conditions. Proteomics is the study of the proteome. For example, a "cellular proteome" may include the collection of proteins found in a particular cell type under a particular set of environmental conditions, such as exposure to hormone stimulation. An organism's complete proteome may include the complete set of proteins from all of the various cellular proteomes. A proteome may also include the collection of proteins in certain sub-cellular biological systems. For example, all of the proteins in a virus can be called a viral proteome. As used herein, the term "proteome" include subsets of a proteome, including but not limited to a kinome; a secretome; a receptome (e.g., GPCRome); an immunoproteome; a nutriproteome; a proteome subset defined by a post-translational modification (e.g., phosphorylation, ubiquitination, methylation, acetylation, glycosylation, oxidation, lipidation, and/or nitrosylation), such as a phosphoproteome (e.g., phosphotyrosine-proteome, tyrosine-kinome, and tyrosine-phosphatome), a glycoproteome, etc.; a proteome subset associated with a tissue or organ, a developmental stage, or a physiological or pathological condition; a proteome subset associated a cellular process, such as cell cycle, differentiation (or de-differentiation), cell death, senescence, cell migration, transformation, or metastasis; or any combination thereof. As used herein, the term "proteomics" refers to quantitative analysis of the proteome within cells, tissues, and bodily fluids, and the corresponding spatial distribution of the proteome within the cell and within tissues. Additionally, proteomics studies include the dynamic state of the proteome, continually changing in time as a function of biology and defined biological or chemical stimuli.

The terminal amino acid at one end of a peptide or polypeptide chain that has a free amino group is referred to herein as the "N-terminal amino acid" (NTAA). The terminal amino acid at the other end of the chain that has a free carboxyl group is referred to herein as the "C-terminal amino acid" (CTAA). The amino acids making up a peptide may be numbered in order, with the peptide being "*n*" amino acids in length. As used herein, NTAA is considered the $n^{th}$ amino acid (also referred to herein as the "*n* NTAA"). Using this nomenclature, the next amino acid is the *n-1* amino acid, then the *n-2* amino acid, and so on down the length of the peptide from the N-terminal end to C-terminal end. In certain embodiments, an NTAA, CTAA, or both may be modified or labeled with a moiety or a chemical moiety.

As used herein, the term "barcode" refers to a nucleic acid molecule of about 2 to about 30 bases (*e.g.,* 2, 3, 4, 5, 6, 7, 8, 9, 10, 11, 12, 13, 14, 15, 16, 17, 18, 19, 20, 21, 22, 23, 24, 25, 26, 27, 28, 29 or 30 bases) providing a unique identifier tag or origin information for a polypeptide, a binding agent, a set of binding agents from a binding cycle, a sample polypeptides, a set of samples, polypeptides within a compartment (*e.g.*, droplet, bead, or separated location), polypeptides within a set of compartments, a fraction of polypeptides, a set of polypeptide fractions, a spatial region or set of spatial regions, a library of polypeptides, or a library of binding agents. A barcode can be an artificial sequence or a naturally occurring sequence. In certain embodiments, each barcode within a population of barcodes is different. In other embodiments, a portion of barcodes in a population of barcodes is different, *e.g.,* at least about 10%, 15%, 20%, 25%, 30%, 35%, 40%, 45%, 50%, 55%, 60%, 65%, 70%, 75%, 80%, 85%, 90%, 95%, 97%, or 99% of the barcodes in a population of barcodes is different. A population of barcodes may be randomly generated or non-randomly generated. In certain embodiments, a population of barcodes are error correcting barcodes. Barcodes can be used to computationally deconvolute the multiplexed sequencing data and identify sequence reads derived from an individual polypeptide, sample, library, etc. A barcode can also be used for deconvolution of a collection of polypeptides that have been distributed into small compartments for enhanced mapping. For example, rather than mapping a peptide back to the proteome, the peptide is mapped back to its originating protein molecule or protein complex.

As used herein, the term "coding tag" refers to a polynucleotide with any suitable length, *e.g.,* a nucleic acid molecule of about 2 bases to about 100 bases, including any integer including 2 and 100 and in between, that comprises identifying information for its associated binding agent. A "coding tag" may also be made from a "sequenceable polymer" (*see, e.g.,* Niu et al., 2013, Nat. Chem. 5:282-292; Roy et al., 2015, Nat. Commun. 6:7237; Lutz, 2015, Macromolecules 48:4759-4767). A coding tag may comprise an encoder sequence, which is optionally flanked by one spacer on one side or optionally flanked by a spacer on each side. A coding tag may also be comprised of an optional UMI and/or an optional

binding cycle-specific barcode. A coding tag may be single stranded or double stranded. A double stranded coding tag may comprise blunt ends, overhanging ends, or both. A coding tag may refer to the coding tag that is directly attached to a binding agent, to a complementary sequence hybridized to the coding tag directly attached to a binding agent (*e.g.,* for double stranded coding tags), or to coding tag information present in an extended recording tag. In certain embodiments, a coding tag may further comprise a binding cycle specific spacer or barcode, a unique molecular identifier, a universal priming site, or any combination thereof.

As used herein, the term "spacer" (Sp) refers to a nucleic acid molecule of about 1 base to about 20 bases (*e.g.*, 1, 2, 3, 4, 5, 6, 7, 8, 9, 10, 11, 12, 13, 14, 15, 16, 17, 18, 19, or 20 bases) in length that is present on a terminus of a recording tag or coding tag. In certain embodiments, a spacer sequence flanks an encoder sequence of a coding tag on one end or both ends. Following binding of a binding agent to a polypeptide, annealing between complementary spacer sequences on their associated coding tag and recording tag, respectively, allows transfer of binding information through a primer extension reaction or ligation to the recording tag, coding tag, or a di-tag construct. Sp' refers to spacer sequence complementary to Sp. Preferably, spacer sequences within a library of binding agents possess the same number of bases. A common (shared or identical) spacer may be used in a library of binding agents. A spacer sequence may have a "cycle specific" sequence in order to track binding agents used in a particular binding cycle. The spacer sequence (Sp) can be constant across all binding cycles, be specific for a particular class of polypeptides, or be binding cycle number specific. Polypeptide class-specific spacers permit annealing of a cognate binding agent's coding tag information present in an extended recording tag from a completed binding/extension cycle to the coding tag of another binding agent recognizing the same class of polypeptides in a subsequent binding cycle via the class-specific spacers. Only the sequential binding of correct cognate pairs results in interacting spacer elements and effective primer extension. A spacer sequence may comprise sufficient number of bases to anneal to a complementary spacer sequence in a recording tag to initiate a primer extension (also referred to as polymerase extension) reaction, or provide a "splint" for a ligation reaction, or mediate a "sticky end" ligation reaction. A spacer sequence may comprise a fewer number of bases than the encoder sequence within a coding tag.

As used herein, the term "recording tag" refers to a moiety, *e.g.,* a chemical coupling moiety, a nucleic acid molecule, or a sequenceable polymer molecule (*see, e.g.,* Niu et al., 2013, Nat. Chem. 5:282-292; Roy et al., 2015, Nat. Commun. 6:7237; Lutz, 2015, Macromolecules 48:4759-4767) to which identifying information of a coding tag can be transferred, or from which identifying information about the macromolecule (*e.g.*, UMI information) associated with the recording tag can be transferred to the coding tag. Identifying information can comprise any information characterizing a molecule such as information pertaining to sample, fraction, partition, spatial location, interacting neighboring molecule(s), cycle number, etc. Additionally, the presence of UMI information can also be classified as identifying information. In certain embodiments, after a binding agent binds to a polypeptide, information from a coding tag linked to a binding agent can be transferred to the recording tag associated with the polypeptide while the binding agent is bound to the polypeptide. In other embodiments, after a binding agent binds to a polypeptide, information from a recording tag associated with the polypeptide can be transferred to the coding tag linked to the binding agent while the binding agent is bound to the polypeptide. A recoding tag may be directly linked to a polypeptide, linked to a polypeptide via a multifunctional linker, or associated with a polypeptide by virtue of its proximity (or co-localization) on a solid support. A recording tag may be linked via its 5' end or 3' end or at an internal site, as long as the linkage is compatible with the method used to transfer coding tag information to the recording tag or *vice versa.* A recording tag may further comprise other functional components, *e.g.*, a universal priming site, unique molecular identifier, a barcode (*e.g.,* a sample barcode, a fraction barcode, spatial barcode, a compartment tag, etc.), a spacer sequence that is complementary to a spacer sequence of a coding tag, or any combination thereof. The spacer sequence of a recording tag is preferably at the 3'-end of the recording tag in embodiments where polymerase extension is used to transfer coding tag information to the recording tag.

As used herein, the term "primer extension", also referred to as "polymerase extension", refers to a reaction catalyzed by a nucleic acid polymerase (e.g., DNA polymerase) whereby a nucleic acid molecule (*e.g.*, oligonucleotide primer, spacer sequence) that anneals to a complementary strand is extended by the polymerase, using the complementary strand as template.

As used herein, the term "unique molecular identifier" or "UMI" refers to a nucleic acid molecule of about 3 to about 40 bases (3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 bases) in length providing a unique identifier tag for each macromolecule, polypeptide or binding agent to which the UMI is linked. A polypeptide UMI can be used to computationally deconvolute sequencing data from a plurality of extended recording tags to identify extended recording tags that originated from an individual polypeptide. A polypeptide UMI can be used to accurately count originating polypeptide molecules by collapsing NGS reads to unique UMIs. A binding agent UMI can be used to identify each individual molecular binding agent that binds to a particular polypeptide. For example, a UMI can be used to identify the number of individual binding events for a binding agent specific for a single amino acid that occurs for a particular peptide molecule. It is understood that when UMI and barcode are both referenced in the context of a binding agent or polypeptide, that the barcode refers to identifying information other that the UMI for the individual binding agent or polypeptide (*e.g.*, sample barcode, compartment barcode, binding cycle barcode).

As used herein, the term "universal priming site" or "universal primer" or "universal priming sequence" refers to a nucleic acid molecule, which may be used for library amplification and/or for sequencing reactions. A universal priming site may include, but is not limited to, a priming site (primer sequence) for PCR amplification, flow cell adaptor sequences that anneal to complementary oligonucleotides on flow cell surfaces enabling bridge amplification in some next generation sequencing platforms, a sequencing priming site, or a combination thereof. Universal priming sites can be used for other types of amplification, including those commonly used in conjunction with next generation digital sequencing. For example, extended recording tag molecules may be circularized and a universal priming site used for rolling circle amplification to form DNA nanoballs that can be used as sequencing templates (Drmanac et al., 2009, Science 327:78-81). Alternatively, recording tag molecules may be circularized and sequenced directly by polymerase extension from universal priming sites (Korlach et al., 2008, Proc. Natl. Acad. Sci. 105:1176-1181). The term "forward" when used in context with a "universal priming site" or "universal primer" may also be referred to as "5'" or "sense". The term "reverse" when used in context with a "universal priming site" or "universal primer" may also be referred to as "3'" or "antisense".

As used herein, the term "extended recording tag" refers to a recording tag to which information of at least one binding agent's coding tag (or its complementary sequence) has been transferred following binding of the binding agent to a polypeptide. Information of the coding tag may be transferred to the recording tag directly (*e.g.,* ligation) or indirectly (*e.g.,* primer extension). Information of a coding tag may be transferred to the recording tag enzymatically or chemically. An extended recording tag may comprise binding agent information of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60, 65, 70, 75, 80, 85, 90, 95, 100, 125, 150, 175, 200 or more coding tags. The base sequence of an extended recording tag may reflect the temporal and sequential order of binding of the binding agents identified by their coding tags, may reflect a partial sequential order of binding of the binding agents identified by the coding tags, or may not reflect any order of binding of the binding agents identified by the coding tags. In certain embodiments, the coding tag information present in the extended recording tag represents with at least 25%, 30%, 35% , 40%, 45%, 50%, 55%, 60%, 65%, 70%, 75%, 80%, 85%, 90%, 91%, 92%, 93%, 94%, 95%, 96%, 97%, 98%, 99%, or 100% identity the polypeptide sequence being analyzed. In certain embodiments where the extended recording tag does not represent the polypeptide sequence being analyzed with 100% identity, errors may be due to off-target binding by a binding agent, or to a "missed" binding cycle (*e.g.*, because a binding agent fails to bind to a polypeptide during a binding cycle, because of a failed primer extension reaction), or both.

As used herein, the term "extended coding tag" refers to a coding tag to which information of at least one recording tag (or its complementary sequence) has been transferred following binding of a binding agent, to which the coding tag is joined, to a polypeptide, to which the recording tag is associated. Information of a recording tag may be transferred to the coding tag directly (*e.g.,* ligation), or indirectly (*e.g.,* primer extension). Information of a recording tag may be transferred enzymatically or chemically. In certain embodiments, an extended coding tag comprises information of one recording tag, reflecting one binding event. As used herein, the term "di-tag" or "di-tag construct" or "di-tag molecule" refers to a nucleic acid molecule to which information of at least one recording tag (or its complementary sequence) and at least one coding tag (or its complementary sequence) has been transferred following binding of a binding agent, to which the coding tag is joined, to a polypeptide, to which the recording tag is associated. Information of a recording tag and coding tag may be transferred to the di-tag indirectly (*e.g.*, primer extension). Information of a recording tag may be transferred enzymatically or chemically. In certain embodiments, a di-tag comprises a UMI of a recording tag, a compartment tag of a recording tag, a universal priming site of a recording tag, a UMI of a coding tag, an encoder sequence of a coding tag, a binding cycle specific barcode, a universal priming site of a coding tag, or any combination thereof.

As used herein, the term "solid support", "solid surface", or "solid substrate", or "sequencing substrate", or "substrate" refers to any solid material, including porous and nonporous materials, to which a polypeptide can be associated directly or indirectly, by any means known in the art, including covalent and non-covalent interactions, or any combination thereof. A solid support may be two-dimensional (e.g., planar surface) or three-dimensional (e.g., gel matrix or bead). A solid support can be any support surface including, but not limited to, a bead, a microbead, an array, a glass surface, a silicon surface, a plastic surface, a filter, a membrane, a PTFE membrane, a PTFE membrane, a nitrocellulose membrane, a nitrocellulose-based polymer surface, nylon, a silicon wafer chip, a flow through chip, a flow cell, a biochip including signal transducing electronics, a channel, a microtiter well, an ELISA plate, a spinning interferometry disc, a nitrocellulose membrane, a nitrocellulose-based polymer surface, a polymer matrix, a nanoparticle, or a microsphere. Materials for a solid support include but are not limited to acrylamide, agarose, cellulose, dextran, nitrocellulose, glass, gold, quartz, polystyrene, polyethylene vinyl acetate, polypropylene, polyester, polymethacrylate, polyacrylate, polyethylene, polyethylene oxide, polysilicates, polycarbonates, poly vinyl alcohol (PVA), Teflon, fluorocarbons, nylon, silicon rubber, polyanhydrides, polyglycolic acid, polyvinylchloride, polylactic acid, polyorthoesters, functionalized silane, polypropylfumerate, collagen, glycosaminoglycans, polyamino acids, dextran, or any combination thereof. Solid supports further include thin film, membrane, bottles, dishes, fibers, woven fibers, shaped polymers such as tubes, particles, beads, microspheres, microparticles, or any combination thereof. For example, when solid surface is a bead, the bead can include, but is not limited to, a ceramic bead, polystyrene bead, a polymer bead, a polyacrylate bead, a methylstyrene bead, an agarose bead, a cellulose bead, a dextran bead, an acrylamide bead, a solid core bead, a porous

bead, a paramagnetic bead, a glass bead, a controlled pore bead, a silica-based bead, or any combinations thereof. A bead may be spherical or an irregularly shaped. A bead or support may be porous. A bead's size may range from nanometers, e.g., 100 nm, to millimeters, e.g., 1 mm. In certain embodiments, beads range in size from about 0.2 micron to about 200 microns, or from about 0.5 micron to about 5 micron. In some embodiments, beads can be about 1, 1.5, 2, 2.5, 2.8, 3, 3.5, 4, 4.5, 5, 5.5, 6, 6.5, 7, 7.5, 8, 8.5, 9, 9.5, 10, 10.5, 15, or 20 μm in diameter. In certain embodiments, "a bead" solid support may refer to an individual bead or a plurality of beads. In some embodiments, the solid surface is a nanoparticle. In certain embodiments, the nanoparticles range in size from about 1 nm to about 500 nm in diameter, for example, between about 1 nm and about 20 nm, between about 1 nm and about 50 nm, between about 1 nm and about 100 nm, between about 10 nm and about 50 nm, between about 10 nm and about 100 nm, between about 10 nm and about 200 nm, between about 50 nm and about 100 nm, between about 50 nm and about 150, between about 50 nm and about 200 nm, between about 100 nm and about 200 nm, or between about 200 nm and about 500 nm in diameter. In some embodiments, the nanoparticles can be about 10 nm, about 50 nm, about 100 nm, about 150 nm, about 200 nm, about 300 nm, or about 500 nm in diameter. In some embodiments, the nanoparticles are less than about 200 nm in diameter.

As used herein, the term "nucleic acid molecule" or "polynucleotide" refers to a single- or double-stranded polynucleotide containing deoxyribonucleotides or ribonucleotides that are linked by 3'-5' phosphodiester bonds, as well as polynucleotide analogs. A nucleic acid molecule includes, but is not limited to, DNA, RNA, and cDNA. A polynucleotide analog may possess a backbone other than a standard phosphodiester linkage found in natural polynucleotides and, optionally, a modified sugar moiety or moieties other than ribose or deoxyribose. Polynucleotide analogs contain bases capable of hydrogen bonding by Watson-Crick base pairing to standard polynucleotide bases, where the analog backbone presents the bases in a manner to permit such hydrogen bonding in a sequence-specific fashion between the oligonucleotide analog molecule and bases in a standard polynucleotide. Examples of polynucleotide analogs include, but are not limited to xeno nucleic acid (XNA), bridged nucleic acid (BNA), glycol nucleic acid (GNA), peptide nucleic acids (PNAs), γPNAs, morpholino polynucleotides, locked nucleic acids (LNAs), threose nucleic acid (TNA), 2'-O-Methyl polynucleotides, 2'-O-alkyl ribosyl substituted polynucleotides, phosphorothioate polynucleotides, and boronophosphate polynucleotides. A polynucleotide analog may possess purine or pyrimidine analogs, including for example, 7-deaza purine analogs, 8-halopurine analogs, 5-halopyrimidine analogs, or universal base analogs that can pair with any base, including hypoxanthine, nitroazoles, isocarbostyril analogues, azole carboxamides, and aromatic triazole analogues, or base analogs with additional functionality, such as a biotin moiety for affinity binding. In some embodiments, the nucleic acid molecule or oligonucleotide is a modified oligonucleotide. In some embodiments, the nucleic acid molecule or oligonucleotide is a DNA with pseudo-complementary bases, a DNA with protected bases, an RNA molecule, a BNA molecule, an XNA molecule, a LNA molecule, a PNA molecule, a γPNA molecule, or a morpholino DNA, or a combination thereof. In some embodiments, the nucleic acid molecule or oligonucleotide is backbone modified, sugar modified, or nucleobase modified. In some embodiments, the nucleic acid molecule or oligonucleotide has nucleobase protecting groups such as Alloc, electrophilic protecting groups such as thiranes, acetyl protecting groups, nitrobenzyl protecting groups, sulfonate protecting groups, or traditional base-labile protecting groups.

As used herein, "nucleic acid sequencing" means the determination of the order of nucleotides in a nucleic acid molecule or a sample of nucleic acid molecules.

As used herein, "next generation sequencing" refers to high-throughput sequencing methods that allow the sequencing of millions to billions of molecules in parallel. Examples of next generation sequencing methods include sequencing by synthesis, sequencing by ligation, sequencing by hybridization, polony sequencing, ion semiconductor sequencing, and pyrosequencing. By attaching primers to a solid substrate and a complementary sequence to a nucleic acid molecule, a nucleic acid molecule can be hybridized to the solid substrate via the primer and then multiple copies can be generated in a discrete area on the solid substrate by using polymerase to amplify (these groupings are sometimes referred to as polymerase colonies or polonies). Consequently, during the sequencing process, a nucleotide at a particular position can be sequenced multiple times (*e.g.*, hundreds or thousands of times) - this depth of coverage is referred to as "deep sequencing." Examples of high throughput nucleic acid sequencing technology include platforms provided by Illumina, BGI, Qiagen, Thermo-Fisher, and Roche, including formats such as parallel bead arrays, sequencing by synthesis, sequencing by ligation, capillary electrophoresis, electronic microchips, "biochips," microarrays, parallel microchips, and single-molecule arrays (*See e.g.,* Service, Science (2006) 311:1544-1546).

As used herein, "single molecule sequencing" or "third generation sequencing" refers to next-generation sequencing methods wherein reads from single molecule sequencing instruments are generated by sequencing of a single molecule of DNA. Unlike next generation sequencing methods that rely on amplification to clone many DNA molecules in parallel for sequencing in a phased approach, single molecule sequencing interrogates single molecules of DNA and does not require amplification or synchronization. Single molecule sequencing includes methods that need to pause the sequencing reaction after each base incorporation ('wash-and-scan' cycle) and methods which do not need to halt between read steps. Examples of single molecule sequencing methods include single molecule real-time sequencing (Pacific Biosciences), nanopore-based sequencing (Oxford Nanopore), duplex interrupted nanopore sequencing, and direct imaging of DNA using advanced microscopy.

As used herein, "analyzing" the polypeptide means to identify, detect, quantify, characterize, distinguish, or a combination thereof, all or a portion of the components of the polypeptide. For example, analyzing a peptide, polypeptide, or protein includes determining all or a portion of the amino acid sequence (contiguous or non-continuous) of the peptide. Analyzing a polypeptide also includes partial identification of a component of the polypeptide. For example, partial identification of amino acids in the polypeptide protein sequence can identify an amino acid in the protein as belonging to a subset of possible amino acids. Analysis typically begins with analysis of the *n* NTAA, and then proceeds to the next amino acid of the peptide (*i.e., n-1, n-2, n-3,* and so forth). This is accomplished by elimination of the *n* NTAA, thereby converting the *n-1* amino acid of the peptide to an N-terminal amino acid (referred to herein as the *"n-1* NTAA"). Analyzing the peptide may also include determining the presence and frequency of post-translational modifications on the peptide, which may or may not include information regarding the sequential order of the post-translational modifications on the peptide. Analyzing the peptide may also include determining the presence and frequency of epitopes in the peptide, which may or may not include information regarding the sequential order or location of the epitopes within the peptide. Analyzing the peptide may include combining different types of analysis, for example obtaining epitope information, amino acid sequence information, post-translational modification information, or any combination thereof.

The term "unmodified" (also "wild-type" or "native") as used herein is used in connection with biological materials such as nucleic acid molecules and proteins (*e.g.*, cleavase), refers to those which are found in nature and not modified by human intervention.

The term "modified" or "engineered" (or "variant" or mutant") as used in reference to nucleic acid molecules and protein molecules, *e.g.,* a modified dipeptide cleavase, implies that such molecules are created by human intervention and/or they are non-naturally occurring. The variant, mutant or modified dipeptide cleavase is a polypeptide having an altered amino acid sequence, relative to an unmodified or wild-type dipeptide cleavase. The variant or modified dipeptide cleavase is a polypeptide which differs from a wild-type dipeptide cleavase sequence by one or more amino acid substitutions, deletions, additions, or combinations thereof. A variant, mutant or modified dipeptide cleavase can contain 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or more amino acid differences (*e.g.*, mutations) compared to the wild-type cleavase. A variant or modified dipeptide cleavase polypeptide generally exhibits at least 25%, 30%, 40%, 50%, 60%, 70%, 80%, 85%, 86%, 87%, 88%, 89%, 90%, 91%, 92%, 93%, 94%, 95%, 96%, 97%, 98%, 99% or more sequence identity to a corresponding wild-type or unmodified dipeptide cleavase. Non-naturally occurring amino acids as well as naturally occurring amino acids are included within the scope of permissible substitutions or additions. A variant, mutant or modified dipeptide cleavase is not limited to any variant, mutant or modified dipeptide cleavase made or generated by a particular method of making and includes, for example, a variant, mutant or modified dipeptide cleavase made or generated by genetic selection, protein engineering, directed evolution, *de novo* recombinant DNA techniques, or combinations thereof. A mutant, variant or modified dipeptide cleavase polypeptide is altered in primary amino acid sequence by substitution, addition, or deletion of amino acid residues. The term "variant" in the context of variant or modified dipeptide cleavase is not be construed as imposing any condition for any particular starting composition or method by which the variant or modified dipeptide cleavase is created. Thus, variant or modified dipeptide cleavase denotes a composition and not necessarily a product produced by any given process. A variety of techniques including genetic selection, protein engineering, recombinant methods, chemical synthesis, or combinations thereof, may be employed.

In some embodiments, variants of a modified dipeptide cleavase displaying only non-substantial or negligible differences in structure can be generated by making conservative amino acid substitutions in the modified dipeptide cleavase. By doing this, modified dipeptide cleavase variants that comprise a sequence having at least 90% (90%, 91%, 92%, 93%, 94%, 95%, 96%, 97%, 98%, and 99%) sequence identity with the modified dipeptide cleavase sequences provided in the attached Sequence Listing can be generated, retaining at least one functional activity, e g., cleavase activity for a given substrate. Examples of conservative amino acid changes are known in the art. Examples of non-conservative amino acid changes that are likely to cause major changes in protein structure are those that cause substitution of (a) a hydrophilic residue, e.g., serine or threonine, for (or by) a hydrophobic residue, e.g., leucine, isoleucine, phenylalanine, valine or alanine; (b) a cysteine or proline for (or by) any other residue; (c) a residue having an electropositive side chain, e.g., lysine, arginine, or histidine, for (or by) an electronegative residue, e.g., glutamic acid or aspartic acid; or (d) a residue having a bulky side chain, e.g., phenylalanine, for (or by) one not having a side chain, e g., glycine. Methods of making targeted amino acid substitutions, deletions, truncations, and insertions are generally known in the art. For example, amino acid sequence variants can be prepared by mutations in the DNA. Methods for polynucleotide alterations are well known in the art, for example, Kunkel et al. (1987) Methods in Enzymol. 154:367-382; U.S. Pat. No. 4,873,192 and the references cited therein.

The term "sequence identity" as used herein refers to the sequence identity between genes or proteins at the nucleotide or amino acid level, respectively. "Sequence identity" is a measure of identity between proteins at the amino acid level and a measure of identity between nucleic acids at nucleotide level. The protein sequence identity may be determined by comparing the amino acid sequence in a given position in each sequence when the sequences are aligned. Similarly, the nucleic acid sequence identity may be determined by comparing the nucleotide sequence in a given position

in each sequence when the sequences are aligned. "Sequence identity" means the percentage of identical subunits at corresponding positions in two sequences when the two sequences are aligned to maximize subunit matching, i.e., taking into account gaps and insertions. Sequence identity is present when a subunit position in both of the two sequences is occupied by the same nucleotide or amino acid, e.g., if a given position is occupied by an adenine in each of two DNA molecules, then the molecules are identical at that position. For example, if 7 positions in a sequence of 10 nucleotides in length are identical to the corresponding positions in a second 10-nucleotide sequence, then the two sequences have 70% sequence identity. Methods for the alignment of sequences for comparison are well known in the art, such methods include GAP, BESTFIT, BLAST, FASTA and TFASTA. The BLAST algorithm calculates percent sequence identity and performs a statistical analysis of the similarity between the two sequences. The software for performing BLAST analysis is publicly available through the National Center for Biotechnology Information (NCBI) website.

The terms "corresponding to position(s)" or "position(s) ... with reference to position(s)" of or within a polypeptide or a polynucleotide, such as recitation that nucleotides or amino acid positions "correspond to" nucleotides or amino acid positions of a disclosed sequence, such sequence set forth in the Sequence Listing, refers to nucleotides or amino acid positions identified in the polynucleotide or in the polypeptide upon alignment with the disclosed sequence using a standard alignment algorithm, such as the BLAST algorithm (NCBI). For example, one skilled in the art can identify a residue in a given polypeptide at a position corresponding to position 191 of SEQ ID NO: 13 by making a BLASTP alignment of the polypeptide sequence together with SEQ ID NO: 13, and find the residue in the polypeptide that is aligned with the residue 191 of SEQ ID NO: 13. By aligning the sequences, one skilled in the art can identify corresponding residues in a given polypeptide, for example, by using conserved and identical amino acid residues in the alignment as guides. Similarly, one skilled in the art can identify any given amino acid residue in a given polypeptide at a position corresponding to a particular position of a reference sequence, such as set forth in the Sequence Listing, by performing alignment of the polypeptide sequence with the reference sequence (for example, by BLASTP publicly available through the NCBI website), matching the corresponding position of the reference sequence with the position in polypeptide sequence and thus identifying the amino acid residue within the polypeptide.

As used herein, domain (such as a sequence of amino acid residues) refers to a portion of a molecule, such as a protein or encoding nucleic acid, that is structurally and/or functionally distinct from other portions of the molecule and is identifiable. For example, domains include those portions of a polypeptide chain that can form an independently folded structure within a protein made up of one or more structural motifs and/or that is recognized by virtue of a functional activity, such as binding activity. A protein can have one, or more than one, distinct domains. For example, a domain can be identified, defined or distinguished by homology of the primary sequence or structure to related family members, such as homology to motifs. In another example, a domain can be distinguished by its function, such as an ability to interact with a biomolecule, such as a cognate binding partner. A domain independently can exhibit a biological function or activity such that the domain independently or fused to another molecule can perform an activity, such as, for example binding. A domain can be a linear sequence of amino acids or a non-linear sequence of amino acids. Many polypeptides contain a plurality of domains. Such domains are known, and can be identified by those of skill in the art. For exemplification herein, definitions are provided, but it is understood that it is well within the skill in the art to recognize particular domains by name. If needed, appropriate software can be employed to identify domains.

As used herein, the term "alkyl" refers to and includes saturated linear and branched univalent hydrocarbon structures and combination thereof, having the number of carbon atoms designated (*i.e.,* $C_1$-$C_{10}$ or $C_{1-10}$ means one to ten carbons). Particular alkyl groups are those having 1 to 20 carbon atoms (a "$C_1$-$C_{20}$ alkyl"). More particular alkyl groups are those having 1 to 8 carbon atoms (a "$C_1$-$C_8$ alkyl"), 3 to 8 carbon atoms (a "$C_3$-$C_8$ alkyl"), 1 to 6 carbon atoms (a "$C_1$-$C_6$ alkyl"), 1 to 5 carbon atoms (a "$C_1$-$C_5$ alkyl"), or 1 to 4 carbon atoms (a "$C_1$-$C_4$ alkyl"), unless otherwise specified Examples of alkyl include, but are not limited to, groups such as methyl, ethyl, n-propyl, isopropyl, n-butyl, t-butyl, isobutyl, sec-butyl, homologs and isomers of, for example, n-pentyl, n-hexyl, n-heptyl, n-octyl, and the like.

As used herein, "alkenyl" as used herein refers to an unsaturated linear or branched univalent hydrocarbon chain or combination thereof, having at least one site of olefinic unsaturation (*i.e.,* having at least one moiety of the formula C=C) and having the number of carbon atoms designated (*i.e.,* $C_2$-$C_{10}$ means two to ten carbon atoms). The alkenyl group may be in "cis" or "trans" configurations, or alternatively in "E" or "Z" configurations. Particular alkenyl groups are those having 2 to 20 carbon atoms (a "$C_2$-$C_{20}$ alkenyl"), having 2 to 8 carbon atoms (a "$C_2$-$C_8$ alkenyl"), having 2 to 6 carbon atoms (a "$C_2$-$C_6$ alkenyl"), or having 2 to 4 carbon atoms (a "$C_2$-$C_4$ alkenyl"). Examples of alkenyl include, but are not limited to, groups such as ethenyl (or vinyl), prop-1-enyl, prop-2-enyl (or allyl), 2-methylprop-1-enyl, but-1-enyl, but-2-enyl, but-3-enyl, buta-1,3-dienyl, 2-methylbuta-1,3-dienyl, homologs and isomers thereof, and the like.

The term "aminoalkyl" refers to an alkyl group that is substituted with one or more $-NH_2$ groups. In certain embodiments, an aminoalkyl group is substituted with one, two, three, four, five or more $-NH_2$ groups. An aminoalkyl group may optionally be substituted with one or more additional substituents as described herein.

As used herein, "aryl" or "Ar" refers to an unsaturated aromatic carbocyclic group having a single ring (*e.g.,* phenyl) or multiple condensed rings (*e.g.,* naphthyl or anthryl) which condensed rings may or may not be aromatic. In one variation, the aryl group contains from 6 to 14 annular carbon atoms. An aryl group having more than one ring where at least

one ring is non-aromatic may be connected to the parent structure at either an aromatic ring position or at a non-aromatic ring position. In one variation, an aryl group having more than one ring where at least one ring is non-aromatic is connected to the parent structure at an aromatic ring position. In some embodiments, phenyl is a preferred aryl group.

As used herein, the term "arylalkyl" refers to an aryl group, as defined herein, appended to the parent molecular moiety through an alkyl group, as defined herein. Representative examples of arylalkyl include, but are not limited to, benzyl, 2- phenylethyl, 3-phenylpropyl, 2-naphth-2-ylethyl, and the like.

As used herein, the term "cycloalkyl" refers to and includes cyclic univalent hydrocarbon structures, which may be fully saturated, mono- or polyunsaturated, but which are non-aromatic, having the number of carbon atoms designated (*e.g.*, $C_1$-$C_{10}$ means one to ten carbons). Cycloalkyl can consist of one ring, such as cyclohexyl, or multiple rings, such as adamantly, but excludes aryl groups. A cycloalkyl comprising more than one ring may be fused, spiro or bridged, or combinations thereof. In some embodiments, the cycloalkyl is a cyclic hydrocarbon having from 3 to 13 annular carbon atoms. In some embodiments, the cycloalkyl is a cyclic hydrocarbon having from 3 to 8 annular carbon atoms (a "$C_3$-$C_8$ cycloalkyl"). Examples of cycloalkyl include, but are not limited to, cyclopropyl, cyclobutyl, cyclopentyl, cyclohexyl, 1-cyclohexenyl, 3-cyclohexenyl, cycloheptyl, norbornyl, and the like.

As used herein, the "halogen" represents chlorine, fluorine, bromine, or iodine. The term "halo" represents chloro, fluoro, bromo, or iodo.

The term "haloalkyl" refers to an alkyl group as described above, wherein one or more hydrogen atoms on the alkyl group have been replaced by a halo group. Examples of such groups include, without limitation, fluoroalkyl groups, such as fluoroethyl, trifluoromethyl, difluoromethyl, trifluoroethyl and the like.

As used herein, the term "heteroaryl" refers to and includes unsaturated aromatic cyclic groups having from 1 to 10 annular carbon atoms and at least one annular heteroatom, including but not limited to heteroatoms such as nitrogen, oxygen and sulfur, wherein the nitrogen and sulfur atoms are optionally oxidized, and the nitrogen atom(s) are optionally quaternized. It is understood that the selection and order of heteroatoms in a heteroaryl ring must conform to standard valence requirements and provide an aromatic ring character, and also must provide a ring that is sufficiently stable for use in the reactions described herein. Typically, a heteroaryl ring has 5-6 ring atoms and 1-4 heteroatoms, which are selected from N, O and S unless otherwise specified; and a bicyclic heteroaryl group contains two 5-6 membered rings that share one bond and contain at least one heteroatom and up to 5 heteroatoms selected from N, O and S as ring members. A heteroaryl group can be attached to the remainder of the molecule at an annular carbon or at an annular heteroatom, in which case the heteroatom is typically nitrogen. Heteroaryl groups may contain additional fused rings (*e.g.,* from 1 to 3 rings), including additionally fused aryl, heteroaryl, cycloalkyl, and/or heterocyclyl rings. Examples of heteroaryl groups include, but are not limited to, pyrazolyl, imidazolyl, triazolyl, pyrrolyl, pyridyl, pyrimidyl, pyrazinyl, pyridazinyl, triazinyl, thiophenyl, furanyl, thiazolyl, and the like.

As used herein, the term "heterocycle", "heterocyclic", or "heterocyclyl" refers to a saturated or an unsaturated non-aromatic group having from 1 to 10 annular carbon atoms and from 1 to 4 annular heteroatoms, such as nitrogen, sulfur or oxygen, and the like, wherein the nitrogen and sulfur atoms are optionally oxidized, and the nitrogen atom(s) are optionally quaternized. A heterocyclyl group may have a single ring or multiple condensed rings, but excludes heteroaryl groups. A heterocycle comprising more than one ring may be fused, spiro or bridged, or any combination thereof. In fused ring systems, one or more of the fused rings can be aryl or heteroaryl. Examples of heterocyclyl groups include, but are not limited to, tetrahydropyranyl, dihydropyranyl, piperidinyl, piperazinyl, pyrrolidinyl, thiazolinyl, thiazolidinyl, tetrahydrofuranyl, tetrahydrothiophenyl, 2,3-dihydrobenzo[b]thiophen-2-yl, 4-amino-2-oxopyrimidin-1(2H)-yl, and the like.

The term "substituted" means that the specified group or moiety bears one or more substituents in place of a hydrogen atom of the unsubstituted group, including, but not limited to, substituents such as alkoxy, acyl, acyloxy, carbonylalkoxy, acylamino, amino, aminoacyl, aminocarbonylamino, aminocarbonyloxy, cycloalkyl, cycloalkenyl, aryl, heteroaryl, aryloxy, cyano, azido, halo, hydroxyl, nitro, carboxyl, thiol, thioalkyl, cycloalkyl, cycloalkenyl, alkyl, alkenyl, alkynyl, heterocyclyl, aralkyl, aminosulfonyl, sulfonylamino, sulfonyl, oxo, carbonylalkylenealkoxy and the like. The term "unsubstituted" means that the specified group bears no substituents. The term "optionally substituted" means that the specified group is unsubstituted or substituted by one or more substituents and thus includes both substituted and unsubstituted versions of the group. Where the term "substituted" is used to describe a structural system, the substitution is meant to occur at any valency-allowed position on the system.

It is understood that aspects and embodiments of the invention described herein include "consisting" and/or "consisting essentially of" aspects and embodiments.

Throughout this disclosure, various aspects of this invention are presented in a range format. It should be understood that the description in range format is merely for convenience and brevity and should not be construed as an inflexible limitation on the scope of the invention. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Other objects, advantages and features of the present invention will become apparent from the following specification taken in conjunction with the accompanying drawings.

## I. MODIFIED OR ENGINEERED CLEAVASES

In another aspect, provided herein is a modified or an engineered cleavase comprising a mutation, *e.g*., one or more amino acid modification(s), in an unmodified cleavase, wherein: said modified or engineered cleavase is derived from a dipeptidyl peptidase of *Thermomonas hydrothermalis* or *Caldithrix abyssii* and removes or is configured to remove a single N-terminally modified amino acid from a target polypeptide. In some embodiments, the present modified or engineered cleavase is configured to cleave the peptide bond between a N-terminally modified amino acid residue and a penultimate terminal amino acid residue of the target polypeptide.

The present modified or engineered cleavase can comprise any suitable active site. For example, the present modified or engineered cleavase can comprise an active site that interacts with the amide bond between the N-terminally modified amino acid residue and a penultimate terminal amino acid residue of the target polypeptide.

The present modified or engineered cleavase can be derived from any suitable type of dipeptidyl peptidase. For example, the present modified or engineered cleavase can be derived from a protein or enzyme classified as a S46 dipeptidyl peptidase (*see e.g.,* Shakh M.A. Rouf, Yuko Ohara-Nemoto, Tomonori Hoshino, Taku Fujiwara, Toshio Ono, Takayuki K. Nemoto, Discrimination based on Gly and Arg/Ser at position 673 between dipeptidyl-peptidase (DPP) 7 and DPP11, widely distributed DPPs in pathogenic and environmental gram-negative bacteria, Biochimie, Volume 95, Issue 4, 2013, Pages 824-832, ISSN 0300-9084), or a functional homolog or fragment thereof.

The present modified or engineered cleavase can remove or can be configured to remove any suitable single N-terminally modified amino acid from a target polypeptide. For example, the present modified or engineered cleavase can remove or can be configured to remove a N-terminal amino acid that is labeled with a chemical or an enzymatic reagent or moiety.

The present modified or engineered cleavase can remove or can be configured to remove any suitable single N-terminally modified amino acid from a target polypeptide containing any suitable N-terminal modification (NTM), such as synthetic NTM. In another example, NTM can comprise an amino acid moiety and/or has a size, *e.g.,* length axis or volume, shape, and/or configuration similar to or exceeding a natural amino acid. In some embodiments, NTM can be a bipartite N-terminal modification that comprises a natural or unnatural amino acid portion (NTMaa) and a N-terminal blocking group ($NTM_{blk}$). The amino acid-like portion (NTMaa) and the N-terminal blocking group ($NTM_{blk}$) can be connected or linked by any suitable bond or linkage. For example, the amino acid portion (NTMaa) and the N-terminal blocking group ($NTM_{blk}$) can be connected with an amide bond.

In some embodiments, NTM does not comprise an amino acid moiety. In some embodiments, NTM comprises a N-terminal blocking group ($NTM_{blk}$) and does not comprise a NTMaa group. In some embodiments, NTM can be a bipartite N-terminal modification that comprises a small (or small molecule) chemical entity having a size, *e.g.,* length axis or volume, shape, and/or configuration similar to or exceeding a natural amino acid, and a N-terminal blocking group ($NTM_{blk}$). The small (or small molecule) chemical entity and the N-terminal blocking group ($NTM_{blk}$) can be connected or linked by any suitable bond or linkage. For example, the small (or small molecule) chemical entity and the N-terminal blocking group can be connected with an amide bond. The small (or small molecule) chemical entity can have any suitable size, *e.g.,* length axis or volume. For example, the small (or small molecule) chemical entity can have a size, *e.g.,* length axis of about 5-10 Å and volume of about 100 - 1000 $Å^3$. In some embodiments, the small (or small molecule) chemical entity has a length axis of about 5, 6, 7, 8, 9 or 10 Å, or any range thereof. In some embodiments, , the small (or small molecule) chemical entity has a volume of about 100, 200, 300, 400, 500, 600, 700, 800, 900, 1000 $Å^3$ or any range thereof.

In another example, the N-terminal modification can comprise a chemical label.

In some embodiments, a chemical reagent for the N-terminal modification is selected from the group consisting of: 2-aminobenzamide, 2-(N-methylamino)-benzamide, 2-(*N*-acetylamine)-benzamide, 2-(N-benzylamine)-benzamide, 4-methylbenzamide, 4-(dimethylamino)benzamide, nicotinamide, 3-aminonicotinamide, 2-pyrazinecarbonyl, 5-amino-2-fluoro-isonicotinamide, 2-carboxylic acid pyrazinecarbonyl, 3,6-difluoro-2-carboxybenzamide, 4-chloro-2-aminobenzamide, 4-nitro-2-aminobenzamide, 4-methoxy-2-aminobenzamide, 4-carboxylic acid-2-aminobenzamide, 5-(trifluoromethyl-2-aminobenzamide, 4-(trifluoromethyl-2-aminobenzamide, 6-fluoro-2-aminobenzamide, 4-fluoro-2-aminobenzamide, 5-methoxy-2-aminobenzamide, 4-fluorobenzamide, 4-(trifluoromethyl)benzamide, 8-fluoroisoquinolinium, 1-hydroxy-2,3,1-benzodiazaborinine-2(1H)-carbonyl, Succinamide, 3,6-Difluoropyridine-2-carbamide, 2-Fluoronicotinamide, 5-Bromo-2-hydroxynicotinamide, 4-(Trifluoromethyl)pyrimidine-5-carbamide, 2-Oxo-1,2-dihydropyridine-3-carbamide, 5-Methyl-2-aminobenzamide, 6-Fluoropicolinamide, 3-Methyl-2-aminobenzamide, 4-Methyl--2-aminobenzamide, 2-Amino-6-methylbenzamide, 2-Amino-6-fluorobenzamide, 2-Amino-5-fluorobenzoamide, 2-Amino-3-fluorobenzoamide, 2-Amino-4-fluorobenzoamide, 2-Aminonicotinamide, 4-Aminonicotinamide, 3-Aminopicolinamide, or a derivative thereof. In some embodiments, the chemical reagent for the N-terminal modification is an isatoic anhydride, an isonicotinic anhydride, an azaisatoic anhydride, a succinic anhydride, an aryl activated ester, a heteroaryl activated ester, a non-aromatic ring activated ester, or a derivative thereof. In some embodiments, the chemical reagent for the N-terminal modification is selected from the group consisting of wherein the chemical reagent is selected from the group consisting of 4-Nitrophenyl Anthranilate, N-Methyl-isatoic anhydride, N-acetyl-isatoic anhydride, N-benzyl-isatoic anhydride, 4-methylbenzoic acid, 4-(dimethylamino)benzoyl chloride, nicotinic acid-NHS, 3-aminonicotinic acid, 2-pyrazinecarbonyl chloride, 5-amino-2-fluoro-isonicotinic acid, 2,3-pyrazinedicarboxylic anhydride, 3,6-difluorophthalic anhydride, 4-chloroisatoic anhydride, 4-nitroisatoic anhydride, 7-methoxy-1h-benzo[d][1,3]oxazine-2,4-dione, 4-carboxylic acid isatoic anhydride, 6-(Trifluoromethyl)-2,4-dihydro-1h-3,1-benzoxazine-2,4-dione, 7-(Trifluoromethyl)-lh-benzo[d][1,3]oxazine-2,4-dione, 6-fluoroisatoic anhydride, 4-fluoroisatoic anhydride, 5-methoxyisatoic anhydride, 4-fluorobenzoic acid anhydride, 4-(trifluoromethyl)benzoic acid anhydride, 2-ethynyl-6-fluorobenzaldehyde, 1-hydroxy-2,3,1-benzodiazaborinine-2(1H)-carboxylic acid, Isatoic anhydride, Succinic anhydride 3,6-Difluoropyridine-2-carboxylic acid, 2-Fluoronicotinic acid, 5-Bromo-2-hydroxynicotinic acid, 4-(Trifluoromethyl)pyrimidine-5-carboxylic acid, 2-Oxo-1,2-dihydropyridine-3-carboxylic acid, 5-Methylisatoic anhydride, 6-Fluoropicolinic acid, 3-Methylisatoic anhydride, 4-Methyl-isatoic anhydride, 2-Amino-6-methylbenzoic acid, 2-Amino-6-fluorobenzoic acid, 2-Amino-5-fluorobenzoic acid, 2-Amino-3-fluorobenzoic acid, 2-Amino-4-fluorobenzoic acid, 2-Aminonicotinic acid, 4-Aminonicotinic acid, 3-Aminopicolinic acid, or a derivative thereof.

The present modified or engineered cleavase can comprise any suitable amino acid sequence variation(s) as compared with the amino acid sequence of the unmodified cleavase. For example, the present modified or engineered cleavase can comprise an amino acid sequence that exhibits at least 50 % identity, at least 60 % identity, at least 70 % identity, at least 80 % identity, or at least 90 %, or at least 95 %, or more identity with the unmodified cleavase.

The present or engineered modified cleavase can comprise any suitable type of mutation(s). For example, wherein the mutation can comprise an amino acid substitution, deletion, addition, or a combination thereof.

The present modified or engineered cleavase can remove or can be configured to remove a single N-terminally modified amino acid from a target polypeptide with any suitable length. For example, the length of the target polypeptide can be greater than 4 amino acids, greater than 5 amino acids, greater than 6 amino acids, greater than 7 amino acids, greater than 8 amino acids, greater than 9 amino acids, greater than 10 amino acids, greater than 11 amino acids, greater than 12 amino acids, greater than 13 amino acids, greater than 14 amino acids, greater than 15 amino acids, greater than 20 amino acids, greater than 25 amino acids, or greater than 30 amino acids.

The present modified or engineered cleavase can comprise mutation(s) at any suitable site(s). For example, the present modified or engineered cleavase can comprise a modification within its substrate binding site. Substrate binding site of a cleavase is comprised of amino acid residues that are involved in interaction with the substrate during substrate recognition and cleavage. The specificity for the substrate is due to the favorable binding interaction of the substrate amino acid side chains with residues that form the substrate binding site of the cleavase (also called specificity pocket). For example, the binding site of dipeptidyl aminopeptidases comprises residues that are involved in interaction with the N-terminal amino group of a polypeptide (these residues form an amine binding site that is a part of the substrate binding site), and residues that are involved in interaction with P1 and P2 residues of the polypeptide. For modified dipeptidyl aminopeptidases amino acid residues in the substrate binding site are modified to interact with the NTM of a labeled polypeptide, and also with P1 or P2 residues of the polypeptide. In some cases, amino acid residues in the substrate binding site of a modified dipeptidyl aminopeptidase are modified such that the modified dipeptidyl aminopeptidase would not recognize the N-terminal amino group of a polypeptide, and thus the modified dipeptidyl aminopeptidase would not cleave unlabeled polypeptide. The substrate binding site of a dipeptidyl cleavase can be determined for example using crystal structure of the dipeptidyl cleavase with its substrate or with an inhibitor, mimicking the substrate.

In another example, the present modified or engineered cleavase can comprise a modification within its catalytic domain. In still another example, the present modified or engineered cleavase can comprise a modification within its chymotrypsin fold. In yet another example, the present modified or engineered cleavase can comprise a modification at an amine binding site. In yet another example, the present modified or engineered cleavase can comprise a modification in its S1 and/or S2 sites. In yet another example, the present modified or engineered cleavase can comprise a modification for improving accessibility to the active site of the modified or engineered cleavase.

In some embodiments, the present modified or engineered cleavase is derived from a dipeptidyl peptidase of *Thermomonas hydrothermalis* comprising an amino acid sequence set forth in SEQ ID NO:33 (wild type (WT) sequence with the signal peptide) or SEQ ID NO:31 (WT sequence without the signal peptide).

The present modified or engineered cleavase can comprise any suitable amino acid sequence variations as compared with the amino acid sequence of the unmodified cleavase. For example, the present modified or engineered cleavase can comprise an amino acid sequence that exhibits at least 30 % identity, at least 40 % identity, at least 50 % identity, at least 60 % identity, at least 70 % identity, at least 80 % identity, at least 90 % or more identity or at least 95 % or more identity to the amino acid sequence set forth in SEQ ID NO:33 or SEQ ID NO:31, or a specific binding fragment thereof.

In some embodiments, the present modified or engineered cleavase has a mutation, with reference to positions of SEQ ID NO: 33, selected from the group consisting of N214X, W215X, R219X, N329X, N333X, A671X, D673X, G674X,

N682X, M692X, I651X, and a combination thereof, X being one of the 20 naturally occurring amino acids other than the amino acid residue of the unmodified dipeptidyl peptidase at the mutated position. In some embodiments, the present modified or engineered cleavase has one or more amino acid modification(s) of N214M, W215G, R219T, N329R, D673A, and/or G674V with reference to positions of SEQ ID NO: 33.

In some embodiments, the present modified or engineered cleavase exhibits the substrate specificity of the above modified or engineered cleavase. In present some embodiments, the modified or engineered cleavase comprises an amino acid sequence that comprises a catalytic domain, an amine binding site, or S1 and/or S2 sites with at least 30 % identity, at least 40 % identity, at least 50 % identity, at least 60 % identity, at least 70 % identity, at least 80 % identity, or at least 90 %, 95 %, or more identity with the catalytic domain, the amine binding site, or the S1 and/or S2 sites of the above modified or engineered cleavase. By definition, when referring to proteases, the S1 site is defined as the region of the protease that binds to the amino acid just upstream (amino side) of the cleavage position and the S2 site binds to the amino acid two residues upstream (amino side) to the cleavage position. For a native DPP which binds to the N-terminal dipeptide of a peptide, the S2 site binds to the N-terminal amino acid and the S1 site binds to the penultimate amino acid. In the modified or engineered Cleavase, derived from a DPP, the S2 site can be used to participate in binding to the N-terminal modification (NTM), and the S1 site used to bind to the NTAA residue, creating a single modified amino acid cleavage.

In some embodiments, the present modified or engineered cleavase is derived from a dipeptidyl peptidase of *Caldithrix abyssii* comprising an amino acid sequence set forth in SEQ ID NO:34 (WT sequence with the signal peptide) or SEQ ID NO:32 (WT sequence without the signal peptide).

The present modified or engineered cleavase can comprise any suitable amino acid sequence variations as compared with the amino acid sequence of the unmodified cleavase. For example, the present modified or engineered cleavase can comprise an amino acid sequence that exhibits at least 30 % identity, at least 40 % identity, at least 50 % identity, at least 60 % identity, at least 70 % identity, at least 80 % identity, at least 90 % or more identity or at least 95 % or more identity to the amino acid sequence set forth in SEQ ID NO:34 or SEQ ID NO:32, or a specific binding fragment thereof.

In some embodiments, the present modified or engineered cleavase has a mutation, with reference to positions of SEQ ID NO: 34, selected from the group consisting of N207M, W208X, R212X, N322X, D663X, and a combination thereof, X being one of the 20 naturally occurring amino acids other than the amino acid residue of the unmodified dipeptidyl peptidase at the mutated position. In some embodiments, the present modified or engineered cleavase has one or more amino acid modification(s) of N207M, W208G, R212V, N322I, D663A, or a combination thereof, with reference to positions of SEQ ID NO: 34.

In some embodiments, the present modified or engineered cleavase exhibits the substrate specificity of the above modified or engineered cleavase. In present some embodiments, the modified or engineered cleavase comprises an amino acid sequence that comprises a catalytic domain, an amine binding site, or S1 and/or S2 sites, with at least 30 % identity, at least 40 % identity, at least 50 % identity, at least 60 % identity, at least 70 % identity, at least 80 % identity, or at least 90 % or more identity with the catalytic domain, the amine binding site, or the S1 and/or S2 sites of the above modified or engineered cleavase.

A nucleic acid encoding the above modified or engineered cleavase is provided herein. A vector, *e.g.,* an expression vector, comprising the nucleic acid encoding the above modified or engineered cleavase is also provided herein. A host cell comprising the above nucleic acid or the vector is further provided herein. The host cell can be any suitable type of cell. For example, the host cell can be a mammalian or human host cell.

In another aspect, provided herein is a modified dipeptide cleavase comprising a mutation, *e.g.*, one or more amino acid modification(s) in an unmodified dipeptide cleavase, wherein the modified dipeptide cleavase removes or is configured to remove a labeled terminal dipeptide from a polypeptide. In some embodiments, the modified dipeptide cleavase is configured to remove a single labeled dipeptide (the terminal and penultimate terminal amino acids) from the C-terminus or N-terminus of a polypeptide. In some embodiments, the modified dipeptide cleavase is derived from a wild-type or unmodified dipeptide cleavase. In some cases, the dipeptide removed contains a terminal labeled amino acid residue that is an N-terminal amino acid. In some embodiments, the dipeptide removed contains a terminal labeled amino acid residue that is a C-terminal amino acid. In some aspects, a labeled amino acid (removed as part of the dipeptide) is a terminal amino acid that is modified by treating with a chemical reagent. In some aspects, the modified dipeptide cleavase comprises an active site that interacts with an amide bond (*e.g.,* the amide bond between a penultimate and antepenultimate terminal amino acid residues of the polypeptide). In some embodiments, the modified dipeptide cleavase contains a mutation that is an amino acid substitution, deletion, addition, or any combinations thereof.

In some embodiments, the modified dipeptide cleavase exhibits activity that is different from the activity of the unmodified or wild-type dipeptide cleavase. "Unmodified dipeptide cleavase" or "wild-type dipeptide cleavase" as used herein refers to any natural or wild-type exopeptidase, or a functional homolog or fragment thereof, that possesses catalytic activity to remove a dipeptide from the terminus of a polypeptide (*e.g.*, from the C-terminus or N-terminus of a polypeptide). The unmodified or wild-type dipeptide cleavase may be an exopeptidase that catalyzes the cleavage of an penultimate peptide bond to release a dipeptide from the peptide chain. The unmodified or wild-type dipeptide cleavase

removes unlabeled or unmodified dipeptides from the peptide chain. The unmodified or wild-type dipeptide cleavase may be a proteolytic enzyme such as an aminopeptidase or a carboxypeptidase. The unmodified dipeptide cleavase described herein may be used to refer to a protein classified by the Enzyme Commission (EC) as EC 3.4.14, EC 3.4.15, MEROPS S9, MEROPS S46, MEROPS M49, or a functional homolog or fragment thereof. The unmodified dipeptide cleavase described herein may be used to refer to a dipeptidyl peptidase, a dipeptidyl aminopeptidase, a peptidyl-dipeptidase, or a dipeptidyl carboxypeptidase.

A "modified dipeptide cleavase" or "variant dipeptide cleavase" refers to any exopeptidase that has been modified from a unmodified or wild-type dipeptide cleavase as described. The modified or variant dipeptide cleavase may be derived from an unmodified or wild-type dipeptide cleavase (*e.g.* a dipeptidyl peptidase, a dipeptidyl aminopeptidase, a peptidyl-dipeptidase, a dipeptidyl carboxypeptidase). As compared to an unmodified or wild-type dipeptide cleavase which removes an unlabeled P1-P2 terminal amino acids from a polypeptide as a dipeptide at a time, a modified dipeptide cleavase removes or is configured to remove a labeled terminal dipeptide from a polypeptide, or a single labeled terminal amino acid (such as N-terminal amino acid or NTAA). In some embodiments, a modified dipeptide cleavase preferentially removes a labeled P1-P2 terminal dipeptide from the polypeptide as compared to the cleavage of an unlabeled P1-P2 terminal dipeptide from the polypeptide. In some embodiments, a modified dipeptide cleavase removes only a labeled P1 terminal residue from the polypeptide as compared to the cleavage of an unlabeled P1-P2 terminal dipeptide from the polypeptide. In the present disclosure P1 is a terminal amino acid residue of a polypeptide, such as N-terminal amino acid (NTAA), and P2 is a penultimate terminal amino acid residue of a polypeptide.

In some embodiments, the modified dipeptide cleavase removes a dipeptide comprising a labeled terminal amino acid, *e.g.,* a labeled NTAA. In some cases, the modified dipeptide cleavase removes a dipeptide comprising a labeled C-terminal amino acid (CTAA). In some embodiments, the modified dipeptide cleavase derived from the dipeptide cleavase is configured to cleave the peptide bond between a penultimate terminal labeled amino acid residue and a antepenultimate terminal amino acid residue of the polypeptide.

In another example, the peptide bond between the P1 and P2 can be cleaved using a modified cleavase. In some embodiments, the peptide bond between the P1 and P2 is cleaved using an above descried modified or engineered cleavase (*see e.g.,* the above Section I.) In some embodiments, the peptide bond between the P1 and P2 is cleaved using a modified or an engineered cleavase described and/or claimed in U.S. provisional application serial Nos. 62/823,927, filed March 26, 2019, 62/824,157, filed March 26, 2019, and 62/931,737, filed November 6, 2019, and in application WO 2020/198264 published on October 01, 2020.

Information regarding various known unmodified or wild-type exopeptidases is available from databases such as MEROPS and/or the BRENDA enzyme information system (*See e.g.,* Schomburg et al., J Biotechnol. (2017) 261:194-206). A protein may be classified using more than one classification system. The Enzyme Commission (EC) sets forth a numbering system for the classification of enzyme based upon specificity using recommendations from Nomenclature Committee of the International Union of Biochemistry and Molecular Biology (IUBMB) for describing each type of characterized enzyme for which an EC (Enzyme Commission) number has been provided (*See e.g.,* Bairoch A., (2000) Nucleic Acids Res. 28:304-305). In some aspects, the unmodified or wild-type dipeptide cleavase is a protein classified in EC 3.4.14, EC 3.4.15, MEROPS S9, MEROPS S46, MEROPS M49, or a homolog thereof. In some aspects, the unmodified or wild-type dipeptide cleavase is a protein provided in **Tables 1, 2, 3, 4, 5A-5B**, or a homolog thereof. In some embodiments, the modified dipeptide cleavase is derived from a protein classified EC 3.4.14, EC 3.4.15, MEROPS S9, MEROPS S46, MEROPS M49, or a functional homolog or fragment thereof (as provided in **Tables 1, 2, 3, 4, 5A-5B**).

| **Table 1. Exemplary Dipeptide Cleavases from EC 3.4.14** | |
|---|---|
| **Enzyme Commission Number** | **Name** |
| 3.4.14.1 | dipeptidyl-peptidase I |
| 3.4.14.2 | dipeptidyl-peptidase II |
| 3.4.14.4 | dipeptidyl-peptidase III |
| 3.4.14.5 | dipeptidyl-peptidase IV |
| 3.4.14.6 | dipeptidyl-dipeptidase |
| 3.4.14.11 | Xaa-Pro dipeptidyl-peptidase |
| 3.4.14.13 | gamma-D-glutamyl-L-ly sine dipeptidyl-peptidase |

| Table 2. Exemplary Dipeptide Cleavases from EC 3.4.15 | |
|---|---|
| **Enzvme Commission Number** | **Name** |
| 3.4.15.1 | peptidyl-dipeptidase A |
| 3.4.15.3 | dipeptidyl carboxypeptidase |
| 3.4.15.4 | peptidyl-dipeptidase B |
| 3.4.15.5 | peptidyl-dipeptidase Dcp |

| Table 3. Exemplary Dipeptide Cleavases from MEROPS S9 | |
|---|---|
| ***MEROPS* ID** | **Name** |
| S09.003 | dipeptidyl-peptidase IV (eukaryote) |
| S09.009 | dipeptidyl-peptidase 4 (bacteria-type 1) |
| S09.012 | dipeptidyl-peptidase V/ dipeptidyl-peptidase 5 |
| S09.013 | dipeptidyl-peptidase 4 (bacteria-type 2) |
| S09.018 | dipeptidyl-peptidase 8 |
| S09.019 | dipeptidyl-peptidase 9 |
| S09.056 | dipeptidyl-peptidase IV, membrane-type (protistan) |
| S09.075 | dipeptidyl-peptidase 5 (*Porphyromonas sp.*) |
| Unassigned | subfamily S9B unassigned peptidases |

| Table 4. Exemplary Dipeptide Cleavases from MEROPS S46 | |
|---|---|
| ***MEROPS* ID** | **Name** |
| S46.001 | dipeptidyl-peptidase 7 |
| S46.002 | dipeptidyl-peptidase 11 |
| S46.003 | dipeptidyl-peptidase BII |
| S46.004 | BF9343_2924 g.p. |

| Table 5A. Exemplary Dipeptide Cleavases from MEROPS M49 | |
|---|---|
| ***MEROPS* ID** | **Name** |
| M49.001 | dipeptidyl-peptidase III |
| M49.003 | dipeptidyl-peptidase IIIB *(Bacteroides thetaiotaomicron-type*) |
| M49.004 | dipeptidyl-peptidase III (*Saccharomyces-type*) |

| Table 5B. Other Exemplary Dipeptide Cleavases | |
|---|---|
| ***MEROPS* ID** | **Name** |
| C01.070 | dipeptidyl-peptidase I |
| S28.002 | dipeptidyl-peptidase II |
| S15.001 | Xaa-Pro dipeptidyl-peptidase |
| S9G.084 | dipeptidyl-peptidase IV beta |

Various peptidases (*e.g.*, cleavases) that sequentially cleave off dipeptides or tripeptides from unsubstituted N-terminals of oligopeptides have been identified. Cleavases as described herein refer to enzymes that are classified under

the Enzyme Commission (EC) Class 3 of hydrolases. Dipeptidyl peptidases (DPPs from Enzyme Commission number 3.4.14; **Table 1)** are a class of exopeptidases which digest dipeptides (two amino acid residues, P1-P2) from the N-terminal end of a peptide, typically in a processive manner. Peptidyl dipeptidases also known as dipeptidyl carboxypeptidases (EC 3.4.15; **Table** 2) act from the C-terminal end in removing dipeptides in a processive manner. In some embodiments, the unmodified or wild-type dipeptide cleavase is an exoaminopeptidase or an exopeptidase. In some aspects, the unmodified or wild-type dipeptide cleavase is a metallopeptidase, *e.g.*, a zinc-dependent metallopeptidase or a zinc-dependent hydrolase. In some aspects, the unmodified or wild-type dipeptide cleavase is a serine exopeptidase or a serine protease. DPPs typically recognize the N-terminal alpha amine, and cleave the peptide bond between the penultimate and antepenultimate amino acid residues of a polypeptide (P2-P3). *See e.g.,* Sanderink et al., J. Clin. Chem. Clin. Biochem. (1988) 26:795-807) and Baral et al., J Biol Chem (2008) 283(32): 22316-22324.

In some embodiments, the modified dipeptide cleavase exhibits activity including the removal of a labeled terminal dipeptide from polypeptides or proteins (*e.g.*, from the N-terminus or C-terminus). In a general manner, the peptidase activity is capable of removing the amino acids $Xaa_1$ and $Xaa_2$ from the terminus of a peptide, polypeptide, or protein, wherein Xaa may represent any amino acid residue selected from the group consisting of Ala, Arg, Asn, Asp, Cys, Gln, Glu, Gly, His, Ile, Leu, Lys, Met, Phe, Pro, Ser, Thr, Trp, Tyr, and Val. It will be understood that the modified dipeptide cleavase of the present disclosure may be unspecific as to the amino acid sequence of the peptide, polypeptide, or protein to be cleaved. In some embodiments, the modified dipeptide cleavase is partially specific or selective. In some aspects, the modified dipeptide cleavase preferentially cleaves or removes some amino acids at the P1 and/or P2 position of the peptide over others. In some cases, the modified dipeptide cleavase preferentially cleaves or removes a class of amino acids over others, *e.g.*, preferentially removing hydrophobic amino acids over other classes of amino acids. In some aspects, the modified dipeptide cleavase may also have a preference for one or more amino acids at the second, third, fourth, fifth, etc. positions from the terminal amino acid. In some cases, the modified dipeptide cleavase exhibits specificity to subsets of amino acids and preferentially removes 1, 2, 3, 4, 5, 6, 7, 8, 9, 10, 15 or more specific terminal amino acid over others.

In some embodiments, the modified dipeptide cleavase is a polypeptide having an altered amino acid sequence, relative to an unmodified or wild-type dipeptide cleavase. In some cases, the modified dipeptide cleavase is a polypeptide which differs from a wild-type dipeptide cleavase sequence by one or more amino acid substitutions, deletions, additions, or combinations thereof. A variant or modified dipeptide cleavase can contain 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or more mutations, *e.g.*, amino acid differences, compared to the wild-type cleavase.

In some embodiments, the variant or modified dipeptide cleavase polypeptide generally exhibits at least 30%, 40%, 50%, 60%, 70%, 80%, 85%, 86%, 87%, 88%, 89%, 90%, 91%, 92%, 93%, 94%, 95%, 96%, 97%, 98%, 99% or more sequence identity to a corresponding wild-type or unmodified dipeptide cleavase. In some embodiments, the wild-type or unmodified dipeptide cleavase comprises the amino acid sequence of any one of SEQ ID NO: 5-8, 10-16, 20, 33, 34, a mature sequence thereof that excludes a signal peptide, or a portion thereof containing the active site. In some embodiments, the variant or modified dipeptide cleavase polypeptide generally exhibits at least 30%, 40%, 50%, 60%, 70%, 80%, 85%, 86%, 87%, 88%, 89%, 90%, 91%, 92%, 93%, 94%, 95%, 96%, 97%, 98%, 99% or more sequence identity to a wild-type or unmodified dipeptide cleavase set forth in SEQ ID NOs: 5-8, 10-16, 20, 33, 34, or a mature sequence thereof that excludes a signal peptide.

It is within the level of a skilled artisan to identify the corresponding position of a mutation or modification, *e.g.*, amino acid substitution, in a dipeptide cleavase polypeptide, including a portion thereof, such as by alignment with a reference sequence. In some embodiments, the unmodified or reference dipeptide cleavase polypeptide generally exhibits at least 30%, 40%, 50%, 60%, 70%, 80%, 85%, 86%, 87%, 88%, 89%, 90%, 91%, 92%, 93%, 94%, 95%, 96%, 97%, 98%, 99% or more sequence identity to any of the sequences set forth in SEQ ID NOs: 5-8 and 10-16, 20, 33, 34. For example, corresponding residues can be determined by alignment of a reference sequence with a sequence provided herein (for example, sequences set forth in SEQ ID NOs: 5-8, 10-16, 20, 33, 34, or a functional homolog or fragment thereof) using known alignment methods. By aligning the sequences, one skilled in the art can identify corresponding residues, for example, using conserved and identical amino acid residues as guides. In some cases, while the numbering (positions) of the residues provided herein may differ from a reference sequence, using the alignment method will allow determination of corresponding residues.

In some embodiments, the modified dipeptide cleavase comprises a mutation, *e.g.,* one or more amino acid modification(s), in an unmodified dipeptide cleavase, wherein the unmodified dipeptide cleavase is a dipeptidyl peptidase 3. Dipeptidyl peptidase 3 (also known as dipeptidyl peptidase III, dipeptidyl aminopeptidase III, dipeptidyl arylamidase III, enkephalinase B, red cell angiotensinase, DPP3, or DPP III) is a metalloproteinase (zinc-dependent) that sequentially removes dipeptides (two amino acid residues) from the N-terminus of short peptides. Wild-type or unmodified DPP3 is classified in the M49 family (MEROPS database identifier M49.001). In some cases, the unmodified dipeptidyl peptidase 3 exhibits at least 30%, 40%, 50%, 60%, 70%, 80%, 85%, 86%, 87%, 88%, 89%, 90%, 91%, 92%, 93%, 94%, 95%, 96%, 97%, 98%, 99% or more sequence identity to UniProt Accession No. Q9NY33 as set forth in SEQ ID NO: 5, UniProt

Accession No. Q08225 as set forth in SEQ ID NO: 6, UniProt Accession No. Q8A6N1 as set forth in SEQ ID NO: 7, UniProt Accession No. H1XW48 as set forth in SEQ ID NO: 8, or UniProt Accession No. O55096 as set forth in SEQ ID NO: 15 (*See e.g.,* Prajapati et al., FEBS J. 2011; 278(18):3256-276; Fukasawa et al., Biochem J. 1998 Jan 15; 329(Pt 2): 275-282; Fukasawa et al., J Amino Acids. 2011; 2011: 574816). DPP3 preferentially digests peptides that are 3 to 10 amino acids in length. DPP3 harbors a unique HEXXGH catalytic motif (SEQ ID NO: 1). Both histidines in this motif along with the glutamate residue of a second conserved EEXRAE/D motif are involved in zinc coordination (SEQ ID NO: 2). In some cases, C-terminal peptide modifications do not affect the activity of DPP3 enzymes. *See* Kumar et al., Sci Rep. (2016) 6:23787. Several substrate-bound structures of DPP3 have been solved, including in complex with peptides that have an N-terminal tyrosine. An N-terminal tyrosine is structurally similar to a phenylisothiocyanate (PITC), nitro-PITC, sulfo-PITC, or a phenylisocyanate version of these modifiers, and these substrate-bound structures may be useful for a targeted active-site design approach. In some embodiments, provided is a modified dipeptide cleavase derived from a dipeptidyl peptidase 3 that cleaves labeled terminal amino dipeptides, *e.g.*, dipeptides containing a labeled N-terminal amino acid residue.

In some embodiments, the modified dipeptide cleavase comprises a mutation, *e.g.,* one or more amino acid modification(s), in an unmodified dipeptide cleavase, wherein the unmodified dipeptide cleavase is a dipeptidyl peptidase 5. Dipeptidyl peptidase 5 is also known as allergen Tri m 4 (*Trichophyton mentagrophytes*), allergen Tri r 4 (*Trichophyton rubrum*), allergen Tri t 4 (*Trichophyton tonsurans*), dipeptidyl-peptidase V, DPP V, and secreted alanyl dipeptidyl peptidase (*Aspergillus oryzae*). Wild-type or unmodified dipeptidyl peptidase 5 is classified in the peptidase family S9 (MEROPS database identifier S09.012). Wild-type or unmodified dipeptidyl peptidase 5 has been observed to catalyze the hydrolysis of X-Ala, His-Ser, and Ser-Tyr dipeptides at a neutral pH optimum *(See e.g.,* Beauvais et al., J Biol Chem. 1997; 272(10):6238-44). Wild-type or unmodified dipeptidyl peptidase 5 is described as a secreted dipeptidyl peptidase which contains the consensus sequences of the catalytic site of the nonclassical serine proteases. In some cases, the unmodified dipeptide cleavase exhibits at least 30%, 40%, 50%, 60%, 70%, 80%, 85%, 86%, 87%, 88%, 89%, 90%, 91%, 92%, 93%, 94%, 95%, 96%, 97%, 98%, 99% or more sequence identity to UniProt Accession No. P0C959 as set forth in SEQ ID NO: 10 or UniProt Accession No. B2RIT0 as set forth in SEQ ID NO: 16. In some embodiments, the mutations, *e.g.,* one or more amino acid modifications *(e.g.,* substitutions, deletions, additions) in the modified dipeptide cleavase is in reference to the amino acid sequence set forth in reference to positions of SEQ ID NO: 10 or 16.

In some embodiments, the modified dipeptide cleavase comprises a mutation, *e.g.,* one or more amino acid modification(s), in an unmodified dipeptide cleavase, wherein the unmodified dipeptide cleavase is a dipeptidyl peptidase 7 (DPP7). Wild-type or unmodified DPP7 is classified in S46 protease family (MEROPS database identifier S46.001). Wild-type or unmodified DPP7 has been observed to catalyze the removal of dipeptides from the N-terminus of oligopeptides, including a broad specificity for both aliphatic and aromatic residues in the P1 position, with glycine or proline being not acceptable in this position *(See e.g.,* Banbula et al., J. Biol. Chem. 2001, 276:6299-6305). DPP7 has been shown to exhibit activity for cleaving the synthetic substrates Met-Leu-methylcoumaryl-7-amide (Met-Leu-MCA), Leu-Arg-MCA, and Lys-Ala-MCA (Rouf et al., FEBS Open Bio. 2013; 3:177-83). In some cases, the unmodified dipeptide cleavase exhibits at least 30%, 40%, 50%, 60%, 70%, 80%, 85%, 86%, 87%, 88%, 89%, 90%, 91%, 92%, 93%, 94%, 95%, 96%, 97%, 98%, 99% or more sequence identity to UniProt Accession No. B2RKV3 as set forth in SEQ ID NO: 11. In some embodiments, the mutations, *e.g.,* one or more amino acid modification(s) *(e.g.,* substitutions, deletions, additions) in the modified dipeptide cleavase is in reference to the amino acid sequence set forth in reference to positions of SEQ ID NO: 11.

In some embodiments, the modified dipeptide cleavase comprises a mutation, *e.g.,* one or more amino acid modification(s), in an unmodified dipeptide cleavase, wherein the unmodified dipeptide cleavase is a dipeptidyl peptidase 11. Dipeptidyl peptidase 11 is also known as Asp/Glu-specific dipeptidyl-peptidase or DPP11. Wild-type or unmodified dipeptidyl peptidase 11 is classified in S46 protease family (MEROPS database identifier S46.002), and shares 38.7% sequence identity with dipeptidyl peptidase 7. Wild-type or unmodified dipeptidyl peptidase 11 has been observed to catalyze the removal of dipeptides from the N-terminus of oligopeptides, including removing dipeptides from oligopeptides with the penultimate N-terminal Asp and Glu and has a P2-position preference to hydrophobic residues *(See e.g.,* Ohara-Nemoto et al., J Biol Chem. 2011; 286(44):38115-27). In some cases, the unmodified dipeptide cleavase exhibits at least 30%, 40%, 50%, 60%, 70%, 80%, 85%, 86%, 87%, 88%, 89%, 90%, 91%, 92%, 93%, 94%, 95%, 96%, 97%, 98%, 99% or more sequence identity to UniProt Accession No. B2RID1 or F8WQK8 as set forth in SEQ ID NO: 12 and 14, respectively. In some embodiments, the mutations, *e.g.,* one or more amino acid modifications (*e.g.,* substitutions, deletions, additions) in the modified dipeptide cleavase is in reference to the amino acid sequence set forth in reference to positions of SEQ ID NO: 12. In some embodiments, the mutations, *e.g.,* one or more amino acid modifications (*e.g.,* substitutions, deletions, additions) in the modified dipeptide cleavase is in reference to the amino acid sequence set forth in reference to positions of SEQ ID NO: 14.

In some embodiments, the modified dipeptide cleavase comprises a mutation, *e.g.,* one or more amino acid modification(s), in an unmodified dipeptide cleavase, wherein the unmodified dipeptide cleavase is a dipeptidyl aminopeptidase BII (DAP BII or dipeptidyl peptidase BII). Wild-type or unmodified DAP BII catalyzes the removal of dipeptides from the amino terminus of peptides (*See e.g.,* Ogasawara et al., J. Bacteriol. 1996, 178:6288-6295); Sakamoto et al.,

Scientific Reports 2014, 4:4977). DAP BII is a serine protease that belongs to the serine peptidase family S46 (MEROPS database identifier S46.003). The amino acid sequence of the catalytic unit of DAP BII exhibits significant similarity to those classified in the clan PA endopeptidases. In some cases, the unmodified dipeptide cleavase exhibits at least 30%, 40%, 50%, 60%, 70%, 80%, 85%, 86%, 87%, 88%, 89%, 90%, 91%, 92%, 93%, 94%, 95%, 96%, 97%, 98%, 99% or more sequence identity to UniProt Accession No. V5YM14 as set forth in SEQ ID NO: 13. In some embodiments, the modified dipeptide cleavase contains one or more amino acids modifications in the catalytic domain of an unmodified DAP BII (*e.g.,* residues 1-252 and residues 550 to 698 of SEQ ID NO: 13). In some embodiments, the mutations, *e.g.,* one or more amino acid modifications (*e.g.,* substitutions, deletions, additions) in the modified dipeptide cleavase is in reference to the amino acid sequence set forth in reference to positions of SEQ ID NO: 13. It has been shown that the unmodified or wild-type DAP BII hydrolyses peptides from the N-terminus of oligopeptides and small proteins, cleaving dipeptide units (NH2-P2-P1-) when the second (P1) residue is Ala, Leu, Ile, Phe, Tyr, Arg, or His (but not Pro) (*See e.g.,* Sakamoto et al., Scientific Reports 2014, 4:4977).

In some embodiments, the modified dipeptide cleavase is derived from DAP BII and removes or is configured to remove a labeled terminal dipeptide from a polypeptide. In some embodiments, the modified dipeptide cleavase has one or more amino acid modifications (*e.g*. substitutions, deletions, additions, or combinations thereof) in an unmodified DAP BII cleavase corresponding to any one or more of positions 126, 188, 189, 190, 191, 192, 196, 238, 302, 306, 307, 310, 525, 528, 546, 604, 650, 651, 665, and/or 692, with reference to positions of SEQ ID NO: 13. In some embodiments, the modified dipeptide cleavase comprises one or more amino acid modifications in an unmodified dipeptide cleavase, corresponding to positions 126, 188, 189, 190, 191, 192, 196, 238, 302, 306, 307, 310, 525, 528, 546, 604, 650, 651, 665, and/or 692, with reference to positions of SEQ ID NO: 13, and comprises an amino acid sequence that exhibits at least 30 % identity, at least 40 % identity, at least 50 % identity, at least 60 % identity, at least 70 % identity, at least 80 % identity, or at least 90 % or more identity to any of SEQ ID NOs: 17-19 or 23-28. In some embodiments, the modified dipeptide cleavase contains a functional fragment of any of the provided sequences (*e.g*., a functional fragment of any of SEQ ID NOs: 17-19 or 23-28).

In some embodiments, the modified dipeptide cleavase has one or more amino acid modifications (*e.g*. substitutions, deletions, additions, or combinations thereof) in an unmodified DAP BII cleavase or fragment thereof corresponding to any one or more of positions 183, 184, 185, 186, 187, 188, 189, 190, 191, 192, 193, 194, 195, 196, 197, 198, 199, 200, 201, and/or 202, with reference to positions of SEQ ID NO: 13. In some embodiments, the modified dipeptide cleavase has one or more amino acid modifications (*e.g*. substitutions, deletions, additions, or combinations thereof) in an unmodified DAP BII cleavase or fragment thereof corresponding to any one or more of positions 188, 189, 190, 191, 192, 302, and/or 310, with reference to positions of SEQ ID NO: 13. In some embodiments, the modified dipeptide cleavase has one or more amino acid modifications (*e.g*. substitutions, deletions, additions, or combinations thereof) in an unmodified DAP BII cleavase or fragment thereof corresponding to any one or more of positions 191, 192, 196, 306, and/or 650, with reference to positions of SEQ ID NO: 13. In some embodiments, the modified dipeptide cleavase has one or more amino acid modifications (*e.g*. substitutions, deletions, additions, or combinations thereof) in an unmodified DAP BII cleavase or fragment thereof corresponding to any one or more of positions 323-544 with reference to positions of SEQ ID NO: 13. In some embodiments, the modified dipeptide cleavase has one or more amino acid modifications (*e.g*. substitutions, deletions, additions, or combinations thereof) in an unmodified DAP BII cleavase or fragment thereof corresponding to any one or more of positions 310, 651, 655, and/or 656 with reference to positions of SEQ ID NO: 13. In some embodiments, the modified dipeptide cleavase has one or more amino acid modifications (*e.g*. substitutions, deletions, additions, or combinations thereof) in an unmodified DAP BII cleavase or fragment thereof corresponding to any one or more of positions 627, 628, 630, 648, 651, 655, and/or 669, with reference to positions of SEQ ID NO: 13.

In some embodiments, the modified dipeptide cleavase is derived from DAP BII and removes or is configured to remove a labeled terminal dipeptide from a polypeptide. In some embodiments, the modified dipeptide cleavase has one or more amino acid modifications (*e.g*. substitutions, deletions, additions, or combinations thereof) in an unmodified DAP BII cleavase corresponding to any one or more of positions 126, 183, 184, 185, 186, 187, 188, 189, 190, 191, 192, 193, 194, 195, 196, 197, 198, 199, 200, 201, 202, 238, 302, 306, 307, 310, 525, 528, 546, 604, 627, 628, 630, 648, 650, 651, 655, 656, 665, 669, and/or 692, with reference to positions of SEQ ID NO: 13.

In some embodiments, the modified dipeptide cleavase has one or more amino acid substitutions selected from the group consisting of A126T, D188V, I189A, D190S, N191L, N191M, W192G, R196S, R196T, R196V, G238V, A302W, N306R, T307K, N310K, N525K, A528V, F546L, A604V, D650A, G651V, K6651, and/or K692N, with reference to positions of SEQ ID NO: 13, or a conservative amino acid substitution thereof. In some embodiments, the one or more amino acid modification is N191M/W192G/R196T/N306R/D650A, N191M/W192G/R196V/N306R/D650A, D188V/I189A/D190S/N191L/W192G/R196S/A302W/N310K/D650A, N191M/W192G/R196T/N306R/T307K/D650A, N191M/W192G/R196T/N306R/N525K/A528V/A604V/D650A/K692N, A126T/N191M/W192G/R196T/G238V/N306R/D650A, N191M/W192G/R196T/N306R/F546L/D650A, N191M/W192G/R196T/N306R/D650A/G651V/K665I, or N191M/W192G/R196T/N306R/D650A/G651V.

In some embodiments, the modified dipeptide cleavase has an amino acid sequence that has at least at least 30 % identity, at least 40 % identity, at least 50 % identity, at least 60 % identity, at least 70 % identity, at least 80 % identity, or at least 90 % or more identity to any of SEQ ID NOs: 17-19, 23-28, or a specific binding fragment thereof. In some embodiments, the specific binding fragment has a length ranging from about 10 amino acids to about 400 amino acids, from about 10 amino acids to about 300 amino acids, from about 10 amino acids to about 200 amino acids, from about 10 amino acids to about 100 amino acids, or from about 10 amino acids to about 50 amino acids. In some specific examples, the modified dipeptide cleavase comprises the sequence of amino acids set forth in any of SEQ ID NOs: 17-19, 23-28, or a sequence of amino acids that exhibits at least 95% sequence identity to any of SEQ ID NOs: 17-19, 23-28, or a specific binding fragment thereof. In some examples, the modified dipeptide cleavase contains one or more of the amino acid substitutions provided in SEQ ID NOs: 17-19 or 23-28. In some aspects, the modified dipeptide cleavase comprises one or more amino acid modifications in an unmodified dipeptide cleavase, corresponding to positions 188, 189, 190, 191, 192, 196, 302, 306, 310, and/or 650, with reference to positions of SEQ ID NO: 13, and has an amino acid sequence that has at least 30 % identity, at least 40 % identity, at least 50 % identity, at least 60 % identity, at least 70 % identity, at least 80 % identity, or at least 90 % or more identity to any of SEQ ID NOs: 17-19 or 23-28. In some aspects, the modified dipeptide cleavase comprises one or more amino acid modifications in an unmodified dipeptide cleavase, corresponding to positions 191, 192, 196, 306, and/or 650, with reference to positions of SEQ ID NO: 13, and has an amino acid sequence that has at least 30 % identity, at least 40 % identity, at least 50 % identity, at least 60 % identity, at least 70 % identity, at least 80 % identity, or at least 90 % or more identity to any of SEQ ID NOs: 17-19 or 23-28. In some embodiments, the modified dipeptide cleavase exhibits the substrate specificity of any one of the sequences in SEQ ID NOs: 17-19 or 23-28. In some embodiments, the modified dipeptide cleavase has the cleaving activity of any one of the sequences in SEQ ID NOs: 17-19 or 23-28.

In some embodiments, the modified dipeptide cleavase has an amino acid sequence that comprises a catalytic domain with at least at least 30 % identity, at least 40 % identity, at least 50 % identity, at least 60 % identity, at least 70 % identity, at least 80 % identity, or at least 90 % or more identity with the catalytic domain of any of SEQ ID NOs: 17-19 or 23-28. In some embodiments, the modified dipeptide cleavase has an amino acid sequence that comprises an amine binding site with at least at least 30 % identity, at least 40 % identity, at least 50 % identity, at least 60 % identity, at least 70 % identity, at least 80 % identity, or at least 90 % or more identity with the amine binding site of any of SEQ ID NOs: 17-19 or 23-28. In some embodiments, the modified dipeptide cleavase has an amino acid sequence that comprises a loop domain with at least 30 % identity, at least 40 % identity, at least 50 % identity, at least 60 % identity, at least 70 % identity, at least 80 % identity, or at least 90 % or more identity with the loop domain of any of SEQ ID NOs: 17-19 or 23-28.

In some specific examples, a desired modified dipeptide cleavase may exhibit reduced bias towards specific amino acids in the P1 or P2 position of the polypeptide. In some embodiments, such modified dipeptide cleavases may be obtained by targeting the P1 or P1 pocket of the wildtype or unmodified enzyme in genetic selection. In some cases, the residues N310, G651, S655, and/or V656 with reference to positions of SEQ ID NO: 13 may be targeted to reduce bias. In some cases, the residues N215, W216, R220, N330, and/or D674 with reference to positions of SEQ ID NO: 20 may be targeted to reduce bias.

**Table 6** also provides exemplary amino acid substitutions in sequences of exemplary modified cleavases by reference to positions of the indicated SEQ ID NOs. In some examples, the modified cleavase contains one or more of the amino acid substitutions provided in **Table 6.**

**Table 6. Exemplary Modified Cleavases**

| Mutation(s) | SEQ ID NO |
|---|---|
| D188V/I189A/D1905/N191L/W192G/R196S/A302W/N310K/D650A | 17 |
| N191M/W192G/R196T/N306R/D650A | 18 |
| N191M/W192G/R196V/N306R/D650A | 19 |
| N191M1W192G/R196T/N306R/T307K/D650A | 23 |
| N191M1W192G/R196T/N306R/N525K/A528V/A604V/D650A/K692N | 24 |
| A126T/N191M/W192G/R196T/G238V/N306R/D650A | 25 |
| N191M1W192G/R196T/N306R/F546L/D650A | 26 |
| N191M1W192G/R196T/N306R/D650A/G651V/K665I | 27 |
| N191M1W192G/R196T/N306R/D650A/G651V | 28 |

In some embodiments, the removed dipeptide comprises an amino acid that is labeled or modified by a chemical

reagent or enzymatic reagent. In some embodiments, selection of the appropriate label with an appropriately engineered or modified dipeptide cleavase enables cleavage of a labeled terminal dipeptide. In some embodiments, the active site and/or amino acid binding site(s) of the unmodified dipeptide cleavase is modified. In some embodiments, the modified dipeptide cleavase comprises a mutation or modification within its substrate binding site, at the boundary of the substrate binding site, or a combination thereof. In some embodiments, the modified dipeptide cleavase is derived from a dipeptide cleavase (*e.g.,* a dipeptidyl peptidase, a dipeptidyl aminopeptidase, a peptidyl-dipeptidase, or a dipeptidyl carboxypeptidase) modified to fit and recognize a label (*e.g.* a chemical label or a chemical modification).

In some embodiments, the modified dipeptide cleavase comprises an amino acid mutation (*e.g*., modifications, substitutions, deletions, additions, or combinations thereof) compared to the wild-type dipeptide cleavase in its substrate binding site, at the boundary of the substrate binding site, in the catalytic domain, in the P1 or P2 pocket, in a chymotrypsin fold, at an amine binding site, in the loop domain, or a combination thereof. In some embodiments, the modified dipeptide cleavase comprises an amino acid mutation (*e.g*., substitutions, deletions, additions, or combinations thereof) compared to the wild-type dipeptide cleavase polypeptide in the hinge region of the cleavase. In some embodiments, the modified dipeptide cleavase comprises an amino acid mutation compared to the wild-type dipeptide cleavase polypeptide in the binding cleft of the cleavase. In some cases, the modified dipeptide cleavase comprises an amino acid mutation compared to the wild-type dipeptide cleavase polypeptide in the inter-lobe cleft of the cleavase. In some embodiments, the modified dipeptide cleavase comprises an amino acid mutation compared to the wild-type dipeptide cleavase polypeptide in the alpha amine binding region of the cleavase. For example, the modified dipeptide cleavase exhibits reduced alpha amine binding compared to the wild-type cleavase polypeptide. *See e.g.,* Kumar et al., Sci Rep. (2016) 6:23787.

In some embodiments, the modified dipeptide cleavase comprises an amino acid mutation (*e.g*., modifications, substitutions, deletions, additions, or combinations thereof) compared to the wild-type dipeptide cleavase polypeptide in the chymotrypsin fold of the cleavase. In some embodiments, the modified dipeptide cleavase comprises an amino acid mutation (*e.g*., modifications, substitutions, deletions, additions, or combinations thereof) compared to the wild-type dipeptide cleavase polypeptide in at an amine binding site. In some embodiments, the modified dipeptide cleavase comprises an amino acid mutation (*e.g*., modifications, substitutions, deletions, additions, or combinations thereof) compared to the wild-type dipeptide cleavase polypeptide in the loop domain. In some aspects, the modified dipeptide cleavase comprises an amino acid mutation (*e.g*., modifications, substitutions, deletions, additions, or combinations thereof) compared to the wild-type dipeptide cleavase polypeptide for improving accessibility to the active site of the modified dipeptide cleavase. In some cases, the modified dipeptide cleavase exhibits greater accessibility of the substrate (*e.g*., polypeptide) to the active site compared to the unmodified dipeptide cleavase. For example, the modified dipeptide cleavase may allow larger substrates to access the active site.

In some embodiments, the modified dipeptide cleavase exhibits altered activity, substrate binding capability, or cleavage characteristics compared to the unmodified dipeptide cleavase. In some embodiments, the modified dipeptide cleavase is modified in the catalytic motif or catalytic domain of the unmodified dipeptide cleavase (*e.g*., the HEXXGH catalytic motif as set forth in SEQ ID NO: 1). In some embodiments, the mutations, *e.g*., one or more amino acid modifications (*e.g*., substitutions, deletions, additions) corresponds to positions 316, 391, and/or 394 with reference to positions of SEQ ID NO: 5. In some embodiments, the mutations, *e.g*., one or more amino acid modifications (e.g., substitutions, deletions, additions) corresponds to amino acid residue positions 419, 420, 421, 422, 423, 424, 425, 426, or a combination thereof, with reference to positions of SEQ ID NO: 5.

In some embodiments, the unmodified dipeptide cleavase is a metallopeptidase. In some embodiments, the modified dipeptide cleavase is a metallopeptidase. In some embodiments, the modified dipeptide cleavase is a zinc-dependent metallopeptidase or a zinc-dependent hydrolase or derived from such. Some known metallopeptidase are characterized by the presence of a conventional catalytic signature motif HEXXH. In some aspects, the two His residues of the HEXXH motif contribute to coordinate the divalent metal ion (*e.g.,* $Zn^{2+}$, $Mn^{2+}$, $Co^{2+}$, $Ni^{2+}$, $Cu^{2+}$). For example, the modified dipeptide cleavase requires the presence of or contact with specific metal ions (*e.g.,* zinc ions, chloride ions) for activation. In some embodiments, function of the modified dipeptide cleavase can be modulated or controlled by the presence or absence of metal ions, or by contacting with metal chelating agents.

In some embodiments, the modified dipeptide cleavase exhibits altered binding affinity and/or specificity to specific substrates compared to the unmodified dipeptide cleavase. For example, the modified dipeptide cleavase exhibits increased binding affinity and/or specificity for labeled terminal amino acids compared to the unmodified dipeptide cleavase. For example, the modified dipeptide cleavase may not remove an unlabeled terminal dipeptide from the polypeptide. In some cases, the modified dipeptide cleavase does not remove a terminal dipeptide that does not contain a labeled amino acid. In comparison, a wild-type or unmodified dipeptide cleavase does not remove dipeptides comprising a labeled amino acid. In some embodiments, the modified dipeptide cleavase exhibits decreased binding affinity and/or specificity for a substrate compared to the unmodified dipeptide cleavase. In some embodiments, the modified dipeptide cleavase exhibits one or more desired characteristics, such as binding rate, rate of hydrolysis, rate of release. In some embodiments, the modified dipeptide cleavase can be removed or released from the polypeptide at a desired rate.

In some of any such embodiments, a reaction with a modified dipeptide cleavase can be enhanced by recruiting

the modified dipeptide cleavase to the labeled terminal amino acid. For example, one or more modified dipeptide cleavases can be recruited to the labeled terminal amino acid of the polypeptide via hybridization of complementary universal priming sequences a DNA tag or sequence associated with the modified dipeptide cleavase and a DNA tag or sequence associated with the polypeptide to be treated with the modified dipeptide cleavase(s). This hybridization step may improve the effective affinity of the modified dipeptide cleavase for the labeled terminal amino acid (*e.g.,* NTAA). In some cases, after the labeled terminal amino acid is removed as a dipeptide, it may diffuse away, and the associated modified dipeptide cleavase can be removed by stripping the hybridized DNA tag.

In some embodiments, the modified dipeptide cleavase is attached to an anchoring sequence. In some cases, the modified dipeptide cleavase is attached to the anchoring sequence directly or indirectly. In some cases, the anchoring sequence is complementary to a sequence attached to the polypeptides. In some embodiments, the anchoring sequence is a universal sequence or a universal DNA tag. In some embodiments, the polypeptide is also attached to a universal sequence. In some examples, the anchoring sequence on the modified dipeptide cleavase brings the enzyme in proximity to the polypeptide. In some embodiments, the anchoring sequence brings the enzyme in proximity or co-localizes the modified dipeptide cleavase to the polypeptide. In some embodiments, this co-localization of the modified dipeptide cleavase and the polypeptide aids in binding and/or removal of the labeled dipeptide from said polypeptide.

In any of the embodiments provided herein, recruitment of one or more modified dipeptide cleavases to the terminal amino acid of the polypeptide may be enhanced by utilizing a chimeric modified dipeptide cleavase containing a first tethering moiety and a second tethering moiety associated with the polypeptide, or is colocalized with the polypeptide, wherein the first tethering moiety is configured to form a stable complex with the second tethering moiety upon contact (moieties are capable of a binding reaction with each other). For example, the polypeptide may be immobilized on a solid support, and the second tethering moiety is attached to the solid support in proximity to the polypeptide, or attached directly to the polypeptide. Examples of first and second tethering moieties include biotin-streptavidin pair, two complementary polynucleotide molecules that form a stable double strand complex upon contact, or other known in the art molecules that can strongly interact upon contact under physiological conditions (or under conditions used in a cleavase assay). In one example, a modified dipeptide cleavase is a low affinity enzyme (> μM Kd) and it is recruited to the polypeptide associated with a biotin using a streptavidin-chimeric modified dipeptide cleavase. In some cases, the efficiency of modified dipeptide cleavase to remove labeled terminal amino acid can be improved due to the increase in effective local concentration as a result of the first tethering moiety-second tethering moiety interaction. In some cases, this approach effectively increases the affinity KD of the modified dipeptide cleavase from μM to subpicomolar. A number of different bioconjugation recruitment strategies can also be employed. An azide modified PITC is commercially available (4-Azidophenyl isothiocyanate, Sigma), allowing a number of simple transformations of azide-PITC into other bioconjugates of PITC, such as biotin-PITC via a click chemistry reaction with alkyne-biotin. In some aspects, after the labeled terminal amino acid is removed, it may diffuse away with the associated modified dipeptide cleavase from the polypeptide.

In some embodiments, the modified dipeptide cleavase can be a single polypeptide chain or a multimer (dimers or higher order multimers) of at least two polypeptide chains. Thus, monomeric, dimeric, and higher order multimeric modified dipeptide cleavase polypeptides are within the scope of the defined term. Multimeric polypeptides can be homomultimeric (of identical polypeptide chains) or heteromultimeric (of non-identical polypeptide chains). In some embodiments, the modified dipeptide cleavase is a monomeric enzyme. In some embodiments, the modified dipeptide cleavase is a fusion molecule or a chimeric molecule. For example, the modified dipeptide cleavase may be attached or associated, directly or indirectly via a linker, to a oligonucleotide. In some specific cases, the modified dipeptide cleavase may be joined to a moiety such as a SpyTag/SpyCatcher or SnoopTag/SnoopCatcher.

### A. Labeled Terminal Amino Acid

In some embodiments, the terminal amino acid of a peptide removed by the modified dipeptide cleavase is labeled or modified. A label can comprise any suitable material or moiety. Any suitable molecule or materials may be employed for this purpose, including proteins, amino acids, nucleic acids, carbohydrates, chemical moieties, and small molecules. In some embodiments, a suitable label is capable of fitting in the binding pocket of the modified dipeptide cleavase. In some aspects, the labeling of a terminal amino acid is performed in a manner that is nucleic acid-compatible (*e.g.*, the labeling is performed in a manner that is not damaging to nucleic acids). In some embodiments, a suitable label enables the modified dipeptide cleavase to remove a labeled dipeptide from the polypeptide. The terminal amino acid of the polypeptides may be labeled by any suitable methods. In some examples, the terminal amino acid is labeled chemically or enzymatically. In some embodiments, the terminal amino acid is labeled by a reagent that is or comprises a chemical agent, an enzyme, and/or a biological agent. In some cases, the terminal amino acid is labeled with a chemical label or moiety.

In some embodiments, a precursor polypeptide (*e.g.*, an unlabeled polypeptide) is contacted with a reagent for labeling the terminal amino acid of the precursor polypeptide to provide a polypeptide prepared for treatment with the modified dipeptide cleavase. In some cases, the contacting of the precursor polypeptide with the reagent for labeling the

terminal amino acid is performed prior to contacting the polypeptide with a modified dipeptide cleavase. In some aspects, the modified dipeptide cleavase is contacted with a polypeptide that has been labeled or modified. In some cases, the contacting of the precursor polypeptide with the reagent for labeling the terminal amino acid and contacting the polypeptide with a modified dipeptide cleavase are performed simultaneously or substantially simultaneously.

In some embodiments, the dipeptide for removal or removed by the modified dipeptide cleavase comprises an amino acid that is labeled with a chemical label. In some examples, the amino acid in the dipeptide for removal by the modified dipeptide cleavase is labeled with a chemical reagent. In some aspects, the labeling of a terminal amino acid by treating with a chemical reagent is performed in a manner that is nucleic acid-compatible (*e.g.*, the labeling is performed under conditions that is not damaging to nucleic acids).

In some embodiments, the modified dipeptide cleavase removes dipeptides comprising amino acid(s) that are labeled, such as a chemically-modified or labeled (*e.g.,* PTC/DNP/acetyl/Cbz-modified or labeled) amino acids on a polypeptide. In some cases, the labeled amino acid is removed as part of a terminal dipeptide. In some embodiments, the modified dipeptide cleavase removes a dipeptide comprising an N-terminal amino acid having a PTC/DNP/acetyl/Cbz group present as the label.

In some embodiments, at least one amino acid (as part of a dipeptide) for removal by the modified dipeptide cleavase, which may be the terminal amino acid of a dipeptide to be removed by the dipeptide cleavase, is labeled with a reagent selected from the group consisting of a phenyl isothiocyanate (PITC), a nitro-PITC, a sulfo-PITC, a phenyl isocyanate (PIC), a nitro-PIC, a sulfo-PIC, benzyloxycarbonyl chloride or carbobenzoxy chloride (Cbz-Cl), N-(Benzyloxycarbonyloxy)succinimide (Cbz-OSu or Cbz-O-NHS), a 1-fluoro-2,4-dinitrobenzene (Sanger's reagent, DNFB), dansyl chloride (DNS-Cl, or 1-dimethylaminonaphthalene-5-sulfonyl chloride), 4-sulfonyl-2-nitrofluorobenzene (SNFB), an anhydride, 2-Pyridinecarboxaldehyde, 2-Formylphenylboronic acid, 2-Acetylphenylboronic acid, 1-Fluoro-2,4-dinitrobenzene, 4-Chloro-7-nitrobenzofurazan, Pentafluorophenylisothiocyanate, 4-(Trifluoromethoxy)-phenylisothiocyanate, 4-(Trifluoromethyl)-phenylisothiocyanate, 3-(Carboxylic acid)-phenylisothiocyanate, 3-(Trifluoromethyl)-phenylisothiocyanate, 1-Naphthylisothiocyanate, N-nitroimidazole-1-carboximidamide, N,N'-Bis(pivaloyl)-1H-pyrazole-1-carboxamidine, N,N'-Bis(benzyloxycarbonyl)-1H-pyrazole-1-carboxamidine, an acetylating reagent, a guanidinylation reagent, a thioacylation reagent, a thioacetylation reagent, a thiobenzylation reagent, and a diheterocyclic methanimine reagent, or a derivative thereof.

In some embodiments, the terminal amino acid for removal by the modified dipeptide cleavase, which may be the terminal amine of a dipeptide to be removed by the dipeptide cleavase, is labeled with an anhydride or derivative thereof. In some embodiments, the reagent for labeling the amino acid for removal by the modified dipeptide cleavase is selected from the group consisting of: S-Acetylmercaptosuccinic anhydride, cis-Aconitic anhydride, 4-Amino-1,8-naphthalic anhydride, endo-Bicyclo[2.2.2]oct-5-ene-2,3-dicarboxylic anhydride, 5-Bromoisatoic anhydride, Bromomaleic anhydride, 4-Bromo-1,8-naphthalic anhydride, Citraconic anhydride, Crotonic anhydride, trans-1,2-Cyclohexanedicarboxylic anhydride, 1-Cyclopentene-1,2-dicarboxylic anhydride, 2,3-Dichloromaleic anhydride, 3,6-Dichlorophthalic anhydride, 3,6-Difluorophthalic anhydride, Diglycolic anhydride, 2,2-Dimethylglutaric anhydride, 3,3-Dimethylglutaric anhydride, 2,3-Dimethylmaleic anhydride, 2,2-Dimethylsuccinic anhydride, (2-Dodecen-1-yl)succinic anhydride, Dodecenylsuccinic anhydride, Glutaric anhydride, Hexafluoroglutaric anhydride, Hexahydro-4-methylphthalic anhydride, Homophthalic anhydride, 3-Hydroxyphthalic anhydride, Itaconic anhydride, Maleic anhydride, 3-Methylglutaric anhydride, N-Methylisatoic anhydride, Methylsuccinic anhydride, 1,8-Naphthalic anhydride, 3-Nitro-1,8-naphthalic anhydride, 4-Nitro-1,8-naphthalic anhydride, 3-Nitrophthalic anhydride, 4-Nitrophthalic anhydride, 2-Octen-1-ylsuccinic anhydride, 2,5-Oxazolidinedione, 2-Phenylglutaric anhydride, Phenylmaleic anhydride, Phenylsuccinic anhydride, N-Phthaloyl-DL-glutamic anhydride, 2,3-Pyrazinedicarboxylic anhydride, 3,4-Pyridinedicarboxylic anhydride, Succinic anhydride, 4-Sulfo-1,8-naphthalic anhydride, Tetrabromophthalic anhydride, Tetrachlorophthalic anhydride, Tetrafluorophthalic anhydride, 3,4,5,6-Tetrahydrophthalic anhydride, 3,3-Tetramethyleneglutaric anhydride, Trimellitic anhydride chloride, and 2-(Triphenylphosphoranylidene)succinic anhydride. *See e.g.* Staiger et al., J. Org. Chem. 1959, 24, 9, 1214-1219; Jiang et al. J. Org. Chem. 2019, 84, 4, 2022-2031; U.S. Patent No. 9,867,883.

In some examples, in preparation for treatment with a modified dipeptide cleavase of the invention, a polypeptide is treated with a chemical reagent that comprises an isatoic anhydride, an isonicotinic anhydride, an azaisatoic anhydride, a succinic anhydride, or a derivative of one of these, and the terminal amino acid of the polypeptide is modified, or labeled, by the chemical reagent. Specific examples of labeling of a terminal amino acid of a polypeptide to be treated with a modified dipeptide cleavase of the invention include:

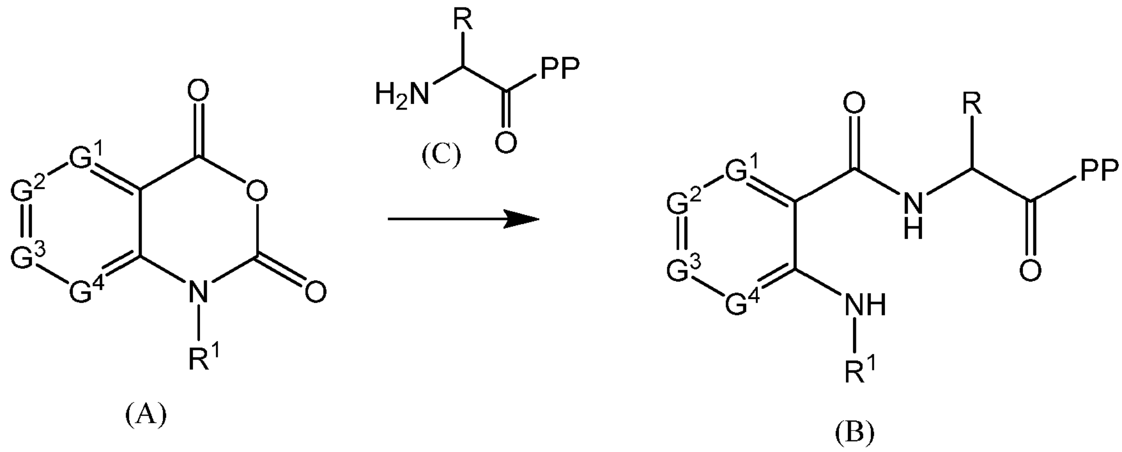

wherein:

$G^1$-$G^4$ are each independently selected from CH, CX, and N;
X at each occurrence is independently selected from $C_1$-$C_2$ alkyl,, $NO_2$, $C_1$-$C_2$ haloalkyl, $C_1$-$C_2$ haloalkoxy, halo, -$OR^2$, -$N(R^2)_2$, -$SR^2$, $SO_2R^3$, $SO_3R^2$, -$B(OR^2)_2$, $C(=O)R^2$, CN, $CON(R^2)_2$, -$COOR^2$, -C(-O)Ar, and tetrazole;
R represents the side chain of an amino acid, e.g. one of the side chains of the 20 common amino acids;
$R^1$ is selected from H, $R^3$, $C(-O)R^2$, -$C(=O)N(R^2)_2$, -C(=O)Ar, and -$SO_2N(R^2)_2$;
$R^2$ is independently at each occurrence selected from H and $C_1$-$C_2$ alkyl;
$R^3$ is independently at each occurrence selected from $C_1$-$C_2$ alkyl;
Ar is independently selected at each occurrence from phenyl, pyridinyl, pyrimidinyl, pyridazinyl, and pyrazinyl, each of which is optionally substituted by one or two groups selected from halo, CN, $NO_2$, $C_1$-$C_2$ alkyl, $C_1$-$C_2$ haloalkyl, $C_1$-$C_2$ haloalkoxy, and -$OR^2$; and
PP represents a portion of a polypeptide, particularly the portion of a polypeptide being prepared for treatment with a modified dipeptide cleavase of the invention excluding the N-terminal amino acid. Thus the compound of Formula (C) is typically a polypeptide for use in the methods of the invention, and R represents the side chain of the terminal amino acid of the polypeptide.

In preferred embodiments, the terminal amino acid shown in Formula (C) is in the L-configuration when R is not H.

The compounds of Formula (B) are polypeptides, sometimes referred to as labeled polypeptides, that have been prepared for use in the modified dipeptide cleavase reactions described herein.

In some aspects, the amino acid for removal by the modified dipeptide cleavase is labeled with an exemplary reagent derived from an isatoic anhydride, an isonicotinic anhydride or an azaisatoic anhydride, especially compounds of Formula (A) as described herein. In some embodiments, the amino acid for removal by the modified dipeptide cleavase is labeled with an exemplary reagent selected from the list consisting of N-Methyl-isatoic anhydride, N-acetyl-isatoic anhydride, 4-carboxylic acid isatoic anhydride, 5-methoxy-isatoic anhydride, 5-nitro-isatoic anhydride, 4-chloro-isatoic anhydride, 4-fluoro-isatoic anhydride, 6-fluoro-isatoic anhydride, N-benzyl-isatoic anhydride, 4-trifluoromethyl-isatoic anhydride, 5-trifluoromethyl-isatoic anhydride, 4-nitro-isatoic anhydride, 4-methoxy-isatoic anhydride, and 5-Amino-2-fluoro-isonicotinic anhydride (6-fluoro-1H-pyrido[3,4-d][1,3]oxazine-2,4-dione), or a derivative thereof. In some examples, the labeled amino acid or dipeptide removed by the action of a modified dipeptide cleavase of the invention comprises an optionally substituted benzamide, typically one derived from any of the optionally substituted isatoic anhydrides disclosed herein, including a compound of Formula (B) as described herein.

In other favored embodiments of the invention, in preparation for treatment with a modified dipeptide cleavase of the invention, the polypeptide is treated with a chemical reagent that comprises a succinic anhydride, a phthalic anhydride, a pyrazinedicarboxylic anhydride, or a derivative of one of these, and the terminal amino acid is modified, or labeled, by the chemical reagent.

Additional specific examples of reactions for labeling of a terminal amino acid of a polypeptide to be treated with a modified dipeptide cleavase of the invention include:

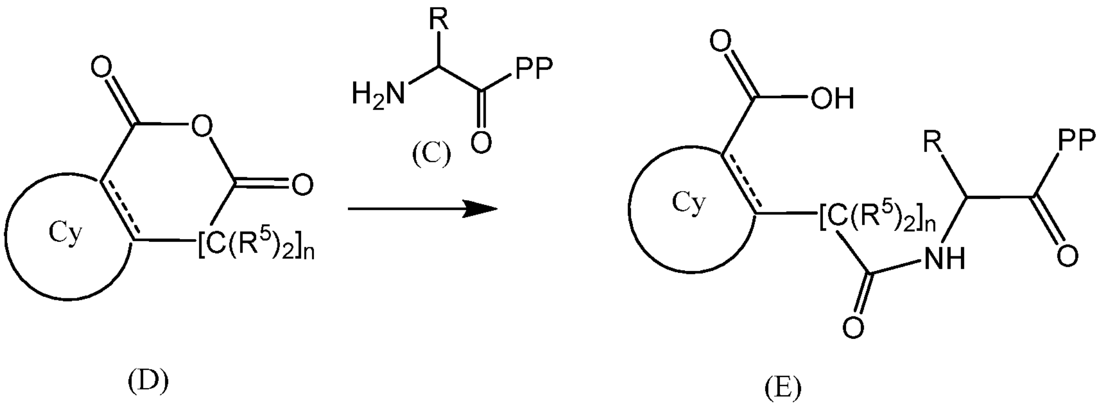

wherein:
n is 0 or 1;

Ring Cy represents a 5- or 6-membered ring or an 8-10 membered bicyclic ring that may be absent or present; when present, ring Cy may be saturated, unsaturated, or aromatic, and the dashed bond may be a single bond, double bond, or aromatic bond;

when Cy is present, it may be a carbocyclic ring, or it may contain one or two heteroatoms selected from N, O and S as ring members;
when Ring Cy is present, it is optionally substituted with one to six groups (or with one to four groups when Cy is aromatic) selected from halo, CN, $NO_2$, $C_1$-$C_2$ alkyl, $C_1$-$C_2$ haloalkyl, $C_1$-$C_2$ haloalkoxy, and -$OR^4$;

when ring Cy is absent, the dashed bond may be a single bond or a double bond, and the dashed bond is optionally substituted by one or two groups selected from halo, CN, $C_1$-$C_2$ alkyl, $C_1$-$C_2$ haloalkyl, $C_1$-$C_2$ haloalkoxy, $CO_2R^4$, and -$OR^4$;

R represents the side chain of an amino acid, e.g. one of the side chains of the 20 common amino acids;
$R^4$ is independently selected at each occurrence from H, $C_1$-$C_2$ alkyl, and $C_1$-$C_2$ haloalkyl;
$R^5$ is independently selected at each occurrence from H, halo, $C_1$-$C_2$ alkyl, $C_1$-$C_2$ haloalkyl, $C_1$-$C_2$ alkoxy, and $C_1$-$C_2$ haloalkoxy;
PP represents a portion of a polypeptide, particularly the portion of a polypeptide being prepared for treatment with a modified dipeptide cleavase of the invention excluding the N-terminal amino acid. Thus the compound of Formula (C) is typically a polypeptide for use in the methods of the invention, and R represents the side chain of the terminal amino acid of the polypeptide.

In preferred embodiments of these reagents of Formula (D), ring Cy is absent when n is 1. In additional preferred embodiments of the chemical reagents of Formula (D), ring Cy is present and is a phenyl ring or a 2,3-pyrazine ring, each of which is optionally substituted as described above, and n is 0.

In preferred embodiments, the terminal amino acid shown in Formula (C) is in the L-configuration when R is not H.

The compounds of Formula (E) are polypeptides, sometimes referred to as labeled polypeptides, that have been prepared for use in the modified dipeptide cleavase reactions described herein.

In some embodiments, the amino acid for removal by the modified dipeptide cleavase is labeled with an exemplary reagent derived from succinic anhydride, or a compound of Formula (D) wherein ring Cy is absent and the dashed bond represents a single bond. In some embodiments, the reagent is 3,6, difluorophthalic anhydride, 2,3 pyrazinedicarboxylic anhydride, or succinic anhydride. In some examples, the removed labeled amino acid or dipeptide comprises 4-carboxybutylamide.

In some embodiments, in preparation for treatment with a modified dipeptide cleavase of the invention, a polypeptide is treated with any suitable chemical reagent that is capable of forming an amide bond with the α-amine of the polypeptide N-terminus. A number of chemical reagents react with terminal amines of the polypeptide to form a modified polypeptide with an amide bond linking the polypeptide to the modification; this N-terminal modified polypeptide can be a substrate for a modified dipeptide cleavase. Chemical reagents that react with amines to form an amide bond are known from the field of peptide coupling, including but not limited to: acyl halides (chlorides, fluorides, bromides), acyl imidazoles, O-acyl isoureas, activated esters [N-hydroxysuccinimide (NHS or HOSu),N-hydroxysulfosuccinimide (sulfo-NHS) p-nitrophenyl (PNP), Pentafluorophenyl (Pfp), 4-sulfo-2,3,5,6,-tetrafluorophenyl, 2,4,5-trichlorophenol, N-hydroxy-5-norbornene-2,3-dicarboximide (HONB), 3-hydroxy-4-oxo-3,4-dihydro-1,2,3-benzotriazine (HODhbt), hydroxybenzotriazole (HOBt), 1-hydroxy-7-azabenzotriazole (HOAt), 1-Hydroxy-1H-1,2,3-triazole-4-carboxylate (HOCt)], Ethyl (2Z)-2-cyano-2-hydroxyiminoacetate (Oxyma)], alkyl esters, carbodiimides, etc. (Hermanson (2013) Bioconjugation Techniques, Academic Press; Montalbetti et al., (2005) Tetrahedron 61: 10827-10852; Montalbetti et al., Wiley Encyclopedia of Chemical Biology: 1-17. de Figueiredo et al., (2016) Chem Rev 116(19): 12029-12122). N-terminal modifications can be installed with an amide bond linking to the polypeptide via enzymatic methods as well (Philpott et al., (2018) Green Chemistry 20(15): 3426-3431). An example of labeling a polypeptide with a PNP ester is provided with 4-Nitrophenyl Anthranilate which can be used to label a polypeptide under the following conditions: 4-Nitrophenol anthranilate (PNPA) is dissolved in DMSO at 100 mM; and PNPA used at 10 mM with 1 mM peptide in 1XPBS (pH 8.5) or 100 mM NaHCO3 carbonate buffer (pH 8.5) in 10% DMSO for 37 °C for 1 hr. The resulting peptide product generated is equivalent to labeling a peptide with isatoic anhydride, and generates a 2-aminobenzamide-modified peptide suitable as a substrate for a modified dipeptide cleavase (e.g., derived from DAP BII) as illustrated in Table 8.

In some examples, in preparation for treatment with a modified dipeptide cleavase of the invention, a polypeptide is treated with a chemical reagent that comprises an amine-protected activated ester to form an amide bond and the terminal amino acid of the polypeptide is modified, or labeled, by the chemical reagent. This modified polypeptide can then be further appropriately treated to remove the designated protecting group, yielding a modified polypeptide for treatment with a modified dipeptide cleavase.

Specific examples of labeling of a terminal amino acid of a polypeptide to be treated with a modified dipeptide cleavase of the invention include:

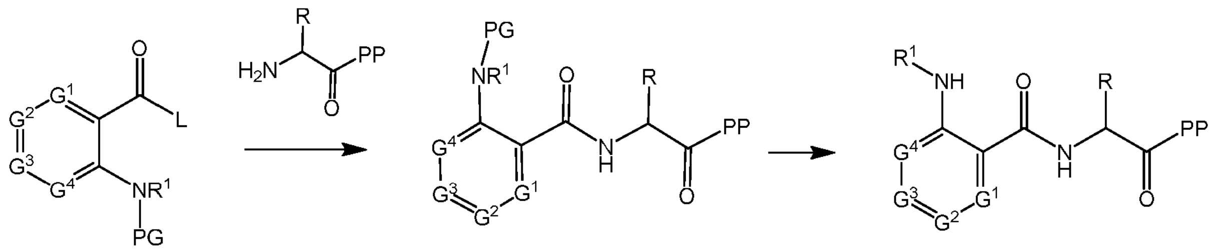

wherein:

$G^1$-$G^4$ are each independently selected from CH, CX, and N;

X at each occurrence is independently selected from H, $C_1$-$C_2$ alkyl, $NO_2$, $C_1$-$C_2$ haloalkyl, $C_1$-$C_2$ haloalkoxy, halo, $-OR^2$, $-N(R^2)_2$, $-SR^2$, $SO_2R^3$, $SO_3R^2$, $-B(OR^2)_2$, $C(=O)R^2$, CN, $CON(R^2)_2$, $-COOR^2$, -C(-O)Ar, and tetrazole;

R represents the side chain of an amino acid, e.g. one of the side chains of the 20 common amino acids;

$R^1$ is selected from H, $R^3$, $C(-O)R^2$, $-C(=O)N(R^2)_2$, -C(=O)Ar, and $-SO_2N(R^3)_2$;

$R^2$ is independently at each occurrence selected from H and $C_1$-$C_2$ alkyl;

$R^3$ is independently at each occurrence selected from $C_1$-$C_2$ alkyl;

Ar is independently selected at each occurrence from phenyl, pyridinyl, pyrimidinyl, pyridazinyl, and pyrazinyl, each of which is optionally substituted by one or two groups selected from halo, CN, $NO_2$, $C_1$-$C_2$ alkyl, $C_1$-$C_2$ haloalkyl, $C_1$-$C_2$ haloalkoxy, and $-OR^2$;

L is a leaving group selected from halo, N-hydroxysuccinimide (NHS), N-hydroxybenzotriazole, sulfo N-hydroxysuccinimide (sulfoNHS), 2,3,4,5,6-pentafluorophenol (pFP), 4-sulfo-2,3,5,6-tetrafluoro phenol, chloro, 4-nitrophenol, and -O(C=)-O-(C1-6 alkyl);

optionally, $-NR^1$-PG can be replaced by $-N_3$; and

PG is H or a nitrogen protecting groups which may be selected from tert-butyloxycarbonyl (Boc), 2,2,2-trichloroethoxycarbonyl (Troc), 2-(trimethylsilyl)ethoxycarbonyl (Teoc), carboxylbenzyl (Cbz), para-nitrocarboxylbenzyl ($p-NO_2Cbz$), allyloxycarbonyl (Alloc), 9-fluorenylmethoxycarbonyl (Fmoc), para-azidocarboxylbenzyl ($p-N_3Cbz$), 2,2,6,6-tetramethylpiperidin-1-yloxycarbonyl (Tempoc), and other N-protecting groups.

In some examples, in preparation for treatment with a modified dipeptide cleavase of the invention, a polypeptide is treated with 4-Nitrophenyl Anthranilate.

In some embodiments, the chemical reagent for modifying or labeling the amino acid for removal by the modified dipeptide cleavase is one or more of any of the compounds of Formula (A) or (D), described herein, or a salt or conjugate thereof.

In some embodiments, the chemical reagent for modifying or labeling the amino acid for removal as a dipeptide by the modified dipeptide cleavase is one or more of any of the compounds of Formula (I), (II), (III), (IV), or (AB), described herein, or a salt or conjugate thereof.

In some embodiments, the reagent for modifying or labeling the amino acid for removal as a dipeptide by the modified dipeptide cleavase comprises a compound selected from the group consisting of a compound of Formula (I):

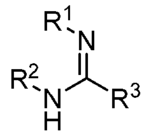

(I)

or a salt or conjugate thereof,
wherein

$R^1$ and $R^2$ are each independently H, $C_{1-6}$alkyl, cycloalkyl, -C(O)$R^a$, -C(O)O$R^b$, or -$S(O)_2R^c$;
$R^a$, $R^b$, and $R^c$ are each independently H, $C_{1-6}$alkyl, $C_{1-6}$haloalkyl, arylalkyl, aryl, or heteroaryl, wherein the $C_{1-6}$alkyl, $C_{1-6}$haloalkyl, arylalkyl, aryl, and heteroaryl are each unsubstituted or substituted;
$R^3$ is heteroaryl, -N$R^d$C(O)O$R^e$, or -S$R^f$, wherein the heteroaryl is unsubstituted or substituted;
$R^d$, $R^e$, and $R^f$ are each independently H or $C_{1-6}$alkyl.

In some embodiments, when $R^3$ is

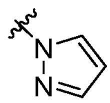

,

$R^1$ and $R^2$ are not both H. In some embodiments of Formula (I), both $R^1$ and $R^2$ are H. In some embodiments, neither $R^1$ nor $R^2$ are H. In some embodiments, one of $R^1$ and $R^2$ is $C_{1-6}$alkyl. In some embodiments, one of $R^1$ and $R^2$ is H, and the other is $C_{1-6}$alkyl, cycloalkyl, -C(O)$R^a$, -C(O)O$R^b$, or -$S(O)_2R^c$. In some embodiments, one or both of $R^1$ and $R^2$ is $C_{1-6}$alkyl. In some embodiments, one or both of $R^1$ and $R^2$ is cycloalkyl. In some embodiments, one or both of $R^1$ and $R^2$ is -C(O)$R^a$. In some embodiments, one or both of $R^1$ and $R^2$ is -C(O)O$R^b$. In some embodiments, one or both of $R^1$ and $R^2$ is -$S(O)_2R^c$. In some embodiments, one or both of $R^1$ and $R^2$ is -$S(O)_2R^c$, wherein $R^c$ is $C_{1-6}$alkyl, $C_{1-6}$haloalkyl, arylalkyl, aryl, or heteroaryl. In some embodiments, $R^1$ is

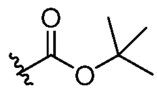

.

In some embodiments, $R^2$ is

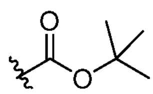

.

In some embodiments, both $R^1$ and $R^2$ are

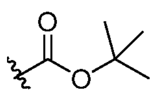

.

In some embodiments, $R^1$ or $R^2$ is

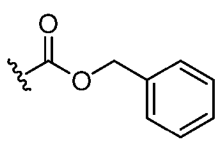

.

In some embodiments of the compound of Formula (I), $R^3$ is a monocyclic heteroaryl group. In some embodiments of Formula (I), $R^3$ is a 5- or 6-membered monocyclic heteroaryl group. In some embodiments of Formula (I), $R^3$ is a 5- or 6-membered monocyclic heteroaryl group containing one or more N. Preferably, $R^3$ is selected from pyrazole, imidazole, triazole and tetrazole, and is linked to the amidine of Formula (I) via a nitrogen atom of the pyrazole, imidazole, triazole or tetrazole ring, and $R^3$ is optionally substituted by a group selected from halo, $C_{1-3}$ alkyl, $C_{1-3}$ haloalkyl, and nitro. In some embodiments, $R^3$ is

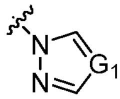
,

wherein $G_1$ is N, CH, or CX where X is halo, $C_{1-3}$ alkyl, $C_{1-3}$ haloalkyl, or nitro. In some embodiments, $R^3$ is

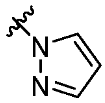

or , where X is Me, F, Cl, $CF_3$, or $NO_2$. In some embodiments, $R^3$ is

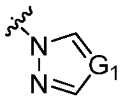
,

wherein $G_1$ is N or CH. In some embodiments, $R^3$ is

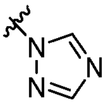
.

In some embodiments, $R^3$ is a bicyclic heteroaryl group. In some embodiments, $R^3$ is a 9- or 10-membered bicyclic heteroaryl group. In some embodiments, $R^3$ is

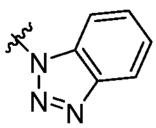
or
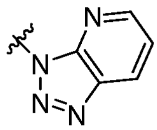
.

In some embodiments, the compound of Formula (I) is

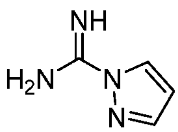
.

In some embodiments, the compound of Formula (I) is not

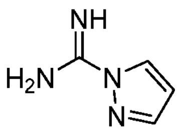
.

In some embodiments, the compound of Formula (I) is selected from the group consisting of

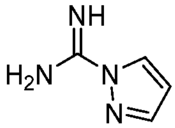, 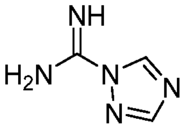, 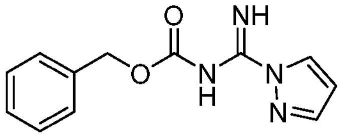, 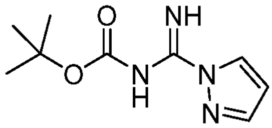,

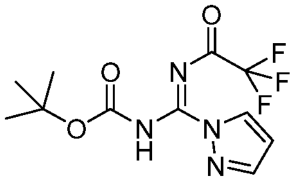, 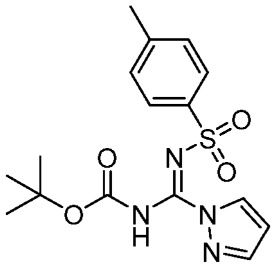, 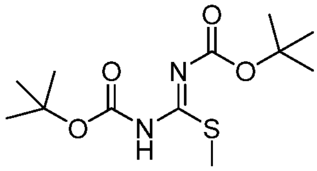,

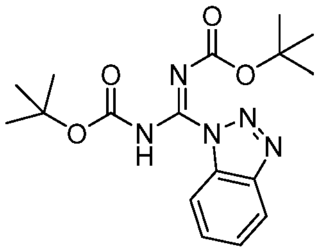, and 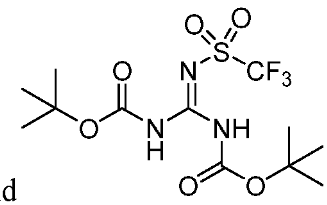,

and optionally also including

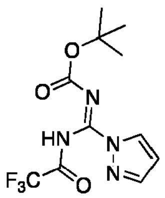 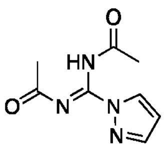 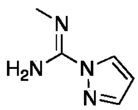 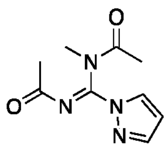 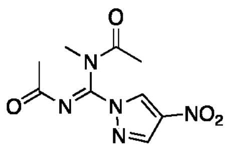 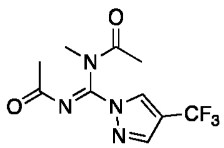

(N-Boc,N'-trifluoroacetyl-pyrazolecarboxamidine, N,N'-bisacetyl-pyrazolecarboxamidine, N-methyl-pyrazolecarboxamidine, N,N'-bisacetyl-N-methyl-pyrazolecarboxamidine, N,N'-bisacetyl-N-methyl-4-nitro-pyrazolecarboxamidine, and N,N'-bisacetyl-N-methyl-4-trifluoromethyl-pyrazolecarboxamidine), or a salt or conjugate of any of these.

In some embodiments, the chemical reagent additionally comprises Mukaiyama's reagent (2-chloro-1-methylpyridinium iodide). In some embodiments, the reagent comprises at least one compound of Formula (I) and Mukaiyama's reagent.

In some embodiments, the chemical reagent comprising a cyanamide derivative is used to label one or more amino acids of the polypeptide. (*See, e.g.,* Kwon et al., Org. Lett. 2014, 16, 6048-6051).

In some embodiments, the chemical reagent comprises a compound selected from the group consisting of a compound of Formula (II):

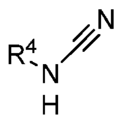 (II)

or a salt or conjugate thereof,
wherein
$R^4$ is H, $C_{1-6}$alkyl, cycloalkyl, -C(O)$R^g$, or -C(O)O$R^g$; and
$R^g$ is H, $C_{1-6}$alkyl, $C_{2-6}$alkenyl, $C_{1-6}$haloalkyl, or arylalkyl, wherein the $C_{1-6}$alkyl, $C_{2-6}$alkenyl, $C_{1-6}$haloalkyl, and arylalkyl are each unsubstituted or substituted.

In some embodiments, a reagent comprising an isothiocyanate derivative is used to label the terminal amino acid (e.g., NTAA) of a polypeptide. (*See, e.g.,* Martin et al., Organometallics. 2006, 34, 1787-1801).

In some embodiments, the chemical reagent comprises a compound selected from the group consisting of a compound of Formula (III):

$R^5$-N=C=S (III)

or a salt or conjugate thereof, wherein
$R^5$ is $C_{1-6}$alkyl, $C_{2-6}$alkenyl, cycloalkyl, heterocyclyl, aryl or heteroaryl;

wherein the $C_{1-6}$alkyl, $C_{2-6}$alkenyl, cycloalkyl, heterocyclyl, aryl or heteroaryl are each unsubstituted or substituted with one or more groups selected from the group consisting of halo, -$NR^hR^i$, -$S(O)_2R^j$, or heterocyclyl;
$R^h$, $R^i$, and $R^j$ are each independently H, $C_{1-6}$alkyl, $C_{1-6}$haloalkyl, arylalkyl, aryl, or heteroaryl, wherein the $C_{1-6}$alkyl, $C_{1-6}$haloalkyl, arylalkyl, aryl, and heteroaryl are each unsubstituted or substituted.

In some embodiments of Formula (III), $R^5$ is substituted phenyl. In some embodiments, $R^5$ is substituted phenyl substituted with one or more groups selected from halo, -$NR^hR^i$, -$S(O)_2R^j$, or heterocyclyl. In some embodiments, $R^5$ is unsubstituted $C_{1-6}$alkyl. In some embodiments, $R^5$ is substituted $C_{1-6}$alkyl. In some embodiments, $R^5$ is substituted $C_{1-6}$alkyl, substituted with one or more groups selected from halo, -$NR^hR^i$, -$S(O)_2R^j$, or heterocyclyl. In some embodiments, $R^5$ is unsubstituted $C_{2-6}$alkenyl. In some embodiments, $R^5$ is $C_{2-6}$alkenyl. In some embodiments, $R^5$ is substituted $C_{2-6}$alkenyl, substituted with one or more groups selected from halo, -$NR^hR^i$, -$S(O)_2R^j$, or heterocyclyl. In some embodiments, $R^5$ is unsubstituted aryl. In some embodiments, $R^5$ is substituted aryl. In some embodiments, $R^5$ is aryl, substituted with one or more groups selected from halo, -$NR^hR^i$, -$S(O)_2R^j$, or heterocyclyl. In some embodiments, $R^5$ is unsubstituted cycloalkyl. In some embodiments, $R^5$ is substituted cycloalkyl. In some embodiments, $R^5$ is cycloalkyl, substituted with one or more groups selected from halo, -$NR^hR^i$, -$S(O)_2R^j$, or heterocyclyl. In some embodiments, $R^5$ is unsubstituted heterocyclyl. In some embodiments, $R^5$ is substituted heterocyclyl. In some embodiments, $R^5$ is heterocyclyl, substituted with one or more groups selected from halo, -$NR^hR^i$, -$S(O)_2R^j$, or heterocyclyl. In some embodiments, $R^5$ is unsubstituted heteroaryl. In some embodiments, $R^5$ is substituted heteroaryl. In some embodiments, $R^5$ is heteroaryl, substituted with one or more groups selected from halo, -$NR^hR^i$, -$S(O)_2R^j$, or heterocyclyl.

In some embodiments, the compound of Formula (III) is trimethylsilyl isothiocyanate (TMSITC) or pentafluorophenyl isothiocyanate (PFPITC).

In some embodiments, the compound is not trifluoromethyl isothiocyanate, allyl isothiocyanate, dimethylaminoazobenzene isothiocyanate, 4-sulfophenyl isothiocyanate, 3-pyridyl isothiocyanate, 2-piperidinoethyl isothiocyanate, 3-(4-morpholino) propyl isothiocyanate, or 3-(diethylamino)propyl isothiocyanate.

In some embodiments, the reagent is or comprises an alkyl amine. In some embodiments, the reagent additionally comprises DIPEA, trimethylamine, pyridine, and/or N-methylpiperidine. In some embodiments, the reagent additionally comprises pyridine and triethylamine in acetonitrile. In some embodiments, the reagent additionally comprises N-methylpiperidine in water and/or methanol.

In some embodiments, the polypeptide is also contacted with a carbodiimide compound.

In some embodiments, the chemical reagent comprises a carbodiimide derivative (*See, e.g.,* Chi et al., 2015, Chem. Eur. J. 2015, 21, 10369-10378).

In some embodiments, the NTAA of a polypeptide is labeled via acylation. (*See, e.g.,* Protein Science (1992), I, 582-589).

In some embodiments, the chemical reagent comprises a compound selected from the group consisting of a compound of Formula (IV):

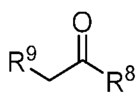 (IV)

or a salt or conjugate thereof,
wherein

$R^8$ is halo or -$OR^m$;
$R^m$ is H, $C_{1-6}$alkyl, or heterocyclyl; and
$R^9$ is hydrogen, halo, or $C_{1-6}$haloalkyl.

In some embodiments of Formula (IV), $R^8$ is halo. In some embodiments, $R^8$ is chloro. In some embodiments, $R^8$

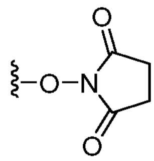
.

In some embodiments, $R^9$ is hydrogen. In some embodiments, $R^9$ is halo, such as bromo. In some embodiments, the compound of Formula (IV) is selected from acetyl chloride, acetyl anhydride, and acetyl-NHS. In some embodiments, the compound is not acetyl anhydride or acetyl-NHS.

In some embodiments, the polypeptide is also contacting with a peptide coupling reagent. In some embodiments, the peptide coupling reagent is a carbodiimide compound. In some embodiments, the carbodiimide compound is diisopropylcarbodiimide (DIC) or 1-ethyl-3-(3-dimethylaminopropyl)carbodiimide (EDC). In some embodiments, the method includes contacting with at least one compound of Formula (I) and a carbodiimide compounds, such as DIC or EDC.

In some embodiments, the chemical reagent comprises a conjugate of Formula (I), Formula (II), Formula (III), or Formula (IV). In some embodiments, the reagent used to modify the terminal amino acid of a polypeptide comprises a compound of Formula (I), Formula (II), Formula (III), or Formula (IV) conjugated to a ligand.

In some embodiments, the chemical reagent comprises a conjugate of Formula (I)-Q, Formula (II)-Q, Formula (III)-Q, or Formula (IV)-Q, wherein Formula (I)-(IV) are as defined above, and Q is a ligand.

In some embodiments, the ligand Q is a pendant group or binding site *(e.g.,* the site to which the binding agent binds). In some embodiments, the polypeptide binds covalently to a binding agent. In some embodiments, the polypeptide comprises a terminal amino acid which includes a ligand group that is capable of covalent binding to a binding agent. In certain embodiments, the polypeptide comprises a labeled NTAA with a compound of Formula (I)-Q, Formula (II)-Q, Formula (III)-Q, or Formula (IV)-Q, , wherein the Q binds covalently to a binding agent. In some embodiments, a coupling reaction is carried out to create a covalent linkage between the polypeptide and the binding agent (*e.g.,* a covalent linkage between the ligand Q and a functional group on the binding agent).

In some embodiments, the chemical reagent comprises a conjugate of Formula (I)-Q

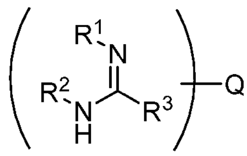
(I)-Q

wherein $R^1$, $R^2$, and $R^3$ are as defined above and Q is a ligand.

In some embodiments, the chemical reagent comprises a conjugate of Formula (II)-Q

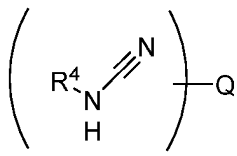
(II)-Q

wherein $R^4$ is as defined above, and Q is a ligand.

In some embodiments, the chemical reagent comprises a conjugate of Formula (III)-Q

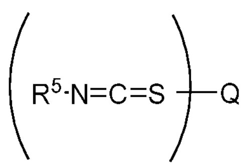
(III)-Q

wherein $R^5$ is as defined above and Q is a ligand.

In some embodiments, the chemical reagent comprises a conjugate of Formula (IV)-Q

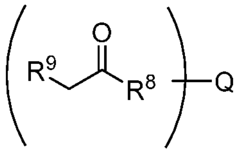 (IV)-Q

wherein $R^8$ and $R^9$ are as defined above and Q is a ligand.

In some embodiments, Q is selected from the group consisting of $-C_{1-6}$alkyl, $-C_{2-6}$alkenyl, $-C_{2-6}$alkynyl, aryl, heteroaryl, heterocyclyl, -N=C=S, -CN, $-C(O)R^n$, $-C(O)OR^o$, $--SR^p$ or $-S(O)_2R^q$; wherein the $-C_{1-6}$alkyl, $-C_{2-6}$alkenyl, $-C_{2-6}$alkynyl, aryl, heteroaryl, and heterocyclyl are each unsubstituted or substituted, and $R^n$, $R^o$, $R^p$, and $R^q$ are each independently selected from the group consisting of $-C_{1-6}$alkyl, $-C_{1-6}$haloalkyl, $-C_{2-6}$alkenyl, $-C_{2-6}$alkynyl, aryl, heteroaryl, and heterocyclyl. In some embodiments, Q is selected from the group consisting of

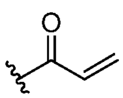, 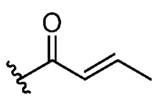, 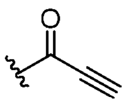, 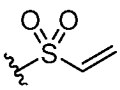, 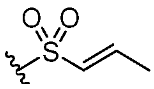, 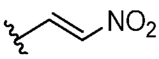, 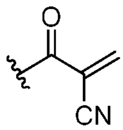, 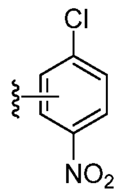,

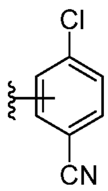, 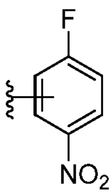, 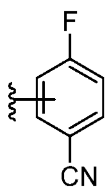, 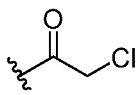, 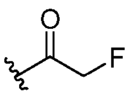, 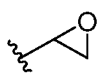, 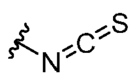, 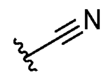,

and

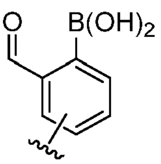.

In some embodiments, Q is a fluorophore. In some embodiments, Q is selected from a lanthanide, europium, terbium, XL665, d2, quantum dots, green fluorescent protein, red fluorescent protein, yellow fluorescent protein, fluorescein, rhodamine, eosin, Texas red, cyanine, indocarbocyanine, ocacarbocyanine, thiacarbocyanine, merocyanine, pyridyloxadole, benzoxadiazole, cascade blue, nile red, oxazine 170, acridine orange, proflavin, auramine, malachite green crystal violet, porphine phtalocyanine, and bilirubin.

Provided in other aspects are reagents used in labeling the terminal amino acid or dipeptide for removal by the modified dipeptide cleavase with more than one label.

In some embodiments, labeling the terminal amino acid (e.g., NTAA) or amino acid for removal as a dipeptide by the modified dipeptide cleavase includes using a first reagent and a second reagent. In some embodiments, the terminal amino acid is concurrently or sequentially labeled with the first reagent and the second reagent. In some embodiments, the first reagent comprises a compound selected from the group consisting of a compound of Formula (I), (II), (III), (IV), and (IV), or a salt or conjugate thereof, as described herein.

In some embodiments, the second reagent comprises a compound of Formula (Va) or (Vb):

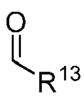 (Va)

or a salt or conjugate thereof,
wherein

$R^{13}$ is H, $C_{1-6}$alkyl, aryl, heteroaryl, cycloalkyl, or heterocyclyl, wherein the $C_{1-6}$alkyl, aryl, heteroaryl, cycloalkyl, and heterocyclyl are each unsubstituted or substituted; or

$R^{13}$-X (Vb)

wherein
$R^{13}$ is $C_{1-6}$alkyl, aryl, heteroaryl, cycloalkyl, or heterocyclyl, each of which is unsubstituted or substituted; and
X is a halogen.

In some embodiments of Formula (Va), $R^{13}$ is H. In some embodiments, $R^{13}$ is methyl. In some embodiments, $R^{13}$ is ethyl, propyl, isopropyl, butyl, isobutyl, secbutyl, pentyl, or hexyl. In some embodiments, $R^{13}$ is $C_{1-6}$alkyl, which is substituted. In some embodiments, $R^{13}$ is $C_{1-6}$alkyl, which is substituted with aryl, heteroaryl, cycloalkyl, or heterocyclyl. In some embodiments, $R^{13}$ is $C_{1-6}$alkyl, which is substituted with aryl. In some embodiments, $R^{13}$ is - $CH_2CH_2Ph$, $-CH_2Ph$, $-CH(CH_3)Ph$, or $-CH(CH_3)Ph$.

In some embodiments of Formula (Vb), $R^{13}$ is methyl. In some embodiments, $R^{13}$ is ethyl, propyl, isopropyl, butyl, isobutyl, secbutyl, pentyl, or hexyl. In some embodiments, $R^{13}$ is $C_{1-6}$alkyl, which is substituted. In some embodiments, $R^{13}$ is $C_{1-6}$alkyl, which is substituted with aryl, heteroaryl, cycloalkyl, or heterocyclyl. In some embodiments, $R^{13}$ is $C_{1-6}$alkyl, which is substituted with aryl. In some embodiments, $R^{13}$ is $-CH_2CH_2Ph$, $-CH_2Ph$, $-CH(CH_3)Ph$, or - $CH(CH_3)Ph$.

In some embodiments, the reagent for modifying or labeling the terminal amino acid for removal as part of a dipeptide by the modified dipeptide cleavase comprises formaldehyde. In some embodiments, the reagent for modifying or labeling the terminal amino acid comprises methyl iodide.

In some embodiments, the polypeptide is also contacted with a reducing agent. In some embodiments, the reducing agent comprises a borohydride, such as $NaBH_4$, $KBH_4$, $ZnBH_4$, $NaBH_3CN$ or $LiBu_3BH$. In some embodiments, the reducing agent comprises an aluminum or tin compound, such as $LiAlH_4$ or SnCl. In some embodiments, the reducing agent comprises a borane complex, such as $B_2H_6$ and dimethyamine borane. In some embodiments, the reagent additionally comprises $NaBH_3CN$.

In some embodiments, the reagents that may be used to label the terminal amino acid (*e.g.,* NTAA) include: 4-sulfophenyl isothiocyanate (sulfo-PITC), 4-nitrophenyl isothiocyanate (nitro-PITC), 3-pyridyl isothiocyanate (PYITC), a phenyl isocyanate (PIC), a nitro-PIC, a sulfo-PIC, an anhydride (*e.g.*, an isatoic anhydride, an isonicotinic anhydride, an azaisatoic anhydride, a succinic anhydride), 2-piperidinoethyl isothiocyanate (PEITC), 3-(4-morpholino) propyl isothiocyanate (MPITC), 3-(diethylamino)propyl isothiocyanate (DEPTIC) (Wang et al., 2009, Anal Chem 81: 1893-1900), (1-fluoro-2,4-dinitrobenzene (Sanger's reagent, DNFB), dansyl chloride (DNS-Cl, or 1-dimethylaminonaphthalene-5-sulfonyl chloride), 4-sulfonyl-2-nitrofluorobenzene (SNFB), acetylation reagents, amidination (guanidinylation) reagents (including PCA and PCA derivatives), 2-carboxy-4,6-dinitrochlorobenzene, 7-methoxycoumarin acetic acid, a thioacylation reagent, a thioacetylation reagent, and/or a thiobenzylation reagent. Many of these reagents are unreactive or minimally reactive with DNA including PITC, nitro-PITC, sulfo-PITC, PYITC, and guanidinylation reagents (*e.g.*, PCA compounds). If the amino acid is blocked to labeling, there are a number of approaches to unblock the terminus, such as removing N-acetyl blocks with acyl peptide hydrolase (APH) (Farries, Harris et al., 1991, Eur. J. Biochem. 196:679-685). Methods of unblocking the N-terminus of a peptide are known in the art *(see, e.g.,* Krishna et al., 1991, Anal. Biochem. 199:45-50; Leone et al., 2011, Curr. Protoc. Protein Sci., Chapter 11:Unit11.7; Fowler et al., 2001, Curr. Protoc. Protein Sci., Chapter 11: Unit 11.7).

Dansyl chloride reacts with the free amine group of a peptide to yield a dansyl derivative of the NTAA. DNFB and SNFB react the $\alpha$-amine groups of a peptide to produce DNP-NTAA, and SNP-NTAA, respectively. Additionally, both DNFB and SNFB also react with the with $\varepsilon$-amine of lysine residues. DNFB also reacts with tyrosine and histidine amino acid residues. In some embodiments, SNFB has better selectivity for amine groups than DNFB (Carty et al., J Biol Chem (1968) 243(20): 5244-5253). In certain embodiments, lysine $\varepsilon$-amines are pre-blocked with an organic anhydride prior to polypeptide protease digestion into peptides.

Isothiocyanates, in the presence of ionic liquids, have been shown to have enhanced reactivity to primary amines. Ionic liquids are excellent solvents (and serve as a catalyst) in organic chemical reactions and can enhance the reaction of isothiocyanates with amines to form thioureas. Moreover, ionic liquids may act as absorbers of microwave radiation to further enhance reactivity (Martinez-Palou, J. Mex. Chem. Soc (2007) 51(4): 252-264). An example is the use of the ionic liquid 1-butyl-3-methyl-imidazolium tetraflouoraborate [Bmim][BF4] for rapid and efficient functionalization of aromatic and aliphatic amines by phenyl isothiocyanate (PITC) (Le, Chen et al. 2005).

In some embodiments, the peptide may be labeled by treating with a chemical reagent comprising a compound of Formula (AB) as shown in the scheme below:

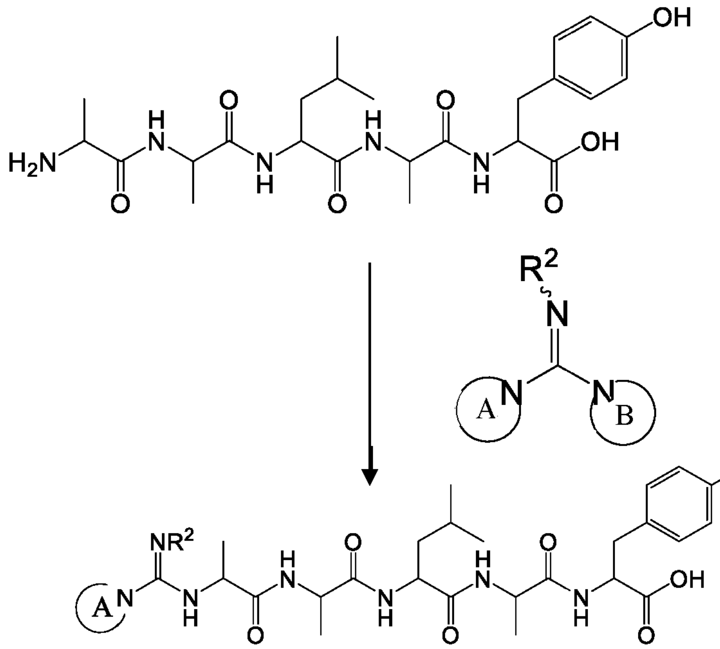

In some embodiments, the peptide treated with a chemical reagent to modify the N-terminal amino acid (NTAA) of peptides is treated with a diheterocyclic methanimine reagent. In some embodiments, the reagent for modifying or labeling the terminal amino acid for removal as part of a dipeptide by the modified dipeptide cleavase comprises a compound of Formula (AB):

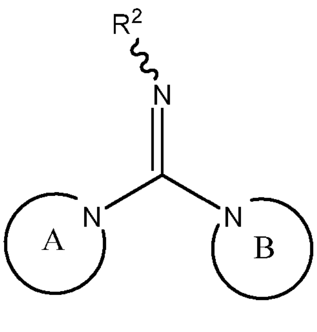

(AB)

wherein:

$R^2$ is H, $R^4$, OH, $OR^4$, $NH_2$, or $-NHR^4$;
$R^4$ is $C_{1-6}$ alkyl, which is optionally substituted with one or two members selected from halo, $C_{1-3}$ alkyl, $C_{1-3}$ alkoxy, $C_{1-3}$ haloalkyl, phenyl, 5-membered heteroaryl, and 6-membered heteroaryl, wherein each phenyl, 5-membered heteroaryl, and 6-membered heteroaryl is optionally substituted with one or two members selected from halo, -OH, $C_{1-3}$ alkyl, $C_{1-3}$ alkoxy, $C_{1-3}$ haloalkyl, $NO_2$, CN, COOR", and $CON(R")_2$,
where each R" is independently H or $C_{1-3}$ alkyl;

ring A and ring B are each independently a 5-membered heteroaryl ring containing up to three N atoms as ring members and each is optionally fused to an additional phenyl or a 5-6 membered heteroaryl ring, and wherein the 5-membered heteroaryl ring and optional fused phenyl or 5-6 membered heteroaryl ring are each optionally substituted with one or two groups selected from $C_{1-4}$ alkyl, $C_{1-4}$ alkoxy, -OH, halo, $C_{1-4}$ haloalkyl, $NO_2$, COOR, $CONR_2$, $-SO_2R^*$, $-NR_2$, phenyl, and 5-6 membered heteroaryl;
wherein each R is independently selected from H and $C_{1-3}$ alkyl optionally substituted with OH, $OR^*$, $-NH_2$, $-NHR^*$, or $-NR^*_2$; and
each R* is $C_{1-3}$ alkyl, optionally substituted with OH, oxo, $C_{1-2}$ alkoxy, or CN;
wherein two R, or two R", or two R* on the same N can optionally be taken together to form a 4-7 membered heterocyclic ring, optionally containing an additional heteroatom selected from N, O and S as a ring member, and optionally substituted with one or two groups selected from halo, $C_{1-2}$ alkyl, OH, oxo, $C_{1-2}$ alkoxy, or CN.

or a salt thereof. In some embodiments, Ring A and Ring B are not both unsubstituted imidazole, and that Ring A and Ring B are not both unsubstituted benzotriazole;

In an example of this embodiment, $R^2$ is H or $R^4$. In these embodiments, In these embodiments, the 5-membered heteroaryl group, when present, can be a 5-membered ring comprising one to three heteroatoms selected from N, O and S as ring members, and the 6-membered heteroaryl group when present can be a 6-membered ring comprising one to three nitrogen atoms as ring members. In some of these embodiments, neither ring A nor ring B is unsubstituted imidazole or unsubstituted benzotriazole. In some embodiments, $R^2$ is H. In some of these embodiments, neither ring A nor ring B is unsubstituted imidazole or unsubstituted benzotriazole.

In some embodiments, Ring A and Ring B are different. In some embodiments, Ring A and Ring B are the same. Specific compounds of this embodiment include:

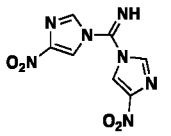
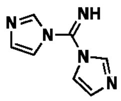
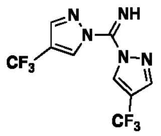
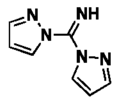
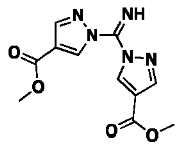
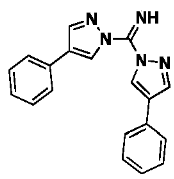

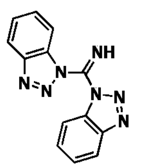
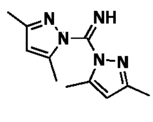
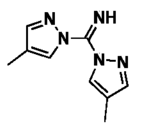
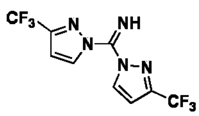
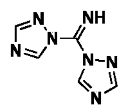

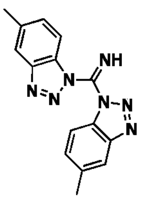
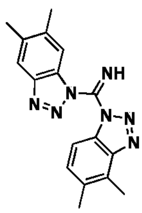
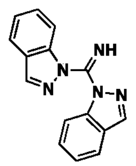
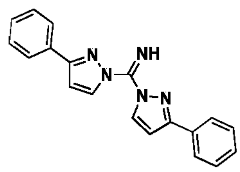
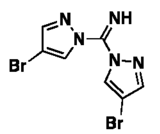

In some aspects, each 5-6 membered heteroaryl ring is independently selected and contains 1 or 2 heteroatoms selected from N, O and S as ring members. In these embodiments, each 5-membered heteroaryl group present can be a 5-membered ring comprising one or two heteroatoms selected from N, O and S as ring members, and the 6-membered heteroaryl group can be a 6-membered ring comprising one to two nitrogen atoms as ring members.

In some specific embodiments, Ring A and Ring B are selected from:

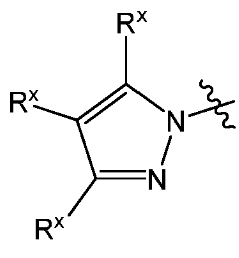
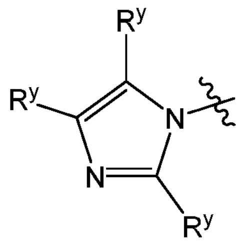

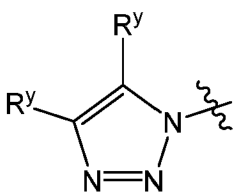
and
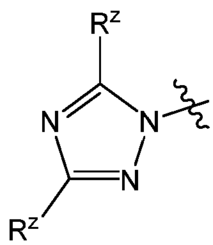

wherein:

each $R^x$, $R^y$ and $R^z$ is independently selected from H, halo, $C_{1-2}$ alkyl, $C_{1-2}$ haloalkyl, $NO_2$, $SO_2(C_{1-2}$ alkyl), $COOR^{\#}$, $C(O)N(R^{\#})_2$, and phenyl optionally substituted with one or two groups selected from halo, $C_{1-2}$ alkyl, $C_{1-2}$ haloalkyl, $NO_2$, $SO_2(C_{1-2}$ alkyl), $COOR^{\#}$, and $C(O)N(R^{\#})_2$,

and two $R^x$, $R^y$ or $R^z$ on adjacent atoms of a ring can optionally be taken together to form a phenyl group, 5-membered heteroaryl group, or 6-membered heteroaryl group fused to the ring, and the fused phenyl, 5-membered heteroaryl, or 6-membered heteroaryl group can optionally be substituted with one or two groups selected from halo, $C_{1-2}$ alkyl, $C_{1-2}$ haloalkyl, $NO_2$, $SO_2(C_{1-2}$ alkyl), $COOR^{\#}$, and $C(O)N(R^{\#})_2$;

wherein each $R^{\#}$ is independently H or $C_{1-2}$ alkyl; and wherein two R# on the same nitrogen can optionally be taken together to form a 4-7 membered heterocycle optionally containing an additional heteroatom selected from N, O and S as a ring member, wherein the 4-7 membered heterocycle is optionally substituted with one or two groups selected from halo, OH, OMe, Me, oxo, $NH_2$, NHMe and $NMe_2$;

or a salt thereof.

In these embodiments, each 5-membered heteroaryl group present can be a 5-membered ring comprising one to three heteroatoms selected from N, O and S as ring members, and the 6-membered heteroaryl group can be a 6-membered ring comprising one to three nitrogen atoms as ring members.

In some embodiments, Ring A and Ring B are the same and are selected from:

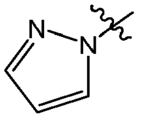
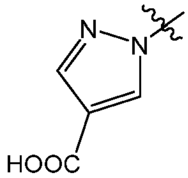
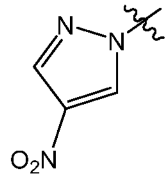

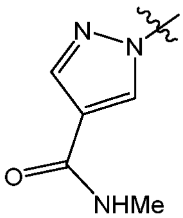
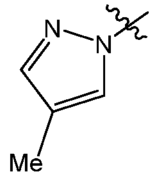
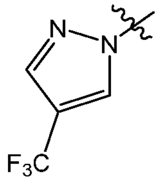

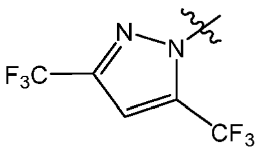
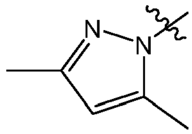
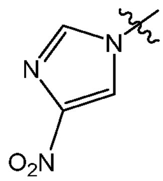

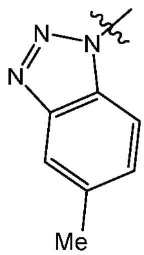
and
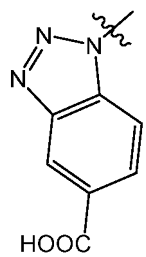

The compound of embodiment 30, which is selected from the following:

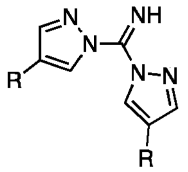

R = H, $CH_3$, $CF_3$, $NO_2$, C(O)$NHCH_3$,

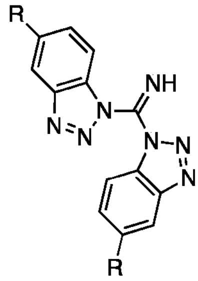

R = H, $CH_3$, $CO_2H$,

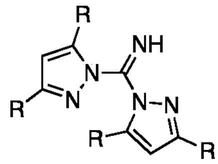

R = $CH_3$, $CF_3$

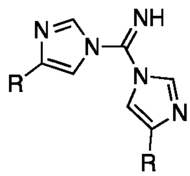

R = H, $NO_2$

**B. Engineering and Genetic Selection**

In some embodiments, the modified dipeptide cleavase provided herein can be made, isolated, engineered, or selected for using any suitable methods. In some cases, the variant or modified dipeptide cleavase polypeptide is altered in primary amino acid sequence compared to the wild-type or unmodified dipeptide cleavase by introducing one or more substitutions, additions, or deletions of amino acid residues. In some embodiments, the modified dipeptide cleavase is derived from a wild-type or unmodified dipeptide cleavase (*e.g.*, a dipeptidyl peptidase, a dipeptidyl aminopeptidase, a peptidyl-dipeptidase, a dipeptidyl carboxypeptidase, or a protein classified in EC 3.4.14, EC 3.4.15, MEROPS S9, MEROPS S46, MEROPS M49, or a functional homolog or fragment thereof) via engineering and genetic selection. A variety of techniques including genetic selection, protein engineering, recombinant methods, chemical synthesis, or combinations thereof, may be employed.

In some embodiments, the modified dipeptide cleavase is engineered using a rational design approach for select activities, substrate binding capability, or other cleaving characteristics. In some embodiments, a rational design approach is based on crystal structure of the unmodified dipeptide cleavase. In some examples, the rational design approach is based on crystal structure of the unmodified dipeptide cleavase with substrates to identify target amino acid residues for modification. In some cases, the modifications may be targeted at residues of specific domains of the unmodified dipeptide cleavase *(See e.g.,* Sakamoto et al., Scientific Reports 2014, 4:4977). In some embodiments, a rational design is used to engineer a modified dipeptide cleavase with modified amino acids in the substrate binding domain of the unmodified dipeptide cleavase. In some embodiments, the mutations, *e.g.*, one or more amino acid modifications (*e.g.*, substitutions, additions, deletions) corresponds to positions 316, 391, 394, or a combination thereof, with reference to positions of SEQ ID NO: 5 or the sequence of a human dipeptidyl peptidase 3 (DPP3) or a homolog thereof. In some embodiments, a rational design is used to engineer a modified dipeptide cleavase that is able to bind or cleave polypeptides of increased length compared to an unmodified dipeptide cleavase. In some embodiments, the mutations, *e.g.,* one or more amino acid modifications *(e.g.,* substitutions, additions, deletions) corresponds to amino acid residue positions 419, 420, 421, 422, 423, 424, 425, 426, or a combination thereof, with reference to positions of SEQ ID NO: 5. In some embodiments, a rational design is used to engineer a modified dipeptide cleavase with modified amino acids in the hinge region of the unmodified dipeptide cleavase.

In some embodiments, the genetic selection or other engineering methods are designed to identify modified dipeptide cleavases that are active on labeled polypeptides (e.g. chemically labeled polypeptides). In some embodiments, the genetic selection or other engineering methods are designed to identify modified dipeptide cleavases that are active on modified or labeled polypeptides having a labeled N-terminal amino acid. In some cases, the size or other characteristics of the moiety or label on the labeled polypeptide is considered in the design of the genetic selection or other engineering methods to obtain a desired modified dipeptide cleavase.

It is understood that references to amino acids, including to specific sequences set forth in the Sequence Listing as SEQ ID NOs used herein to describe domain organization of a wild-type or modified dipeptide cleavase are for illustrative purposes only and are not meant to limit the scope of the embodiments provided. It is understood that

polypeptides and the description of domains thereof are theoretically derived based on homology analysis and alignments with similar molecules. Thus, the exact locus can vary, and is not necessarily the same for each protein. Hence, the specific domain, such as specific binding domain, loop domain, or other functional domain), can be identified in a homolog or enzyme derived from another species using known analyses and alignment methods.

In some examples, amino acids for modification in a wildtype dipeptide cleavase can be chosen using analysis of crystal structure of the wild-type cleavase (*e.g.* wildtype DAP BII) and its substrate to identify contact residues and other residues at the protein interaction interface. This analysis can be performed for example, using Rosetta software suite for macromolecular modeling (Das et al., Annu Rev Biochem (2008) 77:363-382). In some embodiments, using the selected target residues for modification, an alignment of wildtype cleavase sequences of other organisms can be used to identify conserved residues (Crooks et al., Genome Res (2004) 14(6): 1188-1190). Based on this analysis, conserved target residues or corresponding residues in homologs can be modified. In some embodiments, the identified contact residues or other residues of interest are modified to introduce new functions. As shown in **FIG. 4A-4C,** a WebLogo analysis of DAP BII homologs with 60% sequence similarity or identity showed sequence conservation across various residues. For example, sequence conservation was observed for residues at the amine binding sites of DAP BII including positions N215, W216, R220, N330, and D674 in reference to the wildtype DAP BII sequence set forth in SEQ ID NO: 20. In another example, sequence conservation was observed for residues at the amine binding sites of DAP BII including positions G207, K208, F209, G210, G211, D212, I213, D214, N215, W216, M217, W218, P219, R220, H221, T222, G223, A224, F225, A226, A326, and N334, in reference to the wildtype DAP BII sequence set forth in SEQ ID NO: 20.

In some aspects, a rational design approach for engineering DAP BII may be used to target domains or residues such that the resulting modified dipeptide cleavase removes or is configured to remove a labeled N-terminal amino acid (NTAA) using crystal structures of DAP BII in complex with substrates (Sakamoto et al., Scientific Reports 2014, 4:4977). For example, the DAP BII structure in complex with a peptide substrate at the residues N191, W192, R196, N306, and D650 (based on the sequence of the protein set forth in SEQ ID NO: 13; UniProt Accession No. V5YM14) interacts with the peptide N-terminal amine group. Additionally, a loop of approximately 20 residues (residue 183-202 in reference to SEQ ID NO: 13) makes contact with the N-terminal residue and penultimate residue of a bound peptide substrate. These amine binding residues and NTAA and penultimate NTAA binding residues, individually or in combination, may be targeted for modification.

In some aspects, it may be desired to modify the specificity of the unmodified or wildtype cleavase (*See e.g.,* Sakamoto et al., Scientific Reports 2014, 4:4977). In some examples, residues in the S1 subsite or pocket of DAP BII can be targeted to engineer a modified cleavase with preferred specificity (*e.g.*, reduced specificity for a specific amino acid residue at the P1 position of the polypeptide treated with the modified cleavase). In some embodiments, the modified dipeptide cleavase comprises mutations, *e.g.*, one or more amino acid modifications (*e.g.*, substitutions, additions, deletions) corresponding to positions D627, I628, G630, A648, G651, S655, M669, or a combination thereof, with reference to positions of SEQ ID NO: 13.

In some embodiments, a modified dipeptide cleavase variant can be identified using a genetic screen. In some cases, the genetic screen uses a cell-based system. In some embodiments, the genetic screen uses prokaryotic cells, such as *E. coli* strains including *E. coli* variants or mutants. In some embodiments, the genetic screen uses eukaryotic cells, such as yeast two-hydrid systems. In some embodiments, the genetic selection is designed to select for modified dipeptide cleavases with desired characteristics for binding of substrates, cleaving, and/or removal of labeled terminal amino acids.

In some embodiments, carrying out a genetic selection screen involves preparing various dipeptide cleavase genes (*e.g.,* a dipeptidyl peptidase, a dipeptidyl aminopeptidase, a peptidyl-dipeptidase, a dipeptidyl carboxypeptidase) for expression. A plasmid or cosmid containing nucleic acid sequences encoding mutated or modified dipeptide cleavase polypeptides is readily constructed using standard techniques well known in the art. In some embodiments, the expression of any of the dipeptide cleavases (*e.g.*, any of SEQ ID NOs: 5-8, 10-16, 20, 31, 32) may further include a signal sequence. In some cases, the use of a signal sequence may be useful for purification purposes. For example, a periplasm targeting sequence such as PelB can be included in the expression construct. Recombinant vectors can be generated using any of the recombinant techniques known in the art.

In some embodiments, the vectors can include a prokaryotic origin of replication and/or a gene whose expression confers a detectable or selectable marker for propagation and/or selection in prokaryotic systems. Once the vector or DNA sequence containing the constructs has been prepared for expression, the DNA constructs may be introduced into an appropriate host. In some embodiments, prokaryotic hosts can be used including bacteria such as *E. coli., Bacillus, Streptomyces, Pseudomonas, Salmonella, Serratia,* etc. Various techniques may be employed, such as protoplast fusion, calcium phosphate precipitation, electroporation or other conventional techniques. After the fusion, the cells are grown in media and screened for appropriate activities.

In some examples, libraries of mutated dipeptide cleavase genes can be generated by error prone PCR or rational mutagenesis using the crystal structure of the cleavase as a guide, or a combination thereof. Other suitable methods for generating mutations or generating a library may also be used. A library of mutated dipeptide cleavase genes can be subsequently cloned into a vector and transformed into an *E. coli* auxotroph strain (available from CSSC E. coli

Genetic Stock Center at Yale - https://cgsc2.biology.yale.edu/). In some embodiments, the screen involves isolating colonies growing on the selection media and extracting and analyzing plasmid DNA to identify modified dipeptide cleavase polypeptides that remove a labeled terminal dipeptide from a polypeptide. In some embodiments, a screen can be performed to identify and isolate a modified dipeptide cleavase that cleaves or is configured to cleave a polypeptide with a labeled amino acid (*e.g.,* a PITC-labeled NTAA or a Cbz-labeled NTAA, etc). In some embodiments, the genetic screen is aimed at selecting for the binding of the label but not for a specific amino acid, therefore, the screen uses polypeptides with various labeled terminal amino acids. In some embodiments, selecting a modified dipeptide cleavase further includes purifying, characterizing, assessing and/or optimizing of the activity of the modified dipeptide cleavase. The modified dipeptide cleavase may be isolated and purified in accordance with conventional methods, such as extraction, precipitation, chromatography, affinity chromatography, electrophoresis, or the like.

In some embodiments, a genetics screen or other selection methods can also be used to select for and obtain modified dipeptide cleavases with an altered active site of the cleavase or with altered binding pockets of the cleavase. In some embodiments, genetics screen or selection methods can also be used to select for and obtain modified dipeptide cleavases with an altered hinge region of the cleavase. In some embodiments, genetic screen or selection methods can also be used to select for and obtain modified dipeptide cleavases with an altered binding cleft of the cleavase. In some cases, genetic screen or selection methods can also be used to select for and obtain modified dipeptide cleavases with an altered inter-lobe cleft of the cleavase. In some embodiments, genetic screen or selection methods can also be used to select for and obtain modified dipeptide cleavases with an altered alpha amine binding region of the cleavase. For example, the modified dipeptide cleavase exhibits reduced alpha amine binding compared to the wild-type cleavase polypeptide.

In some embodiments, a genetic screen or other selection methods can also be used to select for and obtain modified dipeptide cleavases configured to remove a dipeptide comprising a labeled terminal amino acid from polypeptides of various lengths. In some cases, porin size in the *E. coli* outer membrane limits the peptide length that can be uptaken. In some embodiments, this length limitation is overcome by briefly treating *E. coli* with Tris-EDTA or the small molecule MAC13243 which permeabilizes the *E. coli* outer membrane (*e.g.,* Leive, L. (1974). Ann N Y Acad Sci 235(0): 109-129; Muheim, C. (2017). Scientific Reports 7(1): 17629) and allows uptake of peptides into the periplasmic space. In some embodiments, the modified dipeptide cleavase is capable of cleaving or is configured to remove amino acids from polypeptides that are greater than 5 amino acids in length, greater than 6 amino acids in length, greater than 7 amino acids in length, greater than 8 amino acids in length, greater than 9 amino acids in length, greater than 10 amino acids in length, greater than 15 amino acids in length, greater than 20 amino acids in length, greater than 25 amino acids in length, or greater than 30 amino acids in length. In some embodiments, the modified dipeptide cleavase is capable of cleaving or is configured to remove amino acids from polypeptides that are less than 30 amino acids in length, less than 40 amino acids in length, less than 50 amino acids in length, less than 75 amino acids in length, less than 100 amino acids in length, less than 200 amino acids in length, less than 300 amino acids in length, less than 400 amino acids in length, less than 500 amino acids in length, less than 600 amino acids in length, less than 700 amino acids in length, less than 800 amino acids in length, less than 900 amino acids in length, or less than 1000 amino acids in length. In some embodiments, the modified dipeptide cleavase is capable of cleaving or is configured to remove amino acids from polypeptides that are between 5 to 100 amino acids in length, between 10 to 100 amino acids in length, between 20 to 100 amino acids in length, between 30 to 100 amino acids in length, between 5 to 50 amino acids in length, between 10 to 50 amino acids in length, between 20 to 50 amino acids in length, between 30 to 50 amino acids in length, between 5 to 30 amino acids in length, between 10 to 30 amino acids in length, between 20 to 30 amino acids in length, between 10 to 20 amino acids in length. In some embodiments, the modified dipeptide cleavase is capable of cleaving or is configured to remove amino acids from polypeptides that are between 50 to 1000 amino acids in length, between 100 to 1000 amino acids in length, between 300 to 1000 amino acids in length, between 500 to 1000 amino acids in length, between 10 to 500 amino acids in length, between 50 to 500 amino acids in length, between 100 to 500 amino acids in length, or between 200 to 500 amino acids in length.

In some embodiments, the modified dipeptide cleavase is capable or configured to remove dipeptides from partial or digested proteins and polypeptides (*e.g.*, protein or polypeptide fragments). In some embodiments, the modified dipeptide cleavase is capable or configured to remove dipeptides from whole or undigested proteins and polypeptides.

In some embodiments, the modified dipeptide cleavase removes the terminal dipeptide by contacting the polypeptide with a modified dipeptide cleavase for less than 5 minutes, less than 10 minutes, less than 20 minutes, less than 30 minutes, less than 40 minutes, less than 50 minutes, less than 60 minutes, less than 2 hours, less than 5 hours, less than 8 hours, or less than 10 hours.

In some embodiments, the modified dipeptide cleavase achieves a yield of polypeptides with the terminal dipeptide removed of >30%, >40%, >50%, >60%, >70%, >80%, >90%, >95%, >99% or more by treating the polypeptide with the modified dipeptide cleavase for about less than 15 minutes. In some embodiments, the modified dipeptide cleavase achieves a yield of polypeptides with the terminal dipeptide removed of >30%, >40%, >50%, >60%, >70%, >80%, >90%, >95%, >99% or more by treating the polypeptide with the modified dipeptide cleavase for about less than 30

minutes. In some embodiments, the modified dipeptide cleavase achieves a yield of polypeptides with the terminal dipeptide removed of >30%, >40%, >50%, >60%, >70%, >80%, >90%, >95%, >99% or more by treating the polypeptide with the modified dipeptide cleavase for about less than 45 minutes. In some embodiments, the modified dipeptide cleavase achieves a yield of polypeptides with the terminal dipeptide removed of >30%, >40%, >50%, >60%, >70%, >80%, >90%, >95%, >99% or more by treating the polypeptide with the modified dipeptide cleavase for about less than 1 hour. In some embodiments, the modified dipeptide cleavase achieves a yield of polypeptides with the terminal dipeptide removed of >30%, >40%, >50%, >60%, >70%, >80%, >90%, >95%, >99% or more by treating the polypeptide with the modified dipeptide cleavase for about less than 2 hours. In some embodiments, the modified dipeptide cleavase achieves a yield of polypeptides with the terminal dipeptide removed of >30%, >40%, >50%, >60%, >70%, >80%, >90%, >95%, >99% or more by treating the polypeptide with the modified dipeptide cleavase for about less than 5 hours.

In some embodiments, the modified dipeptide cleavase is capable of cleaving dipeptides or functions at a temperature of higher than about 10° C, higher than about 20° C higher than about 30° C, or higher than about 40° C. In some embodiments, the modified dipeptide cleavase is capable of cleaving terminal dipeptides or functions at a temperature of about 10° C to 20° C, about 10° C to 30° C, about 10° C to 40° C, about 10° C to 50° C, about 10° C to 60° C, about 10° C to 70° C, about 10° C to 80° C, about 10° C to 90° C or about 10° C to 100° C; about 20° C to 30° C, about 20° C to 40° C, about 20° C to 50° C, about 20° C to 60° C, about 20° C to 70° C, about 20° C to 80° C, about 20° C to 90° C, or about 20° C to 100° C; about 30° C to 40° C, about 30° C to 50° C, about 30° C to 60° C; about 50° C to 70° C, about 50° C to 80° C, about 50° C to 90° C, or about 50° C to 100° C. In some embodiments, the modified dipeptide cleavase is capable of cleaving terminal dipeptides at a temperature at which the secondary structure of the polypeptide is disrupted. In some embodiments, the modified dipeptide cleavase functions at about 20 to 25° C. In some embodiments, the method includes contacting the modified dipeptide cleavase with the polypeptide while applying heating. In some embodiments, the heating is achieved by applying microwave energy. In some embodiments of any of the methods provided herein, the contacting of the modified dipeptide cleavase with the polypeptide to remove a terminal dipeptide is performed in the presence of microwave energy.

Provided herein are isolated DNA molecules encoding any of the modified dipeptide cleavases as described in Section I. Also provided are recombinant expression vectors comprising a DNA molecule encoding any of the modified dipeptide cleavases as described in Section I. In some cases, the DNA molecules and recombinant expression vectors are isolated from the genetic engineering and selection methods described. In some cases, a host cell comprising the DNA molecule is also provided. In some embodiments, a fusion protein containing a fragment of a modified dipeptide cleavase is provided.

In some embodiments, provided herein is a method of producing a modified or variant dipeptide cleavase, comprising introducing the nucleic acid molecule according to any one of the embodiments described herein or vector according to any one of the embodiments described herein into a host cell under conditions to express the protein in the cell. Also provided herein are methods for producing any of the modified dipeptide cleavases provided herein including: cultivating a transformed host cell under conditions suitable for expression of the modified dipeptide cleavase, and separating, purifying and/or recovering the mutant organism expressing the modified dipeptide cleavase. In some embodiments, provided herein is a host cell comprising a DNA molecule encoding a modified dipeptide cleavase. In some embodiments, the host cell comprises a recombinant expression vector for expressing a modified dipeptide cleavase. In some embodiments, the method further includes isolating or purifying the variant or modified dipeptide cleavase from the cell.

In some embodiments, provided herein is an engineered cell, expressing the variant or modified dipeptide cleavase polypeptide according to any one of the embodiments described herein or the nucleic acid molecule encoding a variant or modified dipeptide cleavase described herein, or the vector according to any one of the embodiments described herein. In some embodiments, the variant or modified dipeptide cleavase polypeptide contains a signal peptide.

## II. POLYPEPTIDES

In some embodiments, the present disclosure relates to the treatment of polypeptides with any of the modified dipeptide cleavases provided herein. In some embodiments, the labeled terminal amino acid is removed as part of a dipeptide from a polypeptide (including a partial or fragmented polypeptide).

In some embodiments, the terminal amino acid is removed as a dipeptide from a polypeptide that has a length of greater than 4 amino acids, greater than 5 amino acids, greater than 6 amino acids, greater than 7 amino acids, greater than 8 amino acids, greater than 9 amino acids, greater than 10 amino acids, greater than 11 amino acids, greater than 12 amino acids, greater than 13 amino acids, greater than 14 amino acids, greater than 15 amino acids, greater than 20 amino acids, greater than 25 amino acids, or greater than 30 amino acids. In some cases, the length of the polypeptide is greater than 10 amino acids. In some embodiments, the terminal amino acid is removed as a dipeptide from a polypeptide that has a length of less than 30 amino acids, less than 40 amino acids, less than 50 amino acids, less than 75 amino acids, less than 100 amino acids, less than 200 amino acids, less than 300 amino acids, less than 400 amino acids, less than 500 amino

acids, less than 600 amino acids, less than 700 amino acids, less than 800 amino acids, less than 900 amino acids, or less than 1000 amino acids. In some embodiments, the terminal amino acid is removed as a dipeptide from a polypeptide that has a length of between 5 to 100 amino acids, between 10 to 100 amino acids, between 20 to 100 amino acids, between 30 to 100 amino acids, between 5 to 50 amino acids, between 10 to 50 amino acids, between 20 to 50 amino acids, between 30 to 50 amino acids, between 5 to 30 amino acids, between 10 to 30 amino acids, between 20 to 30 amino acids, between 10 to 20 amino acids. In some embodiments, the terminal amino acid is removed as a dipeptide from a polypeptide that has a length of between 50 to 1000 amino acids, between 100 to 1000 amino acids, between 300 to 1000 amino acids, between 500 to 1000 amino acids, between 10 to 500 amino acids, between 50 to 500 amino acids, between 100 to 500 amino acids, or between 200 to 500 amino acids.

In some embodiments, the terminal amino acid is removed as a dipeptide from a partial or digested protein and polypeptide (*e.g.,* a polypeptide fragment). In some embodiments, the terminal amino acid is removed as a dipeptide from a whole or undigested protein and polypeptide.

A polypeptide treated with the modified dipeptide cleavases provided herein and according the methods disclosed herein may be obtained from a suitable source or sample, including but not limited to: biological samples, such as cells (both primary cells and cultured cell lines), cell lysates or extracts, cell organelles or vesicles, including exosomes, tissues and tissue extracts; biopsy; fecal matter; bodily fluids (such as blood, whole blood, serum, plasma, urine, lymph, bile, cerebrospinal fluid, interstitial fluid, aqueous or vitreous humor, colostrum, sputum, amniotic fluid, saliva, anal and vaginal secretions, perspiration and semen, a transudate, an exudate (*e.g.*, fluid obtained from an abscess or any other site of infection or inflammation) or fluid obtained from a joint (normal joint or a joint affected by disease such as rheumatoid arthritis, osteoarthritis, gout or septic arthritis) of virtually any organism, with mammalian-derived samples, including microbiome-containing samples, being preferred and human-derived samples, including microbiome-containing samples, being particularly preferred; environmental samples (such as air, agricultural, water and soil samples); microbial samples including samples derived from microbial biofilms and/or communities, as well as microbial spores; research samples including extracellular fluids, extracellular supernatants from cell cultures, inclusion bodies in bacteria, cellular compartments including mitochondrial compartments, and cellular periplasm.

In certain embodiments, the polypeptide is a protein or a protein complex. Amino acid sequence information and post-translational modifications of the polypeptide are transduced into a nucleic acid encoded library that can be analyzed via next generation sequencing methods. A polypeptide may comprise L-amino acids, D-amino acids, or both. A polypeptide may comprise a standard, naturally occurring amino acid, a modified amino acid (*e.g.*, post-translational modification), an amino acid analog, an amino acid mimetic, or any combination thereof. In some embodiments, the polypeptide is naturally occurring, synthetically produced, or recombinantly expressed. In any of the aforementioned embodiments, the polypeptide may further comprise a post-translational modification.

Standard, naturally occurring amino acids include Alanine (A or Ala), Cysteine (C or Cys), Aspartic Acid (D or Asp), Glutamic Acid (E or Glu), Phenylalanine (F or Phe), Glycine (G or Gly), Histidine (H or His), Isoleucine (I or Ile), Lysine (K or Lys), Leucine (L or Leu), Methionine (M or Met), Asparagine (N or Asn), Proline (P or Pro), Glutamine (Q or Gln), Arginine (R or Arg), Serine (S or Ser), Threonine (T or Thr), Valine (V or Val), Tryptophan (W or Trp), and Tyrosine (Y or Tyr). Non-standard amino acids include selenocysteine, pyrrolysine, and N-formylmethionine, β-amino acids, Homo-amino acids, Proline and Pyruvic acid derivatives, 3-substituted Alanine derivatives, Glycine derivatives, Ring-substituted Phenylalanine and Tyrosine Derivatives, Linear core amino acids, and N-methyl amino acids.

A post-translational modification (PTM) of a polypeptide may be a covalent modification or enzymatic modification. Examples of post-translation modifications include, but are not limited to, acylation, acetylation, alkylation (including methylation), biotinylation, butyrylation, carbamylation, carbonylation, deamidation, deiminiation, diphthamide formation, disulfide bridge formation, eliminylation, flavin attachment, formylation, gamma-carboxylation, glutamylation, glycylation, glycosylation (*e.g.*, N-linked, O-linked, C-linked, phosphoglycosylation), glypiation, heme C attachment, hydroxylation, hypusine formation, iodination, isoprenylation, lipidation, lipoylation, malonylation, methylation, myristolylation, oxidation, palmitoylation, pegylation, phosphopantetheinylation, phosphorylation, prenylation, propionylation, retinylidene Schiff base formation, S-glutathionylation, S-nitrosylation, S-sulfenylation, selenation, succinylation, sulfination, ubiquitination, and C-terminal amidation. A post-translational modification includes modifications of the amino terminus and/or the carboxyl terminus of a peptide, polypeptide, or protein. Modifications of the terminal amino group include, but are not limited to, des-amino, N-lower alkyl, N-di-lower alkyl, and N-acyl modifications. Modifications of the terminal carboxy group include, but are not limited to, amide, lower alkyl amide, dialkyl amide, and lower alkyl ester modifications (*e.g.*, wherein lower alkyl is $C_1$-$C_4$ alkyl). A post-translational modification also includes modifications, such as but not limited to those described above, of amino acids falling between the amino and carboxy termini of a peptide, polypeptide, or protein. Post-translational modification can regulate a protein's "biology" within a cell, e.g., its activity, structure, stability, or localization. Phosphorylation is the most common post-translational modification and plays an important role in regulation of protein, particularly in cell signaling (Prabakaran et al., (2012) Wiley Interdiscip Rev Syst Biol Med 4: 565-583). The addition of sugars to proteins, such as glycosylation, has been shown to promote protein folding, improve stability, and modify regulatory function. The attachment of lipids to proteins enables targeting to the cell membrane. A post-translational

modification can also include modifications to include one or more detectable labels.

In certain embodiments, the polypeptide can be fragmented. For example, the fragmented polypeptide can be obtained by fragmenting a polypeptide, protein or protein complex from a sample, such as a biological sample. The polypeptide, protein or protein complex can be fragmented by any means known in the art, including fragmentation by a protease or endopeptidase. In some embodiments, fragmentation of a polypeptide, protein or protein complex is targeted by use of a specific protease or endopeptidase. A specific protease or endopeptidase binds and cleaves at a specific consensus sequence (*e.g.*, TEV protease which is specific for ENLYFQ\S consensus sequence). In other embodiments, fragmentation of a peptide, polypeptide, or protein is non-targeted or random by use of a non-specific protease or endopeptidase. A non-specific protease may bind and cleave at a specific amino acid residue rather than a consensus sequence (*e.g.*, proteinase K is a non-specific serine protease). Proteinases and endopeptidases are well known in the art, and examples of such that can be used to cleave a protein or polypeptide into smaller peptide fragments include proteinase K, trypsin, chymotrypsin, pepsin, thermolysin, thrombin, Factor Xa, furin, endopeptidase, papain, pepsin, subtilisin, elastase, enterokinase, Genenase™ I, Endoproteinase LysC, Endoproteinase AspN, Endoproteinase GluC, etc. (Granvogl et al., (2007) Anal Bioanal Chem 389: 991-1002). In certain embodiments, a peptide, polypeptide, or protein is fragmented by proteinase K, or optionally, a thermolabile version of proteinase K to enable rapid inactivation. Proteinase K is quite stable in denaturing reagents, such as urea and SDS, enabling digestion of completely denatured proteins.

In some embodiments, the polypeptide is contacted with one or more enzymes in addition to a modified dipeptide cleavase to eliminate the NTAA (*e.g.,* a proline aminopeptidase to remove an N-terminal proline, if present). In some embodiments, the additional enzyme eliminates an NTAA from the polypeptide that is a proline. In some specific examples, the enzyme is a proline aminopeptidase, a proline iminopeptidase (PIP), or a pyroglutamate aminopeptidase (pGAP). In some embodiments, one or more modified dipeptide cleavases are used in combination with other enzymes to treat the polypeptides. In some embodiments, the polypeptide is first contacted with a proline aminopeptidase under conditions suitable to remove an N-terminal proline, if present.

Chemical reagents can also be used to digest proteins into peptide fragments. A chemical reagent may cleave at a specific amino acid residue (*e.g.*, cyanogen bromide hydrolyzes peptide bonds at the C-terminus of methionine residues). Chemical reagents for fragmenting polypeptides or proteins into smaller peptides include cyanogen bromide (CNBr), hydroxylamine, hydrazine, formic acid, BNPS-skatole [2-(2-nitrophenylsulfenyl)-3-methylindole], iodosobenzoic acid, •NTCB +Ni (2-nitro-5-thiocyanobenzoic acid), etc.

In certain embodiments, some polypeptides can be treated with a reagent for enzymatic or chemical elimination. In certain embodiments, following enzymatic or chemical elimination, the resulting polypeptide fragments are approximately the same desired length, *e.g.,* from about 10 amino acids to about 70 amino acids, from about 10 amino acids to about 60 amino acids, from about 10 amino acids to about 50 amino acids, about 10 to about 40 amino acids, from about 10 to about 30 amino acids, from about 20 amino acids to about 70 amino acids, from about 20 amino acids to about 60 amino acids, from about 20 amino acids to about 50 amino acids, about 20 to about 40 amino acids, from about 20 to about 30 amino acids, from about 30 amino acids to about 70 amino acids, from about 30 amino acids to about 60 amino acids, from about 30 amino acids to about 50 amino acids, or from about 30 amino acids to about 40 amino acids. A elimination reaction may be monitored, preferably in real time, by spiking the protein or polypeptide sample with a short test FRET (fluorescence resonance energy transfer) polypeptide comprising a peptide sequence containing a proteinase or endopeptidase elimination site. In the intact FRET peptide, a fluorescent group and a quencher group are attached to either end of the peptide sequence containing the elimination site, and fluorescence resonance energy transfer between the quencher and the fluorophore leads to low fluorescence. Upon elimination of the test peptide by a protease or endopeptidase, the quencher and fluorophore are separated giving a large increase in fluorescence. An elimination reaction can be stopped when a certain fluorescence intensity is achieved, allowing a reproducible elimination end point to be achieved.

### A. Providing the Polypeptide Joined to a Support or in Solution

In some embodiments, polypeptides of the present disclosure are joined to a surface of a solid support (also referred to as "substrate surface"). In some cases, the polypeptides are joined to a solid support prior to contacting with the modified dipeptide cleavase. In some cases, the modified dipeptide cleavase removes a labeled terminal amino acid from a polypeptide that is join (directly or indirectly) to a solid support. In some embodiments, the labeled terminal amino acid is removed as a single amino acid or as part of a dipeptide.

The solid support can be any porous or non-porous support surface including, but not limited to, a bead, a microbead, an array, a glass surface, a silicon surface, a plastic surface, a filter, a membrane, a PTFE membrane, a PTFE membrane, a nitrocellulose membrane, a nitrocellulose-based polymer surface, nylon, a silicon wafer chip, a flow cell, a flow through chip, a biochip including signal transducing electronics, a microtiter well, an ELISA plate, a spinning interferometry disc, a nitrocellulose membrane, a nitrocellulose-based polymer surface, a nanoparticle, or a microsphere. Materials for a solid support include but are not limited to acrylamide, agarose, cellulose, dextran, nitrocellulose, glass,

gold, quartz, polystyrene, polyethylene vinyl acetate, polypropylene, polyester, polymethacrylate, polyacrylate, polyethylene, polyethylene oxide, polysilicates, polycarbonates, poly vinyl alcohol (PVA), Teflon, fluorocarbons, nylon, silicon rubber, polyanhydrides, polyglycolic acid, polyvinylchloride, polylactic acid, polyorthoesters, functionalized silane, polypropylfumerate, collagen, glycosaminoglycans, polyamino acids, or any combination thereof. Solid supports further include thin film, membrane, bottles, dishes, fibers, woven fibers, shaped polymers such as tubes, particles, beads, microparticles, or any combination thereof. For example, when solid surface is a bead, the bead can include, but is not limited to, a polystyrene bead, a polymer bead, a polyacrylate bead, a methylstyrene bead, an agarose bead, a cellulose bead, a dextran bead, an acrylamide bead, a solid core bead, a porous bead, a paramagnetic bead, glass bead, a controlled pore bead, a silica-based bead, or any combinations thereof.

In certain embodiments, a solid support is a bead, which may refer to an individual bead or a plurality of beads. In some embodiments, the bead is compatible with a selected next generation sequencing platform that will be used for downstream analysis (*e.g.*, SOLiD or 454). In some embodiments, a solid support is an agarose bead, a paramagnetic bead, a polystyrene bead, a polymer bead, an acrylamide bead, a solid core bead, a porous bead, a glass bead, or a controlled pore bead. In further embodiments, a bead may be coated with a binding functionality (*e.g.*, amine group, affinity ligand such as streptavidin for binding to biotin labeled polypeptide, antibody) to facilitate binding to a polypeptide.

Proteins, polypeptides, or peptides can be joined to the solid support, directly or indirectly, by any means known in the art, including covalent and non-covalent interactions, or any combination thereof (*see, e.g.,* Chan et al., 2007, PLoS One 2:e1164; Cazalis et al., Bioconj. Chem. 15:1005-1009; Soellner et al., 2003, J. Am. Chem. Soc. 125:11790-11791; Sun et al., 2006, Bioconjug. Chem. 17-52-57; Decreau et al., 2007, J. Org. Chem. 72:2794-2802; Camarero et al., 2004, J. Am. Chem. Soc. 126:14730-14731; Girish et al., 2005, Bioorg. Med. Chem. Lett. 15:2447-2451; Kalia et al., 2007, Bioconjug. Chem. 18:1064-1069; Watzke et al., 2006, Angew Chem. Int. Ed. Engl. 45:1408-1412; Parthasarathy et al., 2007, Bioconjugate Chem. 18:469-476; and Bioconjugate Techniques, G. T. Hermanson, Academic Press (2013)). For example, the peptide may be joined to the solid support by a ligation reaction. Alternatively, the solid support can include an agent or coating to facilitate joining, either direct or indirectly, the peptide to the solid support. Any suitable molecule or materials may be employed for this purpose, including proteins, nucleic acids, carbohydrates and small molecules. For example, in one embodiment the agent is an affinity molecule. In another example, the agent is an azide group, which group can react with an alkynyl group in another molecule to facilitate association or binding between the solid support and the other molecule.

Proteins, polypeptides, or peptides can be joined to the solid support using methods referred to as "click chemistry." For this purpose, any reaction which is rapid and substantially irreversible can be used to attach proteins, polypeptides, or peptides to the solid support. Exemplary reactions include the copper catalyzed reaction of an azide and alkyne to form a triazole (Huisgen 1, 3-dipolar cycloaddition), strain-promoted azide alkyne cycloaddition (SPAAC), reaction of a diene and dienophile (Diels-Alder), strain-promoted alkyne-nitrone cycloaddition, reaction of a strained alkene with an azide, tetrazine or tetrazole, alkene and azide [3+2] cycloaddition, alkene and tetrazine inverse electron demand Diels-Alder (IEDDA) reaction (*e.g.*, m-tetrazine (mTet) or phenyl tetrazine (pTet) and trans-cyclooctene (TCO); or pTet and an alkene), alkene and tetrazole photoreaction, Staudinger ligation of azides and phosphines, and various displacement reactions, such as displacement of a leaving group by nucleophilic attack on an electrophilic atom (Horisawa, Front Physiol (2014). 5: 457; Knall, Hollauf et al., Tetrahedron Lett (2014) 55(34): 4763-4766). Exemplary displacement reactions include reaction of an amine with: an activated ester; an N-hydroxysuccinimide ester; an isocyanate; an isothioscyanate, an aldehyde, an epoxide, or the like.

In some embodiments, the polypeptide and solid support are joined by a functional group capable of formation by reaction of two complementary reactive groups, for example a functional group which is the product of one of the foregoing "click" reactions. In various embodiments, functional group can be formed by reaction of an aldehyde, oxime, 97erivatiz, hydrazide, alkyne, amine, azide, acylazide, acylhalide, nitrile, nitrone, sulfhydryl, disulfide, sulfonyl halide, isothiocyanate, imidoester, activated ester (*e.g.,* N-hydroxysuccinimide ester, pentynoic acid STP ester), ketone, $\alpha$,$\beta$-unsaturated carbonyl, alkene, maleimide, $\alpha$-haloimide, epoxide, aziridine, tetrazine, tetrazole, phosphine, biotin or thiirane functional group with a complementary reactive group. An exemplary reaction is a reaction of an amine (*e.g.*, primary amine) with an N-hydroxysuccinimide ester or isothiocyanate.

In some embodiments, the functional group comprises an alkene, ester, amide, thioester, disulfide, carbocyclic, heterocyclic or heteroaryl group. In further embodiments, the functional group comprises an alkene, ester, amide, thioester, thiourea, disulfide, carbocyclic, heterocyclic or heteroaryl group. In other embodiments, the functional group comprises an amide or thiourea. In some more specific embodiments, functional group is a triazolyl functional group, an amide, or thiourea functional group.

In some embodiments, iEDDA click chemistry is used for immobilizing polypeptides to a solid support since it is rapid and delivers high yields at low input concentrations. In another embodiment, m-tetrazine rather than tetrazine is used in an iEDDA click chemistry reaction, as m-tetrazine has improved bond stability. In another embodiment, phenyl tetrazine (pTet) is used in an iEDDA click chemistry reaction.

In some embodiments, the substrate surface is functionalized with TCO, and the recording tag-labeled protein,

polypeptide, peptide is immobilized to the TCO coated substrate surface via an attached m-tetrazine moiety.

In some embodiments, polypeptides are immobilized to a surface of a solid support by its C-terminus, N-terminus, or an internal amino acid, for example, via an amine, carboxyl, or sulfydryl group. Standard activated supports used in coupling to amine groups include CNBr-activated, NHS-activated, aldehyde-activated, azlactone-activated, and CDI-activated supports. Standard activated supports used in carboxyl coupling include carbodiimide-activated carboxyl moieties coupling to amine supports. Cysteine coupling can employ maleimide, idoacetyl, and pyridyl disulfide activated supports. An alternative mode of peptide carboxy terminal immobilization uses anhydrotrypsin, a catalytically inert derivative of trypsin that binds peptides containing lysine or arginine residues at their C-termini without cleaving them.

In certain embodiments, a polypeptide is immobilized to a solid support via covalent attachment of a solid surface bound linker to a lysine group of the protein, polypeptide, or peptide.

In certain embodiments, a polypeptide is first labeled with a DNA tag, and the chimeric DNA-polypeptide molecule is immobilized to a solid support via nucleic acid hybridization and ligation to a DNA sequence attached to the solid support. In some embodiments, protein and polypeptide fragmentation into peptides can be performed before or after attachment of a DNA tag or DNA recording tag.

### B. Optional Processing of Polypeptides

A sample of polypeptides can undergo protein fractionation methods prior to attachment to a solid support, where proteins or peptides are separated by one or more properties such as cellular location, molecular weight, hydrophobicity, or isoelectric point, or protein enrichment methods. Alternatively, or additionally, protein enrichment methods may be used to select for a specific protein or peptide (*see, e.g.,* Whiteaker et al., (2007) Anal. Biochem. 362:44-54) or to select for a particular post translational modification (*see, e.g.,* Huang et al., (2014) J. Chromatogr. A 1372:1-17). Alternatively, a particular class or classes of proteins such as immunoglobulins, or immunoglobulin (Ig) isotypes such as IgG, can be affinity enriched or selected for analysis. In the case of immunoglobulin molecules, analysis of the sequence and abundance or frequency of hypervariable sequences involved in affinity binding are of particular interest, particularly as they vary in response to disease progression or correlate with healthy, immune, and/or disease phenotypes. Overly abundant proteins can also be subtracted from the sample using standard immunoaffinity methods. Depletion of abundant proteins can be useful for plasma samples where over 80% of the protein constituent is albumin and immunoglobulins. Several commercial products are available for depletion of plasma samples of overly abundant proteins, such as PROTIA and PROT20 (Sigma-Aldrich).

In some embodiments, the methods provided herein may be performed on polypeptides that have been normalized. In some embodiments, subtraction of certain protein species (*e.g.*, highly abundant proteins) from the sample is performed. This can be accomplished, for example, using commercially available protein depletion reagents such as Sigma's PROT20 immuno-depletion kit, which deplete the top 20 plasma proteins. Additionally, it would be useful to have an approach that greatly reduced the dynamic range even further to a manageable 3-4 orders. In certain embodiments, a protein sample dynamic range can be modulated by fractionating the protein sample using standard fractionation methods, including electrophoresis and liquid chromatography (Zhou et al., Anal Chem (2012) 84(2): 720-734), or partitioning the fractions into compartments (e.g., droplets) loaded with limited capacity protein binding beads/resin (e.g. hydroxylated silica particles) (McCormick, Anal Biochem (1989) 181(1): 66-74) and eluting bound protein. Excess protein in each compartmentalized fraction is washed away.

Examples of electrophoretic methods include capillary electrophoresis (CE), capillary isoelectric focusing (CIEF), capillary isotachophoresis (CITP), free flow electrophoresis, gel-eluted liquid fraction entrapment electrophoresis (GELFrEE). Examples of liquid chromatography protein separation methods include reverse phase (RP), ion exchange (IE), size exclusion (SE), hydrophilic interaction, etc. Examples of compartment partitions include emulsions, droplets, microwells, physically separated regions on a flat substrate, etc. Exemplary protein binding beads/resins include silica nanoparticles derivatized with phenol groups or hydroxyl groups (*e.g.*, StrataClean Resin from Agilent Technologies, RapidClean from LabTech, etc.). By limiting the binding capacity of the beads/resin, highly-abundant proteins eluting in a given fraction will only be partially bound to the beads, and excess proteins removed.

## III. EXEMPLARY USE OF MODIFIED DIPEPTIDE CLEAVASE AND RELATED METHODS

Provided herein is a method of treating one or more polypeptides comprising contacting the polypeptide with a modified dipeptide cleavase. In some embodiments, the modified dipeptide cleavase comprises a mutation, *e.g.*, one or more amino acid modifications in an unmodified dipeptide cleavase, wherein the modified dipeptide cleavase removes a labeled terminal dipeptide from a polypeptide. In some embodiments, polypeptides are contacted with any one or more of the modified dipeptide cleavases as described in Section I. In some embodiments, the method further comprises contacting the polypeptide with a reagent for labeling the terminal amino acid. In some embodiments, the contacting with the reagent for labeling the terminal amino acid is with any one or more of the reagents described in Section I.A. In

some embodiments, one or more cycles of contacting the polypeptide with the modified dipeptide cleavase and contacting with a reagent to label the terminal amino acid is performed, such as in a cyclic manner as depicted in **FIG. 2A-2C and Fig. 9.** In some embodiments, the polypeptide is bound to a support. In some embodiments, the method includes joining the polypeptides to a solid support (*e.g.*, directly or indirectly). In some embodiments, the removal of NTAA as part of a dipeptide from a polypeptide using the provided modified dipeptide cleavases can be combined with a chemical method for removing the NTAA from a peptide, such as described in PCT publication number WO 2019/089846.

In some embodiments, the modified dipeptide cleavases provided herein can be used for treating polypeptides to be analyzed and/or sequenced. In some embodiments, the methods are for determining the sequence of at least a portion of the polypeptide. In some embodiments, the provided methods can be used in the context of a degradation-based polypeptide sequencing assay. In some cases, the method may include performing any of the methods as described in International Patent Publication No. WO 2017/192633. In some cases, the sequence of the polypeptide is analyzed by construction of an extended recording tag (*e.g.*, DNA sequence) representing the polypeptide sequence, such as an extended recording tag. In some cases, the methods provided herein apply to or can be used in combination with a ProteoCode™ assay. In some embodiments employing a cyclic degradation-based polypeptide analysis method, the provided modified dipeptide cleavase provides certain advantages. For example, the recognition and removal of labeled amino acids as dipeptides may provide a pause to amino acid removal as compared to an enzyme which removes unlabeled dipeptides, which may continuously remove amino acids from the polypeptide before other steps of the assay can be performed (*e.g.*, binding of the NTAA by a binding agent and recording information of the NTAA to a recording tag). Thus, in some cases, by recognizing and removing labeled dipeptides, the modified dipeptide cleavase removes the NTAA (as part of a dipeptide) only after a labeling step has occurred. Thereby, the modified dipeptide cleavase provides control over the removal of dipeptides (containing a labeled amino acid) compared to the unmodified dipeptide cleavase which removes unlabeled dipeptides.

In some embodiments, a method comprising the modified dipeptide cleavase is conducted in the absence of a condition that degrades nucleic acids (*e.g.,* DNA, such as a recording tag). In some embodiments, the method comprising the modified dipeptide cleavase is conducted in the absence of a chemical condition that degrades nucleic acids. In some embodiments, the method comprising the modified dipeptide cleavase is conducted in conditions compatible with a degradation-based polypeptide sequencing assay (*e.g.*, the methods as described in International Patent Publication No. WO 2017/192633). In some cases, the method comprising the modified dipeptide cleavase is conducted in the presence of conditions compatible with nucleic acids. In some embodiments, the method comprising the modified dipeptide cleavase is conducted in the absence of a strong acid or a strong base. In some aspects, the strong acid is a strong anhydrous acid. In some examples, the method comprising the modified dipeptide cleavase is conducted in the absence of anhydrous TFA.

In some embodiments, the method includes contacting the polypeptide with more than one modified dipeptide cleavase. In some cases, various modified dipeptide cleavases may exhibit different characteristics, for example, binding preferences for polypeptides and/or differences in cleaving dipeptides. In some embodiments, different modified dipeptide cleavases may be used in any of the described methods, as a mixture of enzymes or each separately. In some embodiments, the different modified dipeptide cleavases are contacted with polypeptides simultaneously or sequentially.

In some embodiments, the polypeptide is contacted with one or more additional enzymes to eliminate the NTAA (*e.g.,* a proline aminopeptidase to remove an N-terminal proline, if present). The methods of the invention may include optionally treating the polypeptides with an enzyme to remove one or more NTAAs (*e.g.*, proline aminopeptidase) before, during, or after treatment with any of the provided chemical reagents for labeling the NTAA. The methods of the invention may include optionally treating the polypeptides with an enzyme to remove one or more NTAAs (*e.g.*, proline aminopeptidase) before, during, or after treatment with any of the provided modified dipeptide cleavases. In some embodiments, the enzyme eliminates an NTAA from the polypeptide that is a proline. In some specific examples, the enzyme is a proline aminopeptidase, a proline iminopeptidase (PIP), or a pyroglutamate aminopeptidase (pGAP). In some embodiments, one or more modified dipeptide cleavases are used in combination with other enzymes to treat the polypeptides. In some specific cases, the modified dipeptide cleavase and/or other enzymes are provided as a cocktail.

In some embodiments, the method further comprises contacting the polypeptide with one or more binding agents capable of binding to the terminal amino acid of the polypeptide, wherein each binding agent comprises a coding tag with identifying information regarding the binding agent. In some cases, the binding agent may bind to a labeled terminal amino acid of the polypeptide. In some further embodiments, the method further comprises transferring the identifying information of the coding tag to a recording tag attached to the polypeptide, thereby generating an extended recording tag on the polypeptide. In some particular embodiments, the method further comprises removing or releasing the one or more binding agents from the polypeptide.

In some embodiments, one or more steps of contacting the polypeptide with various reagents, including for example, contacting with the modified dipeptide cleavase, with the reagent to label the terminal amino acid, and/or with binding reagent(s), is repeated in a cyclic manner. In some embodiments, provided is a method for analyzing a polypeptide, comprising the steps of: (a) contacting a polypeptide with a binding agent capable of binding to the terminal amino acid of the polypeptide, wherein each binding agent comprises a coding tag with identifying information regarding

the binding agent; (b) transferring the identifying information of the coding tag to a recording tag associated with each of the polypeptides to generate an extended recording tag; (c) contacting the polypeptide with a reagent to label the terminal amino acid of the polypeptide; and (d) contacting the polypeptide with a modified dipeptide cleavase comprising a mutation, *e.g.*, one or more amino acid modifications in an unmodified dipeptide cleavase, whereby the modified dipeptide cleavase removes a terminal dipeptide labeled by the reagent in step (c) from the polypeptide. In some embodiments, steps (a)-(d) are repeated for "n" binding cycles, wherein the information of each coding tag of each binding agent that binds to the polypeptide is transferred to the extended recording tag generated from the previous binding cycle to generate an nth order extended recording tag. In some embodiments, the method further comprises (b 1) removing or releasing the one or more binding agents from the plurality of polypeptides. In some examples, the polypeptide is contacted with the reagent to label the terminal amino acid of the polypeptide prior to contacting the polypeptide with the modified dipeptide cleavase. In some embodiments, the polypeptides includes a plurality of polypeptides. In some embodiments, the polypeptide is contacted with a plurality of binding agents. In some embodiments, the polypeptide is contacted with two or more binding agents.

In some examples, step (a) is performed before step (b); step (a) is performed before step (c); step (a) is performed before step (d); step (b) is performed before step (c); step (b) is performed before step (d); step (c) is performed before step (a); step (c) is performed before step (b); and/or step (c) is performed before step (d). In some particular embodiments, the steps are performed in the order: (a), (b), (c), and (d). In some particular embodiments, the steps are performed in the order: (c), (a), (b), and (d). In some embodiments, the method further comprises (e) analyzing the nth order extended recording tag. In some embodiments, the method further comprises removing the one or more binding agents. In some embodiments, step (b1) is performed after step (a); step (b1) is performed after step (b); step (b1) is performed before step (c); and/or step (b1) is performed before step (d).

In an exemplary workflow, the treatment and analysis of the polypeptides is as follows: a large collection of polypeptides (*e.g.,* 50 million - 1 billion or more) from a proteolytic digest are immobilized randomly on a single molecule sequencing substrate (*e.g.*, beads) at an appropriate intramolecular spacing. In some cases, the polypeptides are attached to recording tags. In a cyclic manner, the terminal amino acid (*e.g.*, N-terminal amino acid) of each peptide is labeled (*e.g.,* PTC, modified-PTC, Cbz, DNP, SNP, acetyl, guanidinyl, amino guanidinyl, heterocyclic methanimine). In some cases, the labeling of the terminal amino acid can be performed as a later step. The labeled N-terminal amino acid (*e.g.*, PITC-NTAA, Cbz-NTAA, DNP-NTAA, SNP-NTAA, acetyl-NTAA, guanidinylated-NTAA, heterocyclic methanimine-NTAA) of each immobilized peptide is bound by the cognate NTAA binding agent which is attached to a coding tag, and information from the coding tag associated with the bound NTAA binding agent is transferred to the recording tag associated with the immobilized peptide, thereby generating an extended recording tag. In some embodiments, the one or more bindings agents is removed or released from the polypeptides. The labeled NTAA is removed as a dipeptide by contacting with a modified dipeptide cleavase. One or more cycles of the labeling, contacting with the binding agent, transferring identifying information, and removal of the labeled dipeptide can be performed.

In some examples, the final extended recording tag is optionally flanked by universal priming sites to facilitate downstream amplification and/or DNA sequencing. The forward universal priming site (*e.g.*, Illumina's P5-S1 sequence) can be part of the original recording tag design and the reverse universal priming site (*e.g.*, Illumina's P7-S2' sequence) can be added as a final step in the extension of the recording tag. In some embodiments, the addition of forward and reverse priming sites can be done independently of a binding agent.

In some embodiments, the order of the steps in the process for a degradation-based peptide or polypeptide sequencing assay can be reversed or be performed in various orders. For example, in some embodiments, the terminal amino acid labeling can be conducted before and/or after the polypeptide is bound to the binding agent. In some embodiments, contacting with the one or more binding agents is before contacting the polypeptide with the reagent for labeling the terminal amino acid. In some cases, contacting with the one or more binding agents is before contacting the polypeptide with the modified dipeptide cleavase to remove the labeled terminal amino acid.

In some embodiments, the terminal amino acid labeling can be conducted before or after the polypeptide is bound to a support. In some embodiments, the terminal amino acid removal can be conducted before and/or after the polypeptide is bound to the binding agent. In some embodiments, the contacting of the polypeptides with the reagent for labeling the terminal amino acid is before the contacting with the binding agent and the contacting with the one or more binding agents is before the contacting of the polypeptides with the modified dipeptide cleavase. In some embodiments, transferring of the identifying information is performed after the contacting of the polypeptide with the one or more binding agents and before the contacting of the polypeptide with the modified dipeptide cleavase.

In some of any such embodiments, removing the one or more binding agents is after the transferring of identifying information from the coding tag to a recording tag associated with each of the polypeptides to generate an extended recording tag. In some of any such embodiments, removing the one or more binding agents is before contacting the polypeptides with a reagent to label the terminal amino acid of the polypeptide. In some embodiments, removing the one or more binding agents is before contacting the polypeptide with a modified dipeptide cleavase.

In some embodiments, the order of any of the steps of the provided methods for treating the proteins or

polypeptides can be reversed or be performed in various orders.

A. **Attaching Recording Tags to Polypeptides**

In some embodiments, the methods provided comprise contacting polypeptides with the modified dipeptide cleavase and optionally other reagents for polypeptide analysis. In one embodiment, the protein or polypeptide is labeled with DNA recording tags through standard amine coupling chemistries. The ε-amino group (*e.g.,* of lysine residues) and the N-terminal amino group are particularly susceptible to labeling with amine-reactive coupling agents, depending on the pH of the reaction (Mendoza et al., Mass Spectrom Rev (2009) 28(5): 785-815). In a particular embodiment, the recording tag is comprised of a reactive moiety (*e.g.,* for conjugation to a solid surface, a multifunctional linker, or a polypeptide), a linker, a universal priming sequence, a barcode (*e.g.*, compartment tag, partition barcode, sample barcode, fraction barcode, or any combination thereof), an optional UMI, and a spacer (Sp) sequence for facilitating information transfer to/from a coding tag. In some cases, wherein ligation is used, the Sp sequence can serve as an overhang of 1-8 bases. In some cases, the recording tag does not include a spacer. In another embodiment, the protein can be first labeled with a universal DNA tag, and the barcode-Sp sequence (representing a sample, a compartment, a physical location on a slide, etc.) are attached to the protein later through and enzymatic or chemical coupling step. A universal DNA tag comprises a short sequence of nucleotides that are used to label a polypeptide and can be used as point of attachment for a barcode (*e.g.,* compartment tag, recording tag, etc.). For example, a recording tag may comprise at its terminus a sequence complementary to the universal DNA tag. In certain embodiments, a universal DNA tag is a universal priming sequence. Upon hybridization of the universal DNA tags on the labeled protein to complementary sequence in recording tags (*e.g.,* bound to beads), the annealed universal DNA tag may be extended via primer extension, transferring the recording tag information to the DNA tagged protein. In a particular embodiment, the protein is labeled with a universal DNA tag prior to proteinase digestion into peptides. The universal DNA tags on the labeled peptides from the digest can then be converted into an informative and effective recording tag. In some embodiments, protein and polypeptide fragmentation into peptides can be performed before or after attachment of a DNA tag or DNA recording tag.

At least one recording tag is associated or co-localized directly or indirectly with the polypeptide and joined to the solid support. A recording tag may comprise DNA, RNA, or polynucleotide analogs including PNA, gPNA, GNA, HNA, BNA, XNA, TNA, or a combination thereof. A recording tag may be single stranded, or partially or completely double stranded. A recording tag may have a blunt end or overhanging end. In certain embodiments, upon binding of a binding agent to a polypeptide, identifying information of the binding agent's coding tag is transferred to the recording tag to generate an extended recording tag. Further extensions to the extended recording tag can be made in subsequent binding cycles.

A recording tag can be joined to the solid support, directly or indirectly (*e.g.*, via a linker), by any means known in the art, including covalent and non-covalent interactions, or any combination thereof. For example, the recording tag may be joined to the solid support by a ligation reaction. Alternatively, the solid support can include an agent or coating to facilitate joining, either direct or indirectly, of the recording tag, to the solid support. Strategies for immobilizing nucleic acid molecules to solid supports (*e.g.*, beads) have been described in U.S. Patent 5,900,481; Steinberg et al. (2004) Biopolymers 73:597-605; Lund et al., (1988) Nucleic Acids Res. 16: 10861-10880).

In certain embodiments, the co-localization of a polypeptide and associated recording tag is achieved by conjugating polypeptide and recording tag to a bifunctional linker attached directly to the solid support surface (Steinberg et al. (2004) Biopolymers 73:597-605). In further embodiments, a trifunctional moiety is used to derivatize the solid support (*e.g.*, beads), and the resulting bifunctional moiety is coupled to both the polypeptide and recording tag. In other embodiments, the co-localization of a polypeptide and associated recording tag is achieved by coupling the polypeptide to the associated DNA recording tag and ligating the chimera to a DNA decorated solid support surface.

Methods and reagents (*e.g.*, click chemistry reagents and photoaffinity labelling reagents) such as those described for attachment of polypeptides and solid supports, may also be used for attachment of recording tags.

In a particular embodiment, a single recording tag is attached to a polypeptide, preferably via the attachment to a de-blocked N- or C-terminal amino acid. In another embodiment, multiple recording tags are attached to the polypeptide, preferably to the lysine residues or peptide backbone. In some embodiments, a polypeptide labeled with multiple recording tags is fragmented or digested into smaller peptides, with each peptide labeled on average with one recording tag.

In certain embodiments, a polypeptide is first labeled with a DNA recording tag, and the chimeric DNA-polypeptide molecule is immobilized to a solid support via nucleic acid hybridization and ligation to a DNA sequence attached to the solid support.

In certain embodiments, a recording tag comprises an optional, unique molecular identifier (UMI), which provides a unique identifier tag for each polypeptide to which the UMI is associated with. A UMI can be about 3 to about 40 bases, or a subrange thereof, *e.g.*, about 3 to about 30 bases, about 3 to about 20 bases, or about 3 to about 10 bases, or about 3 to about 8 bases. In some embodiments, a UMI is about 3 bases, 4 bases, 5 bases, 6 bases, 7 bases, 8 bases, 9 bases, 10 bases, 11 bases, 12 bases, 13 bases, 14 bases, 15 bases, 16 bases, 17 bases, 18 bases, 19 bases, 20 bases, 25 bases, 30

bases, 35 bases, or 40 bases in length. A UMI can be used to de-convolute sequencing data from a plurality of extended recording tags to identify sequence reads from individual polypeptides. In some embodiments, within a library of polypeptides, each polypeptide is associated with a single recording tag, with each recording tag comprising a unique UMI. In other embodiments, multiple copies of a recording tag are associated with a single polypeptide, with each copy of the recording tag comprising the same UMI. In some embodiments, a UMI has a different base sequence than the spacer or encoder sequences within the binding agents' coding tags to facilitate distinguishing these components during sequence analysis.

In certain embodiments, a recording tag comprises a barcode, *e.g.*, other than the UMI if present. A barcode is a nucleic acid molecule of about 3 to about 30 bases, or a subrange thereof, *e.g.*, about 3 to about 25 bases, about 3 to about 20 bases, about 3 to about 10 bases, about 3 to about 10 bases, about 3 to about 8 bases in length. In some embodiments, a barcode is about 3 bases, 4 bases, 5 bases, 6 bases, 7 bases, 8 bases, 9 bases, 10 bases, 11 bases, 12 bases, 13 bases, 14 bases, 15 bases, 20 bases, 25 bases, or 30 bases in length. In one embodiment, a barcode allows for multiplex sequencing of a plurality of samples or libraries. A barcode may be used to identify a partition, a fraction, a compartment, a sample, a spatial location, or library from which the polypeptide derived. Barcodes can be used to de-convolute multiplexed sequence data and identify sequence reads from an individual sample or library. For example, a barcoded bead is useful for methods involving emulsions and partitioning of samples, *e.g.*, for purposes of partitioning the proteome.

A barcode can represent a compartment tag in which a compartment, such as a droplet, microwell, physical region on a solid support, etc. is assigned a unique barcode. The association of a compartment with a specific barcode can be achieved in any number of ways such as by encapsulating a single barcoded bead in a compartment, *e.g.*, by direct merging or adding a barcoded droplet to a compartment, by directly printing or injecting a barcode reagent to a compartment, etc. The barcode reagents within a compartment are used to add compartment-specific barcodes to the polypeptide or fragments thereof within the compartment. Applied to protein partitioning into compartments, the barcodes can be used to map analysed peptides back to their originating protein molecules in the compartment. This can greatly facilitate protein identification. Compartment barcodes can also be used to identify protein complexes.

In other embodiments, multiple compartments that represent a subset of a population of compartments may be assigned a unique barcode representing the subset.

Alternatively, a barcode may be a sample identifying barcode. A sample barcode is useful in the multiplexed analysis of a set of samples in a single reaction vessel or immobilized to a single solid substrate or collection of solid substrates (*e.g.*, a planar slide, population of beads contained in a single tube or vessel, etc.). Polypeptides from many different samples can be labeled with recording tags with sample-specific barcodes, and then all the samples pooled together prior to immobilization to a solid support, cyclic binding, and recording tag analysis. Alternatively, the samples can be kept separate until after creation of a DNA-encoded library, and sample barcodes attached during PCR amplification of the DNA-encoded library, and then mixed together prior to sequencing. This approach could be useful when assaying analytes (*e.g.*, proteins) of different abundance classes. For example, the sample can be split and barcoded, and one portion processed using binding agents to low abundance analytes, and the other portion processed using binding agents to higher abundance analytes. In a particular embodiment, this approach helps to adjust the dynamic range of a particular protein analyte assay to lie within the "sweet spot" of standard expression levels of the protein analyte.

In certain embodiments, polypeptides from multiple different samples are labeled with recording tags containing sample-specific barcodes. The multi-sample barcoded polypeptides can be mixed together prior to a cyclic binding reaction. In this way, a highly-multiplexed alternative to a digital reverse phase protein array (RPPA) is effectively created (Guo et al., Proteome Sci (2012) 10(1): 56; Assadi, Lamerz et al., Mol Cell Proteomics (2013) 12(9): 2615-2622; Akbani et al. 2014; Mol Cell Proteomics (2014) 13(7): 1625-1643; Creighton et al., Drug Des Devel Ther (2015) 9: 3519-3527). The creation of a digital RPPA-like assay has numerous applications in translational research, biomarker validation, drug discovery, clinical, and precision medicine.

In certain embodiments, a recording tag comprises a universal priming site, *e.g.,* a forward or 5' universal priming site. A universal priming site is a nucleic acid sequence that may be used for priming a library amplification reaction and/or for sequencing. A universal priming site may include, but is not limited to, a priming site for PCR amplification, flow cell adaptor sequences that anneal to complementary oligonucleotides on flow cell surfaces (*e.g.*, Illumina next generation sequencing), a sequencing priming site, or a combination thereof. A universal priming site can be about 10 bases to about 60 bases. In some embodiments, a universal priming site comprises an Illumina P5 primer (5'-AATGATACGGCGACC ACCGA-3' - SEQ ID NO:3) or an Illumina P7 primer (5'-CAAGCAGAAGACGGCATACGAGAT - 3' - SEQ ID NO:4).

In certain embodiments, a recording tag comprises a spacer at its terminus, *e.g.*, 3' end. As used herein reference to a spacer sequence in the context of a recording tag includes a spacer sequence that is identical to the spacer sequence associated with its cognate binding agent, or a spacer sequence that is complementary to the spacer sequence associated with its cognate binding agent. The terminal, *e.g.,* 3', spacer on the recording tag permits transfer of identifying information of a cognate binding agent from its coding tag to the recording tag during the first binding cycle (*e.g.,* via annealing of complementary spacer sequences for primer extension or sticky end ligation).

In one embodiment, the spacer sequence is about 1-20 bases in length or a subrange thereof, *e.g.*, about 2-12

bases in length, or 5-10 bases in length. The length of the spacer may depend on factors such as the temperature and reaction conditions of the primer extension reaction for transferring coding tag information to the recording tag. In some embodiments, the recording tag does not comprise a spacer.

In a preferred embodiment, the spacer sequence in the recording is designed to have minimal complementarity to other regions in the recording tag; likewise, the spacer sequence in the coding tag should have minimal complementarity to other regions in the coding tag. In other words, the spacer sequence of the recording tags and coding tags should have minimal sequence complementarity to components such unique molecular identifiers, barcodes (e.g., compartment, partition, sample, spatial location), universal primer sequences, encoder sequences, cycle specific sequences, etc. present in the recording tags or coding tags.

In some embodiments, the recording tags associated with a library of polypeptides share a common spacer sequence. In other embodiments, the recording tags associated with a library of polypeptides have binding cycle specific spacer sequences that are complementary to the binding cycle specific spacer sequences of their cognate binding agents, which can be useful when using non-concatenated extended recording tags.

In some cases, the collection of extended recording tags can be concatenated. For example, after the binding cycles are complete, the bead solid supports, each bead comprising on average one or fewer than one polypeptide per bead, each polypeptide having a collection of extended recording tags that are co-localized at the site of the polypeptide, are placed in an emulsion. The emulsion is formed such that each droplet, on average, is occupied by at most 1 bead. An optional assembly PCR reaction is performed in-emulsion to amplify the extended recording tags co-localized with the polypeptide on the bead and assemble them in co-linear order by priming between the different cycle specific sequences on the separate extended recording tags (Xiong et al., FEMS Microbiol Rev (2008) 32(3): 522-540). Afterwards the emulsion is broken and the assembled extended recording tags are sequenced.

In another embodiment, the DNA recording tag is comprised of a universal priming sequence (U1), one or more barcode sequences (BCs), and a spacer sequence (Sp1) specific to the first binding cycle. In the first binding cycle, binding agents employ DNA coding tags comprised of an Sp1 complementary spacer, an encoder barcode, and optional cycle barcode, and a second spacer element (Sp2). The utility of using at least two different spacer elements is that the first binding cycle selects one of potentially several DNA recording tags and a single DNA recording tag is extended resulting in a new Sp2 spacer element at the end of the extended DNA recording tag. In the second and subsequent binding cycles, binding agents contain just the Sp2' spacer rather than Sp1'. In this way, only the single extended recording tag from the first cycle is extended in subsequent cycles. In another embodiment, the second and subsequent cycles can employ binding agent specific spacers.

In some embodiments, a recording tag comprises from 5' to 3' direction: a universal forward (or 5') priming sequence, a UMI, and a spacer sequence. In some embodiments, a recording tag comprises from 5' to 3' direction: a universal forward (or 5') priming sequence, an optional UMI, a barcode (*e.g.*, sample barcode, partition barcode, compartment barcode, spatial barcode, or any combination thereof), and a spacer sequence. In some other embodiments, a recording tag comprises from 5' to 3' direction: a universal forward (or 5') priming sequence, a barcode (*e.g.*, sample barcode, partition barcode, compartment barcode, spatial barcode, or any combination thereof), an optional UMI, and a spacer sequence.

Combinatorial approaches may be used to generate UMIs from modified DNA and PNAs. In one example, a UMI may be constructed by "chemical ligating" together sets of short word sequences (4-15mers), which have been designed to be orthogonal to each other (Spiropulos and Heemstra 2012). A DNA template is used to direct the chemical ligation of the "word" polymers. The DNA template is constructed with hybridizing arms that enable assembly of a combinatorial template structure simply by mixing the sub-components together in solution. In certain embodiments, there are no "spacer" sequences in this design. The size of the word space can vary from 10's of words to 10,000's or more words or a subrange thereof. In certain embodiments, the words are chosen such that they differ from one another to not cross hybridize, yet possess relatively uniform hybridization conditions. In one embodiment, the length of the word will be on the order of 10 bases, with about 1000's words in the subset (this is only 0.1% of the total 10-mer word space ~ $4^{10}$ = 1 million words). Sets of these words (1000 in subset) can be concatenated together to generate a final combinatorial UMI with complexity = $1000^n$ power. For 4 words concatenated together, this creates a UMI diversity of $10^{12}$ different elements. These UMI sequences will be appended to the polypeptide at the single molecule level. In one embodiment, the diversity of UMIs exceeds the number of molecules of polypeptides to which the UMIs are attached. In this way, the UMI uniquely identifies the polypeptide of interest. The use of combinatorial word UMI's facilitates readout on high error rate sequencers, (e.g., nanopore sequencers, nanogap tunneling sequencing, etc.) since single base resolution is not required to read words of multiple bases in length. Combinatorial word approaches can also be used to generate other identity-informative components of recording tags or coding tags, such as compartment tags, partition barcodes, spatial barcodes, sample barcodes, encoder sequences, cycle specific sequences, and barcodes. Methods relating to nanopore sequencing and DNA encoding information with error-tolerant words (codes) are known in the art (see, e.g., Kiah et al., 2015, Codes for DNA sequence profiles. IEEE International Symposium on Information Theory (ISIT); Gabrys et al., 2015, Asymmetric Lee distance codes for DNA-based storage. IEEE Symposium on Information Theory (ISIT); Laure et al., 2016, Coding in 2D:

Using Intentional Dispersity to Enhance the Information Capacity of Sequence-Coded Polymer Barcodes. Angew. Chem. Int. Ed. doi:10.1002/anie.201605279; Yazdi et al., 2015, IEEE Transactions on Molecular, Biological and Multi-Scale Communications 1:230-248; and Yazdi et al., 2015, Sci Rep 5:14138). Thus, in certain embodiments, an extended recording tag, an extended coding tag, or a di-tag construct in any of the embodiments described herein is comprised of identifying components (*e.g.*, UMI, encoder sequence, barcode, compartment tag, cycle specific sequence, etc.) that are error correcting codes. In some embodiments, the error correcting code is selected from: Hamming code, Lee distance code, asymmetric Lee distance code, Reed-Solomon code, and Levenshtein-Tenengolts code. For nanopore sequencing, the current or ionic flux profiles and asymmetric base calling errors are intrinsic to the type of nanopore and biochemistry employed, and this information can be used to design more robust DNA codes using the aforementioned error correcting approaches. An alternative to employing robust DNA nanopore sequencing barcodes, one can directly use the current or ionic flux signatures of barcode sequences (U.S. Patent No. 7,060,507), avoiding DNA base calling entirely, and immediately identify the barcode sequence by mapping back to the predicted current/flux signature as described by Laszlo et al. (2014, Nat. Biotechnol. 32:829-833). For example, Laszlo et al. describe the current signatures generated by the biological nanopore, MspA, when passing different word strings through the nanopore, and the ability to map and identify DNA strands by mapping resultant current signatures back to an *in silico* prediction of possible current signatures from a universe of sequences (Laszlo et al., (2014) Nat. Biotechnol. 32:829-833). Similar concepts can be applied to DNA codes and the electrical signal generated by nanogap tunneling current-based DNA sequencing (Ohshiro et al., 2012, Sci Rep 2: 501).

Thus, in certain embodiments, the identifying components of a coding tag, recording tag, or both are capable of generating a unique current or ionic flux or optical signature, wherein the analysis step of any of the methods provided herein comprises detection of the unique current or ionic flux or optical signature in order to identify the identifying components. In some embodiments, the identifying components are selected from an encoder sequence, barcode, UMI, compartment tag, cycle specific sequence, or any combination thereof.

In certain embodiments, all or a substantial amount of the polypeptides (*e.g.*, at least 50%, 55%, 60%, 65%, 70%, 75%, 80%, 85%, 90%, 95%, 96%, 97%, 98%, 99%, or 100%) within a sample are labeled with a recording tag. Attaching of the recording tag to the polypeptides may occur before or after immobilization of the polypeptides to a solid support.

In other embodiments, a subset of polypeptides within a sample are labeled with recording tags. In a particular embodiment, a subset of polypeptides from a sample undergo targeted (analyte specific) labeling with recording tags. Targeted recording tag labeling of proteins may be achieved using target protein-specific binding agents (*e.g.*, antibodies, aptamers, etc.) that are linked a short target-specific DNA capture probe, *e.g.*, analyte-specific barcode, which anneal to complementary target-specific bait sequence, *e.g.*, analyte-specific barcode, in recording tags. The recording tags comprise a reactive moiety for a cognate reactive moiety present on the target protein (*e.g.,* click chemistry labeling, photoaffinity labeling). For example, recording tags may comprise an azide moiety for interacting with alkyne-derivatized proteins, or recording tags may comprise a benzophenone for interacting with native proteins, etc. Upon binding of the target protein by the target protein specific binding agent, the recording tag and target protein are coupled via their corresponding reactive. After the target protein is labeled with the recording tag, the target-protein specific binding agent may be removed by digestion of the DNA capture probe linked to the target-protein specific binding agent. For example, the DNA capture probe may be designed to contain uracil bases, which are then targeted for digestion with a uracil-specific excision reagent (*e.g.*, USER™), and the target-protein specific binding agent may be dissociated from the target protein.

In one example, antibodies specific for a set of target proteins can be labeled with a DNA capture probe that hybridizes with recording tags designed with complementary bait sequence. Sample-specific labeling of proteins can be achieved by employing DNA-capture probe labeled antibodies hybridizing with complementary bait sequence on recording tags comprising of sample-specific barcodes.

In another example, target protein-specific aptamers are used for targeted recording tag labeling of a subset of proteins within a sample. A target specific-aptamer is linked to a DNA capture probe that anneals with complementary bait sequence in a recording tag. The recording tag comprises a reactive chemical or photo-reactive chemical probes (*e.g.* benzophenone (BP)) for coupling to the target protein having a corresponding reactive moiety. The aptamer binds to its target protein molecule, bringing the recording tag into close proximity to the target protein, resulting in the coupling of the recording tag to the target protein.

Photoaffinity (PA) protein labeling using photo-reactive chemical probes attached to small molecule protein affinity ligands has been previously described (Park, Koh et al. 2016). Typical photo-reactive chemical probes include probes based on benzophenone (reactive diradical, 365 nm), phenyldiazirine (reactive carbon, 365 nm), and phenylazide (reactive nitrene free radical, 260 nm), activated under irradiation wavelengths as previously described (Smith et al., Future Med Chem. (2015) 7(2): 159-183). In a preferred embodiment, target proteins within a protein sample are labeled with recording tags comprising sample barcodes using the method disclosed by Li et al., in which a bait sequence in a benzophenone labeled recording tag is hybridized to a DNA capture probe attached to a cognate binding agent (*e.g.,* nucleic acid aptamer (Li et al., Angew Chem Int Ed Engl (2013) 52(36): 9544-9549). For photoaffinity labeled protein targets, the use of DNA/RNA aptamers as target protein-specific binding agents are preferred over antibodies since the

photoaffinity moiety can self-label the antibody rather than the target protein. In contrast, photoaffinity labeling is less efficient for nucleic acids than proteins, making aptamers a better vehicle for DNA-directed chemical or photo-labeling. Similar to photo-affinity labeling, one can also employ DNA-directed chemical labeling of reactive lysine's (or other moieties) in the proximity of the aptamer binding site in a manner similar to that described by Rosen et al. (Rosen et al, Nature Chemistry volume (2014) 6:804-809; Kodal et al., ChemBioChem (2016) 17:1338-1342).

In the aforementioned embodiments, other types of linkages besides hybridization can be used to link the target specific binding agent and the recording tag. For example, the two moieties can be covalently linked, using a linker that is designed to be cleaved and release the binding agent once the captured target protein (or other polypeptide) is covalently linked to the recording tag. A suitable linker can be attached to various positions of the recording tag, such as the 3' end, or within the linker attached to the 5' end of the recording tag.

Recording tags can be attached to the protein, polypeptide, or peptides pre- or post-immobilization to the solid support. For example, proteins, polypeptides, or peptides can be first labeled with recording tags and then immobilized to a solid surface via a recording tag comprising at two functional moieties for coupling. One functional moiety of the recording tag couples to the protein, and the other functional moiety immobilizes the recording tag-labeled protein to a solid support.

In other embodiments, polypeptides are immobilized to a solid support prior to labeling of the proteins, polypeptides or peptides with recording tags. For example, proteins can first be derivatized with reactive groups such as click chemistry moieties. The activated protein molecules can then be attached to a suitable solid support and then labeled with recording tags using the complementary click chemistry moiety. As an example, proteins derivatized with alkyne and mTet moieties may be immobilized to beads derivatized with azide and TCO and attached to recording tags labeled with azide and TCO.

In certain embodiments, the surface of a solid support is passivated (blocked) to minimize non-specific absorption to binding agents. A "passivated" surface refers to a surface that has been treated with outer layer of material to minimize non-specific binding of a binding agent. Methods of passivating surfaces include standard methods from the fluorescent single molecule analysis literature, including passivating surfaces with polymer like polyethylene glycol (PEG) (Pan et al., 2015, Phys. Biol. 12:045006), polysiloxane (e.g., Pluronic F-127), star polymers (*e.g.*, star PEG) (Groll et al., 2010, Methods Enzymol. 472:1-18), hydrophobic dichlorodimethylsilane (DDS) + self-assembled Tween-20 (Hua et al., 2014, Nat. Methods 11:1233-1236), diamond-like carbon (DLC), DLC + PEG (Stavis et al., 2011, Proc. Natl. Acad. Sci. USA 108:983-988), and zwitterionic moiety (e.g., U.S. Patent Application Publication US 2006/0183863). In addition to covalent surface modifications, a number of passivating agents can be employed as well including surfactants like Tween-20, polysiloxane in solution (Pluronic series), poly vinyl alcohol, (PVA), and proteins like BSA and casein. Alternatively, density of proteins, polypeptide, or peptides can be titrated on the surface or within the volume of a solid substrate by spiking a competitor or "dummy" reactive molecule when immobilizing the proteins, polypeptides or peptides to the solid substrate.

In certain embodiments where multiple polypeptides are immobilized on the same solid support, the polypeptides can be spaced appropriately to reduce the occurrence of or prevent a cross-binding or inter-molecular event, *e.g.*, where a binding agent binds to a first polypeptides and its coding tag information is transferred to a recording tag associated with a neighboring polypeptides rather than the recording tag associated with the first polypeptide. To control polypeptide spacing on the solid support, the density of functional coupling groups (*e.g.,* TCO) may be titrated on the substrate surface. In some embodiments, multiple polypeptides are spaced apart on the surface or within the volume (*e.g.*, porous supports) of a solid support at a distance of about 50 nm to about 500 nm, or a subrange thereof, *e.g.,* or about 50 nm to about 400 nm, or about 50 nm to about 300 nm, or about 50 nm to about 200 nm, or about 50 nm to about 100 nm. In some embodiments, multiple polypeptides are spaced apart on the surface of a solid support with an average distance of at least 50 nm, at least 60 nm, at least 70 nm, at least 80 nm, at least 90 nm, at least 100 nm, at least 150 nm, at least 200 nm, at least 250 nm, at least 300 nm, at least 350 nm, at least 400 nm, at least 450 nm, or at least 500 nm. In some embodiments, multiple polypeptides are spaced apart on the surface of a solid support with an average distance of at least 50 nm. In some embodiments, polypeptides are spaced apart on the surface or within the volume of a solid support such that, empirically, the relative frequency of inter- to intra-molecular events is <1:10; <1:100; <1:1,000; or <1:10,000. A suitable spacing frequency can be determined empirically using a functional assay (*see,* Example 31 of International Patent Publication No. WO 2017/192633), and can be accomplished by dilution and/or by spiking a "dummy" spacer molecule that competes for attachments sites on the substrate surface.

For example, PEG-5000 (MW ~ 5000) is used to block the interstitial space between peptides on the substrate surface (*e.g.*, bead surface). In addition, the peptide is coupled to a functional moiety that is also attached to a PEG-5000 molecule. In some embodiments, this is accomplished by coupling a mixture of NHS-PEG-5000-TCO + NHS-PEG-5000-Methyl to amine-derivatized beads. The stoichiometric ratio between the two PEGs (TCO vs. methyl) is titrated to generate an appropriate density of functional coupling moieties (TCO groups) on the substrate surface; the methyl-PEG is inert to coupling. The effective spacing between TCO groups can be calculated by measuring the density of TCO groups on the surface. In certain embodiments, the mean spacing between coupling moieties (e.g., TCO) on the solid surface is at least 50 nm, at least 100 nm, at least 250 nm, or at least 500 nm. After PEG5000-TCO/methyl derivatization of the beads, the

excess $NH_2$ groups on the surface are quenched with a reactive anhydride (*e.g.* acetic or succinic anhydride). Other MW PEGs can also be used for passivation from MW ~ 300 Da to over 50 kDa.

In some embodiments, the spacing is accomplished by titrating the ratio of available attachment molecules on the substrate surface. In some examples, the substrate surface (*e.g.*, bead surface) is functionalized with a carboxyl group (COOH) which is treated with an activating agent (*e.g.*, activating agent is EDC and Sulfo-NHS). In some examples, the substrate surface (*e.g.*, bead surface) comprises NHS moieties. In some embodiments, a mixture of mPEG$_n$-NH2 and NH2-PEG$_n$-mTet is added to the activated beads (wherein n is any number, such as 1-100). The ratio between the mPEG$_3$-$NH_2$ (not available for coupling) and NH2-PEG24-mTet (available for coupling) is titrated to generate an appropriate density of functional moieties available to attach the analyte on the substrate surface. In certain embodiments, the mean spacing between coupling moieties (*e.g.*, $NH_2$-PEG$_4$-mTet) on the solid surface is at least 50 nm, at least 100 nm, at least 250 nm, or at least 500 nm. In some specific embodiments, the ratio of $NH_2$-PEG$_n$-mTet to mPEG$_3$-NH2 is about or greater than 1: 1000, about or greater than 1:10,000, about or greater than 1:100,000, or about or greater than 1:1,000,000. In some further embodiments, the capture nucleic acid attaches to the NH2-PEG$_n$-mTet.

In particular embodiments, the polypeptide(s) and/or the recording tag(s) are immobilized on a substrate or support at a density such that the interaction between (i) a coding agent bound to a first polypeptide (particularly, the coding tag in that bound coding agent), and (ii) a second polypeptide and/or its recording tag, is reduced, minimized, or completely eliminated. Therefore, false positive assay signals resulting from "intermolecular" engagement can be reduced, minimized, or eliminated.

In certain embodiments, the density of the polypeptides and/or the recording tags on a substrate is determined for each type of polypeptide. For example, the longer a denatured polypeptide chain is, the lower the density should be in order to reduce, minimize, or prevent "intermolecular" interactions. In certain aspects, increasing the spacing between the polypeptide molecules and/or the recording tags (*i.e.,* lowering the density) increases the signal to background ratio of the presently disclosed assays.

In some embodiments, the polypeptide molecules and/or the recording tags are deposited or immobilized on a substrate at any suitable average density, e.g., at an average density of about 0.0001 molecule/$\mu m^2$, 0.001 molecule/$\mu m^2$, 0.01 molecule/$\mu m^2$, 0.1 molecule/$\mu m^2$, 1 molecule/$\mu m^2$, about 2 molecules/$\mu m^2$, about 3 molecules/$\mu m^2$, about 4 molecules/$\mu m^2$, about 5 molecules/$\mu m^2$, about 6 molecules/$\mu m^2$, about 7 molecules/$\mu m^2$, about 8 molecules/$\mu m^2$, about 9 molecules/$\mu m^2$, or about 10 molecules/$\mu m^2$. In other embodiments, the polypeptide(s) and/or the recording tag(s) are deposited or immobilized at an average density of about 15, about 20, about 25, about 30, about 35, about 40, about 45, about 50, about 55, about 60, about 65, about 70, about 75, about 80, about 85, about 90, about 95, about 100, about 105, about 110, about 115, about 120, about 125, about 130, about 135, about 140, about 145, about 150, about 155, about 160, about 165, about 170, about 175, about 180, about 185, about 190, about 195, about 200, or about 200 molecules/$\mu m^2$ on a substrate. In other embodiments, the polypeptide(s) and/or the recording tag(s) are deposited or immobilized at an average density of about 1 molecule/$mm^2$, about 10 molecules/$mm^2$, about 50 molecules/$mm^2$, about 100 molecules/$mm^2$, about 150 molecules/$mm^2$, about 200 molecules/$mm^2$, about 250 molecules/$mm^2$, about 300 molecules/$mm^2$, about 350 molecules/$mm^2$, 400 molecules/$mm^2$, about 450 molecules/$mm^2$, about 500 molecules/$mm^2$, about 550 molecules/$mm^2$, about 600 molecules/$mm^2$, about 650 molecules/$mm^2$, about 700 molecules/$mm^2$, about 750 molecules/$mm^2$, about 800 molecules/$mm^2$, about 850 molecules/$mm^2$, about 900 molecules/$mm^2$, about 950 molecules/$mm^2$, or about 1000 molecules/$mm^2$. In still other embodiments, the polypeptide(s) and/or the recording tag(s) are deposited or immobilized on a substrate at an average density between about $1\times10^3$ and about $0.5\times10^4$ molecules/$mm^2$, between about $0.5\times10^4$ and about $1\times10^4$ molecules/$mm^2$, between about $1\times10^4$ and about $0.5\times10^5$ molecules/$mm^2$, between about $0.5\times10^5$ and about $1\times10^5$ molecules/$mm^2$, between about $1\times10^5$ and about $0.5\times10^6$ molecules/$mm^2$, or between about $0.5\times10^6$ and about $1\times10^6$ molecules/$mm^2$. In other embodiments, the average density of the polypeptide(s) and/or the recording tag(s) deposited or immobilized on a substrate can be, for example, between about 1 molecule/$cm^2$ and about 5 molecules/$cm^2$, between about 5 and about 10 molecules/$cm^2$, between about 10 and about 50 molecules/$cm^2$, between about 50 and about 100 molecules/$cm^2$, between about100 and about $0.5\times10^3$ molecules/$cm^2$, between about $0.5\times10^3$ and about $1\times10^3$ molecules/$cm^2$, $1\times10^3$ and about $0.5\times10^4$ molecules/$cm^2$, between about $0.5\times10^4$ and about $1\times10^4$ molecules/$cm^2$, between about $1\times10^4$ and about $0.5\times10^5$ molecules/$cm^2$, between about $0.5\times10^5$ and about $1\times10^5$ molecules/$cm^2$, between about $1\times10^5$ and about $0.5\times10^6$ molecules/$cm^2$, or between about $0.5\times10^6$ and about $1\times10^6$ molecules/$cm^2$.

B. **Cyclic Transfer of Coding Tag Information to Recording Tags**

In the methods described herein, upon binding of a binding agent to a polypeptide, identifying information of its linked coding tag is transferred to a recording tag associated with the polypeptide, thereby generating an "extended recording tag." An extended recording tag may comprise information from a binding agent's coding tag representing each binding cycle performed. However, an extended recording tag may also experience a "missed" binding cycle, *e.g.*, because a binding agent fails to bind to the polypeptide, because the coding tag was missing, damaged, or defective,

because the primer extension reaction failed. Even if a binding event occurs, transfer of information from the coding tag to the recording tag may be incomplete or less than 100% accurate, *e.g.*, because a coding tag was damaged or defective, because errors were introduced in the primer extension reaction). Thus, an extended recording tag may represent 100%, or up to 95%, 90%, 85%, 80%, 75%, 70%, 65%, 60%, 65%, 55%, 50%, 45%, 40%, 35%, 30%, or any subrange thereof, of binding events that have occurred on its associated polypeptide. Moreover, the coding tag information present in the extended recording tag may have at least 30%, 35%, 40%, 45%, 50%, 55%, 60%, 65%, 70%, 75%, 80%, 85%, 90%, 95%, or 100% identity the corresponding coding tags.

In certain embodiments, a binding agent may bind to an NTAA, a CTAA, an intervening amino acid, dipeptide (sequence of two amino acids), tripeptide (sequence of three amino acids), or higher order peptide of a peptide molecule. In some embodiments, each binding agent in a library of binding agents selectively binds to a particular amino acid, for example one of the twenty standard naturally occurring amino acids. The standard, naturally-occurring amino acids include Alanine (A or Ala), Cysteine (C or Cys), Aspartic Acid (D or Asp), Glutamic Acid (E or Glu), Phenylalanine (F or Phe), Glycine (G or Gly), Histidine (H or His), Isoleucine (I or Ile), Lysine (K or Lys), Leucine (L or Leu), Methionine (M or Met), Asparagine (N or Asn), Proline (P or Pro), Glutamine (Q or Gln), Arginine (R or Arg), Serine (S or Ser), Threonine (T or Thr), Valine (V or Val), Tryptophan (W or Trp), and Tyrosine (Y or Tyr). In some embodiments, the binding agent binds to an unmodified or native amino acid. In some examples, the binding agent binds to an unmodified or native dipeptide (sequence of two amino acids), tripeptide (sequence of three amino acids), or higher order peptide of a peptide molecule. A binding agent may be engineered for high affinity for a native or unmodified NTAA, high specificity for a native or unmodified NTAA, or both. In some embodiments, binding agents can be developed through directed evolution of promising affinity scaffolds using phage display.

A binding agent may bind to an N-terminal peptide, a C-terminal peptide, or an intervening peptide of a peptide, polypeptide, or protein molecule. A binding agent may bind to an N-terminal amino acid, C-terminal amino acid, or an intervening amino acid of a peptide molecule. A binding agent may bind to an N-terminal or C-terminal diamino acid moiety. A binding agent may preferably bind to a chemically modified or labeled amino acid. For example, a binding agent may preferably bind to an amino acid that has been functionalized with an acetyl moiety, Cbz moiety, guanyl moiety, dansyl moiety, PTC moiety, DNP moiety, SNP moiety, heterocyclic methanimine moiety, etc., over an amino acid that does not possess said moiety.

In certain embodiments, an extended recording tag may comprise information from multiple coding tags representing multiple, successive binding events. In these embodiments, a single, concatenated extended recording tag can be representative of a single polypeptide. As referred to herein, transfer of coding tag information to a recording tag also includes transfer to an extended recording tag as would occur in methods involving multiple, successive binding events.

In certain embodiments, the binding event information is transferred from a coding tag to a recording tag in a cyclic fashion. Cross-reactive binding events can be informatically filtered out after sequencing by requiring that at least two different coding tags, identifying two or more independent binding events, map to the same class of binding agents (cognate to a particular protein). An optional sample or compartment barcode can be included in the recording tag, as well an optional UMI sequence. The coding tag can also contain an optional UMI sequence along with the encoder and spacer sequences. Universal priming sequences may also be included in extended recording tags for amplification and NGS sequencing.

Coding tag information associated with a specific binding agent may be transferred to a recording tag using a variety of methods. In certain embodiments, information of a coding tag is transferred to a recording tag via primer extension (Chan, McGregor et al. 2015). A spacer sequence on the 3'-terminus of a recording tag or an extended recording tag anneals with complementary spacer sequence on the 3' terminus of a coding tag and a polymerase (*e.g.*, strand-displacing polymerase) extends the recording tag sequence, using the annealed coding tag as a template. In some embodiments, oligonucleotides complementary to coding tag encoder sequence and 5' spacer can be pre-annealed to the coding tags to prevent hybridization of the coding tag to internal encoder and spacer sequences present in an extended recording tag. The 3' terminal spacer, on the coding tag, remaining single stranded, preferably binds to the terminal 3' spacer on the recording tag. In other embodiments, a nascent recording tag can be coated with a single stranded binding protein to prevent annealing of the coding tag to internal sites. Alternatively, the nascent recording tag can also be coated with RecA (or related homologues such as uvsX) to facilitate invasion of the 3' terminus into a completely double stranded coding tag (Bell et al., 2012, Nature 491:274-278). This configuration prevents the double stranded coding tag from interacting with internal recording tag elements, yet is susceptible to strand invasion by the RecA coated 3' tail of the extended recording tag (Bell, et al., 2015, Elife 4: e08646). The presence of a single-stranded binding protein can facilitate the strand displacement reaction.

In some embodiments, a DNA polymerase that is used for primer extension possesses strand-displacement activity and has limited or is devoid of 3'-5 exonuclease activity. Several of many examples of such polymerases include Klenow exo- (Klenow fragment of DNA Pol 1), T4 DNA polymerase exo-, T7 DNA polymerase exo (Sequenase 2.0), Pfu exo-, Vent exo-, Deep Vent exo-, Bst DNA polymerase large fragment exo-, Bca Pol, 9°N Pol, and Phi29 Pol exo-. In a

preferred embodiment, the DNA polymerase is active at room temperature and up to 45°C. In another embodiment, a "warm start" version of a thermophilic polymerase is employed such that the polymerase is activated and is used at about 40°C-50°C. An exemplary warm start polymerase is Bst 2.0 Warm Start DNA Polymerase (New England Biolabs).

Additives useful in strand-displacement replication include any of a number of single-stranded DNA binding proteins (SSB proteins) of bacterial, viral, or eukaryotic origin, such as SSB protein of E. coli, phage T4 gene 32 product, phage T7 gene 2.5 protein, phage Pf3 SSB, replication protein A RPA32 and RPA14 subunits (Wold, 1997); other DNA binding proteins, such as adenovirus DNA-binding protein, herpes simplex protein ICP8, BMRF1 polymerase accessory subunit, herpes virus UL29 SSB-like protein; any of a number of replication complex proteins known to participate in DNA replication, such as phage T7 helicase/primase, phage T4 gene 41 helicase, E. coli Rep helicase, E. coli recBCD helicase, recA, E. coli and eukaryotic topoisomerases (Annu Rev Biochem. (2001) 70:369-413).

Mis-priming or self-priming events, such as when the terminal spacer sequence of the recoding tag primes extension self-extension may be minimized by inclusion of single stranded binding proteins (T4 gene 32, E. coli SSB, etc.), DMSO (1-10%), formamide (1-10%), BSA( 10-100 ug/ml), TMACl (1-5 mM), ammonium sulfate (10-50 mM), betaine (1-3 M), glycerol (5-40%), or ethylene glycol (5-40%), in the primer extension reaction.

Most type A polymerases are devoid of 3' exonuclease activity (endogenous or engineered removal), such as Klenow exo-, T7 DNA polymerase exo- (Sequenase 2.0), and Taq polymerase catalyzes non-templated addition of a nucleotide, preferably an adenosine base (to lesser degree a G base, dependent on sequence context) to the 3' blunt end of a duplex amplification product. For Taq polymerase, a 3' pyrimidine (C>T) minimizes non-templated adenosine addition, whereas a 3' purine nucleotide (G>A) favours non-templated adenosine addition. In some embodiments, using Taq polymerase for primer extension, placement of a thymidine base in the coding tag between the spacer sequence distal from the binding agent and the adjacent barcode sequence (*e.g.*, encoder sequence or cycle specific sequence) accommodates the sporadic inclusion of a non-templated adenosine nucleotide on the 3' terminus of the spacer sequence of the recording tag. In this manner, the extended recording tag (with or without a non-templated adenosine base) can anneal to the coding tag and undergo primer extension.

Alternatively, addition of non-templated base can be reduced by employing a mutant polymerase (mesophilic or thermophilic) in which non-templated terminal transferase activity has been greatly reduced by one or more point mutations, especially in the O-helix region (see U.S. Patent 7,501,237) (Yang et al., Nucleic Acids Res. (2002) 30(19): 4314-4320). Pfu exo-, which is 3' exonuclease deficient and has strand-displacing ability, also does not have non-templated terminal transferase activity.

In another embodiment, polymerase extension buffers are comprised of 40-120 mM buffering agent such as Tris-Acetate, Tris-HCl, HEPES, etc. at a pH of 6-9.

Self-priming/mis-priming events initiated by self-annealing of the terminal spacer sequence of the extended recording tag with internal regions of the extended recording tag may be minimized by including pseudo-complementary bases in the recording/extended recording tag (Lahoud et al., Nucleic Acids Res. (2008) 36:3409-3419), (Hoshika et al., Angew Chem Int Ed Engl (2010) 49(32): 5554-5557). Pseudo-complementary bases show significantly reduced hybridization affinities for the formation of duplexes with each other due the presence of chemical modification. However, many pseudo-complementary modified bases can form strong base pairs with natural DNA or RNA sequences. In certain embodiments, the coding tag spacer sequence is comprised of multiple A and T bases, and commercially available pseudo-complementary bases 2-aminoadenine and 2-thiothymine are incorporated in the recording tag using phosphoramidite oligonucleotide synthesis. Additional pseudocomplementary bases can be incorporated into the extended recording tag during primer extension by adding pseudo-complementary nucleotides to the reaction (Gamper et al., Biochemistry. (2006) 45(22):6978-86).

In some embodiments, to minimize non-specific interaction of the coding tag labeled binding agents in solution with the recording tags of immobilized proteins, competitor (also referred to as blocking) oligonucleotides complementary to recording tag spacer sequences can be added to binding reactions to minimize non-specific interactions. In some embodiments, blocking oligonucleotides are relatively short. Excess competitor oligonucleotides are washed from the binding reaction prior to primer extension, which effectively dissociates the annealed competitor oligonucleotides from the recording tags, especially when exposed to slightly elevated temperatures (*e.g.*, 30-50 °C). Blocking oligonucleotides may comprise a terminator nucleotide at its 3' end to prevent primer extension.

In some embodiments, the coding tag may comprise a hairpin. In certain embodiments, the hairpin comprises mutually complementary nucleic acid regions are connected through a nucleic acid strand. In some embodiments, the nucleic acid hairpin can also further comprise 3' and/or 5' single-stranded region(s) extending from the double-stranded stem segment. In some examples, the hairpin comprises a single strand of nucleic acid.

In certain embodiments, the annealing of the spacer sequence on the recording tag to the complementary spacer sequence on the coding tag is metastable under the primer extension reaction conditions (*i.e.,* the annealing Tm is similar to the reaction temperature). This allows the spacer sequence of the coding tag to displace any blocking oligonucleotide annealed to the spacer sequence of the recording tag.

Coding tag information associated with a specific binding agent may also be transferred to a recording tag via

ligation. Ligation may be a blunt end ligation or sticky end ligation. Ligation may be an enzymatic ligation reaction. Examples of ligases include, but are not limited to CV DNA ligase (see U.S. Patent Publication No. US 2014/0378315), T4 DNA ligase, T7 DNA ligase, T3 DNA ligase, Taq DNA ligase, E. coli DNA ligase, 9°N DNA ligase, Electroligase®. Alternatively, a ligation may be a chemical ligation reaction. In the illustration, a spacer-less ligation is accomplished by using hybridization of a "recording helper" sequence with an arm on the coding tag. The annealed complement sequences are chemically ligated using standard chemical ligation or "click chemistry" (Gunderson et al., Genome Res (1998) 8(11): 1142-1153; Peng et al., European J Org Chem (2010) (22): 4194-4197; El-Sagheeret al., Proc Natl Acad Sci U S A (2011) 108(28): 11338-11343; El-Sagheer et al., Org Biomol Chem (2011) 9(1): 232-235; Sharma et al., Anal Chem (2012) 84(14): 6104-6109; Roloff et al., Bioorg Med Chem (2013) 21(12): 3458-3464; Litovchick et al., Artif DNA PNA XNA (2014) 5(1): e27896; Roloff et al., Methods Mol Biol (2014) 1050:131-141).

In another embodiment, transfer of PNAs can be accomplished with chemical ligation using published techniques. The structure of PNA is such that it has a 5' N-terminal amine group and an unreactive 3' C-terminal amide. Chemical ligation of PNA requires that the termini be modified to be chemically active. This is typically done by derivatizing the 5' N-terminus with a cysteinyl moiety and the 3' C-terminus with a thioester moiety. Such modified PNAs easily couple using standard native chemical ligation conditions (Roloff et al., (2013) Bioorgan. Med. Chem. 21:3458-3464).

In some embodiments, coding tag information can be transferred using topoisomerase. Topoisomerase can be used be used to ligate a topo-charged 3' phosphate on the recording tag to the 5' end of the coding tag, or complement thereof (Shuman et al., 1994, J. Biol. Chem. 269:32678-32684).

As described herein, a binding agent may bind to a post-translationally modified amino acid. Thus, in certain embodiments, an extended recording tag comprises coding tag information relating to amino acid sequence and post-translational modifications of the polypeptide. In some embodiments, detection of internal post-translationally modified amino acids (*e.g.*, phosphorylation, glycosylation, succinylation, ubiquitination, S-Nitrosylation, methylation, N-acetylation, lipidation, etc.) is be accomplished prior to detection and elimination of terminal amino acids (*e.g.*, NTAA or CTAA). In one example, a peptide is contacted with binding agents for PTM modifications, and associated coding tag information are transferred to the recording tag. Once the detection and transfer of coding tag information relating to amino acid modifications is complete, the PTM modifying groups can be removed before detection and transfer of coding tag information for the primary amino acid sequence using N-terminal or C-terminal degradation methods. Thus, resulting extended recording tags indicate the presence of post-translational modifications in a peptide sequence, though not the sequential order, along with primary amino acid sequence information.

In some embodiments, detection of internal post-translationally modified amino acids may occur concurrently with detection of primary amino acid sequence. In one example, an NTAA (or CTAA) is contacted with a binding agent specific for a post-translationally modified amino acid, either alone or as part of a library of binding agents (e.g., library composed of binding agents for the 20 standard amino acids and selected post-translational modified amino acids). Successive cycles of terminal amino acid elimination and contact with a binding agent (or library of binding agents) follow. Thus, resulting extended recording tags indicate the presence and order of post-translational modifications in the context of a primary amino acid sequence.

In certain embodiments, an ensemble of recording tags may be employed per polypeptide to improve the overall robustness and efficiency of coding tag information transfer. The use of an ensemble of recording tags associated with a given polypeptide rather than a single recording tag improves the efficiency of library construction due to potentially higher coupling yields of coding tags to recording tags, and higher overall yield of libraries. The yield of a single concatenated extended recording tag is directly dependent on the stepwise yield of concatenation, whereas the use of multiple recording tags capable of accepting coding tag information does not suffer the exponential loss of concatenation.

For embodiments involving analysis of denatured proteins, polypeptides, and peptides, the bound binding agent and annealed coding tag can be removed following primer extension by using highly denaturing conditions (*e.g.*, 0.1-0.2 N NaOH, 6M Urea, 2.4 M guanidinium isothiocyanate, 95% formamide, etc.).

### C. **Characterization of Polypeptides via Cyclic Rounds of Amino Acid Recognition, Recording Tag Extension, and Amino Acid Removal**

In certain embodiments, the methods for analyzing a polypeptide provided in the present disclosure comprise multiple binding cycles, where the polypeptide is contacted with a plurality of binding agents, and successive binding of binding agents transfers historical binding information in the form of a nucleic acid based coding tag to at least one recording tag associated with the polypeptide. In this way, a historical record containing information about multiple binding events is generated in a nucleic acid format.

In certain embodiments, the concentration of the binding agents in a solution is controlled to reduce background and/or false positive results of the assay.

In some embodiments, the concentration of a binding agent can be at any suitable concentration, *e.g.*, at about 0.0001 nM, about 0.001 nM, about 0.01 nM, about 0.1 nM, about 1 nM, about 2 nM, about 5 nM, about 10 nM, about 20 nM,

about 50 nM, about 100 nM, about 200 nM, about 500 nM, or about 1000 nM. In other embodiments, the concentration of a soluble conjugate used in the assay is between about 0.0001 nM and about 0.001 nM, between about 0.001 nM and about 0.01 nM, between about 0.01 nM and about 0.1 nM, between about 0.1 nM and about 1 nM, between about 1 nM and about 2 nM, between about 2 nM and about 5 nM, between about 5 nM and about 10 nM, between about 10 nM and about 20 nM, between about 20 nM and about 50 nM, between about 50 nM and about 100 nM, between about 100 nM and about 200 nM, between about 200 nM and about 500 nM, between about 500 nM and about 1000 nM, or more than about 1000 nM.

In some embodiments, the ratio between the soluble binding agent molecules and the immobilized polypeptides and/or the recording tags can be at any suitable range, *e.g.,* at about 0.00001:1, about 0.0001:1, about 0.001:1, about 0.01:1, about 0.1:1, about 1:1, about 2:1, about 5:1, about 10:1, about 15:1, about 20:1, about 25:1, about 30:1, about 35:1, about 40:1, about 45:1, about 50:1, about 55:1, about 60:1, about 65:1, about 70:1, about 75:1, about 80:1, about 85:1, about 90:1, about 95:1, about 100:1, about $10^4$:1, about $10^5$:1, about $10^6$:1, or higher, or any ratio in between the above listed ratios. Higher ratios between the soluble binding agent molecules and the immobilized polypeptide(s) and/or the recording tag(s) can be used to drive the binding and/or the coding tag/recoding tag information transfer to completion. This may be particularly useful for detecting and/or analyzing low abundance polypeptides in a sample.

In embodiments relating to methods of analyzing peptide or polypeptides using an N-terminal degradation based approach, following contacting and binding of a first binding agent to an *n* NTAA of a peptide of *n* amino acids and transfer of the first binding agent's coding tag information to a recording tag associated with the peptide, thereby generating a first order extended recording tag, the *n and n-1* NTAA is eliminated as a labeled dipeptide described herein. In some aspects, two or more any of the modified dipeptide cleavases described in Section I can be used in combination to remove the labeled NTAA (as part of a dipeptide). For example, a sample can be treated with a mixture of modified dipeptide cleavase enzymes to achieve removal of various NTAAs in the peptides in the sample. Removal of the *n* labeled NTAA and *n-1* amino acid as a dipeptide by contacting with the modified dipeptide cleavase converts the *n-3* amino acid of the peptide to an N-terminal amino acid, which is referred to herein as an *n-3* NTAA. A second binding agent can be contacted with the peptide and binds to the *n-3* NTAA, and the second binding agent's coding tag information is transferred to the first order extended recording tag thereby generating a second order extended recording tag (*e.g.,* for generating a concatenated $n^{th}$ order extended recording tag representing the peptide). Elimination of the *n-3* labeled NTAA and *n-4* amino acid as a dipeptide by a modified dipeptide cleavase converts the *n-5* amino acid of the peptide to an N-terminal amino acid, which is referred to herein as *n-5* NTAA. Additional binding, transfer, labeling, and removal, can occur as described above up to *n* amino acids to generate an $n^{th}$ order extended recording tag. As used herein, an *n* "order" when used in reference to a binding agent, coding tag, or extended recording tag, refers to the *n* binding cycle, wherein the binding agent and its associated coding tag is used or the *n* binding cycle where the extended recording tag is created. In some embodiments, steps including the NTAA in the described exemplary approach can be performed instead with a CTAA. In some embodiments, the binding agents bind and recognizes the last two amino acids of a polypeptide.

In some embodiments, contacting of the first binding agent and second binding agent to the polypeptide, and optionally any further binding agents (*e.g.*, third binding agent, fourth binding agent, fifth binding agent, and so on), are performed at the same time. For example, the first binding agent and second binding agent, and optionally any further order binding agents, can be pooled together, for example to form a library of binding agents. In another example, the first binding agent and second binding agent, and optionally any further order binding agents, rather than being pooled together, are added simultaneously to the polypeptide. In one embodiment, a library of binding agents comprises at least 20 binding agents that selectively bind to the 20 standard, naturally occurring amino acids.

In other embodiments, the first binding agent and second binding agent, and optionally any further order binding agents, are each contacted with the polypeptide in separate binding cycles, added in sequential order. In certain embodiments, multiple binding agents are used at the same time, in parallel. This parallel approach saves time and reduces non-specific binding by non-cognate binding agents to a site that is bound by a cognate binding agent (because the binding agents are in competition).

The length of the final extended recording tags generated by the methods described herein is dependent upon multiple factors, including the length of the coding tag (*e.g.,* encoder sequence and spacer), the length of the recording tag (*e.g.,* unique molecular identifier, spacer, universal priming site, bar code), the number of binding cycles performed, and whether coding tags from each binding cycle are transferred to the same extended recording tag or to multiple extended recording tags. In some examples, if the coding tag has an encoder sequence of 5 bases that is flanked on each side by a spacer of 5 bases, the coding tag information on the final extended recording tag, which represents the peptide's binding agent history, is 10 bases x number of degradation cycles.

After the final binding cycle and transfer of the final binding agent's coding tag information to the extended recording tag, the tag can be capped by addition of a universal reverse priming site via ligation, primer extension or other methods known in the art. In some embodiments, the universal forward priming site in the recording tag is compatible with the universal reverse priming site that is appended to the final extended recording tag. In some embodiments, a universal reverse priming site is an Illumina P7 primer (5'-CAAGCAGAAGACGGCATACGAGAT - 3' - SEQ ID NO:4) or an Illumina P5 primer (5'-AATGATACGGCGACCACCGA-3' - SEQ ID NO:3). The sense or antisense P7 may be appended,

depending on strand sense of the recording tag. An extended recording tag library can be cleaved or amplified directly from the solid support (*e.g.*, beads) and used in traditional next generation sequencing assays and protocols.

In some embodiments, a primer extension reaction is performed on a library of single stranded extended recording tags to copy complementary strands thereof. In some embodiments, the peptide sequencing assay (*e.g.*, ProteoCode™ assay), comprises several chemical and enzymatic steps in a cyclical progression. In some cases, one advantage of a single molecule assay is the robustness to inefficiencies in the various cyclical chemical/enzymatic steps. In some embodiments, the use of cycle-specific barcodes present in the coding tag sequence allows an advantage to the assay.

D. **Processing and Analysis of Tags**

Extended recording tag and any other tags representing the polypeptide(s) of interest can be processed and analysed using a variety of nucleic acid sequencing methods. Examples of sequencing methods include, but are not limited to, chain termination sequencing (Sanger sequencing); next generation sequencing methods, such as sequencing by synthesis, sequencing by ligation, sequencing by hybridization, polony sequencing, ion semiconductor sequencing, and pyrosequencing; and third generation sequencing methods, such as single molecule real time sequencing, nanopore-based sequencing, duplex interrupted sequencing, and direct imaging of DNA using advanced microscopy.

Suitable sequencing methods for use in the invention include, but are not limited to, sequencing by hybridization, sequencing by synthesis technology (e.g., HiSeq™ and Solexa™, Illumina), SMRT™ (Single Molecule Real Time) technology (Pacific Biosciences), true single molecule sequencing (e.g., HeliScope™, Helicos Biosciences), massively parallel next generation sequencing (e.g., SOLiD™, Applied Biosciences; Solexa and HiSeq™, Illumina), massively parallel semiconductor sequencing (e.g., Ion Torrent), and pyrosequencing technology (e.g., GS FLX and GS Junior Systems, Roche/454), and nanopore sequence (*e.g.*, Oxford Nanopore Technologies).

A library of extended recording tags, extended coding tags, or di-tags may be amplified in a variety of ways. A library of extended recording tags, extended coding tags, or di-tags may undergo exponential amplification, *e.g.,* via PCR or emulsion PCR. Emulsion PCR is known to produce more uniform amplification (Hori, Fukano et al., Biochem Biophys Res Commun (2007) 352(2): 323-328). Alternatively, a library of extended recording tags, extended coding tags, or di-tags may undergo linear amplification, *e.g.,* via *in vitro* transcription of template DNA using T7 RNA polymerase. The library of extended recording tags, extended coding tags, or di-tags can be amplified using primers compatible with the universal forward priming site and universal reverse priming site contained therein. A library of extended recording tags, extended coding tags, or di-tags can also be amplified using tailed primers to add sequence to either the 5'-end, 3'-end or both ends of the extended recording tags, extended coding tags, or di-tags. Sequences that can be added to the termini of the extended recording tags, extended coding tags, or di-tags include library specific index sequences to allow multiplexing of multiple libraries in a single sequencing run, adaptor sequences, read primer sequences, or any other sequences for making the library of extended recording tags, extended coding tags, or di-tags compatible for a sequencing platform. An example of a library amplification in preparation for next generation sequencing is as follows: a 20 μl PCR reaction volume is set up using an extended recording tag library eluted from ~1 mg of beads (~ 10 ng), 200 μM dNTP, 1 μM of each forward and reverse amplification primers, 0.5 μl (1U) of Phusion Hot Start enzyme (New England Biolabs) and subjected to the following cycling conditions: 98° C for 30 sec followed by 20 cycles of 98° C for 10 sec, 60° C for 30 sec, 72° C for 30 sec, followed by 72° C for 7 min, then hold at 4° C.

In certain embodiments, either before, during or following amplification, the library of extended recording tags, extended coding tags, or di-tags can undergo target enrichment. In some embodiments, target enrichment can be used to selectively capture or amplify extended recording tags representing polypeptides of interest from a library of extended recording tags, extended coding tags, or di-tags before sequencing. In some aspects, target enrichment for protein sequencing is challenging because of the high cost and difficulty in producing highly-specific binding agents for target proteins. In some cases, antibodies are notoriously non-specific and difficult to scale production across thousands of proteins. In some embodiments, the methods of the present disclosure circumvent this problem by converting the protein code into a nucleic acid code which can then make use of a wide range of targeted DNA enrichment strategies available for DNA libraries. In some cases, peptides of interest can be enriched in a sample by enriching their corresponding extended recording tags. Methods of targeted enrichment are known in the art, and include hybrid capture assays, PCR-based assays such as TruSeq custom Amplicon (Illumina), padlock probes (also referred to as molecular inversion probes), and the like (*see,* Mamanova et al., (2010) Nature Methods 7: 111-118; Bodi et al., J. Biomol. Tech. (2013) 24:73-86; Ballester et al., (2016) Expert Review of Molecular Diagnostics 357-372; Mertes et al., (2011) Brief Funct. Genomics 10:374-386; Nilsson et al., (1994) Science 265:2085-8).

In one embodiment, a library of extended recording tags, extended coding tags, or di-tags is enriched via a hybrid capture-based assay. In a hybrid-capture based assay, the library of extended recording tags, extended coding tags, or di-tags is hybridized to target-specific oligonucleotides or "bait oligonucleotide" that are labelled with an affinity tag (*e.g.,* biotin). Extended recording tags, extended coding tags, or di-tags hybridized to the target-specific oligonucleotides are

"pulled down" via their affinity tags using an affinity ligand (*e.g.,* streptavidin coated beads), and background (non-specific) extended recording tags are washed away. The enriched extended recording tags, extended coding tags, or di-tags are then obtained for positive enrichment (*e.g.*, eluted from the beads).

For bait oligonucleotides synthesized by array-based *"in situ"* oligonucleotide synthesis and subsequent amplification of oligonucleotide pools, competing baits can be engineered into the pool by employing several sets of universal primers within a given oligonucleotide array. For each type of universal primer, the ratio of biotinylated primer to non-biotinylated primer controls the enrichment ratio. The use of several primer types enables several enrichment ratios to be designed into the final oligonucleotide bait pool.

A bait oligonucleotide can be designed to be complementary to an extended recording tag, extended coding tag, or di-tag representing a polypeptide of interest. The degree of complementarity of a bait oligonucleotide to the spacer sequence in the extended recording tag, extended coding tag, or di-tag can be from 0% to 100%, and any integer in between. This parameter can be easily optimized by a few enrichment experiments. In some embodiments, the length of the spacer relative to the encoder sequence is minimized in the coding tag design or the spacers are designed such that they unavailable for hybridization to the bait sequences. One approach is to use spacers that form a secondary structure in the presence of a cofactor. An example of such a secondary structure is a G-quadruplex, which is a structure formed by two or more guanine quartets stacked on top of each other (Bochman et al., Nat Rev Genet (2012) 13(11):770-780). A guanine quartet is a square planar structure formed by four guanine bases that associate through Hoogsteen hydrogen bonding. The G-quadruplex structure is stabilized in the presence of a cation, *e.g.,* K+ ions vs. Li+ ions.

To minimize the number of bait oligonucleotides employed, a set of relatively unique peptides from each protein can be bioinformatically identified, and only those bait oligonucleotides complementary to the corresponding extended recording tag library representations of the peptides of interest are used in the hybrid capture assay. In some embodiments, sequential rounds or enrichment can also be carried out, with the same or different bait sets.

To enrich the entire length of a polypeptide in a library of extended recording tags, extended coding tags, or di-tags representing fragments thereof (*e.g.*, peptides), "tiled" bait oligonucleotides can be designed across the entire nucleic acid representation of the protein.

In another embodiment, primer extension and ligation-based mediated amplification enrichment (AmpliSeq, PCR, TruSeq TSCA, etc.) can be used to select and module fraction enriched of library elements representing a subset of polypeptides. Competing oligonucleotides can also be employed to tune the degree of primer extension, ligation, or amplification. In the simplest implementation, this can be accomplished by having a mix of target specific primers comprising a universal primer tail and competing primers lacking a 5' universal primer tail. After an initial primer extension, only primers with the 5' universal primer sequence can be amplified. The ratio of primer with and without the universal primer sequence controls the fraction of target amplified. In other embodiments, the inclusion of hybridizing but non-extending primers can be used to modulate the fraction of library elements undergoing primer extension, ligation, or amplification.

Targeted enrichment methods can also be used in a negative selection mode to selectively remove extended recording tags, extended coding tags, or di-tags from a library before sequencing. Thus, in the example described above using biotinylated bait oligonucleotides and streptavidin coated beads, the supernatant is retained for sequencing while the bait-oligonucleotide:extended recording tag, extended coding tag, or di-tag hybrids bound to the beads are not analysed. Examples of undesirable extended recording tags, extended coding tags, or di-tags that can be removed are those representing over abundant polypeptide species, *e.g.,* for proteins, albumin, immunoglobulins, etc.

A competitor oligonucleotide bait, hybridizing to the target but lacking a biotin moiety, can also be used in the hybrid capture step to modulate the fraction of any particular locus enriched. The competitor oligonucleotide bait competes for hybridization to the target with the standard biotinylated bait effectively modulating the fraction of target pulled down during enrichment. The ten orders dynamic range of protein expression can be compressed by several orders using this competitive suppression approach, especially for the overly abundant species such as albumin. Thus, the fraction of library elements captured for a given locus relative to standard hybrid capture can be modulated from 100% down to 0% enrichment.

Additionally, library normalization techniques can be used to remove overly abundant species from the extended recording tag, extended coding tag, or di-tag library. This approach works best for defined length libraries originating from peptides generated by site-specific protease digestion such as trypsin, LysC, GluC, etc. In one example, normalization can be accomplished by denaturing a double-stranded library and allowing the library elements to re-anneal. The abundant library elements re-anneal more quickly than less abundant elements due to the second-order rate constant of bimolecular hybridization kinetics (Bochman, Paeschke et al. 2012). The ssDNA library elements can be separated from the abundant dsDNA library elements using methods known in the art, such as chromatography on hydroxyapatite columns (VanderNoot, et al., 2012, Biotechniques 53:373-380) or treatment of the library with a duplex-specific nuclease (DSN) from Kamchatka crab (Shagin et al., (2002) Genome Res. 12:1935-42) which destroys the dsDNA library elements.

Any combination of fractionation, enrichment, and subtraction methods, of the polypeptides before attachment to the solid support and/or of the resulting extended recording tag library can economize sequencing reads and improve

measurement of low abundance species.

In some embodiments, a library of extended recording tags, extended coding tags, or di-tags is concatenated by ligation or end-complementary PCR to create a long DNA molecule comprising multiple different extended recorder tags, extended coding tags, or di-tags, respectively (Du et al., (2003) BioTechniques 35:66-72; Muecke et al., (2008) Structure 16:837-841; U.S. Patent No. 5,834,252). This embodiment is preferable for nanopore sequencing in which long strands of DNA are analyzed by the nanopore sequencing device.

In some embodiments, direct single molecule analysis is performed on an extended recording tag, extended coding tag, or di-tag (***see, e.g.,*** Harris et al., (2008) Science 320:106-109). The extended recording tags, extended coding tags, or di-tags can be analysed directly on the solid support, such as a flow cell or beads that are compatible for loading onto a flow cell surface (optionally microcell patterned), wherein the flow cell or beads can integrate with a single molecule sequencer or a single molecule decoding instrument. For single molecule decoding, hybridization of several rounds of pooled fluorescently-labelled of decoding oligonucleotides (Gunderson et al., (2004) Genome Res. 14:970-7) can be used to ascertain both the identity and order of the coding tags within the extended recording tag. In some embodiments, the binding agents may be labelled with cycle-specific coding tags as described above (see also, Gunderson et al., (2004) Genome Res. 14:970-7). Cycle-specific coding tags will work for both a single, concatenated extended recording tag representing a single polypeptide, or for a collection of extended recording tags representing a single polypeptide.

Following sequencing of the extended reporter tag, extended coding tag, or di-tag libraries, the resulting sequences can be collapsed by their UMIs and then associated to their corresponding polypeptides and aligned to the totality of the proteome. Resulting sequences can also be collapsed by their compartment tags and associated to their corresponding compartmental proteome, which in a particular embodiment contains only a single or a very limited number of protein molecules. Both protein identification and quantification can easily be derived from this digital peptide information.

In some embodiments, the coding tag sequence can be optimized for the particular sequencing analysis platform. In a particular embodiment, the sequencing platform is nanopore sequencing. In some embodiments, the sequencing platform has a per base error rate of > 1%, > 5%, > 10%, >15%, > 20%, > 25%, or > 30%. For example, if the extended recording tag is to be analyzed using a nanopore sequencing instrument, the barcode sequences (e.g., encoder sequences) can be designed to be optimally electrically distinguishable in transit through a nanopore. Peptide sequencing according to the methods described herein may be well-suited for nanopore sequencing, given that the single base accuracy for nanopore sequencing is still rather low (75%-85%), but determination of the "encoder sequence" should be much more accurate (> 99%). Moreover, a technique called duplex interrupted nanopore sequencing (DI) can be employed with nanopore strand sequencing without the need for a molecular motor, greatly simplifying the system design (Derrington et al., Proc Natl Acad Sci U S A (2010) 107(37): 16060-16065). Readout of the extended recording tag via DI nanopore sequencing requires that the spacer elements in the concatenated extended recording tag library be annealed with complementary oligonucleotides. The oligonucleotides used herein may comprise LNAs, or other modified nucleic acids or analogs to increase the effective Tm of the resultant duplexes. As the single-stranded extended recording tag decorated with these duplex spacer regions is passed through the pore, the double strand region will become transiently stalled at the constriction zone enabling a current readout of about three bases adjacent to the duplex region. In a particular embodiment for DI nanopore sequencing, the encoder sequence is designed in such a way that the three bases adjacent to the spacer element create maximally electrically distinguishable nanopore signals (Derrington et al., Proc Natl Acad Sci U S A (2010) 107(37): 16060-16065). As an alternative to motor-free DI sequencing, the spacer element can be designed to adopt a secondary structure such as a G-quartet, which will transiently stall the extended recording tag, extended coding tag, or di-tag as it passes through the nanopore enabling readout of the adjacent encoder sequence (Shim et al., Nucleic Acids Res (2009) 37(3): 972-982; Zhang et al., mAbs (2016) 8, 524-535). After proceeding past the stall, the next spacer will again create a transient stall, enabling readout of the next encoder sequence, and so forth.

The methods disclosed herein can be used for analysis, including detection, quantitation and/or sequencing, of a plurality of polypeptides simultaneously (multiplexing). Multiplexing as used herein refers to analysis of a plurality of polypeptides in the same assay. The plurality of polypeptides can be derived from the same sample or different samples. The plurality of polypeptides can be derived from the same subject or different subjects. The plurality of polypeptides that are analyzed can be different polypeptides, or the same polypeptide derived from different samples. A plurality of polypeptides includes 2 or more polypeptides, 5 or more polypeptides, 10 or more polypeptides, 50 or more polypeptides, 100 or more polypeptides, 500 or more polypeptides, 1000 or more polypeptides, 5,000 or more polypeptides, 10,000 or more polypeptides, 50,000 or more polypeptides, 100,000 or more polypeptides, 500,000 or more polypeptides, or 1,000,000 or more polypeptides.

Sample multiplexing can be achieved by upfront barcoding of recording tag labeled polypeptide samples. Each barcode represents a different sample, and samples can be pooled prior to cyclic binding assays or sequence analysis. In this way, many barcode-labeled samples can be simultaneously processed in a single tube. This approach is a significant improvement on immunoassays conducted on reverse phase protein arrays (RPPA) (Akbani et al., Mol Cell Proteomics

(2014) 13(7): 1625-1643; Creighton et al., Drug Des Devel Ther (2015) 9: 3519-3527; Nishizuka et al., Drug Metab Pharmacokinet (2016) 31(1): 35-45). In this way, the present disclosure essentially provides a highly digital sample and analyte multiplexed alternative to the RPPA assay with a simple workflow.

## IV. KITS AND RELATED ARTICLES OF MANUFACTURE

Provided herein are kits comprising one or more modified dipeptide cleavase(s) comprising a mutation, *e.g.*, one or more amino acid modifications in an unmodified dipeptide cleavase and a reagent for or labeling the terminal amino acid of a polypeptide. In some aspects, the modified dipeptide cleavase is derived from a dipeptide cleavase and removes a labeled terminal dipeptide from a polypeptide. In some embodiments, the kits also include instructions for using the reagents for treating polypeptide(s) for analysis and/or sequencing. In some embodiments, the kits comprising one or more modified dipeptide cleavase(s) (*e.g.*, as described in Section I) are for use in treating peptide(s), polypeptide(s), and protein(s) for sequencing and/or analysis. In some embodiments, the protein analysis employs barcoding and nucleic acid encoding of molecular recognition events, and/or detectable labels. In some embodiments, the kits also include other components for treating the polypeptide(s) and analysis of the polypeptide(s), including tag(s) (*e.g.,* a DNA tag or a DNA recording tag), solid support(s), and other reagent(s) for preparing the polypeptide(s) and reagent(s) for polypeptide analysis.

In some embodiments, the kits also comprise a modified or engineered cleavase that removes a labeled single amino acid from a polypeptide (*see e.g.,* the above Section I.) In some embodiments, the present kits can comprise a modified or an engineered cleavase described and/or claimed in U.S. provisional application serial Nos. 62/823,927, filed March 26, 2019, 62/824,157, filed March 26, 2019, and 62/931,737, filed November 6, 2019, and in application WO 2020/198264 published on October 01, 2020.

In some embodiments, the kit comprises more than one modified dipeptide cleavase. In some cases, a variety of modified dipeptide cleavases may exhibit different characteristics, for example, preferences for binding polypeptides and/or cleaving amino acids. In some embodiments, two or more modified dipeptide cleavases may be included in the kit as a mixture of enzymes or separately with each modified dipeptide cleavase in a container. In some embodiments, the different modified dipeptide cleavases are contacted with polypeptides simultaneously or sequentially.

In some embodiments, the kit also comprises one or more additional enzyme(s) to eliminate the NTAA (*e.g.,* a proline aminopeptidase). In some specific examples, the additional enzyme is a proline aminopeptidase, a proline iminopeptidase (PIP), or a pyroglutamate aminopeptidase (pGAP). In some embodiments, one or more modified dipeptide cleavases are provided in combination with other enzymes in the kit. In some specific cases, the modified dipeptide cleavase and other enzymes are provided as a cocktail in the kit.

In some embodiments, the kit also comprises one or more buffer(s) or a reaction fluid that comprises the substrate(s), ion(s), and factor(s) necessary for the desired reaction to occur. Buffers including wash buffers, reaction buffers, and binding buffers, elution buffers and the like are known to those or ordinary skill in the arts. In some embodiments, the modified dipeptide cleavase is a metallopeptidase and the kit comprises a buffer comprising metal ions required for activation of the modified dipeptide cleavase. In some examples, the kit comprises the require metal ions required for activation of the modified dipeptide cleavase, *e.g.*, zinc ions or chloride ions. In some embodiments, the kit further comprises metal-chelating agents or other reagents for inactivating the modified dipeptide cleavase. In some embodiments, the kits further include buffers and other components to accompany other reagents described herein. The reagents, buffers, and other components may be provided in vials (such as sealed vials), vessels, ampules, bottles, jars, flexible packaging (*e.g.*, sealed Mylar or plastic bags), and the like. Any of the components of the kits may be sterilized and/or sealed.

In some embodiments, the kits further comprise one or more binding agent(s), wherein each binding agent comprises a coding tag with identifying information regarding the binding agent. In some cases, the kit comprises two or more binding agents. In some examples, the kit comprises a library of binding agents. In some embodiments, the two or more binding agents may be provided in individual containers or as a mixture in a container. In some embodiments, the kit further includes a reagent for transferring the identifying information of the coding tag to a recording tag attached to the polypeptide, wherein the transferring of the identifying information to the recording tag generates an extended recording tag on the polypeptide. In some cases, the reagent for transferring identifying information is a chemical ligation reagent or a biological ligation reagent.

In some embodiments, the kit further includes an amplification reagent for amplifying the extended recording tags.

In some embodiments, the kit further comprises substrate(s) selected from the group consisting of a bead, a porous bead, a magnetic bead, a paramagnetic bead, a porous matrix, an array, a surface, a glass surface, a silicon surface, a plastic surface, a slide, a filter, nylon, a chip, a silicon wafer chip, a flow through chip, a biochip including signal transducing electronics, a well, a microtitre well, a plate, an ELISA plate, a disc, a spinning interferometry disc, a membrane, a nitrocellulose membrane, a nitrocellulose-based polymer surface, a nanoparticle (*e.g.*, comprising a metal

such as magnetic nanoparticles ($Fe_3O_4$), gold nanoparticles, and/or silver nanoparticles), quantum dots, a nanoshell, a nanocage, and a microsphere, or any combination thereof. In some embodiments, the kit comprises a plurality of substrates.

In some embodiments, the kit includes one or more reagent(s) for nucleic acid sequence analysis. In some examples, the reagent for sequence analysis is for use in sequencing by synthesis, sequencing by ligation, sequencing by hybridization, polony sequencing, ion semiconductor sequencing, pyrosequencing, single molecule real-time sequencing, nanopore-based sequencing, or direct imaging of DNA using advanced microscopy, or any combination thereof.

In some embodiments, the kits or articles of manufacture may further comprise instruction(s) on the methods and uses described herein. In some embodiments, the instructions are directed to methods of preparing and treating polypeptides, including the modified dipeptide cleavase provided herein. The kits described herein may also include other materials desirable from a commercial and user standpoint, including other buffers, diluents, filters, syringes, and package inserts with instructions for performing any methods described herein.

Any of the above-mentioned kit components, and any molecule, molecular complex or conjugate, reagent (*e.g.*, chemical or biological reagents, including modified dipeptide cleavases), agent, structure (*e.g.*, support, surface, particle, or bead), reaction intermediate, reaction product, binding complex, or any other article of manufacture disclosed and/or used in the exemplary kits and methods, may be provided separately or in any suitable combination in order to form a kit. The kit may optionally comprise instructions for using the modified dipeptide cleavase.

## V. EXAMPLES

The following examples are offered to support the disclosure.

Compounds used in the invention can be made by methods known in the art in view of the following examples. A representative method for attaching an NTM to a target polypeptide is as follows, using a representative NTM of Formula (5) to attach an NTM to the NTAA of a target polypeptide:

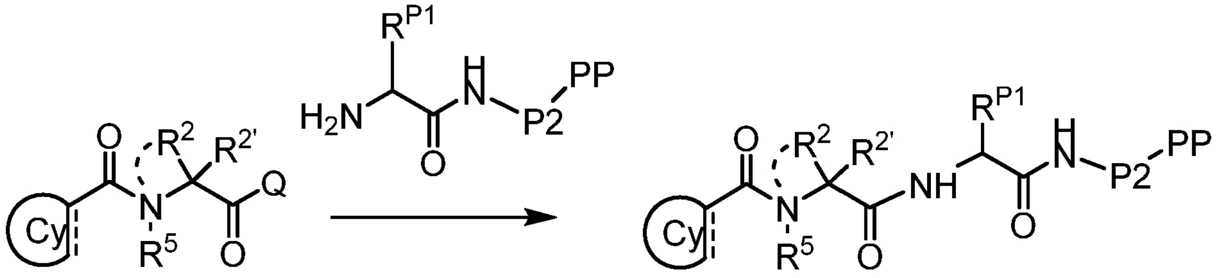

(5)

In this general reaction scheme, $R^{P1}$ is the side chain of the N-terminal amino acid of a target polypeptide, P2 is the penultimate residue of the polypeptide, and PP represents the remainder of the target polypeptide: $R^{P1}$ is typically selected from the 20 common amino acid side chains, optionally protected amino acid side chains, posttranslationally modified amino acid side chains, and unnatural amino acid sidechains; for example a side chain of any of these amino acids: Alanine, aspartic acid, isoaspartic acid, asparagine, N-glycosylated asparagine, glutamic acid, glutamine, glycine, (2-, 3-, or 4-pyridyl-)alanine, phenylglycine, 4-fluorophenylglycine, leucine, isoleucine, valine, dimethylglycine, methionine, methionine sulfoxide, phenylalanine, serine, phosphoserine, O-glycosylated serine, threonine, phosphothreonine, O-glycosylated threonine, cysteine, carbamidomethylcysteine, S-glycosylated cysteine, selenocysteine, sulfenic acid, sulfinic acid, sulfonic acid, tyrosine, sulfotyrosine, phosphotyrosine, nitrosotyrosine, tryptophan, histidine, N-acetyllysine, N-methyllysine, N,N-dimethyllysine, N,N,N-trimethyllysine, N-azidolysine , citrulline, nitroarginine, methylarginine, dimethylarginine, proline, hydroxyproline, or a salt thereof. The features in Formula (5) are as described herein for chemical reagents of Formula (5).

Reagents comprised of active esters (e.g., compounds of Formulas (3)-(9) wherein Q is an $R^Q$ as described for the Formula) are dissolved in one of the following polar organic solvents; acetonitrile (ACN), *N,N*-dimethylformamide (DMF), *N,N*-dimethylacetamide (DMAc), N-methyl-2-pyrrolidone (NMP), sulfolane, dimethylsulfoxide (DMSO), cyrene, 1,3-dimethyl-2-imidazolidinone (DMI), and 1,3-Dimethyl-3,4,5,6-tetrahydro-2(1H)-pyrimidinone (DMPU)

Buffers used for this reaction are typically selected from:

Sodium acetate, potassium acetate, ammonium acetate, sodium phosphate, potassium phosphate, ammonium phosphate, PBS, MES, MOPS, HEPES, Tris-HCl, NEMA, PIPES, HEPPSO, triethylammonium acetate, triethanolammonium acetate, citrate, cit-phos, CAPS, CAPSO, bicarbonate, carbonate-bicarbonate, carbonate, borate, and

bis-tris,
where the pH of the buffer is in a range of 4-12; typically 6-11, and preferably 7-10.

Example 1: Selection, Design, and Isolation of Modified Dipeptide Cleavases

This example describes the selection and isolation of exemplary modified dipeptide cleavases, and engineering of dipeptidyl peptidase 3 (DPP3), dipeptidyl peptidase 5 (DPP5) and dipeptidyl aminopeptidase BII (DAP BII) proteins for selected activities by rational design.

### *A. Genetic Selection for DPP3, DPP5, and DAP BII variants active on modified NTAA peptides*

To identify optimal engineered modified dipeptide cleavases such as DPP3, DPP5, and DAP BII variants, genetic selection is carried out using an amino acid-specific auxotrophic *E. coli* strain (available from CSSC *E. coli* Genetic Stock Center at Yale - https://cgsc2.biology.yale.edu/) that only survives on minimal media plates when supplied with the auxotrophic amino acid or a short peptide containing the auxotrophic amino acid. *See e.g.,* Neuenschwander et al., Nat Biotechnol. (2007) 25(10):1145-1147). This cell-based assay system is used to select variants functional on labeled polypeptides as follows: The cleavase genes (such as DPP3, DPP5, and DAP BII) are separately expressed in an auxotrophic strain supplemented with a Cbz-labeled tetrapeptide (Cbz-AAAR, SEQ ID NO: 21) in which the C-terminal diamino acid acts as the auxotrophic supplement upon native dipeptide uptake and cleavage in the cytosol. Short oligopeptide substrates permeate into the periplasm through outer membrane porin channels but require active transport into the cytoplasm via three main oligopeptide/dipeptide uptake systems in *E. coli:* Opp, Tpp, and Dpp (Abouhamad et al., Mol Microbiol. (1991) 5(5):1035-1047); after uptake into the cytoplasm, short peptides are digested by endogenous endopeptidases within the cytosol. Oligopeptide or dipeptide transport by these three systems is inhibited by N-terminal modified (*e.g.,* Cbz) oligopeptides/dipeptides (Smith et al., Microbiology (1999) 145(Pt 10):2891-901; Payne et al., Arch Biochem Biophys. (2000) 384(1):9-23; Fang et al., J Bacteriol. 2000 May;182(9):2530-2535). The growth of auxotrophic *E. coli* on the Cbz-labeled feedstocks will be inhibited. Genetic selection is accomplished by relieving this inhibition by expression and secretion of functional protein in the periplasm using an appropriate signal peptide (*e.g.* pelB) (Speck et al., Protein Eng Des Sel. (2011) 24(6):473-484; Thie et al., N Biotechnol. (2008) 25(1):49-54). Once in the periplasm, functional protein converts the Cbz-oligopeptide to free auxotrophic dipeptides that are taken up into the cell cytoplasm.

*E. coli* strains auxotrophic for an amino acid; such as arginine, glutamine, or tryptophan; are employed in the genetic selection. Other suitable strains can also be used for selection. The growth media for the genetic selection is M9 minimal media salts supplemented with $MgSO_4$, $CaCl_2$, glucose, and agar. The appropriate Cbz-labeled peptide is also added to the growth media before the solution is poured into a plate to solidify.

A general approach to testing families of cleavase genes (*e.g.,* DPP3, DPP5, and DAP BII), is to select a family in the NCBI database cluster based on the homology of their encoded proteins. A pool of genes that contains a representative from each cluster is selected. The genes for selected proteins are synthesized using codons optimized for expression in *E. coli.* The genes, encoding proteins from various organisms, are pooled and libraries of mutated genes are generated by error prone PCR or rational mutagenesis using the crystal structure of proteins with known structure. Furthermore, a combination of error prone PCR and rational mutagenesis is used to generate additional mutated libraries. The pool of mutated genes is subsequently cloned into a vector which has a promoter that is compatible with gene expression in the auxotroph strains, such as a T5 or arabinose promoter. The library of mutated genes, is cloned into a vector, which adds a periplasmic targeting signal (*e.g.,* pelB) to the N-terminus of the encoded protein. The cloned library is then transformed into an *E. coli* auxotroph strain. After recovery of the transformed cells in rich media (*e.g.,* SOC), the cells are washed with M9 minimal liquid media to remove all traces of proteins that will allow the auxotroph strain to subvert the genetic selection by presenting as false positives. The cells are then spread onto the selection media containing the Cbz-labeled peptide. The plates are incubated at a temperature ranging from 25 to 37 degrees Celsius until colonies are observed. Colonies growing on the selection media are isolated, and plasmid DNA is extracted. The cleavase gene is then sequenced to identify the protein sequence that can remove the Cbz-labeled peptide.

Various lengths and sequences of Cbz-labeled peptides can be used in the genetic selection to generate enzymes with specificities that can remove all 20 modified natural amino acids used in polypeptide synthesis.

### *B. Rational design of DPP3, DPP5, and DAP BII for activity on modified NTAA peptides*

A rational design approach for engineering DPP3 to remove a labeled N-terminal amino acid (NTAA) is guided using crystal structures of DPP3 in complex with substrates. In structures of human DPP3 in complex with substrates, the residues Glu 316, Asn 391, and Asn 394 (based on the sequence of the protein set forth in SEQ ID NO: 5; UniProt Accession No. Q9NY33) make hydrogen bonding interactions with the peptide N-terminal amine group. These residues, individually or in combination, are altered to select for modified dipeptidyl peptidases that accommodate a labeled NTAA.

Due to the lack of crystal structure of DPP5 with substrate, comparative modeling tools such as Rosetta macromolecular modeling suite is used to generate a homology model of DPP5. Based on the model and sequence analysis, the loop between Thr127-Thr180 (based on NCBI reference sequence WP_012457755.1, SEQ ID NO: 16) is identified to be a hypothetical region for binding native N-terminal amino acid and thus a region for engineering to recognize modified amino acid. Multiple acidic residues that can bind N-terminal amine in this region are highly conserved, including Asp142, Asp153 and Asp160. Multiple approaches can be used to explore this loop region, including error prone PCR, site saturated mutagenesis, and replacement with homologous loops from Blast search. These different diversification strategies are built into a library via Kunkel based approach using *in vitro* generated oligos by PCR or commercially synthesized oligos. In addition to testing small changes in the loop region, *in vitro* recombination can also be used to combine large sequence changes, and/or error prone PCR of the full length to explore regions outside of the loop. Mutational scanning and random error prone based approach can identify hotspot region for next round of library creation and screening.

A rational design approach for engineering DAP BII to remove a labeled N-terminal amino acid (NTAA) as a dipeptide is guided using crystal structures of DAP BII in complex with substrates (Sakamoto et al., Scientific Reports 2014, 4:4977). In the DAP BII structure in complex with a peptide substrate, the residues N191, W192, R196, N306, and D650 (based on the sequence of the protein set forth in SEQ ID NO: 13; UniProt Accession No. V5YM14) make hydrogen bonding interactions with the peptide N-terminal amine group. Additionally, in the native DAP BII crystal structure, a loop of approximately 20 residues (residue 183-202) makes contact with the N-terminal residue and penultimate residue of a bound peptide substrate. These amine binding residues and NTAA and penultimate NTAA binding residues, individually or in combination, are altered to select for modified dipeptide cleavases that cleaves a dipeptide containing the labeled NTAA residue with minimal bias. A combinatorial variant libraries of these residues with Kunkel and related methods (Kunkel, T (1985). PNAS 82(2): 488-492) are created, and genetic selection libraries are used to screen for variants with altered activities toward modified N-terminal amino acid. An error prone based library is built based on the hits from initial screening for next round of library creation and screening.

***C. Engineered variants of DPP3 active on longer peptides***

In some cases, DPP3 enzymes may be limited in their maximal peptide substrate length. For example, the human enzyme has a peptide substrate length limit of 8 to 10 amino acids. A genetic selection is carried out to identify modified dipeptidyl peptidase enzymes that are able to cleave peptide sizes or lengths that are increased compared to the unmodified dipeptidyl peptidase. The porin size in the *E. coli* outer membrane limits the peptide length that can be uptaken to five or six amino acids. To subvert this size limit, biotinylated peptides up to 31 amino acids in length that can be uptaken by *E. coli* via the biotin transporter are used as *in vivo* substrates for a DPP3 enzyme.

A rational design approach for increasing the peptide length that can be cleaved by DPP3 is carried out using crystal structures of DPP3 in complex with substrates. In the structure of human DPP3 in complex with the eight amino acid peptide, DRVYIHPF (SEQ ID NO: 9), the region of DPP3 which constrains the peptide length is deduced. For example, amino acid residues 419-426 (numbered according to human DPP3 set forth in SEQ ID NO: 5) is targeted for mutagenesis or removal to allow DPP3 to be active with longer peptides.

***D. Purification and characterization conditions***

Selected cleavase enzymes (*e.g.*, DPP3, DPP5, DAP BII) are produced with a purification tag, such as a six histidine tag, and purified using the tag. Fluorescent or colorimetric substrates are generated by Cbz modifying amino acids that are conjugated to a molecule produces a signal upon cleavage of the Cbz modified amino acid. The amino acid conjugated substrates that are used include amino acid-nitroanilides, amino acid-β-naphthylamides, and amino acid-amidomethyl coumarins. These substrates are used to rapidly assay and optimize the activity of selected modified enzymes.

Example 2: Labeling of N-terminal amino acid (NTAA) of peptides with chemical compounds mimicking "amino-acid" like profiles.

This example describes the labeling of the N-terminal amino acid by treating the polypeptides with various chemical reagents. Addition of a benzyloxycarbonyl (Cbz), phenylisothiocyanate (PITC) or PITC derivative to the N-terminus of a peptide resembles adding a tyrosine or phenylalanine to the N-terminal amino acid, which is a natural substrate for dipeptidyl peptidase such as DPP3, DPP5, or DAP BII. The main distinguishing feature is the absence of an N-terminal amine group. Several reagents for labeling the N-terminal amino acid were tested for the ability to efficiently label the N-terminus. The chemical reagents for labeling the amino acid were also tested for its effect on modifying native DNA. For example, four different N-terminal modifying reagents are shown:

Pyridyl-ITC

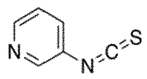

Nitro-PITC

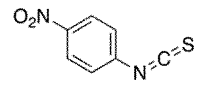

Sulfo-PITC

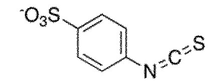

Guanidinylation Reagent

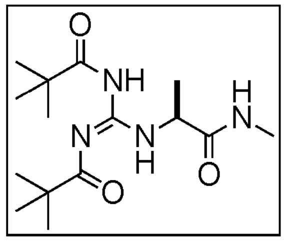

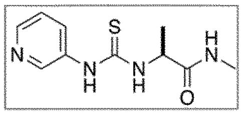

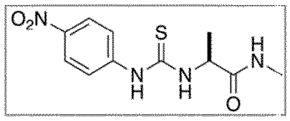

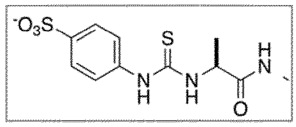

The modifying reagents for labeling the peptides include three isothiocyanates (Pyridyl-ITC, Nitro-PITC and Sulfo-ITC). In some cases, isocyanates could be used in place of isothiocyanates to create a urea (oxygen) rather than thiourea (sulfur) in the final modified NTAA. The fourth reagent shown is a proprietary guanidinylation derivative. Pyridyl-ITC is from a class of known Edman modifying reagents based on isothiocyanates, which generate an N-terminus that self-eliminates under acidic conditions. Nitro-PITC and Sulfo-PITC are more active Edman derivatives of phenylisothiocyanate (PITC). All isothiocyanate reagents were tested for peptide NTAA modification of two exemplary peptides (a peptide with an N-terminal G (NT-G) = GRFSGIY (SEQ ID NO: 29); a peptide with an N-terminal W (NT-W) = WTQIFGA (SEQ ID NO: 30)) under aqueous conditions. The PITC-related derivatizations were performed at 60°C for 15 min. in 1X PBS buffer with 25 mM of the indicated reagent. The guandinylation derivatization was performed at 60°C for 1 hour in 1X PBS buffer (pH 7.4) with 10% DMSO using 15 mM of the guandinylation reagent. A total of 50 equivalents of the reagent was used in the solution assay. LC-MS was used to quantitate conversion efficiency of the peptides by the various modifying reagents.

In all cases, for the modifying reagents for labeling the peptides shown, quantitative modification was observed without any DNA modification. PITC was also run as a control, but under the conditions tested did not generate complete modification. As shown in FIG. 3, high-yield labeling of the peptides with Pyridyl-ITC, Nitro-PITC and Sulfo-ITC and high-yield guanidinylation was observed with both peptides.

Example 3: Selection, Isolation, and Assessment of DAP BII Derived Modified Dipeptide Cleavases

This example describes the generation of libraries of variant DAP BII genes and identification of active modified dipeptide cleavases from genetic selection.

A DAP BII library was generated substantially as described in Example 1, using a DAP BII library that targeted various combinations of residues selected from positions 188, 189, 190, 191, 192, 196, 302, 306, 310, and 650 (based on the sequence of the protein set forth in SEQ ID NO: 13). The variant DAP BII libraries were transformed into an arginine auxotroph strain of *E. coli,* which has a deletion in the *argA* gene (strain JW2786-1). The cleavase genes were expressed with a periplasm targeting sequence PelB signal sequence. Genetic selection was performed on the transformed *E. coli* using M9 minimal media agar plates supplemented with arginine N-terminal modified peptides. The plates were incubated at 35 °C until colonies appeared. In the selection, cells harboring a modified (*e.g.* DAP BII) cleavase that is active against N-terminally modified arginine peptides (AAAR (SEQ ID NO: 21)) will cleave the peptide and release arginine as a part of the AR dipeptide. This release of arginine will enable the cells to survive. An exemplary chemical reagent, isatoic anhydride, was used to label the N-terminal of arginine-containing peptides. The plates were incubated at 35 °C until colonies appeared. From the surviving cells, plasmid DNA was subsequently isolated and sequenced to identify the mutations that generate an active modified dipeptide cleavase that recognizes labeled amino acids.

Using the described genetic selection approach, mutations in DAP BII were identified in exemplary active modified dipeptide cleavases derived from wildtype DAP BII genes. Candidates that were identified in the genetic selection were confirmed by purification of the encoded enzyme which was subjected to in-solution assays. The cleavase gene encodes a hexa-histidine tag fused to the C-terminus of the protein, which enables the cleavase to be purified via immobilized metal affinity chromatography. Purified modified dipeptide cleavase candidates were assayed in reaction mixtures consisting of HEPES (50 mM, pH 7.5), EDTA (1 mM), cleavase enzyme (100 nM to 1 μM), and N-terminal 2-aminobenzamide-labeled peptide with the sequence AAGVAMPGAEDDVVGSGSK($N_3$) as set forth in SEQ ID NO: 22 (100 μM). The reactions were incubated between 25 °C and 37 °C for 30 min to 3 h. Reaction mixtures were then analyzed via LC-MS for product identification and results are shown in **Table 7.**

| Table 7. LC-MS data of reaction products. | | | |
|---|---|---|---|
| **Product 1 Observed Mass** | **Product 2 Observed Mass** | **Expected Mass for [2-aminobenzamide-AA] (M+H)** | **Expected Mass for [GVAMPGAEDDVVGSGSK(azide)] (M/2)** |
| 279.2 | 801 | 279.1 | 801 |

Three exemplary dipeptide cleavases containing the sequences as set forth in SEQ ID NOs: 17, 18, and 19 were identified and shown to exhibit similar cleaving activity as shown in **Table 7.** The confirmed active modified dipeptide cleavases contained mutations D188V/I189A/D190S/N191L/W192G/R196S/A302W/N310K/D650A, N191M/W192G/R196T/N306R/D650A, or N191M/W192G/R196V/N306R/D650A, where the exemplary amino acid substitutions are designated by amino acid position number corresponding to the respective reference unmodified DAP BII sequence set forth in SEQ ID NO:13. The amino acid position is indicated in the middle, with the corresponding unmodified (*e.g.* wild-type) amino acid listed before the number and the identified variant amino acid substitution listed after the number. As shown, the LC-MS data identified two reaction products, product 1 has an observed mass of 279.2 and product 2 has an observed mass of 801. The expected mass for 2-aminobenzamide-AA is 279.1 (M+H). The expected mass for the C-terminal product of 2-aminobenzamide-AAGVAMPGAEDDVVGSGSK($N_3$) after cleavage (GVAMPGAEDDVVGSGSK($N_3$); SEQ ID NO: 53) is 801 (M/2). These data demonstrate that the identified modified dipeptide cleavases were removing the expected labeled dipeptide (2-aminobenzamide-AA) from the treated polypeptides. The same cleavase may accommodate cleavage of a single terminal amino acid from a polypeptide, when the bipartite label (2-aminobenzamide-alanine) is used, and the substrate is 2-aminobenzamide-Ala-labeled polypeptide.

Starting from the identified dipeptide cleavase set forth in SEQ ID NO: 18, error prone PCR combined with Kunkel mutagenesis was further used to generate libraries of ~$10^9$ complexity with precise control over a desired mutation frequency range (Holland et al., J Immunol Methods. (2013) 394(1-2):55-61). Expression and selection was performed substantially as described above for genetic selection. Identified and purified modified dipeptide cleavase candidates were assessed using the in-solution assays substantially as described above to test cleavage of the 2-aminobenzamide-AAGVAMPGAEDDVVGSGSK($N_3$) peptide and LC-MS was performed for product identification. Confirmed modified dipeptide cleavases shown in **Table 8** were observed to remove the expected labeled dipeptide (2-aminobenzamide-AA, or M15-AA) from the treated polypeptides. In the table, the exemplary amino acid substitutions are designated by amino acid position number corresponding to the respective reference unmodified DAP BII sequence set forth in SEQ ID NO:13. The amino acid position is indicated in the middle, with the corresponding unmodified (*e.g.* wild-type) amino acid listed before the number and the identified variant amino acid substitution listed after the number.

| Table 8. Exemplary Modified Dipeptide Cleavases | |
|---|---|
| **SEQ ID NO** | **Mutations** |
| 23 | N191M/W192G/R196T/N306R/T307K/D650A |
| 24 | N191M/W192G/R196T/N306R/N525K/A528V/A604V/D650A/K692N |
| 25 | A126T/N191M/W192G/R196T/G238V/N306R/D650A |
| 26 | N191M/W192G/R196T/N306R/F546L/D650A |
| 27 | N191M/W192G/R196T/N306R/D650A/G651V/K665I |
| 28 | N191M/W192G/R196T/N306R/D650A/G651V |

Exemplary cleavases that removed a single labeled terminal amino acid were selected and shown to exhibit similar cleaving activity as shown in **Table 9.** The confirmed active modified dipeptide cleavases contained mutations as set forth in **Table 10,** where the exemplary amino acid substitutions are designated by amino acid position number corresponding to the respective reference unmodified DAP BII sequence set forth in SEQ ID NO:13. The amino acid position is indicated in the middle, with the corresponding unmodified (*e.g.*, wild-type) amino acid listed before the number and the identified variant amino acid substitution listed after the number. As shown, the LC-MS data identified a product with an observed mass of 836.4, which matches the expected mass of the C-terminal product, AGVAMPGAEDDVVGSGSK($N_3$) (SEQ ID NO:54), after cleavage and removal of the labeled terminal amino acid (A). These data demonstrate that the described process modified a wildtype dipeptide cleavase, DAP BII, which naturally removes unlabeled dipeptides, to remove a single labeled terminal amino acid.

| Table 9. LC-MS data of Cleavase reaction products. | |
|---|---|
| **Product 1 Observed Mass** | **Expected Mass for [AGVAMPGAEDDVVGSGSK(azide)] (M/2)** |
| 836.4 | 836.4 |

| Table 10: Exemplary Modified Cleavases | | | |
|---|---|---|---|
| **SEQ ID NO** | **Mutations** | **Chemical Reagent Used to Label Target Peptide** | **Product Removed by Modified Cleavase** |
| 36 | N191C/W192L/R196K/N306R/N310D/G651Y/S655G/V656G | Isatoic Anhydride | 2-aminobenzamide-P1 |
| 37 | N191C/W192L/N306R/N310D/G651Y/S655G/V656G | Isatoic Anhydride | 2-aminobenzamide-P1 |
| 38 | N191F/W192F/N306R/N310G/G651HN656E | Isatoic Anhydride | 2-aminobenzamide-P1 |
| 39 | N191R/W192L/N306S/N310L/G651T/S655T/V656S | Isatoic Anhydride | 2-aminobenzamide-P1 |
| 40 | N191S/R196H/N306A/D650G | 5-nitro isatoic anhydride | 5-nitro-2-aminobenzamide-P1 |
| 41 | N191T/R196H/N306A/D650G | 5-nitro isatoic anhydride | 5-nitro-2-aminobenzamide-P1 |
| 42 | N191M/R196H/N306A/D650G | 5-nitro isatoic anhydride | 5-nitro-2-aminobenzamide-labeled P1 |
| 43 | N191V/N306A/D650S | Succinic anhydride | Succinic acid-labeled P1 |
| 44 | N191S/N306G/D650S | Succinic anhydride | Succinic acid-labeled P1 |

Example 4: Kinetic study of DAP BII derived modified dipeptide cleavases from genetic selection and error-prone libraries.

This example describes assessment of kinetics of the modified dipeptide cleavases isolated from the genetic selection and error prone libraries described in Example 3. One modified dipeptide cleavase from the original genetic selection (SEQ ID NO: 18) and one modified dipeptide cleavase from the error prone PCR library (SEQ ID NO: 27) were evaluated.

Steady-state kinetics of modified dipeptide cleavase enzymes were determined by using the Michaelis-Menten equation, $v_o = \frac{V_{MAX}[S]}{K_M+[S]}$; where $v_o$ represents the initial velocity of the reaction, $V_{MAX}$ represents the maximum velocity reached by the system at saturation of substrate, S, and $K_M$ represents the Michaelis constant which is equal to $\frac{V_{MAX}}{2}$ and relates to the substrate binding affinity to the enzyme. *See e.g.* Berg et al. Biochemistry. 5th edition. New York: W H Freeman; (2002) Section 8.4, The Michaelis-Menten Model Accounts for the Kinetic Properties of Many Enzymes). These experiments were performed using a clear 96-well plate and monitored by a 96-well plate reader capable of measuring absorbance at 405nm wavelength in 11-second intervals. A standard curve was made using a dilution series of*para*-nitroaniline (pNA) from 1 mM through 0.001 mM in 20% DMA in 80 mM HEPES (pH = 8.0) and the absorbance was monitored at 37 °C to obtain a linear relationship of absorbance at 405nm versus pNA concentration. Then a dilution series (8 mM through 0.125 mM) of target enzyme was made using 80 µL total volume in 100 mM HEPES (pH = 8.0). A stock solution of 2-aminobenzamide-AA-pNA was prepared in DMA resulting in a concentration of 25 mM. From this stock solution, 20 µL was added to the enzyme dilution to bring the total volume to 100 µL and the concentration of 2-aminobenzamide-AA-pNA to 5 mM. The resulting solution was incubated at 37 °C for 5 minutes and monitored on the plate reader at 405nm. The enzyme concentration for the kinetics assay was determined by showing the maximum absorbance achieved was less than the detection limit of the instrument and within the range of the pNA standard curve.

From this, the enzyme concentration selected for an enzyme (containing the amino acid sequences as set forth in SEQ ID NO: 18 and SEQ ID NO: 27) were 1 uM and 0.8 uM, respectively. Using these concentrations, 80 µL of 1.25 uM (SEQ ID NO: 18) or 1.0 uM (SEQ ID NO: 27) were added to 12 wells in a single row and incubated for 10 minutes at 37 °C.

Separately, two different dilution series of 2-aminobenzamide-AA-pNA were prepared by diluting either a 60 mM solution in DMA by a factor of 0.5 for 11 wells or a 50 mM solution in DMA by a factor of 0.6 for 11 wells. The kinetic experiment was performed by adding 20μL of the dilution series of 2-aminobenzamide-AA-pNA to the 80 μL wells of enzyme; bringing the total enzyme concentration to 1 uM or 0.8 uM, respectively and the concentration range of substrate to 12 mM through 0.012 mM or 10 mM through 0.06 mM, respectively. Once the substrate was added, the plate reader scanned the wells by monitoring absorbance at 405nm at 37 °C over a time course of 5 minutes to obtain the rate of product formation as a function of time in minutes. The initial rate of each reaction was obtained by taking the slope of the linear portion of the data at 0-60 seconds. That slope value versus the concentration value of substrate for a specific well was then input into the Michaelis-Menten equation using SigmaPlot to obtain the values shown in **Table 11** and the non-linear relationship shown in **FIG. 5.** The experimental results showed that the modified dipeptide cleavase isolated from the error-prone library approach (SEQ ID NO: 27) exhibits binding affinity of substrate to the binding pocket, by lowering the $K_M$ and increase the catalytic efficiency ( $\frac{k_{cat}}{K_M}$ ) of the enzyme from the original genetic selection (SEQ ID NO: 18) process by > 4-fold.

| Table 11. Kinetic Study Results | | | |
|---|---|---|---|
| SEQ ID NO: 18 | | SEQ ID NO: 27 | |
| $E_T$ | = 1.000 | $E_T$ | = 0.8000 |
| $k_{cat}$ | 90.75 | $k_{cat}$ | 272.9 |
| $K_M$ | 2.957 | $K_M$ | 2.002 |
| $V_{max}$ | = 90.75 | $V_{max}$ | = 218.3 |

Example 5: Development of NTM modification for evolving NTM-P1 anticalin-based binding agents with minimal P2 bias.

**Anticalin scaffold selection and library design.** Lipocalins were used as starting scaffolds for directed evolution toward modified NTAAs. Anticalins have an intrinsic cup-like binding pocket, highly stable structure, good recombinant expression in *E. coli.,* binding pocket evolvability using phage display, and demonstrated potential for strong and specific binding to small molecules. Based on internal data and computational modeling, NTMs were designed such that when combined with the P1 amino acid (N-terminal residue), the NTM-P1 moiety occupies the anticalin $\beta$-barrel core, with the P1 sidechain oriented closer to the surface of the pocket. Many anticalins have an intrinsic ability to bind a modified-dipeptide residue. This design forces the P2 residue (penultimate residue) of the peptide to be located just outside the pocket or affinity determining region and contribute less energy to binding. In particular, an $NTM_{blk}$ comprised of a Abz-L was evaluated, wherein Abz is 2-amino benzyl group and is attached to a Leucine amino acid (2-aminobenzamide-Leu NTM, also called M15-Leu). This $NTM_{blk}$ group was attached to the N-terminal of the peptide by use of an activate ester form (pentafluorylbenzyl). The leucine can be substituted for any other amino acid either natural or non-natural in order to better optimize fit and discrimination between various P1 residues. The M15-Leu NTM was previously used for selection of cleavases (Example 3).

**Library construction, phage panning, and clone characterization.** High diversity (~$10^{10}$) phage libraries using NNK variant site encoding were constructed targeting residues positions within the pocket of the anticalin **(Fig. 6),** the sequence of anticalin is set forth in SEQ ID NO:35. Using standard protocols, phage library was panned against different mod-NTAA target peptides. Clones from the panning output were isolated and characterized using a panel of peptides in a multiplex Luminex binding assay. Specific binders were isolated against a variety of M15-L-NTAAs **(Fig. 7).** An exemplary engineered anticalin comprises an amino acid sequence that has at least 80%, 90%, 95% or more identity to an amino acid sequence set forth in SEQ ID NO:35. In some embodiments, an engineered anticalin comprises a mutation in the scaffold set forth in SEQ ID NO:35 selected from the group consisting of V33T, L36R, Y52R, T54L, L70M, R79S, W81E, F85Q, L96E, N98L, H100T, R101W, Y102H, Y108W, F125S, K127P, K136R, Y140L corresponding to positions of SEQ ID NO:35.

**Evaluation of M15-L-P1P2 peptide binding.** The ProteoCode™ assay was used to generate binding profiles across a set of 288 peptides (17X17 combination of different P1 and P2 residues) for the anticalin binders. To enable ProteoCode™ encoding, the anticalin binders were expressed with a SpyCatcher fusion at the C-terminus of the anticalin enabling easy bio-conjugation of a SpyTag-DNA coding tag chimera. Proximity and binding between binders and different peptides allows the transfer of information from the DNA *coding tag* to the DNA recording tag attached to the queried peptide. The use of Abz-L (M15-LEU) group enabled generation of a set of P1 discriminatory binders, which also minimized the effect of the P2 group on binding/encoding **(Fig. 8).** The same NTM group was used successfully to generate modified cleavases that can recognize and cleave the modified terminal residue of a polypeptide (Example 3). The set of binders and cleavases selected specifically again recognize and cleave the modified terminal residues of a polypeptide

can be used in combination and allows to encode sequentially every or majority of amino acids of the immobilized polypeptide (ProteoCode™ encoding, shown on Fig. 9).

Example 6. Syntheses of exemplary compounds

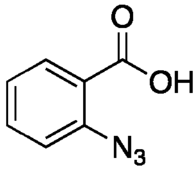

compound [1]

**Synthesis of 2-azidobenzoic acid (compound [1]):** To a 100 mL round-bottom flask equipped with a magnetic stirbar, 1g of isatoic anhydride (6.13 mmol) was dissolved in a mixture of tetrahydrofuran (THF) and 5 equiv. (30.65 mmol) of sodium hydroxide (NaOH) in water. The mixture was stirred vigorously at room temperature for 30 minutes. LCMS of the solution showed that complete hydrolysis of the anhydride had taken place (forming the 2-aminobenzoic acid), so the solution was placed in an ice bath and acidified by addition of 20 equiv. (122.6 mmol) of conc. HCl. To this, 1.2 equiv. of sodium nitrite ($NaNO_2$; 7.36 mmol) dissolved in water was added dropwise and allowed to stir at 0 °C for 20 minutes. Then, 1.5 equiv. of sodium azide ($NaN_3$; 9.195 mmol) was dissolved in water and added dropwise to the solution and proceeded to react for 15 minutes. The upon completion monitored by LCMS, the solution was extracted (3x50 mL) with ethyl acetate (EtOAc), washed with brine, and dried over $Na_2SO_4$. The pooled organic solution was filtered, condensed, taken up in minimal diethyl ether ($Et_2O$), and precipitated with n-heptane. The solution was filtered and the remaining orange-brown powder collected was used without further purification (>99% pure by LC-MS; 932mg, 93% yield).

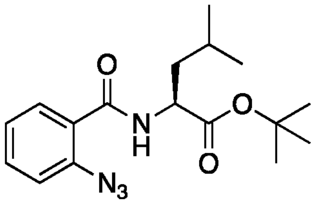

compound [2]

**Synthesis of *N*-(2-azidobenzamid)-L-leucine-*O*-*tert*-butyl ester (compound** [2]): To a 100 mL round-bottom flask containing a magnetic stirbar, 632 mg of compound [1] (3.874 mmol) was added and dissolved in anhydrous *N,N*-dimethylformamide (DMF), followed by 1.2 equiv. of diisopropylethylamine (DIPEA; 4.469 mmol). The solution was allowed to stir at room temperature for 10 minutes and then 1.1 equiv. of COMU ((1-Cyano-2-ethoxy-2-oxoethylidenaminooxy)dimethylamino-morpholino-carbenium hexafluorophosphate; 4.261 mmol) was added to the solution and continued to stir for 30 minutes. In a separate vial, 1.2 equiv. of L-leucine-*O*-*tert*-butyl ester HCl (4.469 mmol) was dissolved in dichloromethane (DCM) and 2.4 equiv. of DIPEA (8.938 mmol). After 30 minutes, the leucine solution was added dropwise to the [1]-containing solution and allowed to react for 18 hours. Upon completion, the solution was diluted in 150 mL of EtOAc and was washed with 1M HCl, then sat. $NaHCO_3$, and lastly brine. The organic layer was dried over $Na_2SO_4$, filtered, and condensed. The remaining oil was dissolved in a minimal volume of DCM and dry-loaded onto silica gel for purification on ISCO CombiFlash (0-50% EtOAc in n-heptane). The fractions containing the desired product [2] were pooled, condensed *in vacuo,* and analyzed by LCMS. This resulted in 1.121g of [2] isolated (>98% purity; 87% yield) as a waxy solid.

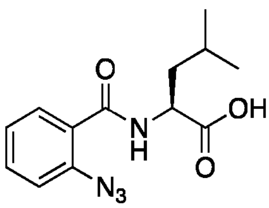

compound [3]

**Synthesis of *N*-(2-azidobenzamid)-L-leucine (compound [3]):** To a 200 mL round-bottom flask containing 1.121g of compound [2], a stirbar was added and the solid was dissolved in 40 mL of DCM. To this solution, 15 mL of trifluoroacetic acid (TFA) was carefully added and the solution was allowed to stir at room temperature for 5 hours. Upon completion (monitored by TLC), the stirbar was removed, washed with DCM and n-heptane, and the solution was condensed *in vacuo.* The remaining residue was washed with n-heptane and condensed *in vacuo* until most of the TFA was removed. The oil was dissolved in a minimal volume of DCM, dry-loaded onto silica gel, and purified on ISCO CombiFlash (0-70% EtOAc in n-heptane). The fractions containing the desired product [3] were pooled, condensed, and analyzed by LCMS. This produced 932 mg of [3] (>99% purity; 99% yield) as an amorphous solid.

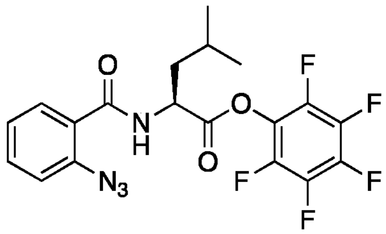

compound [4]

**Synthesis of *N*-(2-azidobenzamid)-L-leucine-*O*-(2,3,4,5,6-pentafluorophenyl) ester (compound [4]):** To a 20 mL amber vial equipped with a stirbar, 296 mg of compound [3] (0.890 mmol) was added and dissolved in 3 mL of anhydrous THF. To this, 1.1 equiv. 2,3,4,5,6-pentafluorophenol (0.980 mmol) was added and stirred until dissolved. In a separate vial, 1.0 equiv. of *N,N'*-dicyclohexylcarbodiimide (DCC; 0.890 mmol) was dissolved in THF and added dropwise to the stirred solution of [3]. The reaction was stirred at 25 °C for 3.5 hours and upon completion was diluted in EtOAc, filtered to remove DCU (dicyclohexylurea), and condensed *in vacuo.* The resulting oil was taken up in minimal volume of DCM and purified by ISCO CombiFlash (0-50% EtOAc in *n*-heptane). The resulting fractions containing the desired product were pooled, condensed, and placed under high vacuum to afford 392 mg of compound [4] as a waxy solid (>95% purity; 99% yield).

Example 7. Evaluating 2-azidobenzamide-LEU-pFP as a suitable modification for modified cleavase recognition and activity.

One of the exemplary modified cleavases identified from genetic selection as described in Example 3 containing the amino acid sequence as set forth in SEQ ID NO: 18 with mutations N191M/W192G/R196T/N306R/D650A was assessed for recognition and activity towards peptides labeled with M15-LEU synthesized as described in the Example 6. A synthetic peptide (IHAGYAW; SEQ ID NO: 45) was functionalized with the compound [4] to show viability of the approach to install M15-LEU to provide selective cleavage. A solution of [4] (150 mM in dimethylacetamide; DMAc) was prepared fresh. The peptide was also dissolved in DMAc to 10 mM concentration. Then in a 1.5 mL tube, 50 μL of acetonitrile and 25 μL MOPS buffer (pH 7.6) were added. To that, 10 μL of the 10mM peptide solution was added in and mixed. Lastly, 15μL of the 150 mM [4] was added in and the solution in the tube was placed in a thermomixer at 40°C for 60 minutes. During the incubation, a solution was prepared by making a 1.25 uM solution of the modified cleavase (having sequence set forth in SEQ ID NO: 18) in 0.1M HEPES buffer (pH 8.0).

After the 60 minute incubation of the IHAGYAW peptide, 100 μL of 0.5M TCEP (tris(2-carboxyethyl)phosphine) solution was added and incubated for 20 minutes at 40 °C to reduce the azide to amine. A 20 μL aliquot was removed and added to the 0.05M MES (2-(N-morpholino)ethanesulfonic acid) with 0.1% Tween 20 at pH 6.4 solution containing the modified cleavase. The modified cleavase and solution of labeled peptide were then incubated at 65°C for 1-18h. The progress of the cleavage event was monitored by taking aliquots of the reaction and injecting on the LC-MS. The results of mass spectrometry analysis have shown that treatment of the peptide with the compound [4] resulted in products with expected molecular weights. Cleavage of the labeled peptide (2-aminobenzamide-LIHAGYAW; SEQ ID NO: 55) was observed to completion after 18 hours. Loss of 2-aminobenzamide-LI was the only cleavage event observed by LC-MS. No remaining 2-aminobenzamide-LIHAGYAW was observed after the 18 hour incubation. These data demonstrate that the tested isolated modified cleavase from genetic selection removed the expected labeled dipeptide from the treated polypeptide, including the exogenous added chemically-labeled leucine as part of the dipeptide (2-aminobenzamide-LI). Using this exemplary approach, the tested modified cleavase derived from a wildtype dipeptide cleavase (DAP BII) was modified to remove a single labeled amino acid (labeled with an exogenous 2-aminobenzamide-LEU, also designated as M15-L) from the polypeptide.

Example 8. Development of thermophilic cleavases for removal of M15-L-P1 from M15-L-modified peptides.

A genetic selection approach was used to evolve thermophilic dipeptidyl peptidase to cleave a single labelled N-terminal amino acid from a peptide similarly to the described in Example 3 (the M15-L NTM was used). High diversity combinatorial libraries on different dipeptidyl peptidase scaffolds were created, and the libraries were transformed into an

*E coli.* selection strain. Structure based design was used to define variant sites for library creation. Peptides with different N-terminally modified P1 amino acids were used to evolve Cleavases for the respective targets.

A genetic selection-based approach to cleavase engineering enables high-throughput enzyme selection (Evnin, L. B., J. R. Vasquez and C. S. Craik (1990). "Substrate specificity of trypsin investigated by using a genetic selection." Proc Natl Acad Sci U S A 87(17): 6659-6663). The selection makes use of short N-terminally modified peptides that contain the auxotrophic amino acid. The peptides readily enter the periplasm of a bacterium but are unable to enter the cytoplasm due to the inability of transporters to recognize the modified N-terminus (Smith, M. W., D. R. Tyreman, G. M. Payne, N. J. Marshall and J. W. Payne (1999). "Substrate specificity of the periplasmic dipeptide-binding protein from Escherichia coli: experimental basis for the design of peptide prodrugs." Microbiology 145 ( Pt 10): 2891-2901). To relieve the amino acid auxotrophy during growth on minimal media, a cleavase scaffold is expressed on a plasmid and targeted to the periplasm, via a pelB leader sequence. The active cleavase variant removes the N-terminally modified amino acid, revealing a native peptide amino terminus. This allows the rest of the peptide, which contains the essential amino acid, to be uptaken and support growth of the bacterium. For studies disclosed herein, an arginine auxotroph was used, which demonstrated an absence of background growth on peptides with the N-terminal M15-LEU modification.

Using this genetic selection approach, an active cleavase variant was identified from an S46 DPP library {N214X,W215X,R219X,N329X,D673X; X = 20 amino acids} and error prone library from *Thermomonas hydrothermalis* with the following amino acid mutations: {N214M,W215G,R219T,N329R,D673A,G674V} with reference to SEQ ID NO: 33 (an unmodified scaffold). Moreover, this variant was further evolved by creating an additional library with variant sites as follows {N214M,W215G,R219T,N329R; N333X,I651X,A671X,D673A,G674X,N682X,M692X; X = any one of 20 natural amino acids; the indicated residue numbers correspond to positions of SEQ ID NO: 33} and by genetic selection generated a set of enzymatic cleavases. Each evolved cleavase was individually assayed on all M15-L-P1 targets. In this assay, individual cleavase clone was expressed and purified, and then incubated with each peptide substrate for 3 hours at 52 °C. The UV absorbance of both product and starting material in the final reaction was measured on HPLC and converted to percentage of conversion. Collectively, they can provide broad activity for removal of almost all M15-L-P1 residues (Fig. 8A and Fig. 8B). The individual data in Fig. 8A is constructed using best conversion rate for each M15-L-P1 targets among all tested Cleavase clones.

Example 9. Engineered dipeptide cleavases can remove single labeled NTAAs of a model polypeptide.

A set of dipeptide cleavase enzymes was evolved from an S46 DPP library as described in Examples 3 and 6 using M15-L-P1 target polypeptides (polypeptide sequences: M15-L-P1-AR, where P1 is one of the 17 natural amino acids, excluding C, K, R) and the dipeptide cleavase scaffold from Thermomonas hydrothermalis (SEQ ID NO: 31 or SEQ ID NO: 33). The enzymes can efficiently cleave M15-L-labeled polypeptides between P1 and P2 amino acid residues, thus are configured to remove a single labeled terminal amino acid from the polypeptide (Fig. 10A and Fig. 10B). To accommodate the M15-L label in the substrate binding site, all modified dipeptide cleavases contained the following mutations at the conserved residues that form an amine binding site in unmodified dipeptidyl aminopeptidases: N214M, W215G, R219T, N329R, D673A (the indicated residue numbers correspond to positions of SEQ ID NO: 33). These mutations are specific to the M15 $NTM_{blk}$ group and may be different for other NTMs including bipartite NTMs of M15 with other amino acid-like groups. At the same time the cleavage efficiency of the evolved enzymes depended on the nature of the P1 residue.

Each evolved cleavase was individually assayed on all M15-L-P1 target polypeptides. In this assay, an individual cleavase clone is expressed and purified, and then incubated with each peptide substrate for 3 hours at 52 °C. Six μM enzyme in 5mM phosphate buffer at pH 8 were used. The UV absorbance of both product and starting material in the final reaction was measured on HPLC and converted to percentage of conversion (Fig. 10A). M15-L-P-AR exhibited poor cleavage efficiency with the set of seven Cleavase clones, but further directed evolution can be used to address this issue. Additionally, efficiency of cleavage reactions were assessed on peptide-DNA fusions. In this assay, peptide substrates were modified to have an azide group at the C-terminal lysine that was linked to dibenzocyclooctyne (DBCO)-activated PEG12 linker connected with a DNA oligo. M15-L-P1-GAEIAGDVAGGK peptides were used (SEQ ID NO: 46), and for D and N as P1, the Gly residue at P2 position was replaced with Val. In Fig. 10B, the cleavage events were monitored by UREA-PAGE assay. It was found that the first selected modified cleavase (M15-L_Z001) provided 100% cleavage for polypeptides with the following M15-L-labeled P1 residues: A, I, L, M, Q, V. Other selected modified cleavases provided 80-100% cleavage for polypeptides with the following groups of M15-L-labeled P1 residues: D,E; S,T; G; N; H,Y; F,W. A broad cleavage of a single labeled terminal amino acid from the polypeptide can be achieved by combining two or more dipeptide cleavases in a set. For example, as shown in Fig. 10A and Fig. 10B, a set of 7 selected dipeptide cleavases can provide broad activity for removal of almost all M15-L-labeled P1 residues from the polypeptide. In another example, a set of two modified dipeptide cleavases can also cleave the majority of M15-L-labeled P1 residues from the polypeptide, except for F, G, H, P, W residues (Fig. 10C). In this assay, short peptides with M15-L-P1-AR sequence are used, same as in Fig. 10A. Other cleavase combinations can be created to achieve a desired level of cleavage specificity, such as different sets of two, three, four or more enzymes.

Importantly, it should be noted that the selected modified dipeptide cleavases does not remove an unlabeled

terminal dipeptide from the polypeptide, as evident from Fig. 10B, showing solid single bands after the cleavage reaction. It means that the selected modified dipeptide cleavases would not continue cleaving the polypeptide after initial cleavage of the modified NTAA. In contrast, unmodified dipeptide cleavase having natural amine binding site residues (N191, W192, R196, N306, D650 corresponding to positions of SEQ ID NO: 33) would continue gradually cleaving the polypeptide, creating multiple bands during the cleavage reaction (Fig. 11). In this experiment, a test peptide LMSHNARGAEDDVVRGGGGK (SEQ ID NO: 47) was derivatized to have an azide group at the C-terminal lysine that was linked to DBCO-activated PEG12 linker connected with a DNA oligo by a click chemistry reaction, and incubated with wild type DAP BII enzyme (40 μM; 1:20 peptide:enzyme ratio) at 30 °C in the following buffer: 20 mM HEPES pH 7.5, 1 mM EDTA, 100 mM NaCl, 10% glycerol. Fig. 11 indicates cleavage results at several time points: 0 min, 5 min, 30 min, 45 min, 60 min (1-5 at the Fig. 11, respectively).

The engineered cleavases were further tested for their ability to cleave labeled polypeptides having different bipartite NTMs. Bipartite NTMs comprise an amino acid-like portion (natural or unnatural amino acid, or a chemical entity having a size, *e.g.,* length axis or volume, shape, and/or configuration similar to a natural amino acid; designated here as NTMaa) and a N-terminal blocking group ($NTM_{blk}$) that provides specificity during the selection process. Such NTM at the N-terminus of a polypeptide would fit in a substrate pocket of a modified dipeptide aminopeptidase, and cause the dipeptide aminopeptidase to cleave a single labeled amino acid from the polypeptide, effectively changing the cleavage mode of the enzyme (Fig. 12A, B, the upper drawings). Initial examinations of bipartite NTMs were performed by a colorimetric assay developed to assess the cleavases' ability to recognize and hydrolyze the NTM-P1 from a small molecule substrate. An initial library was synthesized to probe the individual pocket(s) of the cleavase by independently exploring the $NTM_{blk}$ and NTMaa. The library was composed of either $NTM_{blk}$-AA-pNA or M15-NTMaa-A-pNA substrate compounds and compared against M15-AA-pNA; where pNA is *p*-nitroaniline. In a 96-well transparent, flat-bottom plate, wells containing 45 μL of cleavase (0.5 μM) in 50 mM MES (pH 6.4) with 0.1% Tween 20 was incubated at 65 °C for 5 minutes. Separately, the pNA substrate compounds were prepared in individual 10 mM DMSO solutions. After the incubation period, 5 μL of the substrates in DMSO were added to the individual wells containing cleavase (n=3). The subsequent reactions were monitored by a plate reader at a wavelength of 405 nm for 150 seconds. Assuming steady-state kinetics, initial velocities monitoring the release of free pNA (at 405 nm) from the substrate in the presence of a cleavase enzyme was used to determine whether a $NTM_{blk}$ or NTMaa was a suitable recognition partner to the cleavase; providing down-selection criteria for further testing. Various bipartite NTMs have been tested, including different combinations of a N-terminal blocking group ($NTM_{blk}$) chosen from the following list: M15 (2-aminobenzamide), M18 (pyrazine-2-carboxamide), M19 (3,4-difluorobenzamide), M20 (3-cyano-4-fluorobenzamide) M21 (3-fluoro-4-cyanobenzamide) M22 (3,4-difluoro-2-aminobenzamide) and an "amino acid-like" part (NTMaa) chosen from the following list: Leu (Leucine), Ala (alanine), Gly (glycine), 3PA (3-pyridylalanine), FPG (4-fluorophenylglycine), Phg (phenylglycine), 3AZ (3-azetidine), C5G (cyclopentylglycine), CPG (cyclopropylglycine), and N-alkylated derivatives of these molecules. In one example, cleavage efficiencies of the M15-L_Z001 cleavase were tested against a model polypeptide (AAAEIRGDVRGGK; SEQ ID NO: 49) labeled with the following bipartite NTMs: M15-Leu (designated as M17), M19-Leu, M19-3PA, M19-FPG, M19-Phg. The model polypeptide was derivatized to have an azide group at the C-terminal lysine, attached to DBCO-modified beads by a click chemistry reaction, and incubated with 5 uM of the M15-L_Z001 cleavase in 50mM MES (pH 6.5) supplemented with 0.1% Tween 20 at 65 °C for 1 hour. Beads were washed with 50mM Tris (pH 8.0); and peptides were digested with Trypsin in 50mM Tris (pH 8.0) at 37 °C for 1 hour. In parallel, beads were prepared with the anticipated cleavage product sequence (AAEIRGDVRGGK; SEQ ID NO: 50) and were also digested as a reference control. Cleavage efficiency was determined by comparing the ratios of the N-terminal tryptic peptide product fragment's (AAEIR; SEQ ID NO: 51) mass signal intensity to the internal standard tryptic peptide's (GDVR; SEQ ID NO: 52) mass signal intensity versus the reference control (n=3). The results shown in Fig. 13 demonstrate that the cleavage efficiency of the NTM-labeled NTAA by a given dipeptidyl cleavase can be adjusted by altering the "amino acid-like" part (NTMaa) of the bipartite NTM.

Further bipartite NTMs were screened against a library of modified dipeptidyl cleavases containing substitutions in their substrate binding sites (as shown in the above examples) in order to optimize efficiency of the cleavage and its specificity for the particular bipartite NTM. The screened bipartite NTMs contain one of the following $NTM_{blk}$ structures: 4-methylbenzoic acid, 4-(dimethylamio)benzoic acid, nicotinic acid, 3-aminonicotinic acid, 2-pyrazinecarbooxylic acid, 5-amino-2-fluoro-isonicotinic acid, 2,3-pyrazinedicarboxylic acid, 4,7-Difluoroisobenzofuran-1,3-dicarboxylic acid, 4-chloro-2-aminobenzoic acid, 4-nitro-2-aminobenzoic acid, 7-methoxy-1h-benzo[d][1,3]oxazine-2,4-dione, 4-carboxy-2-aminobenzoic acid, 6-(Trifluoromethyl)-2,4-dihydro-1h-3,1-benzoxazine-2,4-dione, 7-(Trifluoromethyl)-1h-benzo[d][1,3]oxazine-2,4-dione, 6-fluoro-2-aminobenzoic acid, 4-fluoro-2-aminobenzoic acid, 5-methoxy-2-aminobenzoic acid, 4-fluorobenzoic acid, 4-(trifluoromethyl)benzoic acid, 2-ethynyl-6-fluorobenzaldehyde, 2-aminobenzoic acid, Succinic anhydride, 3,6-Difluoropyridine-2-carboxylic acid, 2-Fluoronicotinic acid, 5-Bromo-2-hydroxynicotinic acid, 4-(Trifluoromethyl)pyrimidine-5-carboxylic acid, 2-Oxo-1,2-dihydropyridine-3-carboxylic acid, 5-Methyl-2-aminobenzoic acid, 6-Fluoropicolinic acid, 3-Methyl-2-aminobenzoic acid, 4-Methyl-2-aminobenzoic acid, 2-Amino-6-methylbenzoic acid, 2-Amino-6-fluorobenzoic acid, 2-Amino-5-fluorobenzoic acid, 2-Amino-3-fluorobenzoic acid, 2-Amino-4-fluorobenzoic

acid, 2-Aminonicotinic acid, 4-Aminonicotinic acid, 3-Aminopicolinic acid, 2-Amino-4,5-difluorobenzoic acid, 3,4-difluorobenzoic acid, 3,4,5-difluorobenzoic acid, 3-(Methoxycarbonyl)bicyclo[1.1.1]pentane-1-carboxylic acid, 3,3-Difluorocyclobutane-1-carboxylic acid, 1-Methyl-2-oxo-piperidine-4-carboxylic acid, Tetrahydropyran-4-carboxylic acid, 5-Fluoroorotic acid, 3-Fluoro-4-nitrobenzoic acid, 3-(Difluoromethyl)-1-methyl-1H-pyrazole-4-carboxylic acid, 4-(Difluoromethoxy)benzoic acid, 1-(Difluoromethyl)-1h-pyrazole-3-carboxylic acid, 4-(Methanesulfonylamino)benzoic acid, 5-Fluoro-6-methoxynicotinic acid, Tetrahydro-2H-thiopyran-4-carboxylic acid 1,1-dioxide, 4-(1H-Tetrazol-5-yl)benzoic acid, 1,2,3-Thiadiazole-4-carboxylic acid, 1,3-Benzodioxole-4-carboxylic acid, 2,1,3-Benzoxadiazole-5-carboxylic acid, 1-Benzyl-3-methyl-1h-pyrazole-5-carboxylic acid, 1-Cyclopropyl-6,7-difluoro-1,4-dihydro-4-oxoquinoline-3-carboxylic acid, 3,4-Dichlorobenzoic acid, 5-Fluoro-6-methylpyridine-2-carboxylic acid, 4,5-Dimethyl-2-(1h-pyrrol-1-yl)thiophene-3-carboxylic acid, 1,3-Dimethyl-1h-thieno[2,3-c]pyrazole-5-carboxylic acid, 1-[(4-Fluorobenzene)sulfonyl]piperidine-3-carboxylic acid, 1-(4-Fluorobenzyl)-5-oxopyrrolidine-3-carboxylic acid, 3-Fluoro-4-methoxybenzoic acid, 4-Fluoro-3-nitrobenzoic acid, 6-Fluoro-4-oxochromene-2-carboxylic acid, 3-Fluorophenylacetic acid, 4-Fluoro-3-(trifluoromethyl)benzoic acid, 5-Furan-2-yl-isoxazole-3-carboxylic acid, 1-Isopropyl-2-(trifluoromethyl)-1h-benzimidazole-5-carboxylic acid, Levofloxacin carboxylic acid, 3,5,7-Trifluoroadamantane-1-carboxylic acid, 3,4,5-Trimethoxybenzoic acid, 2-Oxo-2,3-dihydro-1h-benzo[d]imidazole-4-carboxylic acid, 1-Methyl-3-(trifluoromethyl)-1h-pyrazole-5-carboxylic acid, 2-Morpholin-4-yl-isonicotinic acid, 1,3-Oxazole-4-carboxylic acid, 4-Carboxybenzenesulfonamide, 3,4-difluorobenzenesulfonyl chloride. In addition, the screened bipartite NTMs also contain one of the following NTMaa structures: a naturally-occurring amino acid residue (alanine, cysteine, aspartic acid, glutamic acid, phenylalanine, glycine, histidine, isoleucine, lysine, leucine, methionine, asparagine, glutamine, arginine, serine, threonine, valine, tryptophan or tyrosine), 3-(3'-pyridyl)-L-alanine, L-cyclohexylglycine, α-aminoisobutyric acid, 3-(4'-pyridyl)-L-alanine, L-azetidine-2-carboxylic acid, isonipecotic acid, L-phenylglycine, β-(2-thienyl)-L-alanine, 3-(4-thiazolyl)-L-alanine, 1-aminocyclopentane-1-carboxylic acid, (2-trifluoromethyl)-L-Phenylalanine, L-cyclopropylalanine, 3-(2'-pyridyl)-L-alanine, beta-cyano-L-alanine, α-methyl-L-4-Fluorophenylalanine, α-methyl-D-4-fluorophenylalanine, 3-amino-2,2-difluoro-propionic acid, O-sulfo-L-tyrosine sodium salt, L-2-furylalanine, 1-aminocyclopropane-1-carboxylic acid, 3,5-dinitro-L-tyrosine, pentafluoro-L-phenylalanine, 3,5-difluoro-L-phenylalanine, 3-fluoro-L-phenylalanine, N-cyclopentylglycine, 1-(amino)cyclohexanecarboxylic acid, N-methylalanine, 4-amino-tetrahydropyran-4-carboxylic acid, 4-amino-1,1-dioxothiane-4-carboxylic acid, 4-amino-1-methyl-4-piperidinecarboxylic acid, 2-amino-N-(2,4-dimethoxybenzyl)acetamido)acetic acid, or N-alkylated derivatives. In some cases, L- or D-configurations of NTMaa structures were alkylated to prevent racemization.

As a result of this screen, multiple combinations of modified cleavase enzymes that specifically cleave a single N-terminal amino acid of a polypeptide modified by a particular bipartite NTM can be selected. The cleavage of the labeled polypeptide by the selected modified cleavase enzyme can be confirmed by LC-MS as described in Example 3.

Next, it can be shown that specific changes in the conserved residues in the substrate binding site of the cleavase can determine specificity to the particular NTMs. As described above, the selected modified cleavase M15-L_Z001 has the following amino acid substitutions in the amine binding site in comparison to the unmodified dipeptidyl aminopeptidase scaffold: N214M, W215G, R219T, N329R, D673A (the indicated residue numbers correspond to positions of SEQ ID NO: 33). These changes drive specificity of the M15-L_Z001 cleavase towards M15-L-labeled polypeptides. Then, using S46 DPP from *Thermomonas hydrothermalis* as a starting scaffold, combinatorial libraries were created (such as N214X, W215X, R219X, N329X, D673X; the indicated residue numbers correspond to positions of SEQ ID NO: 33), and genetic selection was performed using M19-L-AR peptide as described in the previous examples. Several modified cleavases have been selected that are specific towards a different NTM (M19). The selected clones share a mutation in the amine binding site: N329P, which is different from the N329R substitution present in the M15-L_Z001 cleavase. This substitution was also observed in other cleavase clones selected using different M19-UAA-labeled polypeptides, where UAA designates unnatural amino acids. Thus, N329P mutation drives specificity of modified cleavases towards M19-labeled polypeptides. As an example, the M19_053 cleavase clone selected for the M19-L-labeled peptides shows almost the same substitutions in the amine binding site as in the M15-L_Z001 cleavase, except for N329P: N214M, W215G, R219T, N329P, D673A (the indicated residue numbers correspond to positions of SEQ ID NO: 33). The cleavage efficiencies of the selected clones M19_053 and M15-L_Z001 were compared on a model polypeptide (polypeptide sequence: LAAR, SEQ ID NO: 48) labeled with either M15 or M19 labels (Fig. 14). The cleavage results were monitored in 5, 10, 20, 30 and 60 minutes, and showed that the newly selected cleavase clone M19_053 provided better specificity towards the M19-labeled polypeptide, whereas the M15-L_Z001 cleavase provided better specificity towards the M15-labeled polypeptide.

Next, it can be shown that different dipeptidyl aminopeptidase scaffolds are evolved to have similar amino acid changes in the amine binding site to recognize a particular NTM. In addition to the above-described scaffold from *Thermomonas hydrothermalis,* showing N214M, W215G, R219T, N329R, D673A amino acid changes for the M15-L-labeled polypeptides, two additional scaffolds were similarly and independently evolved to cleave the M15-L-labeled polypeptides, namely dipeptidyl aminopeptidase scaffolds Dap BII from *Pseudoxanthomonas mexicana* (SEQ ID NO: 13) and DPP11 from *Porphyromonas gingivalis* (SEQ ID NO: 12). All three unmodified scaffolds show conservation of the residues N214, W215, R219, N329, D673 in the amine binding site (the given residue numbers correspond to positions of SEQ ID NO: 33), although the sequence identity between Dap BII and DPP11 scaffolds is about 32%, and the sequence

identity between Dap BII and the *Thermomonas hydrothermalis* scaffolds is about 74%. After selection on the M15-L-labeled polypeptides, the scaffold from *Thermomonas hydrothermalis* showed N214M, W215G, R219T, N329R, D673A amino acid changes (the M15-L_Z001 cleavase), whereas the Dap BII scaffold shows corresponding N214M, W215G, R219T, N329R, D673A changes, and the DPP11 scaffold shows corresponding N214M, W215G, R219V, N329I, D673A changes (the indicated residue numbers correspond to positions of SEQ ID NO: 33). Thus, very similar amino acid changes can be selected in the amine binding sites of different dipeptidyl aminopeptidases to recognize a particular specific NTM.

Next, it can be shown that the same dipeptidyl aminopeptidase scaffold can be evolved to recognize and cleave a single terminal amino acid of a polypeptide labeled with different NTMs having various shapes. In addition to bipartite NTMs having an NTMaa part (see above), a relatively small, single part NTM (Fig. 12A, B, lower drawings) can also be used. Similar to described above, using S46 DPP from *Thermomonas hydrothermalis* as a starting scaffold, combinatorial libraries were created (such as N214X, W215X, R219X, N329X, D673X; the residue numbers correspond to positions of SEQ ID NO: 33), and genetic selection was performed using M19- AAAR peptide. Two types of Cleavases can be selected from the same scaffold: P1 cutters that cleave polypeptides after P1 residue, and P2 cutters that cleave polypeptides after P2 residue. P2 cutters are similar to enzymes described above, such as M15-L_Z001 cleavase, having a large substrate-binding pocket that can accommodate two amino acids and a label. P2 cutters selected for the M19 NTM comprise the following exemplary mutations of the conserved residues that form an amine binding site of the cleavase: (a) N214M, W215G, R219T, N329R, D673A; (b) N214M, W215G, R219T, N329P, D673S; (c) N214M, W215G, R219A, N329P, D673A; (d) N214M, W215G, R219V, N329P, D673A (all the indicated residue numbers correspond to positions of SEQ ID NO: 33), sharing significant level of similarity with the M15-L_Z001 cleavase. In contrast, the selected exemplary P1 cutter comprises the following mutations of the conserved residues that form an amine binding site of the cleavase: N214T, W215M, R219K, N329Y (P1 cutter 1, the residue numbers correspond to positions of SEQ ID NO: 33). This mutation pattern differs dramatically from the mutations in the M15-L_Z001 cleavase. This allows for a different geometry of the substrate-binding pocket that can now accommodate a single amino acid and the M19 label. The cleavage reaction of the model peptide M19-AAAR (SEQ ID NO: 21) by the selected P1 cutter 1 was set up as follows: 300 mcM M19-AAAR, 0.1 mcM P1 cutter 1 enzyme in 5 mM sodium phosphate buffer, pH=8, 52 °C, 1 hour incubation time. Reaction mixtures were then analyzed via LC-MS for product identification; the observed mass of the cleaved product was 230.2 Da, which matched well with the expected mass for the M19-A (M+H) part (231.2 Da).

Example 10. Diversity of the S46 DPP family of dipeptidyl cleavases. In order to better understand the diversity of potential dipeptidyl cleavase scaffolds, enzymes from the S46 DPP family (based on MEROPS classification) were downloaded, and aligned using a WebLogo analysis of sequence conservation of DAP BII homologs (Crooks GE, et al., WebLogo: A sequence logo generator, Genome Research, 14:1188-1190, (2004)). These sequences (7903 sequences were assessed) were first clustered at 80% identity to reduce complexity, and cluster centers were used for alignment and WebLogo analysis. All the sequences analyzed after clustering were no more than 80% close to each other. 2125 sequences were aligned and show conservation of N215, W(F)216, R220, N330, and D674 residues in reference to the wildtype DAP BII sequence set forth in SEQ ID NO: 20 (Fig. 15). The height of each stack indicates the sequence conservation at that position (measured in bits), and the height of symbols within the stack reflects the relative frequency of the corresponding amino acid at the indicated position (amino acid numbers correspond to positions of SEQ ID NO: 20). Then, a pairwise identity distribution relative to DapBII (Pseudoxanthomonas mexicana) was calculated and shown in Fig. 16. Fig. 16 depicts results from a WebLogo analysis of sequence conservation of different DAP BII homologs selected to have no more than 80% sequence identity. The most frequent percent identity for DapBII homologs among the family members was found to be from 27 to 36 % identity.

The present disclosure is not intended to be limited in scope to the particular disclosed embodiments, which are provided, for example, to illustrate various aspects of the invention. Various modifications to the compositions and methods described will become apparent from the description and teachings herein. Such variations may be practiced without departing from the true scope of the disclosure and are intended to fall within the scope of the present disclosure. These and other changes can be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled.

## Claims

1. A modified dipeptide cleavase comprising three or more amino acid substitutions in a substrate binding site of a dipeptidyl aminopeptidase, wherein:

   (i) the dipeptidyl aminopeptidase removes or is configured to remove two terminal amino acids from a polypeptide; and

(ii) the modified dipeptide cleavase removes or is configured to remove from the polypeptide having a terminal amino acid residue labeled with a chemical reagent (a) the single labeled terminal amino acid residue or (b) a labeled terminal dipeptide, wherein

the dipeptidyl aminopeptidase comprises an amino acid sequence having at least 30 % sequence identity to the amino acid sequence of SEQ ID NO: 31 and also comprises an asparagine residue at a position corresponding to position 191 of SEQ ID NO: 31, a tryptophan or phenylalanine residue at a position corresponding to position 192 of SEQ ID NO: 31, an arginine residue at a position corresponding to position 196 of SEQ ID NO: 31, an asparagine residue at a position corresponding to position 306 of SEQ ID NO: 31, an aspartate residue at a position corresponding to position 650 of SEQ ID NO: 31; and wherein the modified dipeptide cleavase comprises three or more amino acid substitutions in residues corresponding to positions 191, 192, 196, 306, 650 of SEQ ID NO: 31.

**2.** The modified dipeptide cleavase of claim 1, wherein the modified dipeptide cleavase does not remove an unlabeled terminal dipeptide from the polypeptide.

**3.** The modified dipeptide cleavase of claim 1, which comprises at least four amino acid substitutions in residues corresponding to positions 191, 192, 196, 306, 650 of SEQ ID NO: 31.

**4.** The modified dipeptide cleavase of claim 1, wherein the modified dipeptide cleavase removes or is configured to remove a single N-terminally labeled amino acid of the polypeptide.

**5.** The modified dipeptide cleavase of claim 1, further comprising one or more amino acid substitutions in residues corresponding to positions 310, 628, 648, 651, 659, 669.

**6.** The modified dipeptide cleavase of claim 1, wherein the dipeptidyl aminopeptidase is a protein classified in MEROPS S46, or a functional homolog or fragment thereof.

**7.** The modified dipeptide cleavase of claim 1, wherein the single terminal amino acid or terminal dipeptide is labeled with a N-terminal modification that comprises a N-terminal blocking group ($NTM_{blk}$) and, optionally, a natural or unnatural amino acid portion (NTMaa), wherein the NTMaa comprises a compound selected from the group consisting of: a naturally-occurring amino acid residue, 3-(3'-pyridyl)-L-alanine, L-cyclohexylglycine, α-aminoisobutyric acid, 3-(4'-pyridyl)-L-alanine, L-azetidine-2-carboxylic acid, isonipecotic acid, L-phenylglycine, β-(2-thienyl)-L-alanine, 3-(4-thiazolyl)-L-alanine, 1-aminocyclopentane-1-carboxylic acid, (2-trifluoromethyl)-L-Phenylalanine, L-cyclopropylalanine, 3-(2'-pyridyl)-L-alanine, beta-cyano-L-alanine, α-methyl-L-4-Fluorophenylalanine, α-methyl-D-4-fluorophenylalanine, 3-amino-2,2-difluoro-propionic acid, O-sulfo-L-tyrosine sodium salt, L-2-furylalanine, 1-aminocyclopropane-1-carboxylic acid, 3,5-dinitro-L-tyrosine, pentafluoro-L-phenylalanine, 3,5-difluoro-L-phenylalanine, 3-fluoro-L-phenylalanine, N-cyclopentylglycine, 1-(amino)cyclohexanecarboxylic acid, N-methylalanine, 4-amino-tetrahydropyran-4-carboxylic acid, 4-amino-1,1-dioxothiane-4-carboxylic acid, 4-amino-1-methyl-4-piperidinecarboxylic acid, 2-amino-N-(2,4-dimethoxybenzyl)acetamido)acetic acid, or N-alkylated derivatives;
and the $NTM_{blk}$ comprises a compound selected from the group consisting of: 4-methylbenzoic acid, 4-(dimethylamio) benzoic acid, nicotinic acid, 3-aminonicotinic acid, 2-pyrazinecarbooxylic acid, 5-amino-2-fluoro-isonicotinic acid, 2,3-pyrazinedicarboxylic acid, 4,7-Difluoroisobenzofuran-1,3-dicarboxylic acid, 4-chloro-2-aminobenzoic acid, 4-nitro-2-aminobenzoic acid, 7-methoxy-1h-benzo[d][1,3]oxazine-2,4-dione, 4-carboxy-2-aminobenzoic acid, 6-(Trifluoromethyl)-2,4-dihydro-1h-3,1-benzoxazine-2,4-dione, 7-(Trifluoromethyl)-1h-benzo[d][1,3]oxazine-2,4-dione, 6-fluoro-2-aminobenzoic acid, 4-fluoro-2-aminobenzoic acid, 5-methoxy-2-aminobenzoic acid, 4-fluorobenzoic acid, 4-(trifluoromethyl)benzoic acid, 2-ethynyl-6-fluorobenzaldehyde, 2-aminobenzoic acid, Succinic anhydride, 3,6-Difluoropyridine-2-carboxylic acid, 2-Fluoronicotinic acid, 5-Bromo-2-hydroxynicotinic acid, 4-(Trifluoromethyl)pyrimidine-5-carboxylic acid, 2-Oxo-1,2-dihydropyridine-3-carboxylic acid, 5-Methyl-2-aminobenzoic acid, 6-Fluoropicolinic acid, 3-Methyl-2-aminobenzoic acid, 4-Methyl-2-aminobenzoic acid, 2-Amino-6-methylbenzoic acid, 2-Amino-6-fluorobenzoic acid, 2-Amino-5-fluorobenzoic acid, 2-Amino-3-fluorobenzoic acid, 2-Amino-4-fluorobenzoic acid, 2-Aminonicotinic acid, 4-Aminonicotinic acid, 3-Aminopicolinic acid, 2-Amino-4,5-difluorobenzoic acid, 3,4-difluorobenzoic acid, 3,4,5-difluorobenzoic acid, 3-(Methoxycarbonyl)bicyclo[1.1.1]pentane-1-carboxylic acid, 3,3-Difluorocyclobutane-1-carboxylic acid, 1-Methyl-2-oxo-piperidine-4-carboxylic acid, Tetrahydropyran-4-carboxylic acid, 5-Fluoroorotic acid, 3-Fluoro-4-nitrobenzoic acid, 3-(Difluoromethyl)-1-methyl-1H-pyrazole-4-carboxylic acid, 4-(Difluoromethoxy)benzoic acid, 1-(Difluoromethyl)-1h-pyrazole-3-carboxylic acid, 4-(Methanesulfonylamino)benzoic acid, 5-Fluoro-6-methoxynicotinic acid, Tetrahydro-2H-thiopyran-4-carboxylic acid 1,1-dioxide, 4-(1H-Tetrazol-5-yl)benzoic acid, 1,2,3-Thiadiazole-4-carboxylic acid, 1,3-Benzodioxole-4-carboxylic acid, 2,1,3-Benzoxadiazole-5-carboxylic acid, 1-Benzyl-3-methyl-1h-pyrazole-5-carboxylic acid, 1-Cyclopropyl-6,7-difluoro-1,4-dihydro-4-oxoquinoline-3-carboxylic acid, 3,4-Dichlorobenzoic acid, 5-Fluoro-6-methylpyridine-2-carboxylic acid, 4,5-Dimethyl-2-(1h-pyrrol-1-yl)thiophene-3-carboxylic acid, 1,3-Dimethyl-1h-thieno[2,3-c]pyrazole-5-carboxylic acid, 1-[(4-Fluorobenzene)sulfonyl]piperidine-3-carboxylic acid, 1-(4-Fluorobenzyl)-5-oxopyrrolidine-3-carboxylic acid, 3-Fluoro-4-methoxybenzoic acid, 4-Fluoro-3-nitrobenzoic acid, 6-Fluoro-4-oxochromene-2-carboxylic acid, 3-Fluorophenylacetic acid, 4-Fluoro-3-(trifluoromethyl)benzoic acid, 5-Furan-2-yl-isoxazole-3-carboxylic acid, 1-Isopropyl-2-(trifluoromethyl)-1h-benzimidazole-5-carboxylic acid, Levofloxacin carboxylic acid, 3,5,7-Trifluoroadamantane-1-carboxylic acid, 3,4,5-Trimethoxybenzoic acid, 2-Oxo-2,3-dihydro-1h-benzo[d]imidazole-4-carboxylic acid, 1-Methyl-3-(trifluoromethyl)-1h-pyrazole-5-carboxylic acid, 2-Morpholin-4-yl-isonicotinic acid, 1,3-Oxazole-4-carboxylic acid, 4-Carboxybenzenesulfonamide, 3,4-difluorobenzenesulfonyl chloride.

**8.** A method of treating a polypeptide, comprising the following steps:

labeling a terminal amino acid of the polypeptide with a chemical reagent to produce a labeled polypeptide; and contacting the labeled polypeptide with a modified dipeptide cleavase, wherein the modified dipeptide cleavase comprises three or more amino acid substitutions in a substrate binding site of a dipeptidyl aminopeptidase, wherein:

(i) the dipeptidyl aminopeptidase removes or is configured to remove two terminal amino acids from the polypeptide upon contacting; and
(ii) the modified dipeptide cleavase removes or is configured to remove from the polypeptide having a terminal amino acid labeled with a chemical reagent (a) the single labeled terminal amino acid or (b) a labeled terminal dipeptide, wherein

the dipeptidyl aminopeptidase comprises an amino acid sequence having at least 30 % sequence identity to the amino acid sequence of SEQ ID NO: 31 and also comprises an asparagine residue at a position corresponding to position 191 of SEQ ID NO: 31, a tryptophan or phenylalanine residue at a position corresponding to position 192 of SEQ ID NO: 31, an arginine residue at a position corresponding to position 196 of SEQ ID NO: 31, an asparagine residue at a position corresponding to position 306 of SEQ ID NO: 31, an aspartate residue at a position corresponding to position 650 of SEQ ID NO: 31; and wherein the modified dipeptide cleavase comprises three or more amino acid substitutions in residues corresponding to positions 191, 192, 196, 306, 650 of SEQ ID NO: 31.

**9.** The method of claim 8, wherein the modified dipeptide cleavase does not remove an unlabeled terminal dipeptide from the polypeptide.

**10.** The method of claim 8, wherein the modified dipeptide cleavase comprises at least four amino acid substitutions in residues corresponding to positions 191, 192, 196, 306, 650 of SEQ ID NO: 31.

**11.** The method of claim 8, wherein the single terminal amino acid or terminal dipeptide is labeled with a N-terminal modification that comprises a N-terminal blocking group ($NTM_{blk}$) and, optionally, a natural or unnatural amino acid portion (NTMaa), wherein the NTMaa comprises a compound selected from the group consisting of: a naturally-occurring amino acid residue, 3-(3'-pyridyl)-L-alanine, L-cyclohexylglycine, $\alpha$-aminoisobutyric acid, 3-(4'-pyridyl)-L-alanine, L-azetidine-2-carboxylic acid, isonipecotic acid, L-phenylglycine, $\beta$-(2-thienyl)-L-alanine, 3-(4-thiazolyl)-L-alanine, 1-aminocyclopentane-1-carboxylic acid, (2-trifluoromethyl)-L-Phenylalanine, L-cyclopropylalanine, 3-(2'-pyridyl)-L-alanine, beta-cyano-L-alanine, $\alpha$-methyl-L-4-Fluorophenylalanine, $\alpha$-methyl-D-4-fluorophenylalanine, 3-amino-2,2-difluoro-propionic acid, O-sulfo-L-tyrosine sodium salt, L-2-furylalanine, 1-aminocyclopropane-1-carboxylic acid, 3,5-dinitro-L-tyrosine, pentafluoro-L-phenylalanine, 3,5-difluoro-L-phenylalanine, 3-fluoro-L-phenylalanine, N-cyclopentylglycine, 1-(amino)cyclohexanecarboxylic acid, N-methylalanine, 4-amino-tetrahydropyran-4-carboxylic acid, 4-amino-1,1-dioxothiane-4-carboxylic acid, 4-amino-1-methyl-4-piperidinecarboxylic acid, 2-amino-N-(2,4-dimethoxybenzyl)acetamido)acetic acid, or N-alkylated derivatives;
and the $NTM_{blk}$ comprises a compound selected from the group consisting of: 4-methylbenzoic acid, 4-(dimethylamio) benzoic acid, nicotinic acid, 3-aminonicotinic acid, 2-pyrazinecarbooxylic acid, 5-amino-2-fluoro-isonicotinic acid, 2,3-pyrazinedicarboxylic acid, 4,7-Difluoroisobenzofuran-1,3-dicarboxylic acid, 4-chloro-2-aminobenzoic acid, 4-nitro-2-aminobenzoic acid, 7-methoxy-1h-benzo[d][1,3]oxazine-2,4-dione, 4-carboxy-2-aminobenzoic acid, 6-(Trifluoromethyl)-2,4-dihydro-1h-3,1-benzoxazine-2,4-dione, 7-(Trifluoromethyl)-1h-benzo[d][1,3]oxazine-2,4-dione, 6-fluoro-2-aminobenzoic acid, 4-fluoro-2-aminobenzoic acid, 5-methoxy-2-aminobenzoic acid, 4-fluorobenzoic acid, 4-(trifluoromethyl)benzoic acid, 2-ethynyl-6-fluorobenzaldehyde, 2-aminobenzoic acid, Succinic anhydride, 3,6-Difluoropyridine-2-carboxylic acid, 2-Fluoronicotinic acid, 5-Bromo-2-hydroxynicotinic acid, 4-(Trifluoromethyl)pyrimidine-5-carboxylic acid, 2-Oxo-1,2-dihydropyridine-3-carboxylic acid, 5-Methyl-2-aminobenzoic acid, 6-Fluoropicolinic acid, 3-Methyl-2-aminobenzoic acid, 4-Methyl-2-aminobenzoic acid, 2-Amino-6-methylbenzoic acid, 2-Amino-6-fluorobenzoic acid, 2-Amino-5-fluorobenzoic acid, 2-Amino-3-fluorobenzoic acid, 2-Amino-4-fluorobenzoic acid, 2-Aminonicotinic acid, 4-Aminonicotinic acid, 3-Aminopicolinic acid, 2-Amino-4,5-difluorobenzoic acid, 3,4-difluorobenzoic acid, 3,4,5-difluorobenzoic acid, 3-(Methoxycarbonyl)bicyclo[1.1.1]pentane-1-carboxylic acid, 3,3-Difluorocyclobutane-1-carboxylic acid, 1-Methyl-2-oxo-piperidine-4-carboxylic acid, Tetrahydropyran-4-carboxylic acid, 5-Fluoroorotic acid, 3-Fluoro-4-nitrobenzoic acid, 3-(Difluoromethyl)-1-methyl-1H-pyrazole-4-carboxylic acid, 4-(Difluoromethoxy)benzoic acid, 1-(Difluoromethyl)-1h-pyrazole-3-carboxylic acid, 4-(Methanesulfonylamino)benzoic acid, 5-Fluoro-6-methoxynicotinic acid, Tetrahydro-2H-thiopyran-4-carboxylic acid 1,1-dioxide, 4-(1H-Tetrazol-5-yl)benzoic acid, 1,2,3-Thiadiazole-4-carboxylic acid, 1,3-Benzodioxole-4-carboxylic acid, 2,1,3-Benzoxadiazole-5-carboxylic acid, 1-Benzyl-3-methyl-1h-pyrazole-5-carboxylic acid, 1-Cyclopropyl-6,7-difluoro-1,4-dihydro-4-oxoquinoline-3-carboxylic acid, 3,4-Dichlorobenzoic acid, 5-Fluoro-6-methylpyridine-2-carboxylic acid, 4,5-Dimethyl-2-(1h-pyrrol-1-yl)thiophene-3-carboxylic acid, 1,3-Dimethyl-1h-thieno[2,3-c]pyrazole-5-carboxylic acid, 1-[(4-Fluorobenzene)sulfonyl]piperidine-3-carboxylic acid, 1-(4-Fluorobenzyl)-5-oxopyrrolidine-3-carboxylic acid, 3-Fluoro-4-methoxybenzoic acid, 4-Fluoro-3-nitrobenzoic acid, 6-Fluoro-4-oxochromene-2-carboxylic acid, 3-Fluorophenylacetic acid, 4-Fluoro-3-(trifluoromethyl)benzoic acid, 5-Furan-2-yl-isoxazole-3-carboxylic acid, 1-Isopropyl-2-(trifluoromethyl)-1h-benzimidazole-5-carboxylic acid, Levofloxacin carboxylic acid, 3,5,7-Trifluoroadamantane-1-carboxylic acid, 3,4,5-Trimethoxybenzoic acid, 2-Oxo-2,3-dihydro-1h-benzo[d]imidazole-4-carboxylic acid, 1-Methyl-3-(trifluoromethyl)-1h-pyrazole-5-carboxylic acid, 2-Morpholin-4-yl-isonicotinic acid, 1,3-Oxazole-4-carboxylic acid, 4-Carboxybenzenesulfonamide, 3,4-difluorobenzenesulfonyl chloride.

**12.** The method of claim 8, further comprising a step of contacting the polypeptide with a binding agent configured to bind to the single labeled terminal amino acid or to the labeled terminal dipeptide, wherein the step of labeling the terminal amino acid of the polypeptide is before the step of contacting the polypeptide with the binding agent; and the step of contacting the polypeptide with the binding agent is before the step of contacting the polypeptide with the modified dipeptide cleavase.

**13.** A set of dipeptide cleavase enzymes, comprising at least two different modified dipeptide cleavases, wherein:

(i) each of the modified dipeptide cleavases from the set of dipeptide cleavase enzymes comprises three or more amino acid substitutions in a substrate binding site of a dipeptidyl aminopeptidase, wherein the dipeptidyl aminopeptidase is configured to remove two terminal amino acids from an unlabeled polypeptide;
(ii) each of the modified dipeptide cleavases from the set of dipeptide cleavase enzymes is configured to remove a single labeled terminal amino acid from a polypeptide having the terminal amino acid labeled with a chemical reagent, wherein
the dipeptidyl aminopeptidase comprises an amino acid sequence having at least 30 % sequence identity to the amino acid sequence of SEQ ID NO: 31 and also comprises an asparagine residue at a position corresponding to position 191 of SEQ ID NO: 31, a tryptophan or phenylalanine residue at a position corresponding to position 192 of SEQ ID NO: 31, an arginine residue at a position corresponding to position 196 of SEQ ID NO: 31, an asparagine residue at a position corresponding to position 306 of SEQ ID NO: 31, an aspartate residue at a position corresponding to position 650 of SEQ ID NO: 31; and wherein the modified dipeptide cleavase comprises three or more amino acid substitutions in residues corresponding to positions 191, 192, 196, 306, 650 of SEQ ID NO: 31; and
(iii) the modified dipeptide cleavases from the set of dipeptide cleavase enzymes have different specificities for the labeled terminal amino acids, which the modified dipeptide cleavases are configured to remove.

**14.** A kit for treating a polypeptide, comprising:

(a) a chemical reagent for labeling a terminal amino acid of the polypeptide; and
(b) a set of dipeptide cleavase enzymes, comprising at least two different modified dipeptide cleavases, wherein:

(i) each of the modified dipeptide cleavases from the set of dipeptide cleavase enzymes comprises three or more amino acid substitutions in a substrate binding site of a dipeptidyl aminopeptidase, wherein the dipeptidyl aminopeptidase is configured to remove two terminal amino acids from an unlabeled polypeptide;
(ii) each of the modified dipeptide cleavases from the set of dipeptide cleavase enzymes is configured to remove a single labeled terminal amino acid from a polypeptide having the terminal amino acid labeled with a chemical reagent, wherein

the dipeptidyl aminopeptidase comprises an amino acid sequence having at least 30 % sequence identity to the amino acid sequence of SEQ ID NO: 31 and also comprises an asparagine residue at a position corresponding to position 191 of SEQ ID NO: 31, a tryptophan or phenylalanine residue at a position corresponding to position 192 of SEQ ID NO: 31, an arginine residue at a position corresponding to position 196 of SEQ ID NO: 31, an asparagine residue at a position corresponding to position 306 of SEQ ID NO: 31, an aspartate residue at a position corresponding to position 650 of SEQ ID NO: 31; and wherein the modified dipeptide cleavase comprises three or more amino acid substitutions in residues corresponding to positions 191, 192, 196, 306, 650 of SEQ ID NO: 31; and

(iii) the modified dipeptide cleavases from the set of dipeptide cleavase enzymes have different specificities for the labeled terminal amino acids, which the modified dipeptide cleavases are configured to remove.

**15.** The kit of claim 14, wherein (i) the chemical reagent is configured to attach a N-terminal modification to the terminal amino acid of the polypeptide; (ii) the N-terminal modification comprises a N-terminal blocking group ($NTM_{blk}$) and, optionally, a natural or unnatural amino acid portion (NTMaa); (iii) the NTMaa comprises a compound selected from the group consisting of: a naturally-occurring amino acid residue, 3-(3'-pyridyl)-L-alanine, L-cyclohexylglycine, α-aminoisobutyric acid, 3-(4'-pyridyl)-L-alanine, L-azetidine-2-carboxylic acid, isonipecotic acid, L-phenylglycine, β-(2-thienyl)-L-alanine, 3-(4-thiazolyl)-L-alanine, 1-aminocyclopentane-1-carboxylic acid, (2-trifluoromethyl)-L-Phenylalanine, L-cyclopropylalanine, 3-(2'-pyridyl)-L-alanine, beta-cyano-L-alanine, α-methyl-L-4-Fluorophenylalanine, α-methyl-D-4-fluorophenylalanine, 3-amino-2,2-difluoro-propionic acid, O-sulfo-L-tyrosine sodium salt, L-2-furylalanine, 1-aminocyclopropane-1-carboxylic acid, 3,5-dinitro-L-tyrosine, pentafluoro-L-phenylalanine, 3,5-difluoro-L-phenylalanine, 3-fluoro-L-phenylalanine, N-cyclopentylglycine, 1-(amino)cyclohexanecarboxylic acid, N-methylalanine, 4-amino-tetrahydropyran-4-carboxylic acid, 4-amino-1,1-dioxothiane-4-carboxylic acid, 4-amino-1-methyl-4-piperidinecarboxylic acid, 2-amino-N-(2,4-dimethoxybenzyl)acetamido)acetic acid, or N-alkylated derivatives; and (iv) the $NTM_{blk}$ comprises a compound selected from the group consisting of: 4-methylbenzoic acid, 4-(dimethylamio)benzoic acid, nicotinic acid, 3-aminonicotinic acid, 2-pyrazinecarbooxylic acid, 5-amino-2-fluoro-isonicotinic acid, 2,3-pyrazinedicarboxylic acid, 4,7-Difluoroisobenzofuran-1,3-dicarboxylic acid, 4-chloro-2-aminobenzoic acid, 4-nitro-2-aminobenzoic acid, 7-methoxy-1h-benzo[d][1,3]oxazine-2,4-dione, 4-carboxy-2-aminobenzoic acid, 6-(Trifluoromethyl)-2,4-dihydro-1h-3,1-benzoxazine-2,4-dione, 7-(Trifluoromethyl)-1h-benzo[d][1,3]oxazine-2,4-dione, 6-fluoro-2-aminobenzoic acid, 4-fluoro-2-aminobenzoic acid, 5-methoxy-2-aminobenzoic acid, 4-fluorobenzoic acid, 4-(trifluoromethyl)benzoic acid, 2-ethynyl-6-fluorobenzaldehyde, 2-aminobenzoic acid, Succinic anhydride, 3,6-Difluoropyridine-2-carboxylic acid, 2-Fluoronicotinic acid, 5-Bromo-2-hydroxynicotinic acid, 4-(Trifluoromethyl)pyrimidine-5-carboxylic acid, 2-Oxo-1,2-dihydropyridine-3-carboxylic acid, 5-Methyl-2-aminobenzoic acid, 6-Fluoropicolinic acid, 3-Methyl-2-aminobenzoic acid, 4-Methyl-2-aminobenzoic acid, 2-Amino-6-methylbenzoic acid, 2-Amino-6-fluorobenzoic acid, 2-Amino-5-fluorobenzoic acid, 2-Amino-3-fluorobenzoic acid, 2-Amino-4-fluorobenzoic acid, 2-Aminonicotinic acid, 4-Aminonicotinic acid, 3-Aminopicolinic acid, 2-Amino-4,5-difluorobenzoic acid, 3,4-difluorobenzoic acid, 3,4,5-difluorobenzoic acid, 3-(Methoxycarbonyl)bicyclo[1.1.1]pentane-1-carboxylic acid, 3,3-Difluorocyclobutane-1-carboxylic acid, 1-Methyl-2-oxo-piperidine-4-carboxylic acid, Tetrahydropyran-4-carboxylic acid, 5-Fluoroorotic acid, 3-Fluoro-4-nitrobenzoic acid, 3-(Difluoromethyl)-1-methyl-1H-pyrazole-4-carboxylic acid, 4-(Difluoromethoxy)benzoic acid, 1-(Difluoromethyl)-1h-pyrazole-3-carboxylic acid, 4-(Methanesulfonylamino)benzoic acid, 5-Fluoro-6-methoxynicotinic acid, Tetrahydro-2H-thiopyran-4-carboxylic acid 1,1-dioxide, 4-(1H-Tetrazol-5-yl)benzoic acid, 1,2,3-Thiadiazole-4-carboxylic acid, 1,3-Benzodioxole-4-carboxylic acid, 2,1,3-Benzoxadiazole-5-carboxylic acid, 1-Benzyl-3-methyl-1h-pyrazole-5-carboxylic acid, 1-Cyclopropyl-6,7-difluoro-1,4-dihydro-4-oxoquinoline-3-carboxylic acid, 3,4-Dichlorobenzoic acid, 5-Fluoro-6-methylpyridine-2-carboxylic acid, 4,5-Dimethyl-2-(1h-pyrrol-1-yl)thiophene-3-carboxylic acid, 1,3-Dimethyl-1h-thieno[2,3-c]pyrazole-5-carboxylic acid, 1-[(4-Fluorobenzene)sulfonyl]piperidine-3-carboxylic acid, 1-(4-Fluorobenzyl)-5-oxopyrrolidine-3-carboxylic acid, 3-Fluoro-4-methoxybenzoic acid, 4-Fluoro-3-nitrobenzoic acid, 6-Fluoro-4-oxochromene-2-carboxylic acid, 3-Fluorophenylacetic acid, 4-Fluoro-3-(trifluoromethyl)benzoic acid, 5-Furan-2-yl-isoxazole-3-carboxylic acid, 1-Isopropyl-2-(trifluoromethyl)-1h-benzimidazole-5-carboxylic acid, Levofloxacin carboxylic acid, 3,5,7-Trifluoroadamantane-1-carboxylic acid, 3,4,5-Trimethoxybenzoic acid, 2-Oxo-2,3-dihydro-1h-benzo[d]imidazole-4-carboxylic acid, 1-Methyl-3-(trifluoromethyl)-1h-pyrazole-5-carboxylic acid, 2-Morpholin-4-yl-isonicotinic acid, 1,3-Oxazole-4-carboxylic acid, 4-Carboxybenzenesulfonamide, 3,4-difluorobenzenesulfonyl chloride.

## Patentansprüche

**1.** Modifizierte Dipeptid-Spaltase, umfassend drei oder mehr Aminosäuresubstitutionen an einer Substratbindungsstelle einer Dipeptidylaminopeptidase, wobei:

(i) die Dipeptidylaminopeptidase entfernt oder so konfiguriert ist, dass sie zwei Terminal-Aminosäuren aus einem Polypeptid entfernt; und

(ii) die modifizierte Dipeptid-Cleavase entfernt oder so konfiguriert ist, dass sie aus dem Polypeptid mit einem chemischen Reagenz markierten Terminal-Aminosäurerest (a) den einzelnen markierten Terminal-Aminosäurerest oder (b) ein markiertes Terminal-Dipeptid entfernt, wobei

die Dipeptidylaminopeptidase eine Aminosäuresequenz umfasst, die mindestens 30 % Sequenzidentität mit der Aminosäuresequenz der SEQ-ID-NR aufweist: 31 und einen Asparaginrest an einer Position umfasst, die der Position 191 der SEQ-ID-NR entspricht: 31, ein Tryptophan- oder Phenylalaninrest an einer Position, die der Position 192 der SEQ-ID-NR entspricht: 31, ein Argininrest an einer Position, die der Position 196 der SEQ-ID-NR entspricht: 31, ein Asparaginrest an einer Position, die der Position 306 der SEQ-ID-NR entspricht: 31, ein Aspartatrest an einer Position, die der Position 650 der SEQ-ID-NR entspricht: 31; und wobei die modifizierte Dipeptid-Spaltase drei oder mehr Aminosäuresubstitutionen in Resten umfasst, die den Positionen 191, 192, 196, 306, 650 der SEQ-ID-NR entsprechen: 31.

2. Modifizierte Dipeptid-Cleavase nach Anspruch 1, wobei die modifizierte Dipeptid-Cleavase kein unmarkiertes Terminal-Dipeptid aus dem Polypeptid entfernt.

3. Modifizierte Dipeptid-Cleavase nach Anspruch 1, die mindestens vier Aminosäuresubstitutionen in Resten umfasst, die den Positionen 191, 192, 196, 306, 650 der SEQ-ID-NR entsprechen: 31.

4. Modifizierte Dipeptid-Cleavase nach Anspruch 1, wobei die modifizierte Dipeptid-Cleavase eine einzelne N-terminal markierte Aminosäure des Polypeptids entfernt oder so konfiguriert ist, dass sie diese entfernt.

5. Modifizierte Dipeptid-Cleavase nach Anspruch 1, die zusätzlich eine oder mehrere Aminosäuresubstitutionen in Resten umfasst, die den Positionen 310, 628, 648, 651, 659, 669 entsprechen.

6. Modifizierte Dipeptid-Spaltase nach Anspruch 1, wobei die Dipeptidylaminopeptidase ein Protein ist, das in MEROPS S46 klassifiziert ist, oder ein funktionelles Homolog oder Fragment davon.

7. Modifizierte Dipeptid-Cleavage-Enzym nach Anspruch 1, wobei die einzelne Terminal-Aminosäure oder das Terminal-Dipeptid mit einer N-terminalen Modifikation markiert ist, die eine N-terminale Blockierungsgruppe ($NTM_{blk}$) und optional einen natürlichen oder unnatürlichen Aminosäureabschnitt (NTMaa) umfasst, wobei NTMaa eine Verbindung umfasst, die aus der Gruppe ausgewählt ist, bestehend aus: einem natürlich vorkommenden Aminosäurerest, 3-(3'-Pyridyl)-L-alanin, L-Cyclohexylglycin, α-Aminoisobuttersäure, 3-(4'-Pyridyl)-L-alanin, L-Azetidin-2-carbonsäure, Isonipecolsäure, L-Phenylglycin, β-(2-Thienyl)-L-alanin, 3-(4-Thiazolyl)-L-alanin, 1-Aminocyclopentan-1-carbonsäure, (2-Trifluormethyl)-L-phenylalanin, L-Cyclopropylalanin, 3-(2'-Pyridyl)-L-Alanin, Beta-Cyano-L-Alanin, α-Methyl-L-4-Fluorphenylalanin, α-Methyl-D-4-Fluorphenylalanin, 3-Amino-2,2-Difluorpropionsäure, O-Sulfo-L-Tyrosin-Natriumsalz, L-2-Furylalanin, 1-Aminocyclopropan-1-carbonsäure, 3,5-Dinitro-L-Tyrosin, Pentafluor-L-phenylalanin, 3,5-Difluor-L-phenylalanin, 3-Fluor-L-phenylalanin, N-Cyclopentylglycin, 1-(Amino)cyclohexancarbonsäure, N-Methylalanin, 4-Aminotetrahydropyran-4-carbonsäure, 4-Amino-1,1-dioxothian-4-carbonsäure, 4-Amino-1-methyl-4-piperidincarboxylsäure, 2-Amino-N-(2,4-dimethoxybenzyl)acetamido)essigsäure oder N-alkylierte Derivate;

und der $NTM_{blk}$ eine Verbindung umfasst, die aus der Gruppe ausgewählt ist, bestehend aus: 4-Methylbenzoesäure, 4-(Dimethylamino)benzoesäure, Nikotinsäure, 3-Aminonicotinsäure, 2-Pyrazincarboxylsäure, 5-Amino-2-fluorisonicotinsäure, 2,3-Pyrazindicarbonsäure, 4,7-Difluorisobenzofuran-1,3-dicarbonsäure, 4-Chlor-2-aminobenzoesäure, 4-Nitro-2-aminobenzoesäure, 7-Methoxy-1h-benzo[d][1,3]oxazin-2,4-dion, 4-Carboxy-2-aminobenzoesäure, 6-(Trifluormethyl)-2,4-dihydro-1h-3,1-benzoxazin-2,4-dion, 7-(Trifluormethyl)-1h-benzo[d][1,3]oxazin-2,4-dion, 6-Fluor-2-aminobenzoesäure, 4-Fluor-2-aminobenzoesäure, 5-Methoxy-2-aminobenzoesäure, 4-Fluorbenzoesäure, 4-(Trifluormethyl)benzoesäure, 2-Ethynyl-6-fluorbenzaldehyd, 2-Aminobenzoesäure, Bernsteinsäureanhydrid, 3,6-Difluorpyridin-2-carbonsäure, 2-Fluornicotinsäure, 5-Brom-2-hydroxynicotinsäure, 4-(Trifluormethyl)pyrimidin-5-carbonsäure, 2-Oxo-1,2-dihydropyridin-3-carbonsäure, 5-Methyl-2-aminobenzoesäure, 6-Fluorpicolinsäure, 3-Methyl-2-aminobenzoesäure, 4-Methyl-2-aminobenzoesäure, 2-Amino-6-methylbenzoesäure, 2-Amino-6-fluorbenzoesäure, 2-Amino-5-fluorbenzoesäure, 2-Amino-3-fluorbenzoesäure, 2-Amino-4-fluorbenzoesäure, 2-Aminonicotinsäure, 4-Aminonicotinsäure, 3-Aminopicolinsäure, 2-Amino-4,5-difluorbenzoesäure, 3,4-Difluorbenzoesäure, 3,4,5-Difluorbenzoesäure, 3-(Methoxycarbonyl)bicyclo[1.1.1]pentan-1-carbonsäure, 3,3-Difluorcyclobutan-1-carbonsäure, 1-Methyl-2-oxo-piperidin-4-carbonsäure, Tetrahydropyran-4-carbonsäure, 5-Fluororotinsäure, 3-Fluor-4-nitrobenzoesäure, 3-(Difluormethyl)-1-methyl-1H-pyrazol-4-carbonsäure, 4-(Difluormethoxy)benzoesäure,

1-(Difluormethyl)-1h-pyrazol-3-carbonsäure, 4-(Methansulfonylamino)benzoesäure, 5-Fluor-6-methoxynicotinsäure, Tetrahydro-2H-thiopyran-4-carbonsäure-1,1-dioxid, 4-(1H-Tetrazol-5-yl)benzoesäure, 1,2,3-Thiadiazol-4-carbonsäure, 1,3-Benzodioxol-4-carbonsäure, 2,1,3-Benzoxadiazol-5-carbonsäure, 1-Benzyl-3-methyl-1h-pyrazol-5-carbonsäure, 1-Cyclopropyl-6,7-difluor-1,4-dihydro-4-oxochinolin-3-carbonsäure, 3,4-Dichlorbenzoesäure, 5-Fluor-6-methylpyridin-2-carbonsäure, 4,5-Dimethyl-2-(1h-pyrrol-1-yl)thiophen-3-carbonsäure, 1,3-Dimethyl-1h-thieno[2,3-c]pyrazol-5-carbonsäure, 1-[(4-Fluorbenzol)sulfonyl]piperidin-3-carbonsäure, 1-(4-Fluorbenzyl)-5-oxopyrrolidin-3-carbonsäure, 3-Fluor-4-methoxybenzoesäure, 4-Fluor-3-nitrobenzoesäure, 6-Fluor-4-oxochromen-2-carbonsäure, 3-Fluorphenylessigsäure, 4-Fluor-3-(trifluormethyl)benzoesäure, 5-Furan-2-yl-isoxazol-3-carbonsäure, 1-Isopropyl-2-(trifluormethyl)-1h-benzimidazol-5-carbonsäure, Levofloxacin-Carbonsäure, 3,5,7-Trifluoradamantan-1-carbonsäure, 3,4,5-Trimethoxybenzoesäure, 2-Oxo-2,3-dihydro-1h-benzo[d]imidazol-4-carbonsäure, 1-Methyl-3-(trifluormethyl)-1h-pyrazol-5-carbonsäure, 2-Morpholin-4-yl-isonicotinsäure, 1,3-Oxazol-4-carbonsäure, 4-Carboxybenzolsulfonamid, 3,4-Difluorbenzolsulfonylchlorid.

**8.** Verfahren zur Behandlung eines Polypeptids, umfassend die folgenden Schritte:

Markieren einer Terminal-Aminosäure des Polypeptids mit einem chemischen Reagenz, um ein markiertes Polypeptid herzustellen; und
Inkontaktbringen des markierten Polypeptids mit einer modifizierten Dipeptid-Cleavase, wobei die modifizierte Dipeptid-Cleavase drei oder mehr Aminosäuresubstitutionen in einer Substratbindungsstelle einer Dipeptidylaminopeptidase umfasst, wobei:

(i) die Dipeptidylaminopeptidase entfernt oder so konfiguriert ist, dass sie bei Kontakt zwei Terminal-Aminosäuren aus dem Polypeptid entfernt; und
(ii) die modifizierte Dipeptid-Cleavase entfernt oder so konfiguriert ist, dass sie aus dem Polypeptid mit einem chemischen Reagenz markierte Terminal-Aminosäure (a) die einzelne markierte Terminal-Aminosäure oder (b) ein markiertes Terminal-Dipeptid entfernt, wobei

die Dipeptidylaminopeptidase eine Aminosäuresequenz umfasst, die mindestens 30 % Sequenzidentität mit der Aminosäuresequenz der SEQ-ID-NR aufweist: 31 und einen Asparaginrest an einer Position umfasst, die der Position 191 der SEQ-ID-NR entspricht: 31, ein Tryptophan- oder Phenylalaninrest an einer Position, die der Position 192 der SEQ-ID-NR entspricht: 31, ein Argininrest an einer Position, die der Position 196 der SEQ-ID-NR entspricht: 31, ein Asparaginrest an einer Position, die der Position 306 der SEQ-ID-NR entspricht: 31, ein Aspartatrest an einer Position, die der Position 650 der SEQ-ID-NR entspricht: 31; und wobei die modifizierte Dipeptid-Spaltase drei oder mehr Aminosäuresubstitutionen in Resten umfasst, die den Positionen 191, 192, 196, 306, 650 der SEQ-ID-NR entsprechen: 31.

**9.** Verfahren nach Anspruch 8, wobei die modifizierte Dipeptid-Cleavase kein unmarkiertes Terminal-Dipeptid aus dem Polypeptid entfernt.

**10.** Verfahren nach Anspruch 8, wobei die modifizierte Dipeptid-Cleavase mindestens vier Aminosäuresubstitutionen in Resten umfasst, die den Positionen 191, 192, 196, 306, 650 der SEQ-ID-NR entsprechen: 31.

**11.** Verfahren nach Anspruch 8, wobei die einzelne Terminal-Aminosäure oder das Terminal-Dipeptid mit einer N-terminalen Modifikation markiert ist, die eine N-terminale Blockierungsgruppe ($NTM_{blk}$) und optional einen natürlichen oder unnatürlichen Aminosäureabschnitt (NTMaa) umfasst, wobei NTMaa eine Verbindung umfasst, die aus der Gruppe ausgewählt ist, bestehend aus: einem natürlich vorkommenden Aminosäurerest, 3-(3'-Pyridyl)-L-alanin, L-Cyclohexylglycin, $\alpha$-Aminoisobuttersäure, 3-(4'-Pyridyl)-L-alanin, L-Azetidin-2-carbonsäure, Isonipecolsäure, L-Phenylglycin, $\beta$-(2-Thienyl)-L-alanin, 3-(4-Thiazolyl)-L-alanin, 1-Aminocyclopentan-1-carbonsäure, (2-Trifluormethyl)-L-phenylalanin, L-Cyclopropylalanin, 3-(2'-Pyridyl)-L-Alanin, Beta-Cyano-L-Alanin, $\alpha$-Methyl-L-4-Fluorphenylalanin, $\alpha$-Methyl-D-4-Fluorphenylalanin, 3-Amino-2,2-Difluorpropionsäure, O-Sulfo-L-Tyrosin-Natriumsalz, L-2-Furylalanin, 1-Aminocyclopropan-1-carbonsäure, 3,5-Dinitro-L-Tyrosin, Pentafluor-L-phenylalanin, 3,5-Difluor-L-phenylalanin, 3-Fluor-L-phenylalanin, N-Cyclopentylglycin, 1-(Amino)cyclohexancarbonsäure, N-Methylalanin, 4-Aminotetrahydropyran-4-carbonsäure, 4-Amino-1,1-dioxothian-4-carbonsäure, 4-Amino-1-methyl-4-piperidincarboxylsäure, 2-Amino-N-(2,4-dimethoxybenzyl)acetamido)essigsäure oder N-alkylierte Derivate;
und der $NTM_{blk}$ eine Verbindung umfasst, die aus der Gruppe ausgewählt ist, bestehend aus: 4-Methylbenzoesäure, 4-(Dimethylamino)benzoesäure, Nikotinsäure, 3-Aminonicotinsäure, 2-Pyrazincarboxylsäure, 5-Amino-2-fluorisonicotinsäure, 2,3-Pyrazindicarbonsäure, 4,7-Difluorisobenzofuran-1,3-dicarbonsäure, 4-Chlor-2-aminobenzoesäure, 4-Nitro-2-aminobenzoesäure, 7-Methoxy-1h-benzo[d][1,3]oxazin-2,4-dion, 4-Carboxy-2-aminobenzoesäure,

6-(Trifluormethyl)-2,4-dihydro-1h-3,1-benzoxazin-2,4-dion, 7-(Trifluormethyl)-1h-benzo[d][1,3]oxazin-2,4-dion, 6-Fluor-2-aminobenzoesäure, 4-Fluor-2-aminobenzoesäure, 5-Methoxy-2-aminobenzoesäure, 4-Fluorbenzoesäure, 4-(Trifluormethyl)benzoesäure, 2-Ethynyl-6-fluorbenzaldehyd, 2-Aminobenzoesäure, Bernsteinsäureanhydrid, 3,6-Difluorpyridin-2-carbonsäure, 2-Fluornicotinsäure, 5-Brom-2-hydroxynicotinsäure, 4-(Trifluormethyl)pyrimidin-5-carbonsäure, 2-Oxo-1,2-dihydropyridin-3-carbonsäure, 5-Methyl-2-aminobenzoesäure, 6-Fluorpicolinsäure, 3-Methyl-2-aminobenzoesäure, 4-Methyl-2-aminobenzoesäure, 2-Amino-6-methylbenzoesäure, 2-Amino-6-fluorbenzoesäure, 2-Amino-5-fluorbenzoesäure, 2-Amino-3-fluorbenzoesäure, 2-Amino-4-fluorbenzoesäure, 2-Aminonicotinsäure, 4-Aminonicotinsäure, 3-Aminopicolinsäure, 2-Amino-4,5-difluorbenzoesäure, 3,4-Difluorbenzoesäure, 3,4,5-Difluorbenzoesäure, 3-(Methoxycarbonyl)bicyclo[1.1.1]pentan-1-carbonsäure, 3,3-Difluorcyclobutan-1-carbonsäure, 1-Methyl-2-oxo-piperidin-4-carbonsäure, Tetrahydropyran-4-carbonsäure, 5-Fluororotinsäure, 3-Fluor-4-nitrobenzoesäure, 3-(Difluormethyl)-1-methyl-1H-pyrazol-4-carbonsäure, 4-(Difluormethoxy)benzoesäure, 1-(Difluormethyl)-1h-pyrazol-3-carbonsäure, 4-(Methansulfonylamino)benzoesäure, 5-Fluor-6-methoxynicotinsäure, Tetrahydro-2H-thiopyran-4-carbonsäure-1,1-dioxid, 4-(1H-Tetrazol-5-yl)benzoesäure, 1,2,3-Thiadiazol-4-carbonsäure, 1,3-Benzodioxol-4-carbonsäure, 2,1,3-Benzoxadiazol-5-carbonsäure, 1-Benzyl-3-methyl-1h-pyrazol-5-carbonsäure, 1-Cyclopropyl-6,7-difluor-1,4-dihydro-4-oxochinolin-3-carbonsäure, 3,4-Dichlorbenzoesäure, 5-Fluor-6-methylpyridin-2-carbonsäure, 4,5-Dimethyl-2-(1h-pyrrol-1-yl)thiophen-3-carbonsäure, 1,3-Dimethyl-1h-thieno[2,3-c]pyrazol-5-carbonsäure, 1-[(4-Fluorbenzol)sulfonyl]piperidin-3-carbonsäure, 1-(4-Fluorbenzyl)-5-oxopyrrolidin-3-carbonsäure, 3-Fluor-4-methoxybenzoesäure, 4-Fluor-3-nitrobenzoesäure, 6-Fluor-4-oxochromen-2-carbonsäure, 3-Fluorphenylessigsäure, 4-Fluor-3-(trifluormethyl)benzoesäure, 5-Furan-2-yl-isoxazol-3-carbonsäure, 1-Isopropyl-2-(trifluormethyl)-1h-benzimidazol-5-carbonsäure, Levofloxacin-Carbonsäure, 3,5,7-Trifluoradamantan-1-carbonsäure, 3,4,5-Trimethoxybenzoesäure, 2-Oxo-2,3-dihydro-1h-benzo[d]imidazol-4-carbonsäure, 1-Methyl-3-(trifluormethyl)-1h-pyrazol-5-carbonsäure, 2-Morpholin-4-yl-isonicotinsäure, 1,3-Oxazol-4-carbonsäure, 4-Carboxybenzolsulfonamid, 3,4-Difluorbenzolsulfonylchlorid.

**12.** Verfahren nach Anspruch 8, ferner umfassend einen Schritt des Inkontaktbringens des Polypeptids mit einem Bindemittel, das so konfiguriert ist, dass es an die einzelne markierte terminale Aminosäure oder an das markierte terminale Dipeptid bindet, wobei der Schritt des Markierens der Terminal-Aminosäure des Polypeptids vor dem Schritt des Inkontaktbringens des Polypeptids mit dem Bindemittel erfolgt. und der Schritt des Inkontaktbringens des Polypeptids mit dem Bindemittel erfolgt vor dem Schritt des Inkontaktbringens des Polypeptids mit der modifizierten Dipeptid-Cleavase.

**13.** Satz von Dipeptid-Spaltungsenzymen, umfassend mindestens zwei verschiedene modifizierte Dipeptid-Spaltungsenzyme, wobei:

(i) jede der modifizierten Dipeptid-Cleavasen aus der Gruppe der Dipeptid-Cleavase-Enzyme drei oder mehr Aminosäuresubstitutionen in einer Substratbindungsstelle einer Dipeptidylaminopeptidase umfasst, wobei die Dipeptidylaminopeptidase so konfiguriert ist, dass sie zwei Terminal-Aminosäuren aus einem unmarkierten Polypeptid entfernt;
(ii) jede der modifizierten Dipeptid-Spaltasen aus der Gruppe der Dipeptid-Spaltasen-Enzyme so konfiguriert ist, dass sie eine einzelne markierte Terminal-Aminosäure aus einem Polypeptid entfernt, dessen Terminal-Aminosäure mit einem chemischen Reagenz markiert ist, wobei
die Dipeptidylaminopeptidase eine Aminosäuresequenz umfasst, die mindestens 30 % Sequenzidentität mit der Aminosäuresequenz der SEQ-ID-NR aufweist: 31 und einen Asparaginrest an einer Position umfasst, die der Position 191 der SEQ-ID-NR entspricht: 31, ein Tryptophan- oder Phenylalaninrest an einer Position, die der Position 192 der SEQ-ID-NR entspricht: 31, ein Argininrest an einer Position, die der Position 196 der SEQ-ID-NR entspricht: 31, ein Asparaginrest an einer Position, die der Position 306 der SEQ-ID-NR entspricht: 31, ein Aspartatrest an einer Position, die der Position 650 der SEQ-ID-NR entspricht: 31; und wobei die modifizierte Dipeptid-Spaltase drei oder mehr Aminosäuresubstitutionen in Resten umfasst, die den Positionen 191, 192, 196, 306, 650 der SEQ-ID-NR entsprechen: 31; und
(iii) die modifizierten Dipeptid-Spaltproteasen aus der Gruppe der Dipeptid-Spaltproteasen unterschiedliche Spezifitäten für die markierten Terminal-Aminosäuren aufweisen, die die modifizierten Dipeptid-Spaltproteasen entfernen sollen.

**14.** Kit zur Behandlung eines Polypeptids, umfassend:

(a) ein chemisches Reagenz zum Markieren einer Terminal-Aminosäure des Polypeptids; und
(b) einen Satz von Dipeptid-Spaltungsenzymen, umfassend mindestens zwei verschiedene modifizierte Dipeptid-Spaltungsenzyme, wobei:

(i) jede der modifizierten Dipeptid-Cleavasen aus der Gruppe der Dipeptid-Cleavase-Enzyme drei oder mehr Aminosäuresubstitutionen in einer Substratbindungsstelle einer Dipeptidylaminopeptidase umfasst, wobei die Dipeptidylaminopeptidase so konfiguriert ist, dass sie zwei Terminal-Aminosäuren aus einem unmarkierten Polypeptid entfernt;

(ii) jede der modifizierten Dipeptid-Spaltasen aus der Gruppe der Dipeptid-Spaltasen-Enzyme so konfiguriert ist, dass sie eine einzelne markierte Terminal-Aminosäure aus einem Polypeptid entfernt, dessen Terminal-Aminosäure mit einem chemischen Reagenz markiert ist, wobei

die Dipeptidylaminopeptidase eine Aminosäuresequenz umfasst, die mindestens 30 % Sequenzidentität mit der Aminosäuresequenz der SEQ-ID-NR aufweist: 31 und einen Asparaginrest an einer Position umfasst, die der Position 191 der SEQ-ID-NR entspricht: 31, ein Tryptophan- oder Phenylalaninrest an einer Position, die der Position 192 der SEQ-ID-NR entspricht: 31, ein Argininrest an einer Position, die der Position 196 der SEQ-ID-NR entspricht: 31, ein Asparaginrest an einer Position, die der Position 306 der SEQ-ID-NR entspricht: 31, ein Aspartatrest an einer Position, die der Position 650 der SEQ-ID-NR entspricht: 31; und wobei die modifizierte Dipeptid-Spaltase drei oder mehr Aminosäuresubstitutionen in Resten umfasst, die den Positionen 191, 192, 196, 306, 650 der SEQ-ID-NR entsprechen: 31; und

(iii) die modifizierten Dipeptid-Spaltproteasen aus der Gruppe der Dipeptid-Spaltproteasen unterschiedliche Spezifitäten für die markierten Terminal-Aminosäuren aufweisen, die die modifizierten Dipeptid-Spaltproteasen entfernen sollen.

**15.** Kit nach Anspruch 14, wobei (i) das chemische Reagenz so konfiguriert ist, dass es eine N-terminale Modifikation an die Terminal-Aminosäure des Polypeptids anfügt; (ii) die N-terminale Modifikation eine N-terminale Blockierungsgruppe ($NTM_{blk}$) sowie optional einen natürlichen oder unnatürlichen Aminosäureabschnitt (NTMaa) umfasst; (iii) der NTMaa eine Verbindung umfasst, die aus der Gruppe ausgewählt ist, bestehend aus: einem natürlich vorkommenden Aminosäurerest, 3-(3'-Pyridyl)-L-alanin, L-Cyclohexylglycin, α-Aminoisobuttersäure, 3-(4'-Pyridyl)-L-alanin, L-Azetidin-2-carbonsäure, Isonipecolsäure, L-Phenylglycin, β-(2-Thienyl)-L-alanin, 3-(4-Thiazolyl)-L-alanin, 1-Aminocyclopentan-1-carbonsäure, (2-Trifluormethyl)-L-phenylalanin, L-Cyclopropylalanin, 3-(2'-Pyridyl)-L-Alanin, Beta-Cyano-L-Alanin, α-Methyl-L-4-Fluorphenylalanin, α-Methyl-D-4-Fluorphenylalanin, 3-Amino-2,2-Difluorpropionsäure, O-Sulfo-L-Tyrosin-Natriumsalz, L-2-Furylalanin, 1-Aminocyclopropan-1-carbonsäure, 3,5-Dinitro-L-Tyrosin, Pentafluor-L-phenylalanin, 3,5-Difluor-L-phenylalanin, 3-Fluor-L-phenylalanin, N-Cyclopentylglycin, 1-(Amino)cyclohexancarbonsäure, N-Methylalanin, 4-Aminotetrahydropyran-4-carbonsäure, 4-Amino-1,1-dioxothian-4-carbonsäure, 4-Amino-1-methyl-4-piperidincarboxylsäure, 2-Amino-N-(2,4-dimethoxybenzyl)acetamido)essigsäure oder N-alkylierte Derivate;

und (iv) der $NTM_{blk}$ eine Verbindung umfasst, die aus der Gruppe ausgewählt ist, bestehend aus: 4-Methylbenzoesäure, 4-(Dimethylamino)benzoesäure, Nikotinsäure, 3-Aminonicotinsäure, 2-Pyrazincarboxylsäure, 5-Amino-2-fluorisonicotinsäure, 2,3-Pyrazindicarbonsäure, 4,7-Difluorisobenzofuran-1,3-dicarbonsäure, 4-Chlor-2-aminobenzoesäure, 4-Nitro-2-aminobenzoesäure, 7-Methoxy-1h-benzo[d][1,3]oxazin-2,4-dion, 4-Carboxy-2-aminobenzoesäure, 6-(Trifluormethyl)-2,4-dihydro-1h-3,1-benzoxazin-2,4-dion, 7-(Trifluormethyl)-1h-benzo[d][1,3]oxazin-2,4-dion, 6-Fluor-2-aminobenzoesäure, 4-Fluor-2-aminobenzoesäure, 5-Methoxy-2-aminobenzoesäure, 4-Fluorbenzoesäure, 4-(Trifluormethyl)benzoesäure, 2-Ethynyl-6-fluorbenzaldehyd, 2-Aminobenzoesäure, Bernsteinsäureanhydrid, 3,6-Difluorpyridin-2-carbonsäure, 2-Fluornicotinsäure, 5-Brom-2-hydroxynicotinsäure, 4-(Trifluormethyl)pyrimidin-5-carbonsäure, 2-Oxo-1,2-dihydropyridin-3-carbonsäure, 5-Methyl-2-aminobenzoesäure, 6-Fluorpicolinsäure, 3-Methyl-2-aminobenzoesäure, 4-Methyl-2-aminobenzoesäure, 2-Amino-6-methylbenzoesäure, 2-Amino-6-fluorbenzoesäure, 2-Amino-5-fluorbenzoesäure, 2-Amino-3-fluorbenzoesäure, 2-Amino-4-fluorbenzoesäure, 2-Aminonicotinsäure, 4-Aminonicotinsäure, 3-Aminopicolinsäure, 2-Amino-4,5-difluorbenzoesäure, 3,4-Difluorbenzoesäure, 3,4,5-Difluorbenzoesäure, 3-(Methoxycarbonyl)bicyclo[1.1.1]pentan-1-carbonsäure, 3,3-Difluorcyclobutan-1-carbonsäure, 1-Methyl-2-oxo-piperidin-4-carbonsäure, Tetrahydropyran-4-carbonsäure, 5-Fluororotinsäure, 3-Fluor-4-nitrobenzoesäure, 3-(Difluormethyl)-1-methyl-1H-pyrazol-4-carbonsäure, 4-(Difluormethoxy)benzoesäure, 1-(Difluormethyl)-1h-pyrazol-3-carbonsäure, 4-(Methansulfonylamino)benzoesäure, 5-Fluor-6-methoxynicotinsäure, Tetrahydro-2H-thiopyran-4-carbonsäure-1,1-dioxid, 4-(1H-Tetrazol-5-yl)benzoesäure, 1,2,3-Thiadiazol-4-carbonsäure, 1,3-Benzodioxol-4-carbonsäure, 2,1,3-Benzoxadiazol-5-carbonsäure, 1-Benzyl-3-methyl-1h-pyrazol-5-carbonsäure, 1-Cyclopropyl-6,7-difluor-1,4-dihydro-4-oxochinolin-3-carbonsäure, 3,4-Dichlorbenzoesäure, 5-Fluor-6-methylpyridin-2-carbonsäure, 4,5-Dimethyl-2-(1h-pyrrol-1-yl)thiophen-3-carbonsäure, 1,3-Dimethyl-1h-thieno[2,3-c]pyrazol-5-carbonsäure, 1-[(4-Fluorbenzol)sulfonyl]piperidin-3-carbonsäure, 1-(4-Fluorbenzyl)-5-oxopyrrolidin-3-carbonsäure, 3-Fluor-4-methoxybenzoesäure, 4-Fluor-3-nitrobenzoesäure, 6-Fluor-4-oxochromen-2-carbonsäure, 3-Fluorphenylessigsäure, 4-Fluor-3-(trifluormethyl)benzoesäure, 5-Furan-2-yl-isoxazol-3-carbonsäure, 1-Isopropyl-2-(trifluormethyl)-1h-benzimidazol-5-carbonsäure, Levofloxacin-Carbonsäure, 3,5,7-Trifluoradamantan-1-carbonsäure, 3,4,5-Trimethoxybenzoesäure, 2-Oxo-2,3-dihydro-1h-benzo[d]imidazol-4-carbonsäure, 1-Methyl-3-(trifluormethyl)-1h-pyrazol-5-carbonsäure, 2-Morpholin-4-yl-isonicotinsäure, 1,3-Oxazol-4-carbonsäure,

4-Carboxybenzolsulfonamid, 3,4-Difluorbenzolsulfonylchlorid.

## Revendications

1. Enzyme de clivage dipeptidique modifiée comprenant trois substitutions d'acides aminés ou plus dans un site de liaison au substrat d'une dipeptidyl aminopeptidase, dans laquelle :

   (i) la dipeptidyl aminopeptidase élimine ou est conçue pour éliminer deux acides aminés terminaux d'un polypeptide ; et
   (ii) l'enzyme de clivage dipeptidique modifiée élimine ou est conçue pour éliminer du polypeptide ayant un résidu d'acide aminé terminal marqué avec un réactif chimique (a) le résidu d'acide aminé terminal marqué unique ou (b) un dipeptide terminal marqué, dans laquelle

   la dipeptidyl aminopeptidase comprend une séquence d'acides aminés ayant au moins 30 % d'identité de séquence par rapport à la séquence d'acides aminés de SEQ ID NO : 31 et comprend également un résidu d'asparagine à une position correspondant à la position 191 de SEQ ID NO : 31, un résidu de tryptophane ou de phénylalanine à une position correspondant à la position 192 de SEQ ID NO : 31, un résidu d'arginine à une position correspondant à la position 196 de SEQ ID NO : 31, un résidu d'asparagine à une position correspondant à la position 306 de SEQ ID NO : 31, un résidu d'aspartate à une position correspondant à la position 650 de SEQ ID NO : 31 ; et dans laquelle l'enzyme de clivage dipeptidique modifiée comprend trois substitutions d'acides aminés ou plus dans des résidus correspondant aux positions 191, 192, 196, 306, 650 de SEQ ID NO : 31.

2. Enzyme de clivage dipeptidique modifiée selon la revendication 1, dans laquelle l'enzyme de clivage dipeptidique modifiée n'élimine pas un dipeptide terminal non marqué du polypeptide.

3. Enzyme de clivage dipeptidique modifiée selon la revendication 1, laquelle comprend au moins quatre substitutions d'acides aminés dans des résidus correspondant aux positions 191, 192, 196, 306, 650 de SEQ ID NO : 31.

4. Enzyme de clivage dipeptidique modifiée selon la revendication 1, dans laquelle l'enzyme de clivage dipeptidique modifiée élimine ou est conçue pour éliminer un unique acide aminé marqué N-terminal du polypeptide.

5. Enzyme de clivage dipeptidique modifiée selon la revendication 1, comprenant en outre une ou plusieurs substitutions d'acides aminés dans des résidus correspondant aux positions 310, 628, 648, 651, 659, 669.

6. Enzyme de clivage dipeptidique modifiée selon la revendication 1, dans laquelle la dipeptidyl aminopeptidase est une protéine classée dans MEROPS S46, ou un homologue ou fragment fonctionnel de celle-ci.

7. Enzyme de clivage dipeptidique modifiée selon la revendication 1, dans laquelle l'acide aminé terminal unique ou le dipeptide terminal est marqué avec une modification N-terminale qui comprend un groupe bloquant N-terminal ($NTM_{blk}$) et, éventuellement, une partie d'acide aminé naturelle ou non naturelle (NTMaa), dans laquelle la NTMaa comprend un composé choisi dans le groupe constitué de : résidu d'acide aminé d'origine naturelle, 3-(3'-pyridyl)-L-alanine, L-cyclohexylglycine, acide $\alpha$-aminoisobutyrique, 3-(4'-pyridyl)-L-alanine, acide L-azétidine-2-carboxylique, acide isonipécotique, L-phénylglycine, $\beta$-(2-thiényl)-L-alanine, 3-(4-thiazolyl)-L-alanine, acide 1-aminocyclopentane-1-carboxylique, (2-trifluorométhyl)-L-phénylalanine, L-cyclopropylalanine, 3-(2'-pyridyl)-L-alanine, bêta-cyano-L-alanine, $\alpha$-méthyl-L-4-fluorophénylalanine, $\alpha$-méthyl-D-4-fluorophénylalanine, acide 3-amino-2,2-difluoro-propionique, sel de O-sulfo-L-tyrosine sodium, L-2-furylalanine, acide 1-aminocyclopropane-1-carboxylique, 3,5-dinitro-L-tyrosine, pentafluoro-L-phénylalanine, 3,5-difluoro-L-phénylalanine, 3-fluoro-L-phénylalanine, N-cyclopentylglycine, acide 1-(amino)cyclohexanecarboxylique, N-méthylalanine, acide 4-amino-tétrahydropyrane-4-carboxylique, acide 4-amino-1,1-dioxothiane-4-carboxylique, acide 4-amino-1-méthyl-4-pipéridinecarboxylique, acide 2-amino-N-(2,4-diméthoxybenzyl)acétamido)acétique, ou des dérivés N-alkylés ;
   et le $NTM_{blk}$ comprend un composé choisi dans le groupe constitué de : acide 4-méthylbenzoïque, acide 4-(diméthylamio)benzoïque, acide nicotinique, acide 3-aminonicotinique, acide 2-pyrazinecarbooxylique, acide 5-amino-2-fluoro-isonicotinique, acide 2,3-pyrazinedicarboxylique, acide 4,7-difluoroisobenzofurane-1,3-dicarboxylique, acide 4-chloro-2-aminobenzoïque, acide 4-nitro-2-aminobenzoïque, 7-méthoxy-1H-benzo[d][1,3]oxazine-2,4-dione, acide 4-carboxy-2-aminobenzoïque, 6-(trifluorométhyl)-2,4-dihydro-1H-3,1-benzoxazine-2,4-dione, 7-(trifluorométhyl)-1H-benzo[d][1 ,3]oxazine-2,4-dione, acide 6-fluoro-2-aminobenzoïque, acide 4-fluoro-2-aminobenzoïque, acide 5-méthoxy-2-aminobenzoïque, acide 4-fluorobenzoïque, acide 4-(trifluorométhyl)benzoïque, 2-éthynyl-6-fluorobenzaldéhyde, acide 2-aminobenzoïque, anhydride succinique, acide 3,6-difluoropyridine-2-carboxylique, acide 2-fluoronicotinique, acide 5-bromo-2-hydroxynicotinique, acide 4-(trifluorométhyl)pyrimidine-5-carboxylique, acide 2-oxo-1,2-dihydropyridine-3-carboxylique, acide 5-méthyl-2-aminobenzoïque, acide 6-fluoropicolinique, acide 3-méthyl-2-aminobenzoïque, acide 4-méthyl-2-aminobenzoïque, acide 2-amino-6-méthylbenzoïque, acide 2-amino-6-fluorobenzoïque, acide 2-amino-5-fluorobenzoïque, acide 2-amino-3-fluorobenzoïque, acide 2-amino-4-fluorobenzoïque, acide 2-aminonicotinique, acide 4-aminonicotinique, acide 3-aminopicolinique, acide 2-amino-4,5-difluorobenzoïque, acide 3,4-difluorobenzoïque, acide 3,4,5-difluorobenzoïque, acide 3-(méthoxycarbonyl)bicyclo[1.1.1]pentane-1-carboxylique, acide 3,3-difluorocyclobutane-1-carboxylique, acide 1-méthyl-2-oxo-pipéridine-4-carboxylique, acide tétrahydropyrane-4-carboxylique, acide 5-fluoroorotique, acide 3-fluoro-4-nitrobenzoïque, acide 3-(difluorométhyl)-1-méthyl-1H-pyrazole-4-carboxylique, acide 4-(difluorométhoxy)benzoïque, acide 1-(difluorométhyl)-1H-pyrazole-3-carboxylique, acide 4-(méthanesulfonylamino)benzoïque, acide 5-fluoro-6-méthoxynicotinique, acide tétrahydro-2H-thiopyrane-4-carboxylique 1,1-dioxyde, acide 4-(1H-tétrazol-5-yle)benzoïque, acide 1,2,3-thiadiazole-4-carboxylique, acide 1,3-benzodioxole-4-carboxylique, acide 2,1,3-benzoxadiazole-5-carboxylique, acide 1-benzyl-3-méthyl-1H-pyrazole-5-carboxylique, acide 1-cyclopropyl-6,7-difluoro-1,4-dihydro-4-oxoquinoline-3-carboxylique, acide 3,4-dichlorobenzoïque, acide 5-fluoro-6-méthylpyridine-2-carboxylique, acide 4,5-diméthyl-2-(1H-pyrrol-1-yl)thiophène-3-carboxylique, acide 1,3-diméthyl-1H-thiéno[2,3-c]pyrazole-5-carboxylique, acide 1-[(4-fluorobenzène)sulfonyl]pipéridine-3-carboxylique, acide 1-(4-fluorobenzyl)-5-oxopyrrolidine-3-carboxylique, acide 3-fluoro-4-méthoxybenzoïque, acide 4-fluoro-3-nitrobenzoïque, acide 6-fluoro-4-oxochromène-2-carboxylique, acide 3-fluorophénylacétique, acide 4-fluoro-3-(trifluorométhyl)benzoïque, acide 5-furan-2-yl-isoxazole-3-carboxylique, acide 1-isopropyl-2-(trifluorométhyl)-1H-benzimidazole-5-carboxylique, Lévofloxacine acide carboxylique, acide 3,5,7-trifluoroadamantane-1-carboxylique, acide 3,4,5-triméthoxybenzoïque, acide 2-oxo-2,3-dihydro-1H-benzo[d]imidazole-4-carboxylique, acide 1-méthyl-3-(trifluorométhyl)-1H-pyrazole-5-carboxylique, acide 2-morpholin-4-yl-isonicotinique, acide 1,3-oxazole-4-carboxylique, 4-carboxybenzènesulfonamide, chlorure de 3,4-difluorobenzènesulfonyle.

**8.** Procédé pour le traitement d'un polypeptide, comprenant les étapes suivantes :

marquage d'un acide aminé terminal du polypeptide avec un réactif chimique pour produire un polypeptide marqué ; et
mise en contact du polypeptide marqué avec une enzyme de clivage dipeptidique modifiée, dans lequel l'enzyme de clivage dipeptidique modifiée comprend trois substitutions d'acides aminés ou plus dans un site de liaison au substrat d'une dipeptidyl aminopeptidase, dans lequel :

(i) la dipeptidyl aminopeptidase élimine ou est conçue pour éliminer deux acides aminés terminaux du polypeptide lors de la mise en contact ; et
(ii) l'enzyme de clivage dipeptidique modifiée élimine ou est conçue pour éliminer du polypeptide ayant un acide aminé terminal marqué avec un réactif chimique (a) l'acide aminé terminal marqué unique ou (b) un dipeptide terminal marqué, dans lequel

la dipeptidyl aminopeptidase comprend une séquence d'acides aminés ayant au moins 30 % d'identité de séquence par rapport à la séquence d'acides aminés de SEQ ID NO : 31 et comprend également un résidu d'asparagine à une position correspondant à la position 191 de SEQ ID NO : 31, un résidu de tryptophane ou de phénylalanine à une position correspondant à la position 192 de SEQ ID NO : 31, un résidu d'arginine à une position correspondant à la position 196 de SEQ ID NO : 31, un résidu d'asparagine à une position correspondant à la position 306 de SEQ ID NO : 31, un résidu d'aspartate à une position correspondant à la position 650 de SEQ ID NO : 31 ; et dans lequel l'enzyme de clivage dipeptidique modifiée comprend trois substitutions d'acides aminés ou plus dans des résidus correspondant aux positions 191, 192, 196, 306, 650 de SEQ ID NO : 31.

**9.** Procédé selon la revendication 8, dans lequel l'enzyme de clivage dipeptidique modifiée n'élimine pas un dipeptide terminal non marqué du polypeptide.

**10.** Procédé selon la revendication 8, dans lequel l'enzyme de clivage dipeptidique modifiée comprend au moins quatre substitutions d'acides aminés ou plus dans des résidus correspondant aux positions 191, 192, 196, 306, 650 de SEQ ID NO : 31.

**11.** Procédé selon la revendication 8, dans lequel l'acide aminé terminal unique ou le dipeptide terminal est marqué avec une modification N-terminale qui comprend un groupe de blocage N-terminal ($NTM_{bik}$) et, éventuellement, une partie d'acide aminé naturelle ou non naturelle (NTMaa), dans lequel la NTMaa comprend un composé choisi dans le groupe

constitué de : résidu d'acide aminé d'origine naturelle, 3-(3'-pyridyl)-L-alanine, L-cyclohexylglycine, acide α-aminoisobutyrique, 3-(4'-pyridyl)-L-alanine, acide L-azétidine-2-carboxylique, acide isonipécotique, L-phénylglycine, β-(2-thiényl)-L-alanine, 3-(4-thiazolyl)-L-alanine, acide 1-aminocyclopentane-1-carboxylique, (2-trifluorométhyl)-L-phénylalanine, L-cyclopropylalanine, 3-(2'-pyridyl)-L-alanine, bêta-cyano-L-alanine, α-méthyl-L-4-fluorophénylalanine, α-méthyl-D-4-fluorophénylalanine, acide 3-amino-2,2-difluoro-propionique, sel de O-sulfo-L-tyrosine sodium, L-2-furylalanine, acide 1-aminocyclopropane-1-carboxylique, 3,5-dinitro-L-tyrosine, pentafluoro-L-phénylalanine, 3,5-difluoro-L-phénylalanine, 3-fluoro-L-phénylalanine, N-cyclopentylglycine, acide 1-(amino)cyclohexanecarboxylique, N-méthylalanine, acide 4-amino-tétrahydropyrane-4-carboxylique, acide 4-amino-1,1-dioxothiane-4-carboxylique, acide 4-amino-1-méthyl-4-pipéridinecarboxylique, acide 2-amino-N-(2,4-diméthoxybenzyl)acétamido)acétique, ou des dérivés N-alkylés ;
et le $NTM_{blk}$ comprend un composé choisi dans le groupe constitué de :
acide 4-méthylbenzoïque, acide 4-(diméthylamio)benzoïque, acide nicotinique, acide 3-aminonicotinique, acide 2-pyrazinecarbooxylique, acide 5-amino-2-fluoro-isonicotinique, acide 2,3-pyrazinedicarboxylique, acide 4,7-difluoroisobenzofurane-1,3-dicarboxylique, acide 4-chloro-2-aminobenzoïque, acide 4-nitro-2-aminobenzoïque, 7-méthoxy-1H-benzo[d][1,3]oxazine-2,4-dione, acide 4-carboxy-2-aminobenzoïque, 6-(trifluorométhyl)-2,4-dihydro-1H-3,1-benzoxazine-2,4-dione, 7-(trifluorométhyl)-1H-benzo[d][1,3]oxazine-2,4-dione, acide 6-fluoro-2-aminobenzoïque, acide 4-fluoro-2-aminobenzoïque, acide 5-méthoxy-2-aminobenzoïque, acide 4-fluorobenzoïque, acide 4-(trifluorométhyl)benzoïque, 2-éthynyl-6-fluorobenzaldéhyde, acide 2-aminobenzoïque, anhydride succinique, acide 3,6-difluoropyridine-2-carboxylique, acide 2-fluoronicotinique, acide 5-bromo-2-hydroxynicotinique, acide 4-(trifluorométhyl)pyrimidine-5-carboxylique, acide 2-oxo-1,2-dihydropyridine-3-carboxylique, acide 5-méthyl-2-aminobenzoïque, acide 6-fluoropicolinique, acide 3-méthyl-2-aminobenzoïque, acide 4-méthyl-2-aminobenzoïque, acide 2-amino-6-méthylbenzoïque, acide 2-amino-6-fluorobenzoïque, acide 2-amino-5-fluorobenzoïque, acide 2-amino-3-fluorobenzoïque, acide 2-amino-4-fluorobenzoïque, acide 2-aminonicotinique, acide 4-aminonicotinique, acide 3-aminopicolinique, acide 2-amino-4,5-difluorobenzoïque, acide 3,4-difluorobenzoïque, acide 3,4,5-difluorobenzoïque, acide 3-(méthoxycarbonyl)bicyclo[1.1.1]pentane-1-carboxylique, acide 3,3-difluorocyclobutane-1-carboxylique, acide 1-méthyl-2-oxo-pipéridine-4-carboxylique, acide tétrahydropyrane-4-carboxylique, acide 5-fluoroorotique, acide 3-fluoro-4-nitrobenzoïque, acide 3-(difluorométhyl)-1-méthyl-1H-pyrazole-4-carboxylique, acide 4-(difluorométhoxy)benzoïque, acide 1-(difluorométhyl)-1H-pyrazole-3-carboxylique, acide 4-(méthanesulfonylamino) benzoïque, acide 5-fluoro-6-méthoxynicotinique, acide tétrahydro-2H-thiopyrane-4-carboxylique 1,1-dioxyde, acide 4-(1H-tétrazol-5-yle)benzoïque, acide 1,2,3-thiadiazole-4-carboxylique, acide 1,3-benzodioxole-4-carboxylique, acide 2,1,3-benzoxadiazole-5-carboxylique, acide 1-benzyl-3-méthyl-1H-pyrazole-5-carboxylique, acide 1-cyclopropyl-6,7-difluoro-1,4-dihydro-4-oxoquinoline-3-carboxylique, acide 3,4-dichlorobenzoïque, acide 5-fluoro-6-méthylpyridine-2-carboxylique, acide 4,5-diméthyl-2-(1H-pyrrol-1-yl)thiophène-3-carboxylique, acide 1,3-diméthyl-1H-thiéno[2,3-c]pyrazole-5-carboxylique, acide 1-[(4-fluorobenzène)sulfonyl]pipéridine-3-carboxylique, acide 1-(4-fluorobenzyl)-5-oxopyrrolidine-3-carboxylique, acide 3-fluoro-4-méthoxybenzoïque, acide 4-fluoro-3-nitrobenzoïque, acide 6-fluoro-4-oxochromène-2-carboxylique, acide 3-fluorophénylacétique, acide 4-fluoro-3-(trifluorométhyl)benzoïque, acide 5-furan-2-yl-isoxazole-3-carboxylique, acide 1-isopropyl-2-(trifluorométhyl)-1H-benzimidazole-5-carboxylique, Lévofloxacine acide carboxylique, acide 3,5,7-trifluoroadamantane-1-carboxylique, acide 3,4,5-triméthoxybenzoïque, acide 2-oxo-2,3-dihydro-1H-benzo[d]imidazole-4-carboxylique, acide 1-méthyl-3-(trifluorométhyl)-1H-pyrazole-5-carboxylique, acide 2-morpholin-4-yl-isonicotinique, acide 1,3-oxazole-4-carboxylique, 4-carboxybenzènesulfonamide, chlorure de 3,4-difluorobenzènesulfonyle.

**12.** Procédé selon la revendication 8, comprenant en outre une étape de mise en contact du polypeptide avec un agent de liaison conçu pour se lier à l'acide aminé terminal marqué unique ou au dipeptide terminal marqué, dans lequel l'étape de marquage de l'acide aminé terminal du polypeptide précède l'étape de mise en contact du polypeptide avec l'agent de liaison ; et l'étape de mise en contact du polypeptide avec l'agent de liaison est antérieure à l'étape de mise en contact du polypeptide avec l'enzyme de clivage dipeptidique modifiée.

**13.** Ensemble d'enzymes de clivage dipeptidiques, comprenant au moins deux enzymes de clivages dipeptidiques modifiées différentes, dans lequel :

(i) chacune des enzymes de clivage dipeptidiques modifiées de l'ensemble d'enzymes de clivage dipeptidiques comprend trois substitutions d'acides aminés ou plus dans un site de liaison au substrat d'une dipeptidyl aminopeptidase, dans lequel la dipeptidyl aminopeptidase est conçue pour éliminer deux acides aminés terminaux d'un polypeptide non marqué ;
(ii) chacune des enzymes de clivages dipeptidiques modifiées de l'ensemble d'enzymes de clivage dipeptidiques est conçue pour éliminer un unique acide aminé terminal marqué d'un polypeptide ayant l'acide aminé terminal marqué avec un réactif chimique, dans lequel

la dipeptidyl aminopeptidase comprend une séquence d'acides aminés ayant au moins 30 % d'identité de séquence par rapport à la séquence d'acides aminés de SEQ ID NO : 31 et comprend également un résidu d'asparagine à une position correspondant à la position 191 de SEQ ID NO : 31, un résidu de tryptophane ou de phénylalanine à une position correspondant à la position 192 de SEQ ID NO : 31, un résidu d'arginine à une position correspondant à la position 196 de SEQ ID NO : 31, un résidu d'asparagine à une position correspondant à la position 306 de SEQ ID NO : 31, un résidu d'aspartate à une position correspondant à la position 650 de SEQ ID NO : 31 ; et dans lequel l'enzyme de clivage dipeptidique modifiée comprend trois substitutions d'acides aminés ou plus dans des résidus correspondant aux positions 191, 192, 196, 306, 650 de SEQ ID NO : 31 ; et
(iii) les enzymes de clivage dipeptidiques modifiées de l'ensemble d'enzymes de clivage dipeptidiques ont des spécificités différentes pour les acides aminés terminaux marqués, que les enzymes de clivage dipeptidiques modifiées sont conçues pour éliminer.

**14.** Kit pour le traitement d'un polypeptide, comprenant :

(a) un réactif chimique pour le marquage d'un acide aminé terminal du polypeptide ; et
(b) un ensemble d'enzymes de clivage dipeptidiques, comprenant au moins deux enzymes de clivage dipeptidiques modifiées différentes, dans lequel :

(i) chacune des enzymes de clivages dipeptidiques modifiées de l'ensemble d'enzymes de clivage dipeptidiques comprend trois substitutions d'acides aminés ou plus dans un site de liaison au substrat d'une dipeptidyl aminopeptidase, dans lequel la dipeptidyl aminopeptidase est conçue pour éliminer deux acides aminés terminaux d'un polypeptide non marqué ;
(ii) chacune des enzymes de clivage dipeptidiques modifiées de l'ensemble d'enzymes de clivage dipeptidiques est conçue pour éliminer un unique acide aminé terminal marqué d'un polypeptide ayant l'acide aminé terminal marqué avec un réactif chimique, dans lequel
la dipeptidyl aminopeptidase comprend une séquence d'acides aminés ayant au moins 30 % d'identité de séquence par rapport à la séquence d'acides aminés de SEQ ID NO : 31 et comprend également un résidu d'asparagine à une position correspondant à la position 191 de SEQ ID NO : 31, un résidu de tryptophane ou de phénylalanine à une position correspondant à la position 192 de SEQ ID NO : 31, un résidu d'arginine à une position correspondant à la position 196 de SEQ ID NO : 31, un résidu d'asparagine à une position correspondant à la position 306 de SEQ ID NO : 31, un résidu d'aspartate à une position correspondant à la position 650 de SEQ ID NO : 31 ; et dans lequel l'enzyme de clivage dipeptidique modifiée comprend trois substitutions d'acides aminés ou plus dans des résidus correspondant aux positions 191, 192, 196, 306, 650 de SEQ ID NO : 31 ; et
(iii) les enzymes de clivage dipeptidiques modifiées de l'ensemble d'enzymes de clivage dipeptidiques ont des spécificités différentes pour les acides aminés terminaux marqués, que les enzymes de clivage dipeptidiques modifiées sont conçues pour éliminer.

**15.** Kit selon la revendication 14, dans lequel (i) le réactif chimique est conçu pour attacher une modification N-terminale à l'acide aminé terminal du polypeptide ; (ii) la modification N-terminale comprend un groupe bloquant N-terminal ($NTM_{blk}$) et, éventuellement, une partie d'acide aminé naturelle ou non naturelle (NTMaa) ; (iii) la NTMaa comprend un composé choisi dans le groupe constitué de : résidu d'acide aminé d'origine naturelle, 3-(3'-pyridyl)-L-alanine, L-cyclohexylglycine, acide α-aminoisobutyrique, 3-(4'-pyridyl)-L-alanine, acide L-azétidine-2-carboxylique, acide isonipécotique, L-phénylglycine, β-(2-thiényl)-L-alanine, 3-(4-thiazolyl)-L-alanine, acide 1-aminocyclopentane-1-carboxylique, (2-trifluorométhyl)-L-phénylalanine, L-cyclopropylalanine, 3-(2'-pyridyl)-L-alanine, bêta-cyano-L-alanine, α-méthyl-L-4-fluorophénylalanine, α-méthyl-D-4-fluorophénylalanine, acide 3-amino-2,2-difluoro-propionique, sel de O-sulfo-L-tyrosine sodium, L-2-furylalanine, acide 1-aminocyclopropane-1-carboxylique, 3,5-dinitro-L-tyrosine, pentafluoro-L-phénylalanine, 3,5-difluoro-L-phénylalanine, 3-fluoro-L-phénylalanine, N-cyclopentylglycine, acide 1-(amino)cyclohexanecarboxylique, N-méthylalanine, acide 4-amino-tétrahydropyrane-4-carboxylique, acide 4-amino-1,1-dioxothiane-4-carboxylique, acide 4-amino-1-méthyl-4-pipéridinecarboxylique, acide 2-amino-N-(2,4-diméthoxybenzyl)acétamido)acétique, ou des dérivés N-alkylés ;
et (iv) le $NTM_{blk}$ comprend un composé choisi dans le groupe constitué de : acide 4-méthylbenzoïque, acide 4-(diméthylamio)benzoïque, acide nicotinique, acide 3-aminonicotinique, acide 2-pyrazinecarbooxylique, acide 5-amino-2-fluoro-isonicotinique, acide 2,3-pyrazinedicarboxylique, acide 4,7-difluoroisobenzofurane-1,3-dicarboxylique, acide 4-chloro-2-aminobenzoïque, acide 4-nitro-2-aminobenzoïque, 7-méthoxy-1H-benzo[d][1,3]oxazine-2,4-dione, acide 4-carboxy-2-aminobenzoïque, 6-(trifluorométhyl)-2,4-dihydro-1H-3,1-benzoxazine-2,4-dione, 7-(trifluorométhyl)-1H-benzo[d][1 ,3]oxazine-2,4-dione, acide 6-fluoro-2-aminobenzoïque, acide 4-fluoro-2-aminobenzoïque, acide 5-méthoxy-2-aminobenzoïque, acide 4-fluorobenzoïque, acide 4-(trifluorométhyl)benzoïque, 2-éthynyl-6-fluorobenzaldéhyde, acide 2-aminobenzoïque, anhydride succinique, acide 3,6-difluoropyridine-2-carboxylique, acide 2-fluoronicotinique, acide 5-bromo-2-hydroxynicotinique, acide 4-(trifluorométhyl)pyrimidine-5-carboxylique, acide 2-oxo-1,2-dihydropyridine-3-carboxylique, acide 5-méthyl-2-aminobenzoïque, acide 6-fluoropicolinique, acide 3-méthyl-2-aminobenzoïque, acide 4-méthyl-2-aminobenzoïque, acide 2-amino-6-méthylbenzoïque, acide 2-amino-6-fluorobenzoïque, acide 2-amino-5-fluorobenzoïque, acide 2-amino-3-fluorobenzoïque, acide 2-amino-4-fluorobenzoïque, acide 2-aminonicotinique, acide 4-aminonicotinique, acide 3-aminopicolinique, acide 2-amino-4,5-difluorobenzoïque, acide 3,4-difluorobenzoïque, acide 3,4,5-difluorobenzoïque, acide 3-(méthoxycarbonyl)bicyclo[1.1.1]pentane-1-carboxylique, acide 3,3-difluorocyclobutane-1-carboxylique, acide 1-méthyl-2-oxo-pipéridine-4-carboxylique, acide tétrahydropyrane-4-carboxylique, acide 5-fluoroorotique, acide 3-fluoro-4-nitrobenzoïque, acide 3-(difluorométhyl)-1-méthyl-1H-pyrazole-4-carboxylique, acide 4-(difluorométhoxy)benzoïque, acide 1-(difluorométhyl)-1H-pyrazole-3-carboxylique, acide 4-(méthanesulfonylamino)benzoïque, acide 5-fluoro-6-méthoxynicotinique, acide tétrahydro-2H-thiopyrane-4-carboxylique 1,1-dioxyde, acide 4-(1H-tétrazol-5-yle)benzoïque, acide 1,2,3-thiadiazole-4-carboxylique, acide 1,3-benzodioxole-4-carboxylique, acide 2,1,3-benzoxadiazole-5-carboxylique, acide 1-benzyl-3-méthyl-1H-pyrazole-5-carboxylique, acide 1-cyclopropyl-6,7-difluoro-1,4-dihydro-4-oxoquinoline-3-carboxylique, acide 3,4-dichlorobenzoïque, acide 5-fluoro-6-méthylpyridine-2-carboxylique, acide 4,5-diméthyl-2-(1H-pyrrol-1-yl)thiophène-3-carboxylique, acide 1,3-diméthyl-1H-thiéno[2,3-c]pyrazole-5-carboxylique, acide 1-[(4-fluorobenzène)sulfonyl]pipéridine-3-carboxylique, acide 1-(4-fluorobenzyl)-5-oxopyrrolidine-3-carboxylique, acide 3-fluoro-4-méthoxybenzoïque, acide 4-fluoro-3-nitrobenzoïque, acide 6-fluoro-4-oxochromène-2-carboxylique, acide 3-fluorophénylacétique, acide 4-fluoro-3-(trifluorométhyl)benzoïque, acide 5-furan-2-yl-isoxazole-3-carboxylique, acide 1-isopropyl-2-(trifluorométhyl)-1H-benzimidazole-5-carboxylique, Lévofloxacine acide carboxylique, acide 3,5,7-trifluoroadamantane-1-carboxylique, acide 3,4,5-triméthoxybenzoïque, acide 2-oxo-2,3-dihydro-1H-benzo[d]imidazole-4-carboxylique, acide 1-méthyl-3-(trifluorométhyl)-1H-pyrazole-5-carboxylique, acide 2-morpholin-4-yl-isonicotinique, acide 1,3-oxazole-4-carboxylique, 4-carboxybenzènesulfonamide, chlorure de 3,4-difluorobenzènesulfonyle.

FIG. 1

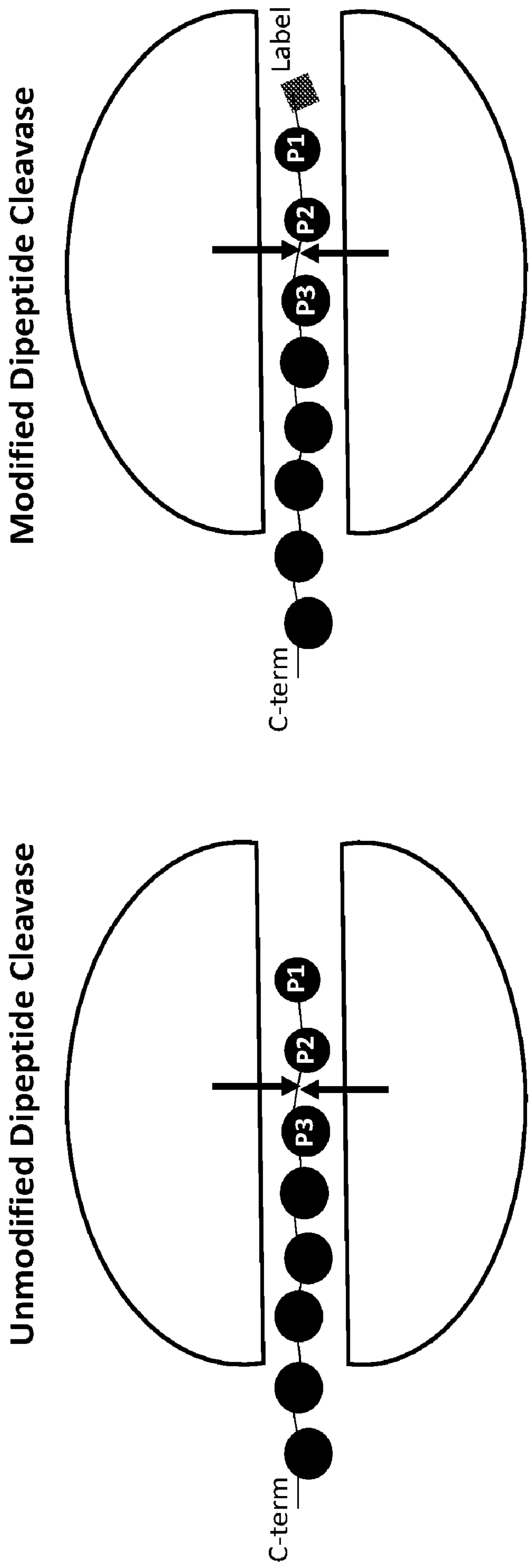
Modified Dipeptide Cleavase
Label
P1
P2
P3
C-term
Unmodified Dipeptide Cleavase
P1
P2
P3
C-term

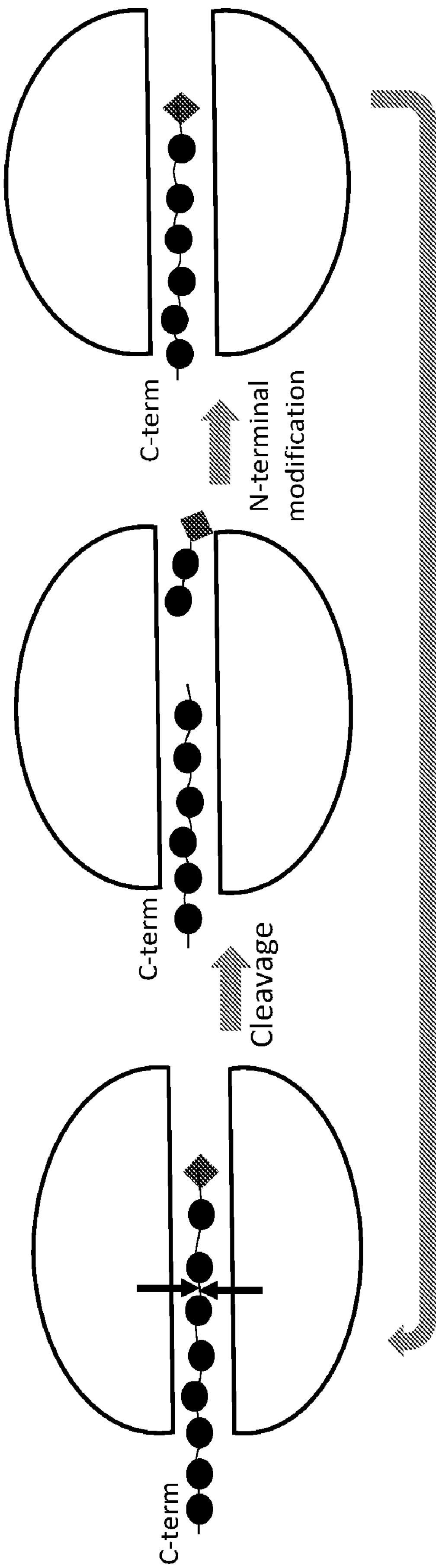


FIG. 2A

FIG. 2B

FIG. 2C

FIG. 3

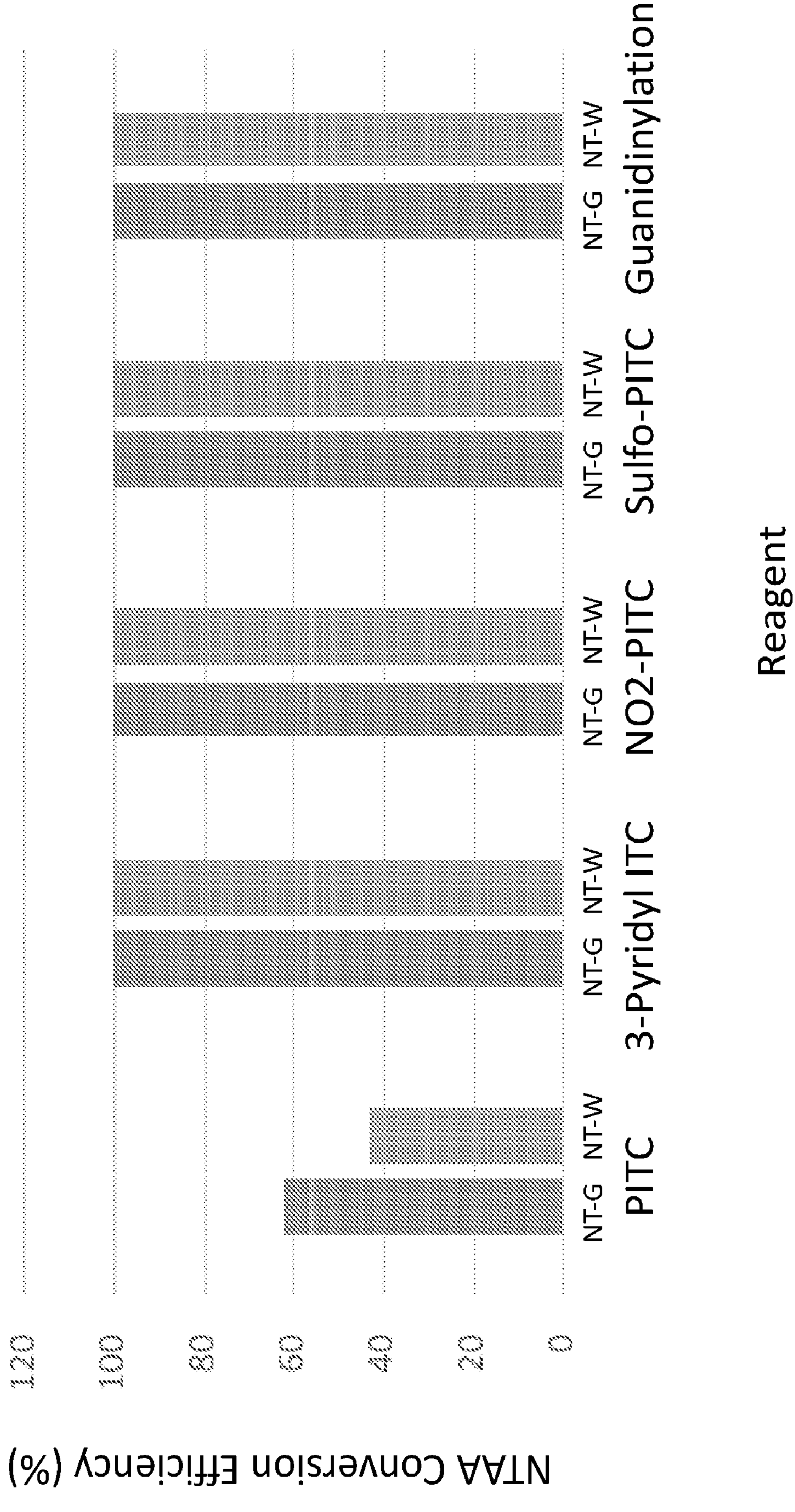
NTAA Conversion Efficiency (%)
0
20
40
60
80
100
120
NT-G
NT-W
PITC
3-Pyridyl ITC
NO2-PITC
Sulfo-PITC
Guanidinylation
Reagent

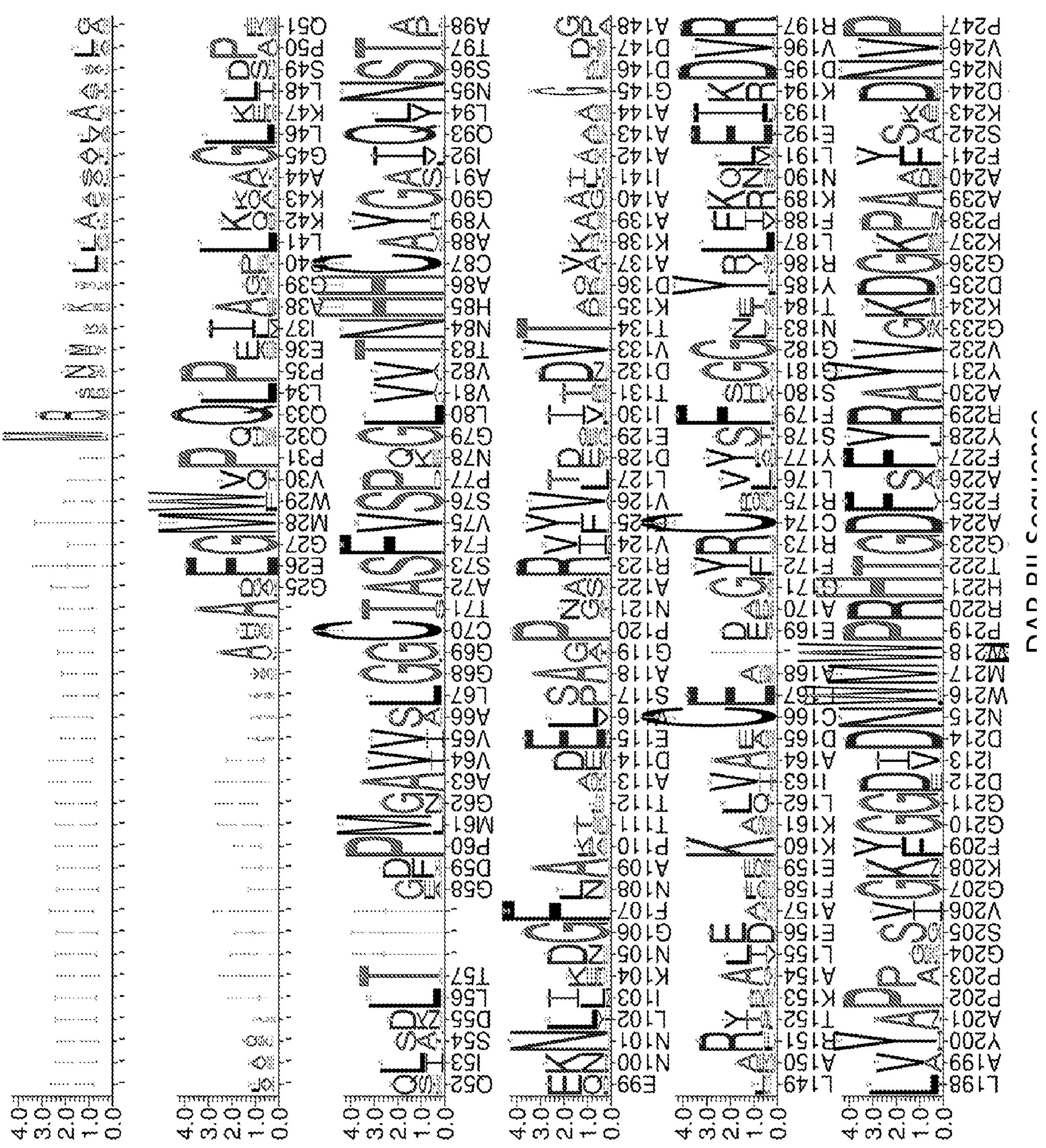

bits
4.0 3.0 2.0 1.0 0.0
Q52 I53 S54 D55 L56 T57 G25 E26 G27 M28 W29 V30 P31 Q32 Q33 L34 P35 E36 I37 A38 G39 P40 L41 K42 K43 A44 G45 L46 K47 L48 S49 P50 Q51
G58 D59 P60 M61 G62 A63 V64 V65 A66 L67 G68 G69 C70 T71 A72 S73 F74 V75 S76 P77 N78 G79 L80 V81 V82 T83 N84 H85 A86 C87 A88 Y89 G90 A91 I92 Q93 L94 N95 S96 T97 A98
E99 N100 N101 L102 I103 K104 N105 G106 F107 N108 A109 P110 T111 T112 A113 D114 E115 V116 S117 A118 G119 P120 N121 A122 R123 V124 A125 V126 L127 D128 E129 I130 T131 D132 V133 T134 K135 D136 A137 K138 A139 A140 I141 A142 A143 A144 G145 D146 D147 A148
L149 A150 R151 T152 K153 A154 L155 E156 A157 F158 E159 K160 K161 L162 I163 A164 D165 C166 A167 A168 E169 A170 G171 F172 R173 C174 R175 L176 Y177 S178 F179 S180 G181 G182 N183 T184 Y185 R186 L187 F188 K189 N190 L191 E192 I193 K194 D195 V196 R197
L198 A199 Y200 A201 P202 P203 G204 S205 V206 G207 K208 F209 G210 G211 D212 I213 D214 N215 W216 M217 W218 P219 R220 H221 T222 G223 A224 F225 A226 F227 Y228 R229 A230 Y231 V232 G233 K234 D235 G236 K237 P238 A239 A240 F241 S242 K243 D244 N245 V246 P247


FIG. 4A

FIG. 4B

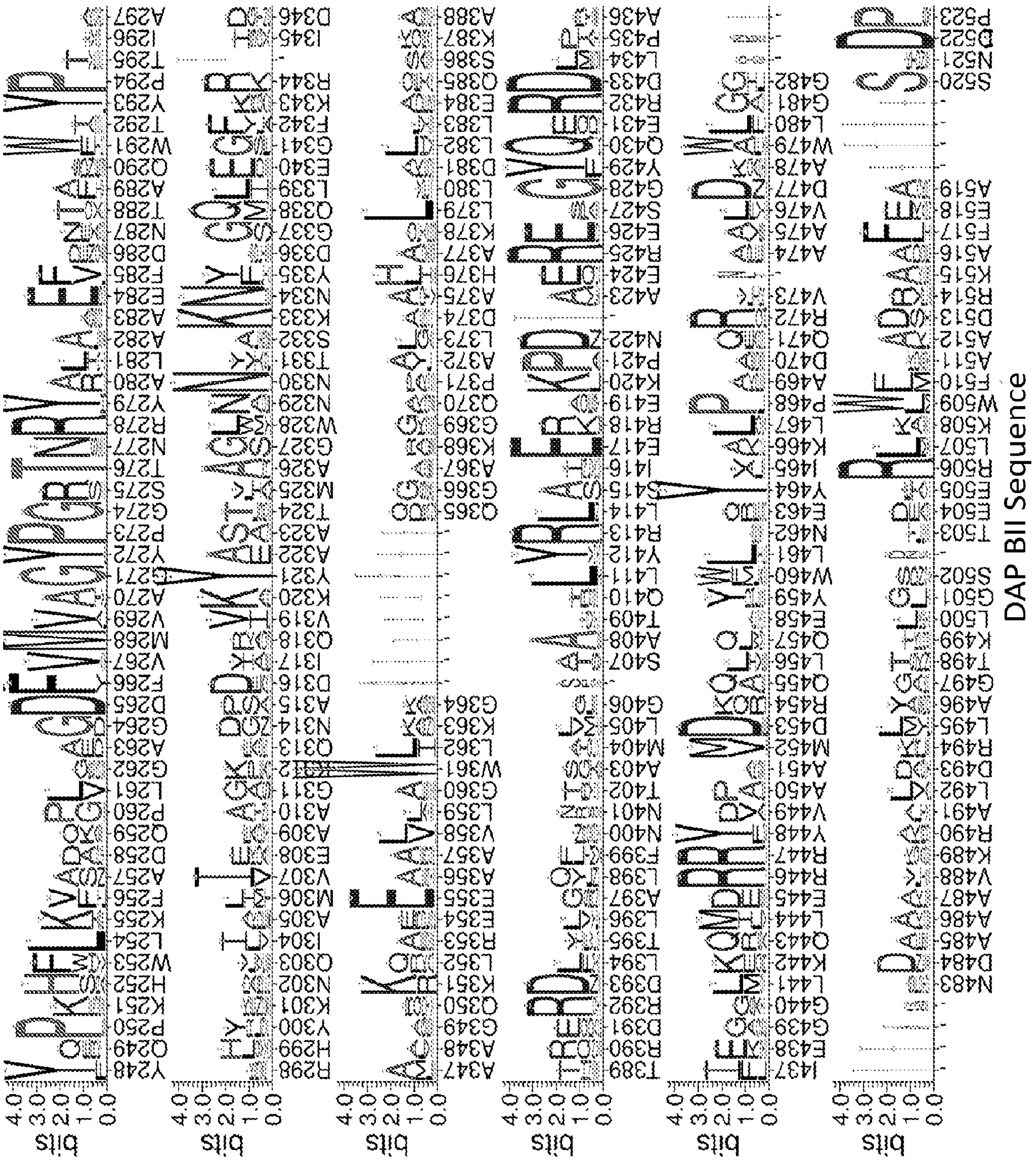

FIG. 4C

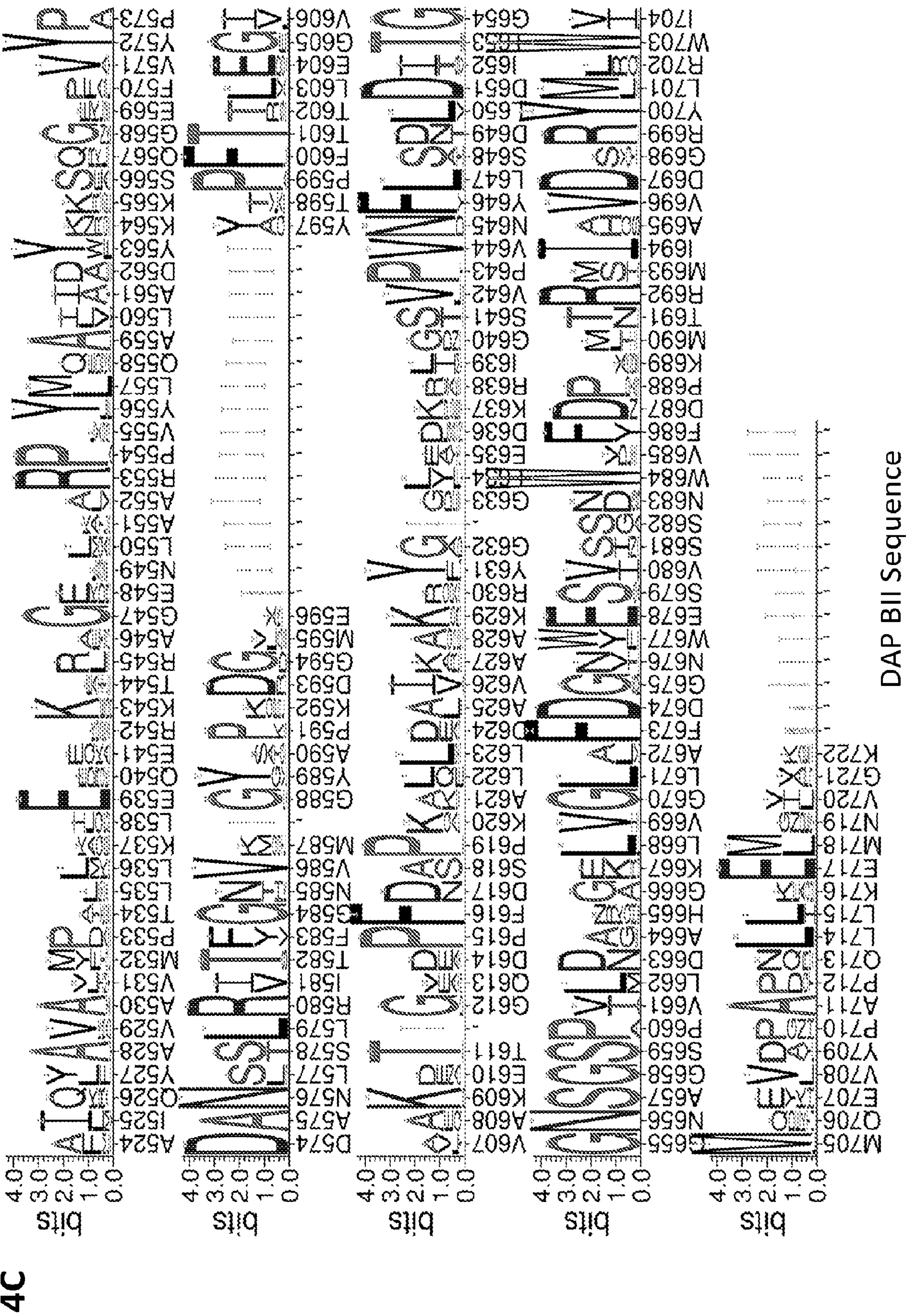

FIG. 5

Michaelis-Menten Kinetics

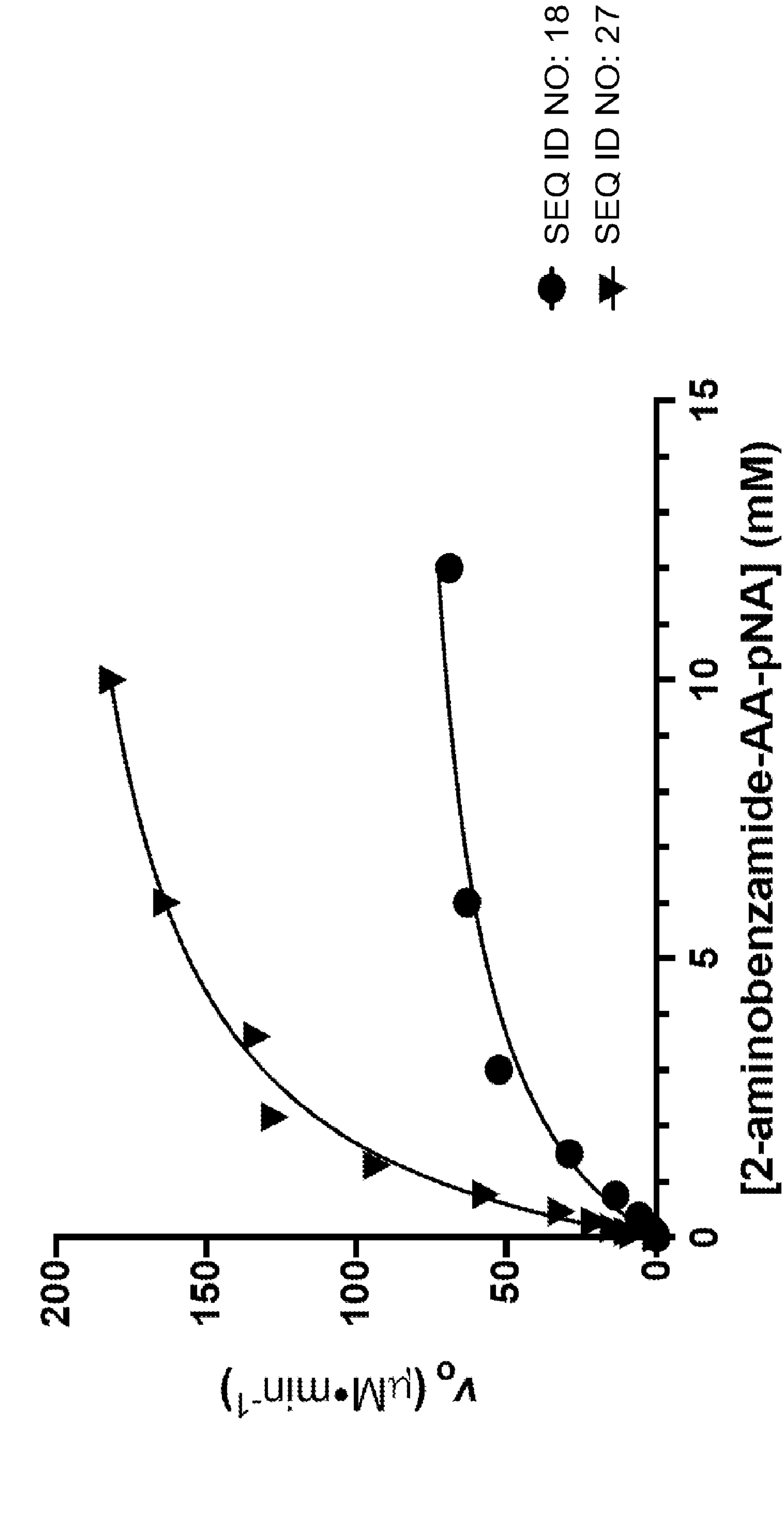

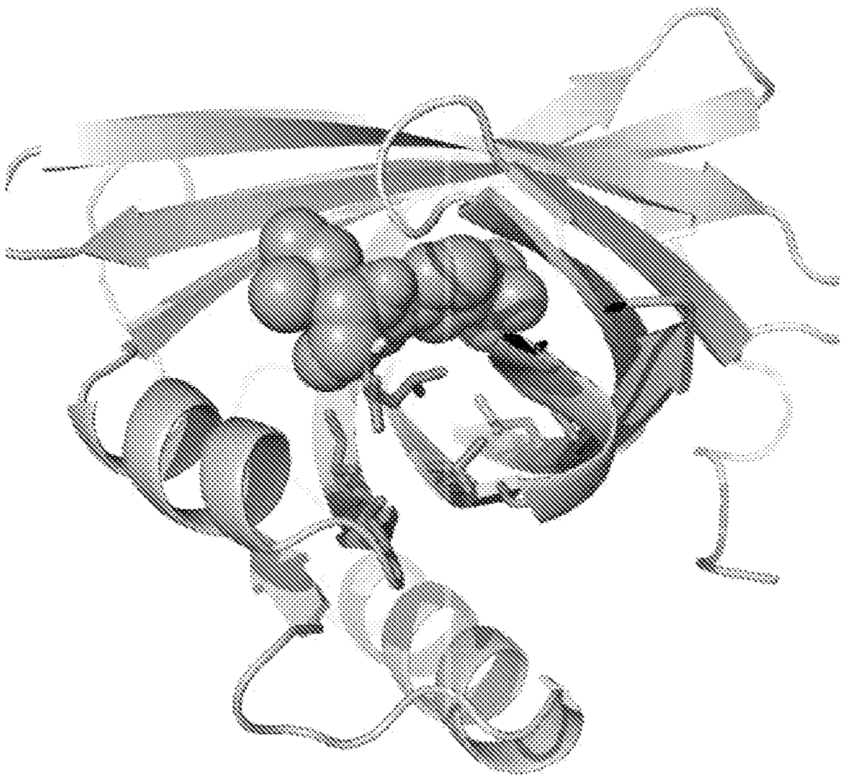

FIG. 6

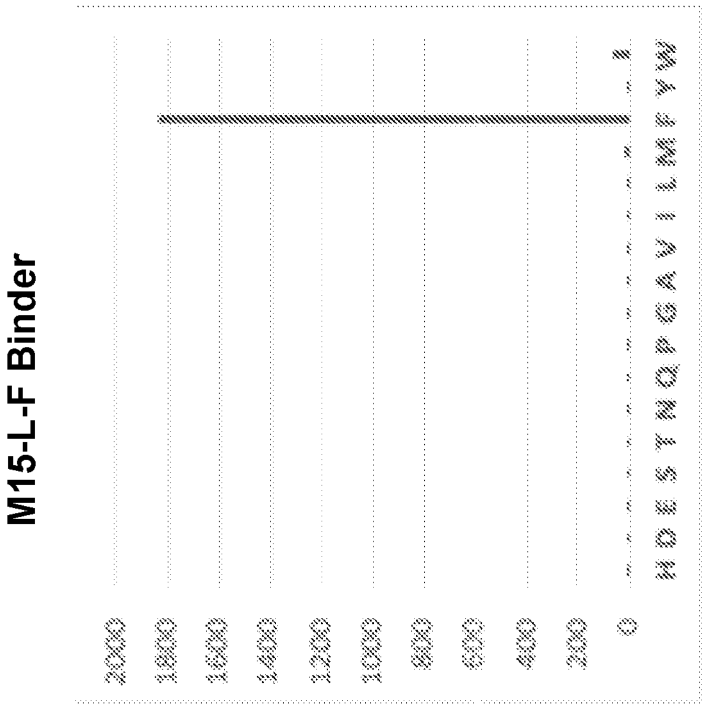
M15-L-F Binder
2000
1800
1600
1400
1200
1000
800
600
400
200
0
H D E S T N Q P G A V I L M F Y W

FIG. 7A

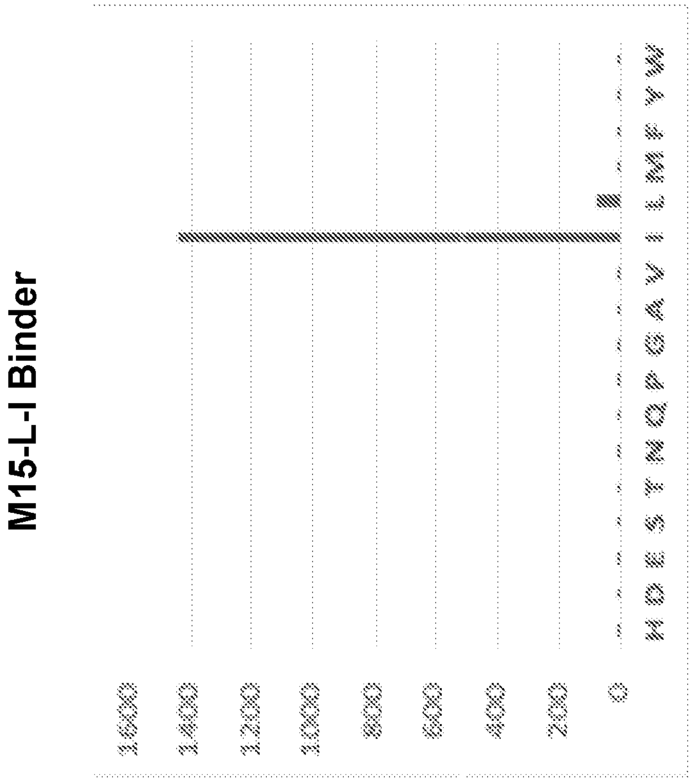
M15-L-I Binder
1600
1400
1200
1000
800
600
400
200
0
H D E S T N Q P G A V I L M F Y W

FIG. 7B

M15-L-I/L Binder

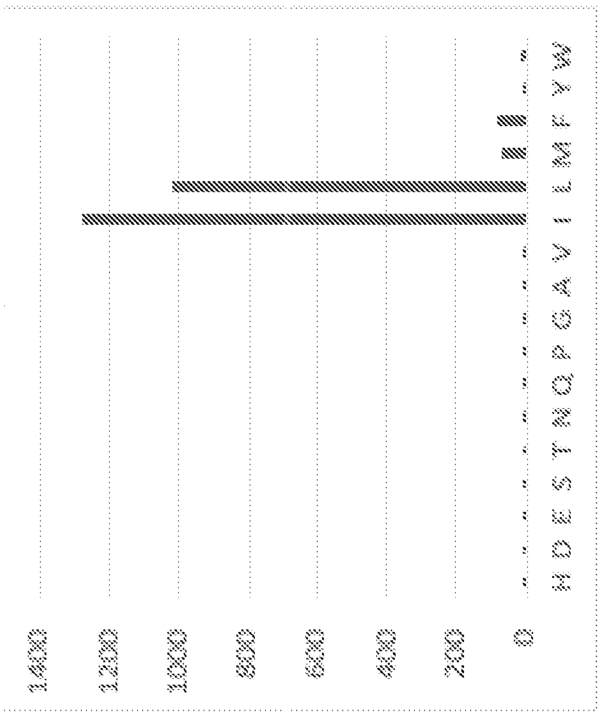


FIG. 7C

M15-L-W Binder

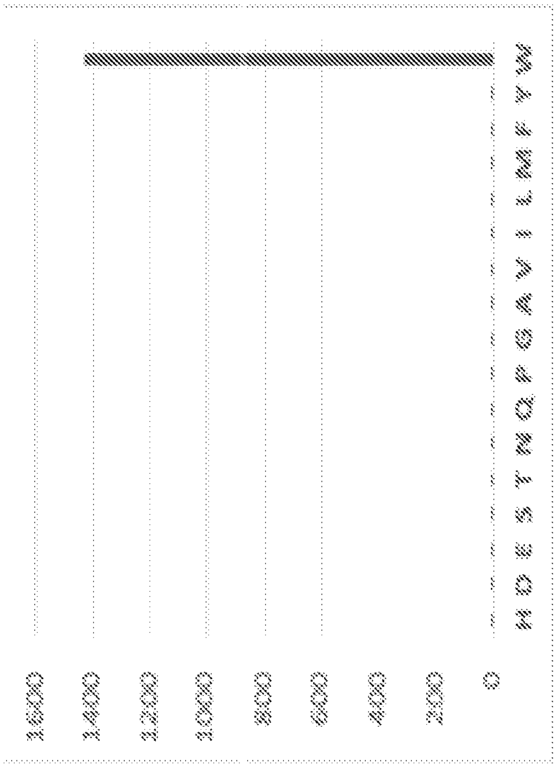


FIG. 7D

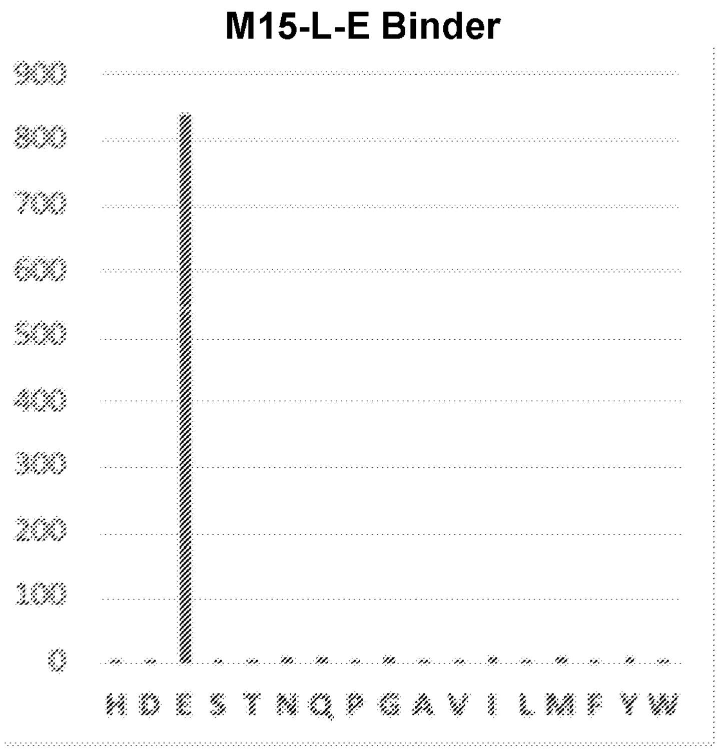
M15-L-E Binder
900
800
700
600
500
400
300
200
100
0
H D E S T N Q P G A V I L M F Y W

FIG. 7E

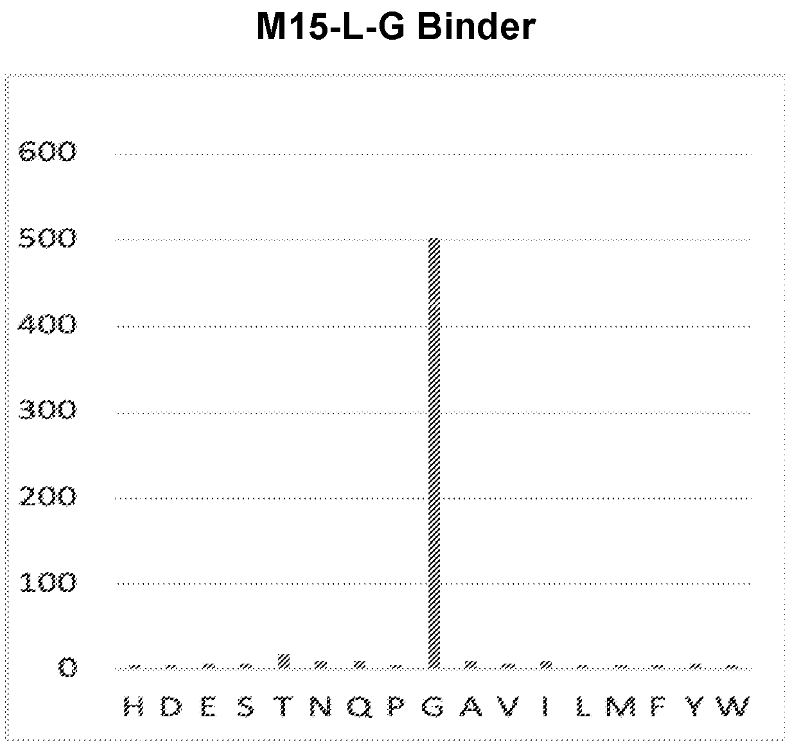
M15-L-G Binder
600
500
400
300
200
100
0
H D E S T N Q P G A V I L M F Y W

FIG. 7F

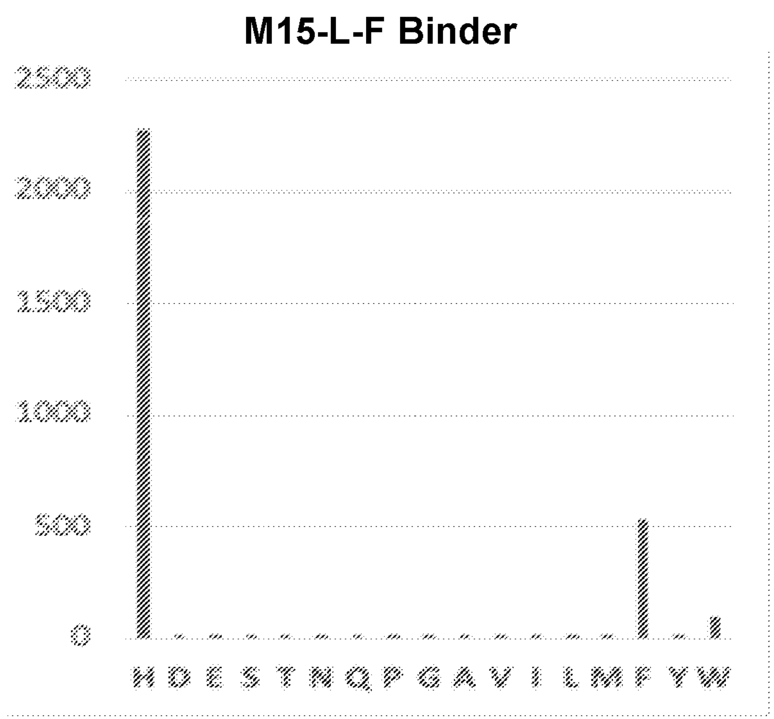
M15-L-F Binder
2500
2000
1500
1000
500
0
H D E S T N Q P G A V I L M F Y W

FIG. 7G

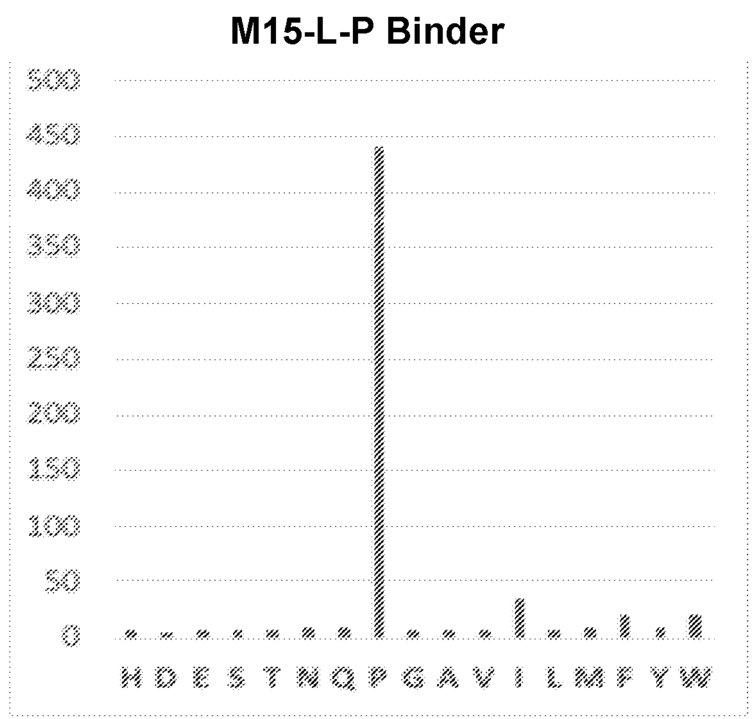
M15-L-P Binder
500
450
400
350
300
250
200
150
100
50
0
H D E S T N Q P G A V I L M F Y W

FIG. 7H

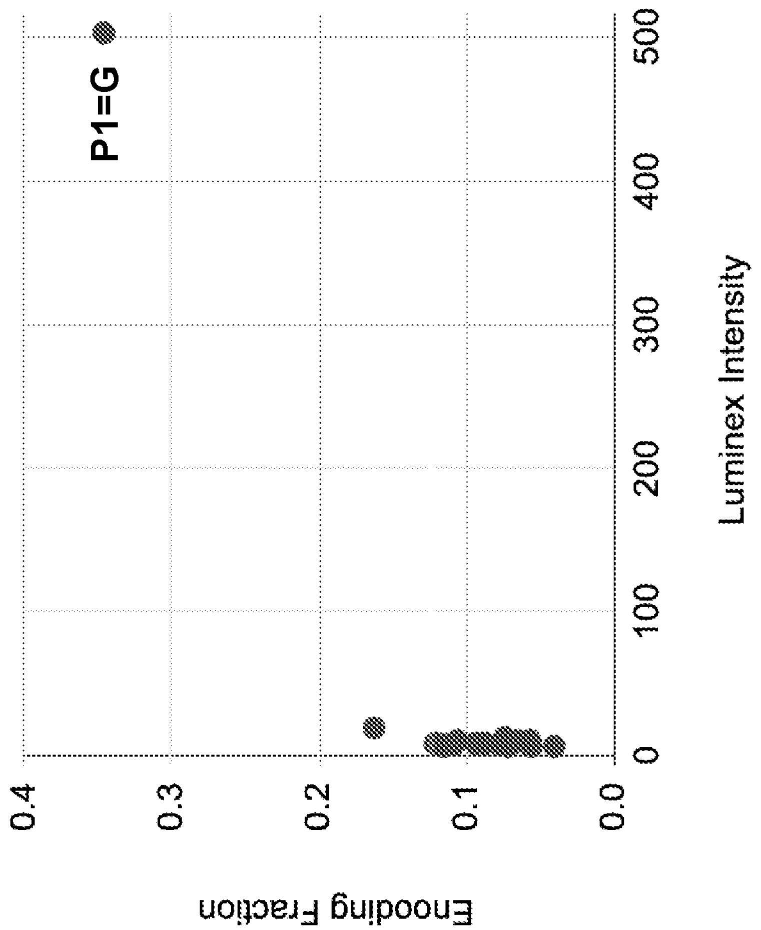

P1=G
Encoding Fraction
0.0
0.1
0.2
0.3
0.4
Luminex Intensity
0
100
200
300
400
500


FIG. 8A

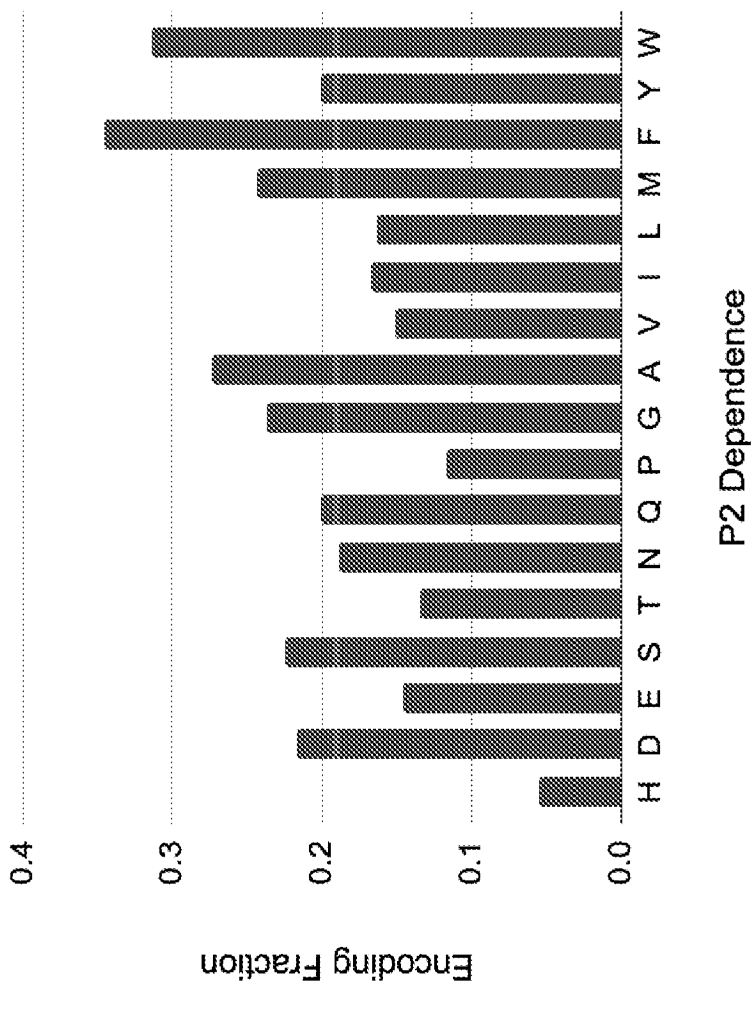
Encoding Fraction
0.0
0.1
0.2
0.3
0.4
H D E S T N Q P G A V I L M F Y W
P2 Dependence

FIG. 8B

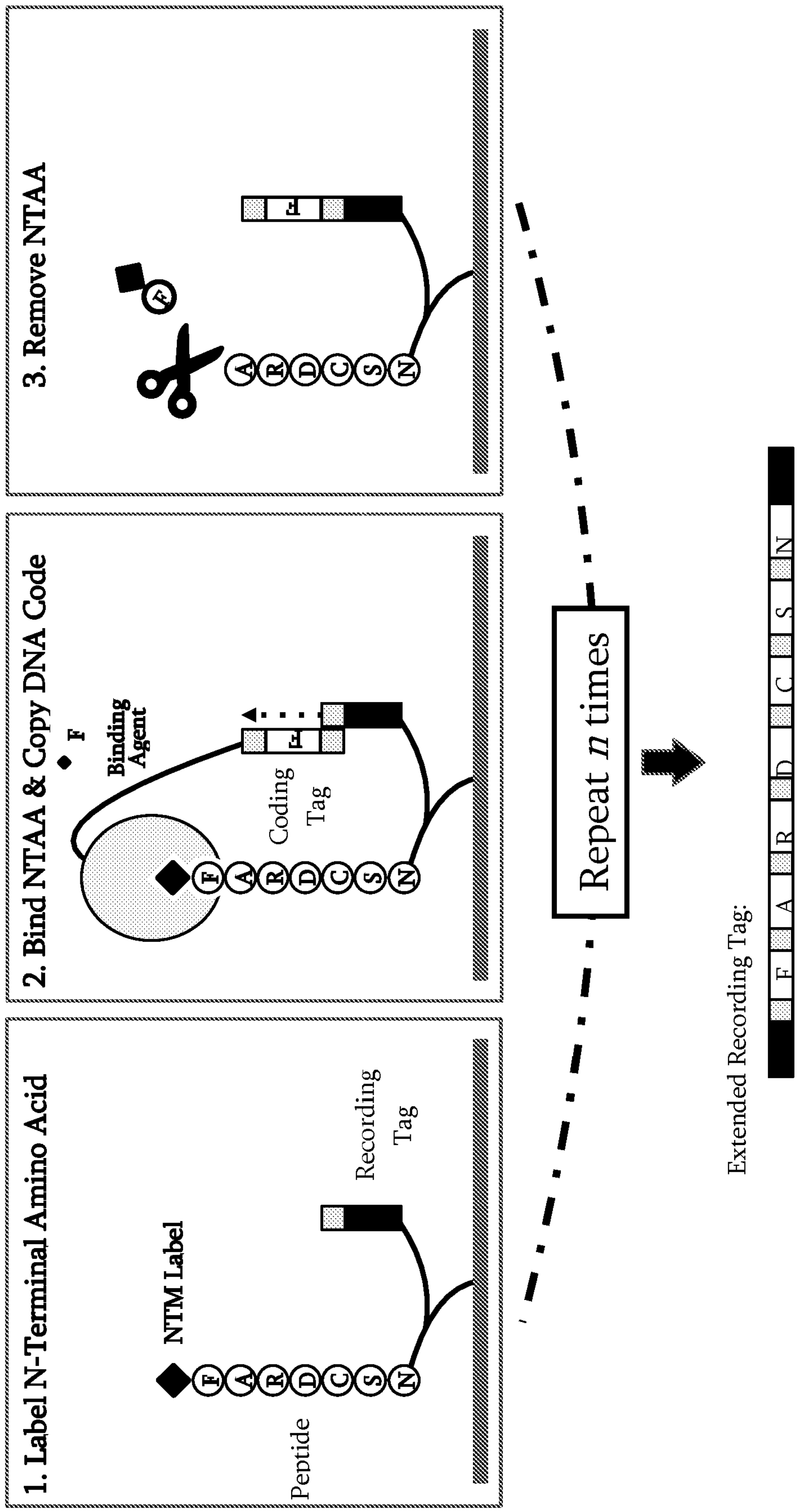
1. Label N-Terminal Amino Acid
NTM Label
Peptide
Recording Tag
F A R D C S N
2. Bind NTAA & Copy DNA Code
F
Binding Agent
Coding Tag
F
3. Remove NTAA
F
Repeat n times
Extended Recording Tag:
F A R D C S N

FIG. 9

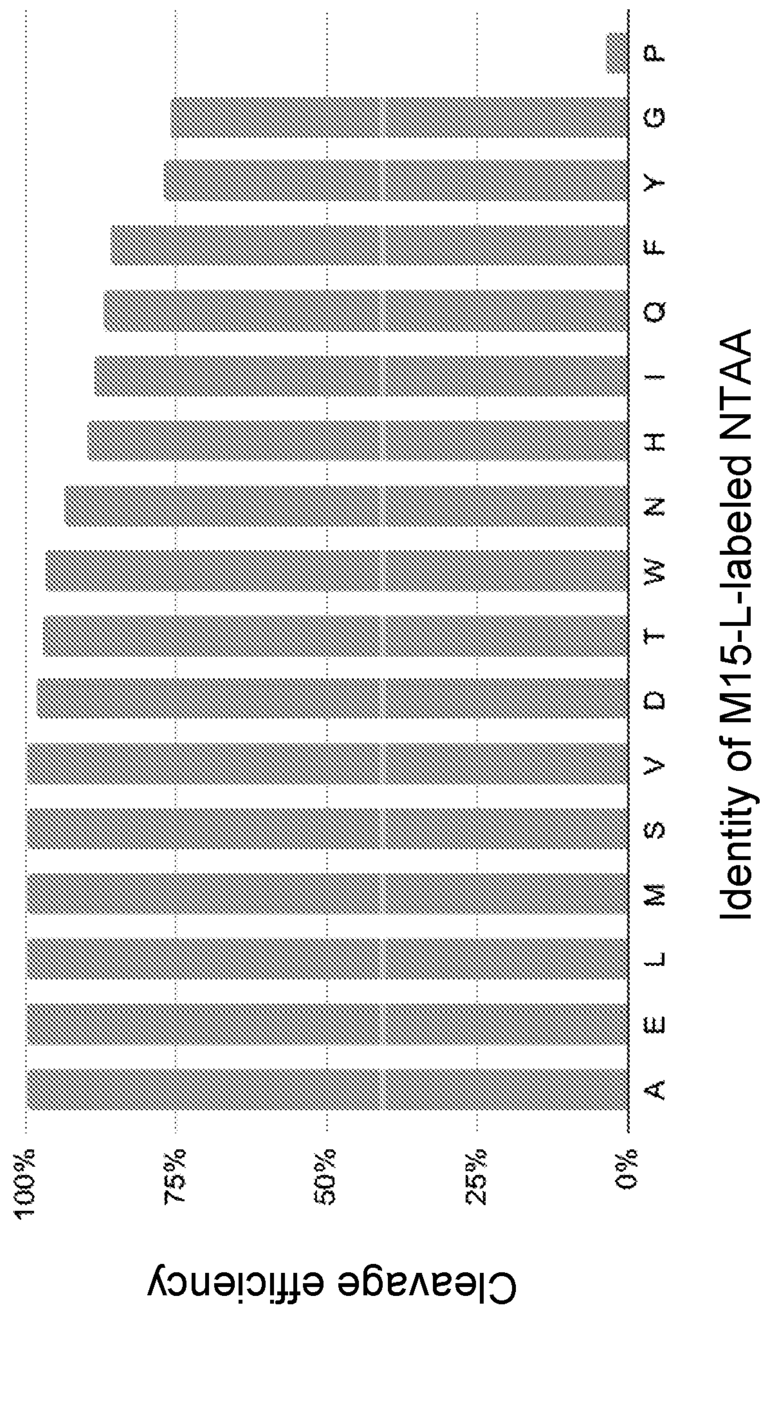
Cleavage efficiency
100%
75%
50%
25%
0%
A E L M S V D T W N H I Q F Y G P
Identity of M15-L-labeled NTAA

FIG. 10A

FIG. 10B

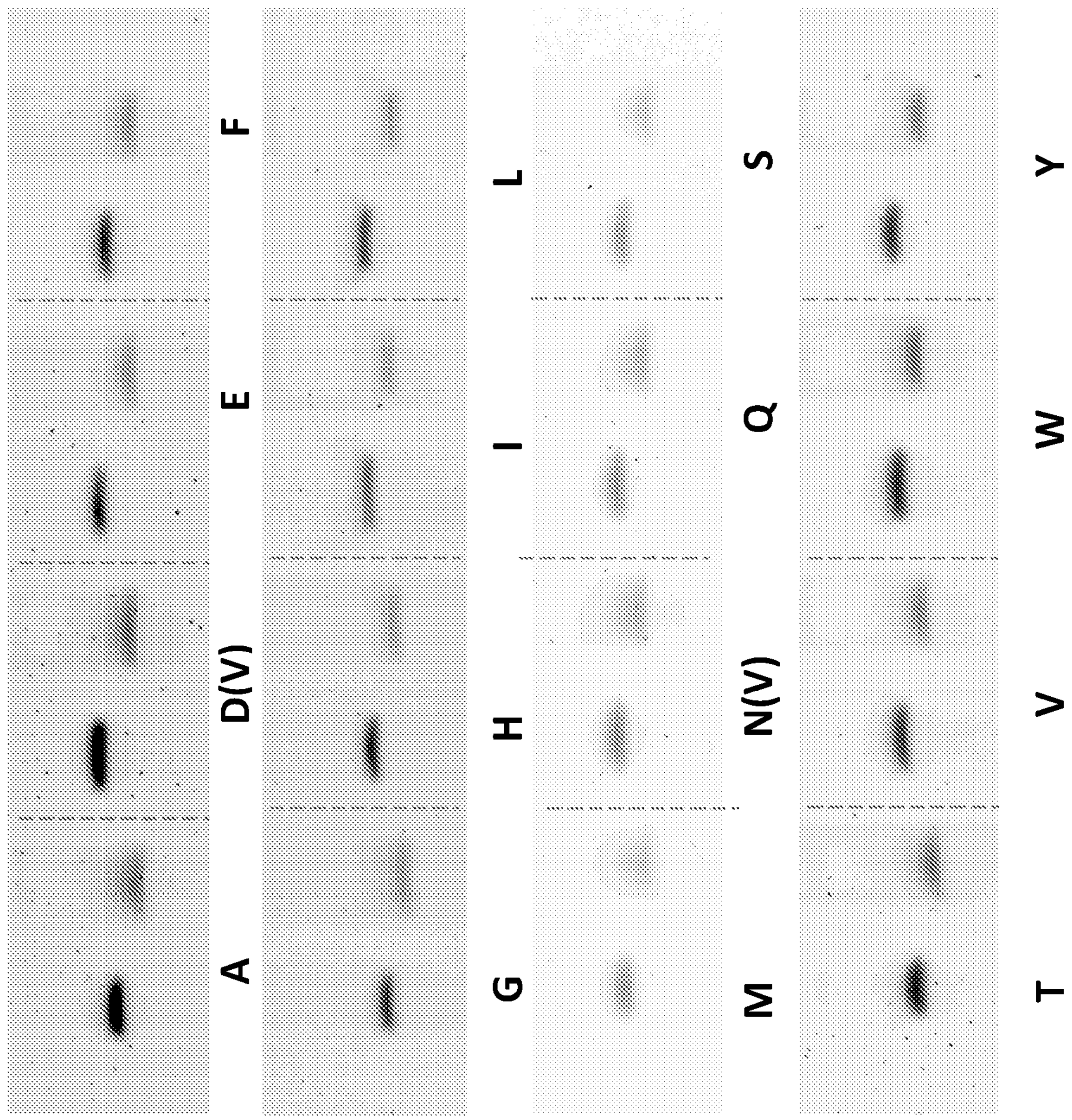

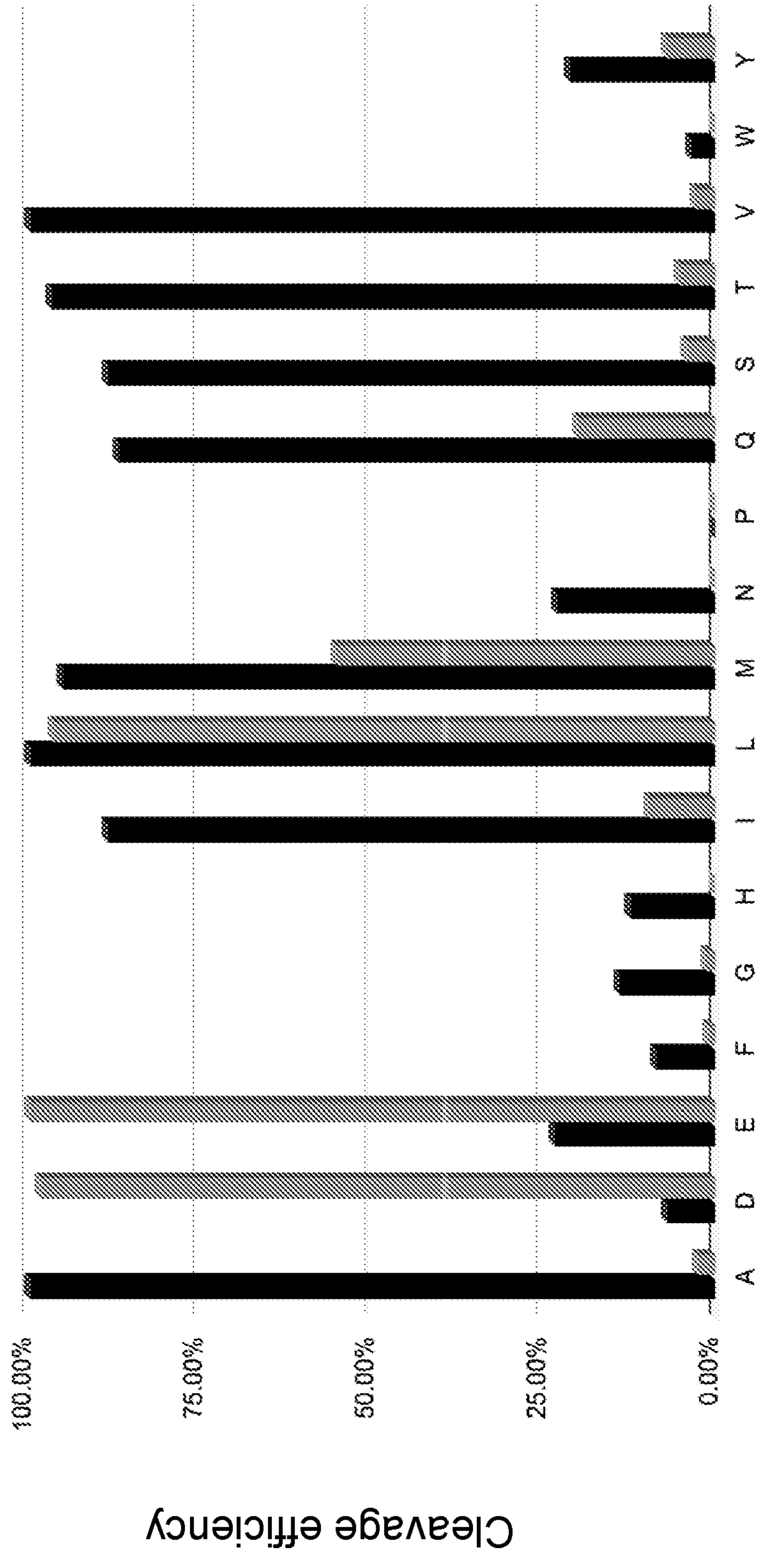
Cleavage efficiency
100.00%
75.00%
50.00%
25.00%
0.00%
A
D
E
F
G
H
I
L
M
N
P
Q
S
T
V
W
Y

FIG. 10C

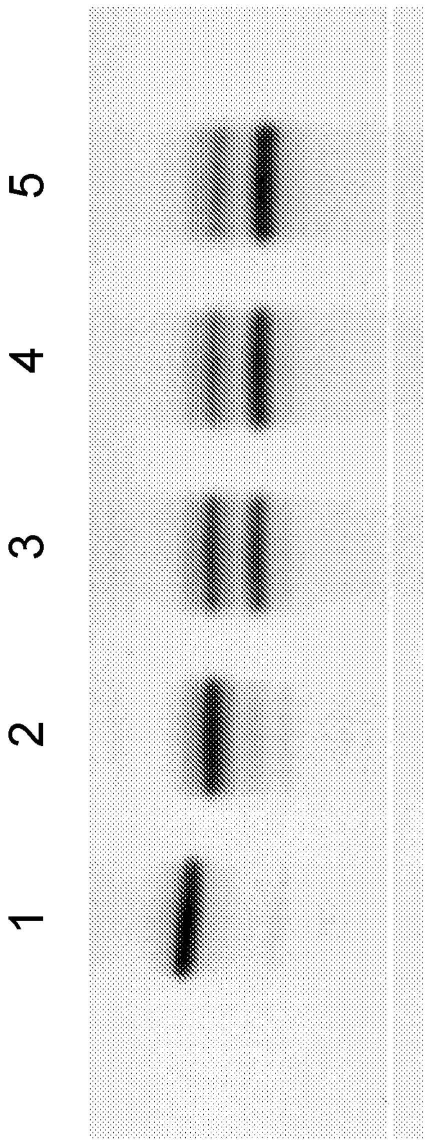


FIG. 11

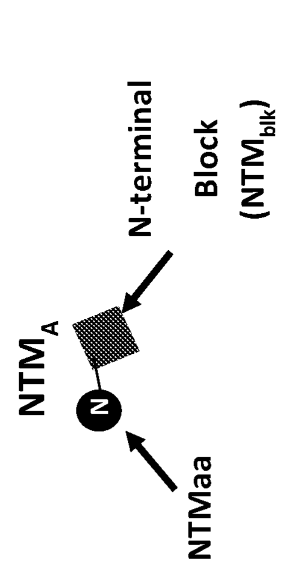


FIG. 12A

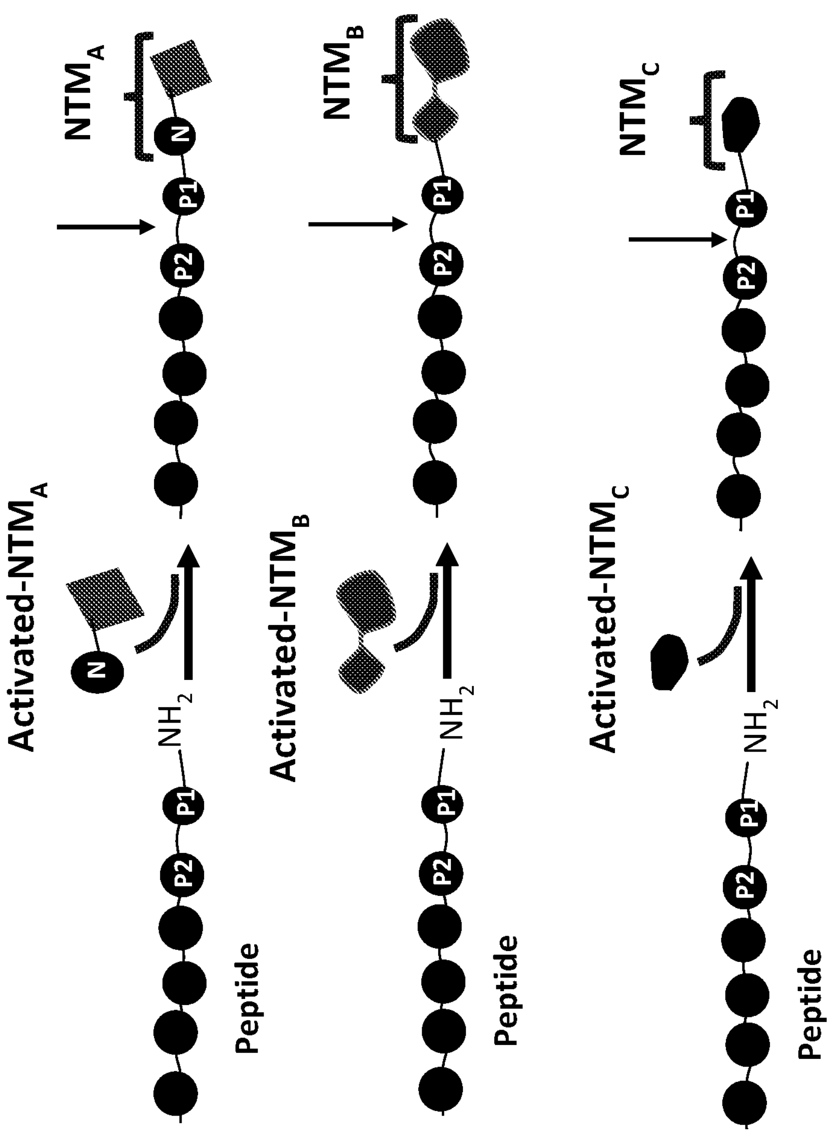


FIG. 12B

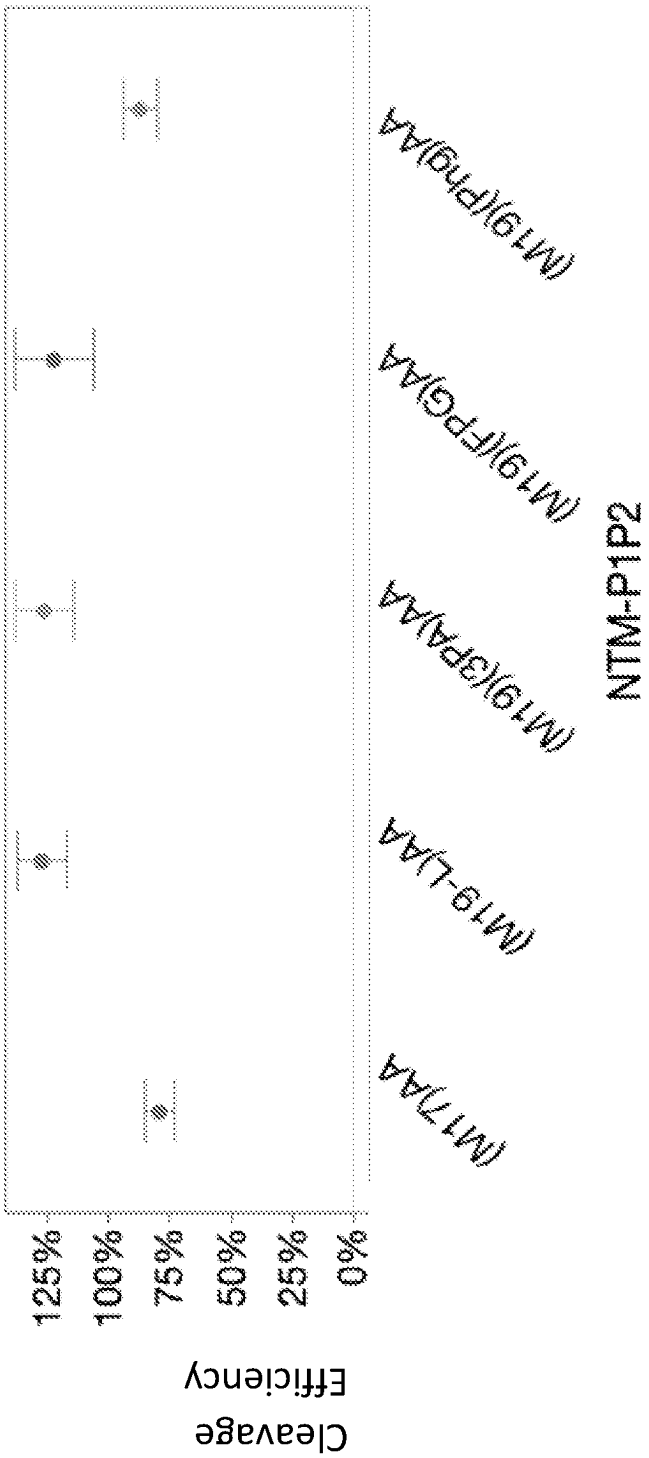
Cleavage Efficiency
125%
100%
75%
50%
25%
0%
(M17)AA
(M19-L)AA
(M19)(3P)AA
(M19)(FPG)AA
(M19)(Plg)AA
NTM-P1P2

FIG. 13

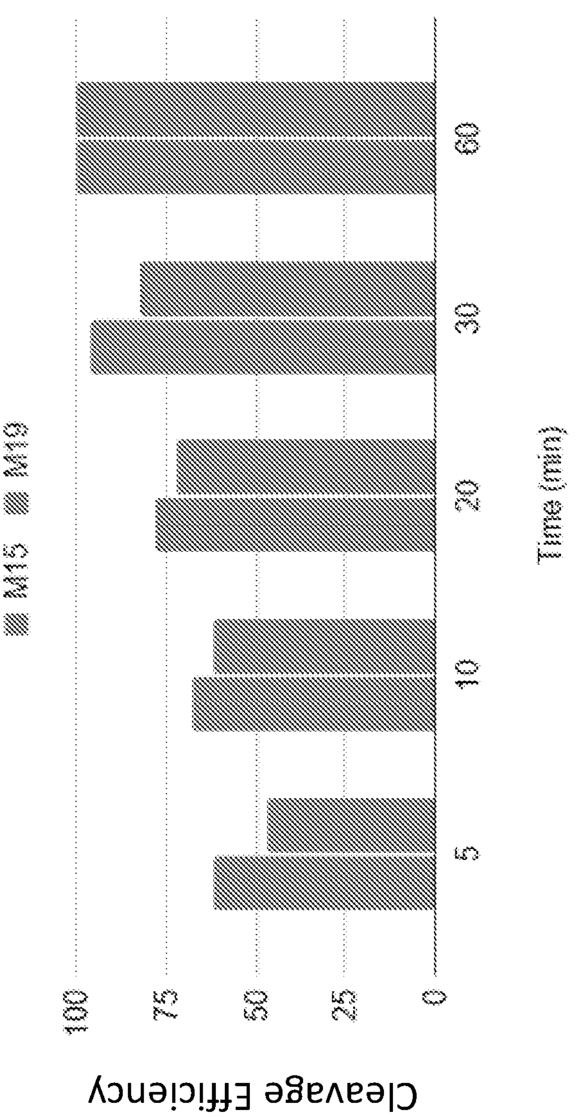


FIG. 14A

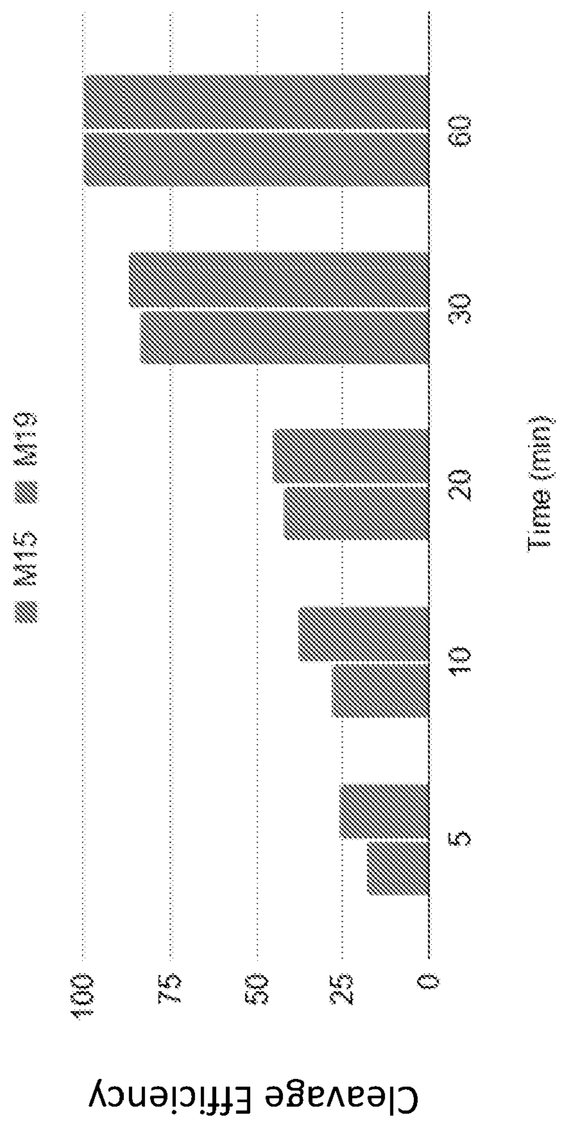


FIG. 14B

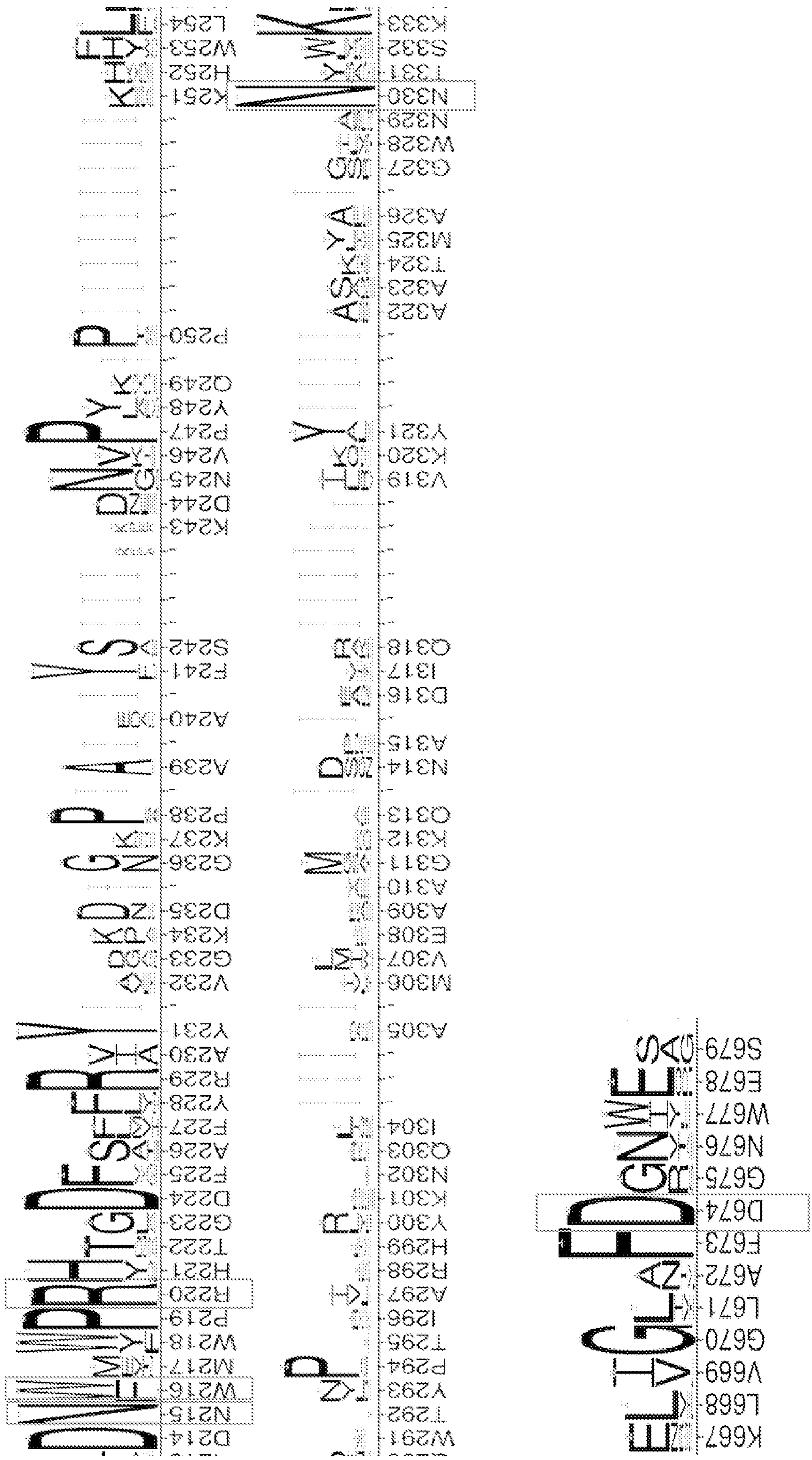

FIG. 15

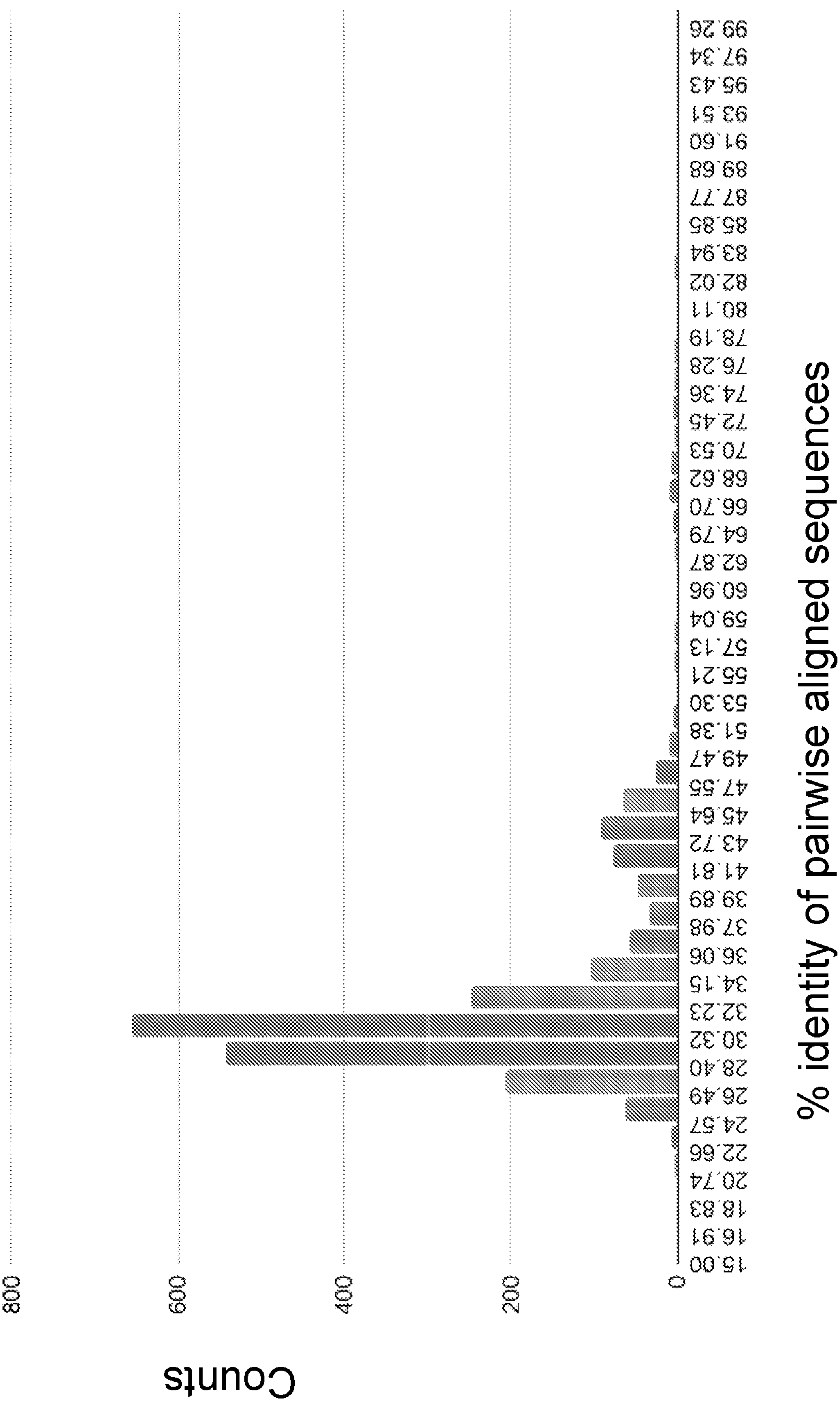
Counts
0
200
400
600
800
% identity of pairwise aligned sequences
15.00
16.91
18.83
20.74
22.66
24.57
26.49
28.40
30.32
32.23
34.15
36.06
37.98
39.89
41.81
43.72
45.64
47.55
49.47
51.38
53.30
55.21
57.13
59.04
60.96
62.87
64.79
66.70
68.62
70.53
72.45
74.36
76.28
78.19
80.11
82.02
83.94
85.85
87.77
89.68
91.60
93.51
95.43
97.34
99.26

FIG. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62994216
- US 63085977
- US 2002164759 A
- WO 2017192633 A
- US 2019246664 A
- WO 2010065322 A
- US 4873192 A
- US 62823927
- US 62824157 B
- US 62931737 B
- WO 2020198264 A
- US 9867883 B
- WO 2019089846 A
- US 5900481 A
- US 7060507 B
- US 20060183863 A
- US 7501237 B
- US 20140378315 A
- US 5834252 A


### Non-patent literature cited in the description


- **SANDERINK et al.** *J. Clin. Chem. Clin. Biochem.*, 1988, vol. 26, 795-807
- **SUZUKI, YOSHIYUKI et al.** Identification of the catalytic triad of family S46 exopeptidases, closely related to clan PA endopeptidases.. *Scientific Reports*, 2014, 4292
- **BORGO et al.** *Protein Sci.*, 2014, vol. 23 (3), 312-320
- **NIU et al.** *Nat. Chem.*, 2013, vol. 5, 282-292
- **ROY et al.** *Nat. Commun.*, 2015, vol. 6, 7237
- **LUTZ**. *Macromolecules*, 2015, vol. 48, 4759-4767
- **DRMANAC et al.** *Science*, 2009, vol. 327, 78-81
- **KORLACH et al.** *Proc. Natl. Acad. Sci.*, 2008, vol. 105, 1176-1181
- *Science*, 2006, vol. 311, 1544-1546
- **KUNKEL et al.** *Methods in Enzymol.*, 1987, vol. 154, 367-382
- **SHAKH M.A. ROUF** ; **YUKO OHARA-NEMOTO** ; **TOMONORI HOSHINO** ; **TAKU FUJIWARA** ; **TOSHIO ONO** ; **TAKAYUKI K. NEMOTO**. Discrimination based on Gly and Arg/Ser at position 673 between dipeptidyl-peptidase (DPP) 7 and DPP11, widely distributed DPPs in pathogenic and environmental gram-negative bacteria. *Biochimie*, 2013, vol. 95 (4), ISSN 0300-9084, 824-832
- **SCHOMBURG et al.** *J Biotechnol.*, 2017, vol. 261, 194-206
- **BAIROCH A.** *Nucleic Acids Res.*, 2000, vol. 28, 304-305
- **BARAL et al.** *J Biol Chem*, 2008, vol. 283 (32), 22316-22324
- **PRAJAPATI et al.** *FEBS J.*, 2011, vol. 278 (18), 3256-276
- **FUKASAWA et al.** *Biochem J.*, 15 January 1998, vol. 329, 275-282
- **FUKASAWA et al.** *J Amino Acids.*, 2011, vol. 2011, 574816
- **KUMAR et al.** *Sci Rep.*, 2016, vol. 6, 23787
- **BEAUVAIS et al.** *J Biol Chem.*, 1997, vol. 272 (10), 6238-44
- **BANBULA et al.** *J. Biol. Chem.*, 2001, vol. 276, 6299-6305
- **ROUF et al.** *FEBS Open Bio.*, 2013, vol. 3, 177-83
- **OHARA-NEMOTO et al.** *J Biol Chem.*, 2011, vol. 286 (44), 38115-27
- **OGASAWARA et al.** *J. Bacteriol.*, 1996, vol. 178, 6288-6295
- **SAKAMOTO et al.** *Scientific Reports*, 2014, vol. 4, 4977
- **STAIGER et al.** *J. Org. Chem.*, 1959, vol. 24 (9), 1214-1219
- **JIANG et al.** *J. Org. Chem.*, 2019, vol. 84 (4), 2022-2031
- **HERMANSON**. Bioconjugation Techniques. Academic Press, 2013
- **MONTALBETTI et al.** *Tetrahedron*, 2005, vol. 61, 10827-10852
- **MONTALBETTI et al.** *Wiley Encyclopedia of Chemical Biology*, 1-17
- **FIGUEIREDO et al.** *Chem Rev*, 2016, vol. 116 (19), 12029-12122
- **PHILPOTT et al.** *Green Chemistry*, 2018, vol. 20 (15), 3426-3431

- **KWON et al.** *Org. Lett.*, 2014, vol. 16, 6048-6051
- **MARTIN et al.** *Organometallics.*, 2006, vol. 34, 1787-1801
- **CHI et al.** *Chem. Eur. J.*, 2015, vol. 21, 10369-10378
- *Protein Science*, 1992, 582-589
- **WANG et al.** *Anal Chem*, 2009, vol. 81, 1893-1900
- **FARRIES, HARRIS et al.** *Eur. J. Biochem.*, 1991, vol. 196, 679-685
- **KRISHNA et al.** *Anal. Biochem.*, 1991, vol. 199, 45-50
- **LEONE et al.** *Curr. Protoc. Protein Sci.*, 2011
- **FOWLER et al.** *Curr. Protoc. Protein Sci.*, 2001
- **CARTY et al.** *J Biol Chem*, 1968, vol. 243 (20), 5244-5253
- **MARTINEZ-PALOU**. *J. Mex. Chem. Soc*, 2007, vol. 51 (4), 252-264
- **DAS et al.** *Annu Rev Biochem*, 2008, vol. 77, 363-382
- **CROOKS et al.** *Genome Res*, 2004, vol. 14 (6), 1188-1190
- **LEIVE, L.** *Ann N Y Acad Sci*, 1974, vol. 235 (0), 109-129
- **MUHEIM, C.** *Scientific Reports*, 2017, vol. 7 (1), 17629
- **PRABAKARAN et al.** *Wiley Interdiscip Rev Syst Biol Med*, 2012, vol. 4, 565-583
- **GRANVOGL et al.** *Anal Bioanal Chem*, 2007, vol. 389, 991-1002
- **CHAN et al.** *PLoS One*, 2007, vol. 2, e1164
- **CAZALIS et al.** *Bioconj. Chem.*, vol. 15, 1005-1009
- **SOELLNER et al.** *J. Am. Chem. Soc.*, 2003, vol. 125, 11790-11791
- **SUN et al.** *Bioconjug. Chem.*, 2006, 17-52, 57
- **DECREAU et al.** *J. Org. Chem.*, 2007, vol. 72, 2794-2802
- **CAMARERO et al.** *J. Am. Chem. Soc.*, 2004, vol. 126, 14730-14731
- **GIRISH et al.** *Bioorg. Med. Chem. Lett.*, 2005, vol. 15, 2447-2451
- **KALIA et al.** *Bioconjug. Chem.*, 2007, vol. 18, 1064-1069
- **WATZKE et al.** *Angew Chem. Int. Ed. Engl.*, 2006, vol. 45, 1408-1412
- **PARTHASARATHY et al.** *Bioconjugate Chem.*, 2007, vol. 18, 469-476
- **G. T. HERMANSON**. Bioconjugate Techniques. Academic Press, 2013
- **HORISAWA**. *Front Physiol*, 2014, vol. 5, 457
- **KNALL, HOLLAUF et al.** *Tetrahedron Lett*, 2014, vol. 55 (34), 4763-4766
- **WHITEAKER et al.** *Anal. Biochem.*, 2007, vol. 362, 44-54
- **HUANG et al.** *J. Chromatogr. A*, 2014, vol. 1372, 1-17
- **ZHOU et al.** *Anal Chem*, 2012, vol. 84 (2), 720-734
- **MCCORMICK**. *Anal Biochem*, 1989, vol. 181 (1), 66-74
- **MENDOZA et al.** *Mass Spectrom Rev*, 2009, vol. 28 (5), 785-815
- **STEINBERG et al.** *Biopolymers*, 2004, vol. 73, 597-605
- **LUND et al.** *Nucleic Acids Res.*, 1988, vol. 16, 10861-10880
- **GUO et al.** *Proteome Sci*, 2012, vol. 10 (1), 56
- **ASSADI, LAMERZ et al.** *Mol Cell Proteomics*, 2013, vol. 12 (9), 2615-2622
- **AKBANI et al.** *Mol Cell Proteomics*, 2014, vol. 13 (7), 1625-1643
- **CREIGHTON et al.** *Drug Des Devel Ther*, 2015, vol. 9, 3519-3527
- **XIONG et al.** *FEMS Microbiol Rev*, 2008, vol. 32 (3), 522-540
- **KIAH et al.** IEEE International Symposium on Information Theory (ISIT). *Codes for DNA sequence profiles.*, 2015
- **GABRYS et al.** IEEE Symposium on Information Theory (ISIT). *Asymmetric Lee distance codes for DNA-based storage.*, 2015
- **LAURE et al.** Coding in 2D: Using Intentional Dispersity to Enhance the Information Capacity of Sequence-Coded Polymer Barcodes.. *Angew. Chem. Int. Ed.*, 2016
- **YAZDI et al.** *IEEE Transactions on Molecular, Biological and Multi-Scale Communications*, 2015, vol. 1, 230-248
- **YAZDI et al.** *Sci Rep*, 2015, vol. 5, 14138
- **LASZLO et al.** *Nat. Biotechnol.*, 2014, vol. 32, 829-833
- **OHSHIRO et al.** *Sci Rep*, 2012, vol. 2, 501
- **SMITH et al.** *Future Med Chem.*, 2015, vol. 7 (2), 159-183
- **LI et al.** *Angew Chem Int Ed Engl*, 2013, vol. 52 (36), 9544-9549
- **ROSEN et al.** *Nature Chemistry*, 2014, vol. 6, 804-809
- **KODAL et al.** *ChemBioChem*, 2016, vol. 17, 1338-1342
- **PAN et al.** *Phys. Biol.*, 2015, vol. 12, 045006
- **GROLL et al.** *Methods Enzymol.*, 2010, vol. 472, 1-18
- **HUA et al.** *Nat. Methods*, 2014, vol. 11, 1233-1236
- **STAVIS et al.** *Proc. Natl. Acad. Sci. USA*, 2011, vol. 108, 983-988
- **BELL et al.** *Nature*, 2012, vol. 491, 274-278
- **BELL et al.** *Elife*, 2015, vol. 4, e08646
- *Annu Rev Biochem.*, 2001, vol. 70, 369-413
- **YANG et al.** *Nucleic Acids Res.*, 2002, vol. 30 (19), 4314-4320

- **LAHOUD et al.** *Nucleic Acids Res.*, 2008, vol. 36, 3409-3419
- **HOSHIKA et al.** *Angew Chem Int Ed Engl*, 2010, vol. 49 (32), 5554-5557
- **GAMPER et al.** *Biochemistry*, 2006, vol. 45 (22), 6978-86
- **GUNDERSON et al.** *Genome Res*, 1998, vol. 8 (11), 1142-1153
- **PENG et al.** *European J Org Chem*, 2010, vol. 22, 4194-4197
- **EL-SAGHEERET**. *Proc Natl Acad Sci U S A*, 2011, vol. 108 (28), 11338-11343
- **EL-SAGHEER et al.** *Org Biomol Chem*, 2011, vol. 9 (1), 232-235
- **SHARMA et al.** *Anal Chem*, 2012, vol. 84 (14), 6104-6109
- **ROLOFF et al.** *Bioorg Med Chem*, 2013, vol. 21 (12), 3458-3464
- **LITOVCHICK et al.** *Artif DNA PNA XNA*, 2014, vol. 5 (1), e27896
- **ROLOFF et al.** *Methods Mol Biol*, 2014, vol. 1050, 131-141
- **ROLOFF et al.** *Bioorgan. Med. Chem.*, 2013, vol. 21, 3458-3464
- **SHUMAN et al.** *J. Biol. Chem.*, 1994, vol. 269, 32678-32684
- **HORI, FUKANO et al.** *Biochem Biophys Res Commun*, 2007, vol. 352 (2), 323-328
- **MAMANOVA et al.** *Nature Methods*, 2010, vol. 7, 111-118
- **BODI et al.** *J. Biomol. Tech.*, 2013, vol. 24, 73-86
- **BALLESTER et al.** *Expert Review of Molecular Diagnostics*, 2016, 357-372
- **MERTES et al.** *Brief Funct. Genomics*, 2011, vol. 10, 374-386
- **NILSSON et al.** *Science*, 1994, vol. 265, 2085-8
- **BOCHMAN et al.** *Nat Rev Genet*, 2012, vol. 13 (11), 770-780
- **VANDERNOOT et al.** *Biotechniques*, 2012, vol. 53, 373-380
- **SHAGIN et al.** *Genome Res.*, 2002, vol. 12, 1935-42
- **DU et al.** *BioTechniques*, 2003, vol. 35, 66-72
- **MUECKE et al.** *Structure*, 2008, vol. 16, 837-841
- **HARRIS et al.** *Science*, 2008, vol. 320, 106-109
- **GUNDERSON et al.** *Genome Res.*, 2004, vol. 14, 970-7
- **DERRINGTON et al.** *Proc Natl Acad Sci U S A*, 2010, vol. 107 (37), 16060-16065
- **SHIM et al.** *Nucleic Acids Res*, 2009, vol. 37 (3), 972-982
- **ZHANG et al.** *mAbs*, 2016, vol. 8, 524-535
- **NISHIZUKA et al.** *Drug Metab Pharmacokinet*, 2016, vol. 31 (1), 35-45
- **NEUENSCHWANDER et al.** *Nat Biotechnol.*, 2007, vol. 25 (10), 1145-1147
- **ABOUHAMAD et al.** *Mol Microbiol.*, 1991, vol. 5 (5), 1035-1047
- **SMITH et al.** *Microbiology*, 1999, vol. 145 (10), 2891-901
- **PAYNE et al.** *Arch Biochem Biophys.*, 2000, vol. 384 (1), 9-23
- **FANG et al.** *J Bacteriol.*, vol. 182 (9), 2530-2535
- **SPECK et al.** *Protein Eng Des Sel.*, 2011, vol. 24 (6), 473-484
- **THIE et al.** *N Biotechnol.*, 2008, vol. 25 (1), 49-54
- **KUNKEL, T**. *PNAS*, 1985, vol. 82 (2), 488-492
- **HOLLAND et al.** *J Immunol Methods.*, 2013, vol. 394 (1-2), 55-61
- **BERG et al.** Biochemistry. W H Freeman, 2002
- **EVNIN, L. B.** ; **J. R. VASQUEZ** ; **C. S. CRAIK**. Substrate specificity of trypsin investigated by using a genetic selection.. *Proc Natl Acad Sci U S A*, 1990, vol. 87 (17), 6659-6663
- **SMITH, M. W.** ; **D. R. TYREMAN** ; **G. M. PAYNE** ; **N. J. MARSHALL** ; **J. W. PAYNE**. Substrate specificity of the periplasmic dipeptide-binding protein from Escherichia coli: experimental basis for the design of peptide prodrugs.. *Microbiology*, 1999, vol. 145, 2891-2901
- **CROOKS GE et al.** *WebLogo: A sequence logo generator, Genome Research*, 2004, vol. 14, 1188-1190